# EUROPEAN PATENT SPECIFICATION

(11) **EP 3 832 746 B1**
(45) Date of publication and mention of the grant of the patent: **09.07.2025**
(21) Application number: 20798411.3
(22) Date of filing: 29.04.2020
(51) Int. Cl.: H10K 85/60, H10K 50/10

(54) **ORGANIC LIGHT EMITTING DEVICE**
ORGANISCHE LICHTEMITTIERENDE VORRICHTUNG
DISPOSITIF ÉLECTROLUMINESCENT ORGANIQUE

(30) Priority: 02.05.2019 KR 20190051622
(43) Date of publication of application: 09.06.2021
(73) Proprietor: LG Chem, Ltd., Seoul 07336, (KR)
(72) Inventor: KIM, Minjun, Daejeon 34122 (KR); JUNG, Min Woo, Daejeon 34122 (KR); LEE, Dong Hoon, Daejeon 34122 (KR); SUH, Sang Duk, Daejeon 34122 (KR); KIM, Seoyeon, Daejeon 34122 (KR)
(74) Representative: Hoffmann Eitle
(86) International application number: PCT/KR2020/005777
(87) International publication number: WO 2020/222569

(56) References cited:
- EP-A1- 3 862 353
- WO-A1-2018/174679
- WO-A1-2018/174681
- KR-A- 20150 024 735
- KR-A- 20170 086 277
- KR-A- 20180 010 144
- KR-A- 20200 038 157

## Description

### FIELD OF THE INVENTION

### CROSS-REFERENCE TO RELATED APPLICATION(S)

This application claims the benefit of Korean Patent Application No. 10-2019-0051622 filed on May 2, 2019 and Korean Patent Application No. 10-2020-0052000 filed on April 29, 2020 in the Korean Intellectual Property Office.

The present disclosure relates to an organic light emitting device.

### BACKGROUND OF THE INVENTION

In general, an organic light emitting phenomenon refers to a phenomenon where electric energy is converted into light energy by using an organic material. The organic light emitting device using the organic light emitting phenomenon has characteristics such as a wide viewing angle, an excellent contrast, a fast response time, an excellent luminance, driving voltage and response speed, and thus many studies have proceeded.

The organic light emitting device generally has a structure which comprises an anode, a cathode, and an organic material layer interposed between the anode and the cathode. The organic material layer frequently has a multilayered structure that comprises different materials in order to enhance efficiency and stability of the organic light emitting device, and for example, the organic material layer may be formed of a hole injection layer, a hole transport layer, a light emitting layer, an electron transport layer, an electron injection layer and the like. In the structure of the organic light emitting device, if a voltage is applied between two electrodes, the holes are injected from an anode into the organic material layer and the electrons are injected from the cathode into the organic material layer, and when the injected holes and electrons meet each other, an exciton is formed, and light is emitted when the exciton falls to a ground state again.

There is a continuing need for the development of new materials for the organic materials used in the organic light emitting devices as described above.

### PRIOR ART LITERATURE

### PATENT LITERATURE

(Patent Literature 0001) Korean Unexamined Patent Publication No. 10-2000-0051826
(Patent Literature 0002) WO 2018/174681 A1

### SUMMARY OF THE INVENTION

### TECHNICAL PROBLEM

It is an object of the present disclosure to provide an organic light emitting device.

### TECHNICAL SOLUTION

Provided herein is an organic light emitting device in accordance with claims 1, 2 and 6. The organic light emitting device is comprising: an anode; a cathode that is provided opposite to the anode; and one or more organic material layers that are provided between the anode and the cathode, wherein the organic material layer includes a compound represented by the following Chemical Formula 1 and a compound represented by the following Chemical Formula 2: 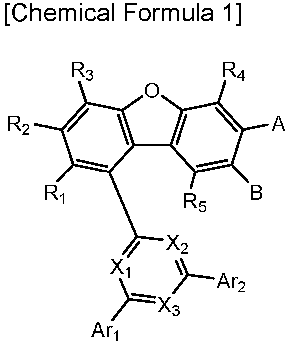 in the Chemical Formula 1,
X₁ to X₃ are each independently N or CR_{5'}, provided that at least one of X₁ to X₃ is N,
Ar₁ and Ar₂ are each independently a substituted or unsubstituted C₆₋₆₀ aryl; or a substituted or unsubstituted C₂₋₆₀ heteroaryl containing at least one of O, N, Si and S,
R₁ to R₅ and R₅, are each independently hydrogen; deuterium; halogen; hydroxy; nitrile; nitro; amino; a substituted or unsubstituted C₂₋₆₀ alkyl; a substituted or unsubstituted C₂₋₆₀ alkoxy; a substituted or unsubstituted C₂₋₆₀ alkenyl; a substituted or unsubstituted C₆₋₆₀ aryl; a substituted or unsubstituted C₂₋₆₀ heteroaryl containing at least one of O, N, Si and S, or R₁ to R₃ combine with an adjacent group to form a condensed ring,
one of A and B is a substituent represented by the following Chemical Formula 1-1, and the other is hydrogen or deuterium, 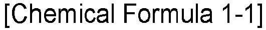 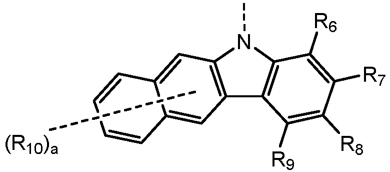
in the Chemical Formula 1-1,
R₆ to R₁₀ are each independently hydrogen; deuterium; halogen; hydroxy; nitrile; nitro; amino; a substituted or unsubstituted C₂₋₆₀ alkyl; a substituted or unsubstituted C₂-₆₀ alkoxy; a substituted or unsubstituted C₂₋₆₀ alkenyl; a substituted or unsubstituted C₆₋₆₀ aryl; a substituted or unsubstituted C₂₋₆₀ heteroaryl containing at least one of O, N, Si and S, or R₆ to R₉ combine with an adjacent group to form a condensed ring,
a is an integer of 1 to 6, 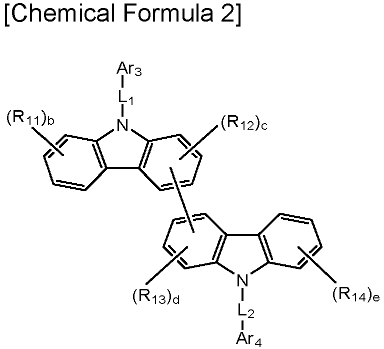
in the Chemical Formula 2,
Ar₃ and Ar₄ are each independently a substituted or unsubstituted C₆₋₆₀ aryl; or a substituted or unsubstituted C₂₋₆₀ heteroaryl containing at least one of O, N, Si and S,
L₁ and L₂ are each independently a single bond; or a substituted or unsubstituted C₆₋₆₀ arylene,
R₁₁ to R₁₄ are each independently hydrogen; deuterium; halogen; hydroxy; nitrile; nitro; amino; a substituted or unsubstituted C₂₋₆₀ alkyl; a substituted or unsubstituted C₂₋₆₀ alkoxy; a substituted or unsubstituted C₂₋₆₀ alkenyl; a substituted or unsubstituted C₆₋₆₀ aryl; a substituted or unsubstituted C₂₋₆₀ heteroaryl containing at least one of O, N, Si and S,
b and e are each independently an integer of 1 to 4, and
c and d are each independently an integer of 1 to 3.

### ADVANTAGEOUS EFFECTS

The organic light emitting device uses the compound represented by Chemical Formula 1 and the compound represented by Chemical Formula 2 as a host material of the light emitting layer, and thereby, can improve the efficiency, achieve low driving voltage and/or improve lifetime characteristics in the organic light emitting device.

### BRIEF DESCRIPTION OF THE DRAWINGS

FIG. 1 shows an example of an organic light emitting device comprising a substrate 1, an anode 2, a light emitting layer 3, and a cathode 4.
FIG. 2 shows an example of an organic light emitting device comprising a substrate 1, an anode 2, a hole injection layer 5, a hole transport layer 6, an electron inhibition layer 7, a light emitting layer 3, a hole blocking layer 8, an electron injection and transport layer 9, and a cathode 4.

### DETAILED DESCRIPTION OF THE EMBODIMENTS

Hereinafter, embodiments of the present disclosure will be described in more detail to facilitate understanding of the invention.

According one embodiment of the present disclosure, there is provided an organic light emitting comprising: an anode; a cathode that is provided opposite to the anode; and one or more organic material layers that are provided between the anode and the cathode, wherein the organic material layer includes a compound represented by Chemical Formula 1 and a compound represented by Chemical Formula 2.

As used herein, the notation 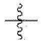 or 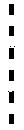 means a bond linked to another substituent group.

As used herein, the term "substituted or unsubstituted" means being unsubstituted or substituted with one or more substituents selected from the group consisting of deuterium; a halogen group; a nitrile group; a nitro group; a hydroxy group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; and a heterocyclic group containing at least one of **N,** O and S atoms, or being unsubstituted or substituted with a substituent to which two or more substituents of the above-exemplified substituents are connected. For example, "a substituent in which two or more substituents are connected" may be a biphenyl group. Namely, a biphenyl group may be an aryl group, or it may be interpreted as a substituent in which two phenyl groups are connected.

In the present disclosure, the carbon number of a carbonyl group is not particularly limited, but is preferably 1 to 40. Specifically, the carbonyl group may be a compound having the following structural formulas, but is not limited thereto.

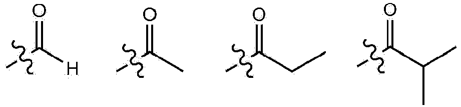

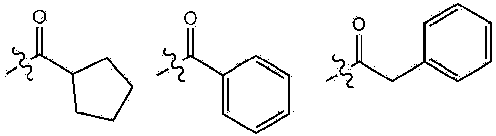

In the present disclosure, an ester group may have a structure in which oxygen of the ester group may be substituted by a straight-chain, branched-chain, or cyclic alkyl group having 1 to 25 carbon atoms, or an aryl group having 6 to 25 carbon atoms. Specifically, the ester group may be a compound having the following structural formulas, but is not limited thereto.

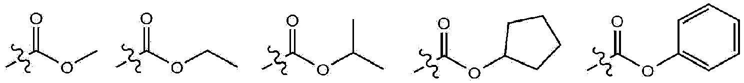

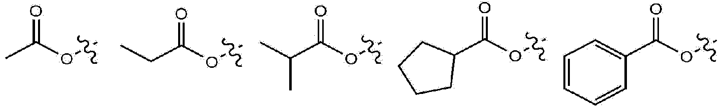

In the present disclosure, the carbon number of an imide group is not particularly limited, but is preferably 1 to 25. Specifically, the imide group may be a compound having the following structural formulas, but is not limited thereto.

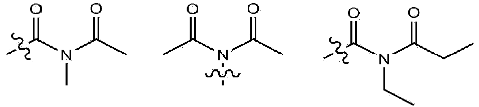

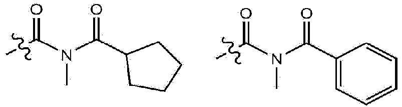

**In** the present disclosure, a silyl group specifically includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but is not limited thereto.

**In** the present disclosure, a boron group specifically includes a trimethylboron group, a triethylboron group, a t-butyldimethylboron group, a triphenylboron group, and a phenylboron group, but is not limited thereto.

**In** the present disclosure, examples of a halogen group include fluorine, chlorine, bromine, or iodine.

**In** the present disclosure, the alkyl group may be straight-chain or branched-chain, and the carbon number thereof is not particularly limited, but is preferably 1 to 40. According to one embodiment, the carbon number of the alkyl group is 1 to 20. According to another embodiment, the carbon number of the alkyl group is 1 to 10. According to another embodiment, the carbon number of the alkyl group is 1 to 6. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethylpropyl, isohexyl, 4-methylhexyl, 5-methylhexyl, and the like, but are not limited thereto.

**In** the present disclosure, the alkenyl group may be straight-chain or branched-chain, and the carbon number thereof is not particularly limited, but is preferably 2 to 40. According to one embodiment, the carbon number of the alkenyl group is 2 to 20. According to another embodiment, the carbon number of the alkenyl group is 2 to 10. According to still another embodiment, the carbon number of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

**In** the present disclosure, a cycloalkyl group is not particularly limited, but the carbon number thereof is preferably 3 to 60. According to one embodiment, the carbon number of the cycloalkyl group is 3 to 30. According to another embodiment, the carbon number of the cycloalkyl group is 3 to 20. According to still another embodiment, the carbon number of the cycloalkyl group is 3 to 6. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present disclosure, an aryl group is not particularly limited, but the carbon number thereof is preferably 6 to 60, and it may be a monocyclic aryl group or a polycyclic aryl group. According to one embodiment, the carbon number of the aryl group is 6 to 30. According to one embodiment, the carbon number of the aryl group is 6 to 20. The aryl group may be a phenyl group, a biphenyl group, a terphenyl group or the like as the monocyclic aryl group, but is not limited thereto. The polycyclic aryl group includes a naphthyl group, an anthracenyl group, a phenanthryl group, a pyrenyl group, a perylenyl group, a chrysenyl group, a fluorenyl group or the like, but is not limited thereto.

In the present disclosure, a fluorenyl group may be substituted, and two substituent groups may be bonded to each other to form a spiro structure. In the case where the fluorenyl group is substituted, 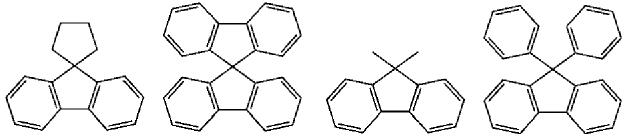 and the like can be formed. However, the structure is not limited thereto.

In the present disclosure, a heterocyclic group is a heterocyclic group containing one or more of O, N, Si and S as a heteroatom, and the carbon number thereof is not particularly limited, but is preferably 2 to 60. Examples of the heterocyclic group include a thiophene group, a furan group, a pyrrole group, an imidazole group, a thiazole group, an oxazol group, an oxadiazol group, a triazol group, a pyridyl group, a bipyridyl group, a pyrimidyl group, a triazine group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzoimidazole group, a benzothiazol group, a benzocarbazole group, a benzothiophene group, a dibenzothiophene group, a benzofuranyl group, a phenanthroline group, an isoxazolyl group, a thiadiazolyl group, a phenothiazinyl group, a dibenzofuranyl group, and the like, but are not limited thereto.

In the present disclosure, the aryl group in the aralkyl group, the aralkenyl group, the alkylaryl group and the arylamine group is the same as the aforementioned examples of the aryl group. In the present disclosure, the alkyl group in the aralkyl group, the alkylaryl group and the alkylamine group is the same as the aforementioned examples of the alkyl group. n the present disclosure, the heteroaryl in the heteroarylamine can be applied to the aforementioned description of the heterocyclic group. In the present disclosure, the alkenyl group in the aralkenyl group is the same as the aforementioned examples of the alkenyl group. In the present disclosure, the aforementioned description of the aryl group may be applied except that the arylene is a divalent group. In the present disclosure, the aforementioned description of the heterocyclic group can be applied except that the heteroarylene is a divalent group. **In** the present disclosure, the aforementioned description of the aryl group or cycloalkyl group can be applied except that the hydrocarbon ring is not a monovalent group but formed by combining two substituent groups. In the present disclosure, the aforementioned description of the heterocyclic group can be applied, except that the heterocyclic group is not a monovalent group but formed by combining two substituent groups.

According one embodiment of the present disclosure, there is provided an organic light emitting comprising: an anode; a cathode that is provided opposite to the anode; and one or more organic material layers that are provided between the anode and the cathode, wherein the organic material layer includes a compound represented by Chemical Formula 1 and a compound represented by Chemical Formula 2.

The organic light emitting device uses the compound represented by Chemical Formula 1 and the compound represented by Chemical Formula 2 as a host material of the light emitting layer, and thereby, can improve the efficiency, achieve low driving voltage and/or improve lifetime characteristics in the organic light emitting device.

Hereinafter, the present disclosure will be described in detail for each configuration.

### Anode and Cathode

As the anode material, generally, a material having a large work function is preferably used so that holes can be smoothly injected into the organic material layer. Specific examples of the anode material include metals such as vanadium, chrome, copper, zinc, and gold, or an alloy thereof; metal oxides such as zinc oxides, indium oxides, indium tin oxides (ITO), and indium zinc oxides (IZO); a combination of metals and oxides, such as ZnO:Al or SNO₂:Sb; conductive polymers such as poly(3-methylthiophene), poly[3,4-(ethylene-1,2-dioxy)thiophene](PEDOT), polypyrrole, and polyaniline, and the like, but are not limited thereto.

As the cathode material, generally, a material having a small work function is preferably used so that electrons can be easily injected into the organic material layer. Specific examples of the cathode material include metals such as magnesium, calcium, sodium, potassium, titanium, indium, yttrium, lithium, gadolinium, aluminum, silver, tin, and lead, or an alloy thereof; a multilayered structure material such as LiF/Al or LiO₂/Al, and the like, but are not limited thereto.

Further, a hole injection layer may be additionally included on the anode. The hole injection layer is composed of a hole injection material, wherein the hole injection material is preferably a compound which has a capability of transporting the holes, thus has a hole injecting effect in the anode and an excellent hole injecting effect to the light emitting layer or the light emitting material, prevents excitons produced in the light emitting layer from moving to a hole injection layer or the electron injection material, and is excellent in the ability to form a thin film.

It is preferable that a HOMO (highest occupied molecular orbital) of the hole injection material is between the work function of the anode material and a HOMO of a peripheral organic material layer. Specific examples of the hole injection material include metal porphyrine, oligothiophene, an arylamine-based organic material, a hexanitrilehexaazatriphenylene-based organic material, a quinacridone-based organic material, a perylene-based organic material, anthraquinone, polyaniline and polythiophene-based conductive polymer, and the like, but are not limited thereto.

### Light emitting layer

The light emitting material included in the light emitting layer is preferably a material which may receive holes and electrons transported from a hole transport layer and an electron transport layer, respectively, and combine the holes and the electrons to emit light in a visible ray region, and has good quantum efficiency to fluorescence or phosphorescence.

The light emitting layer may include a host material and a dopant material. In particular, in the present disclosure, the host material includes the compound represented by Chemical Formula 1 and the compound represented by Chemical Formula 2.

The Chemical Formula 1 may be any one selected from compounds represented by the following Chemical Formulas 1-A, 1-B and 1-C. 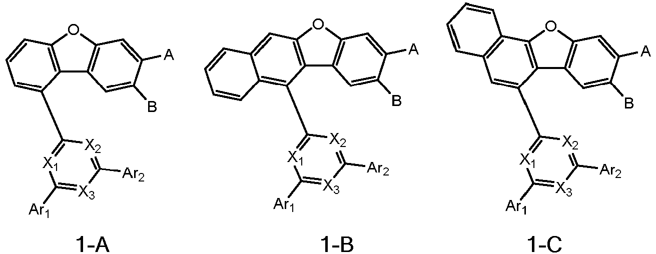 in the Chemical Formulas 1-A, 1-B and 1-C,
X₁, X₂, X₃, Ar₁, Ar₂, A and B are as defined above.

In the Chemical Formula 1, X₁ to X₃ all may be N.

In the Chemical Formula 1, Ar₁ and Ar₂ may be each independently any one selected from the group consisting of:

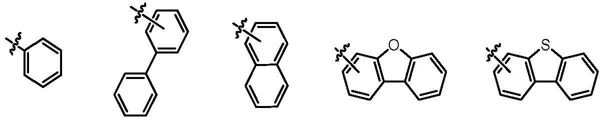

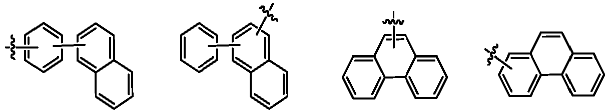

In the Chemical Formulae 1-B and 1-C, Chemical Formula 1-1 may be selected from the group consisting of the following compounds.

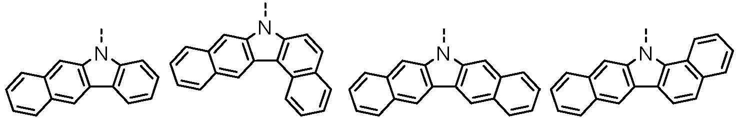

In the Chemical Formula 1-A, Chemical Formula 1.1 is selected from the group consisting of the following compounds.

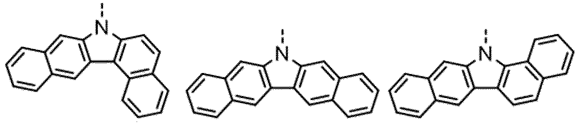

The compound represented by Chemical Formula 1 may be selected from the group consisting of the following compounds.

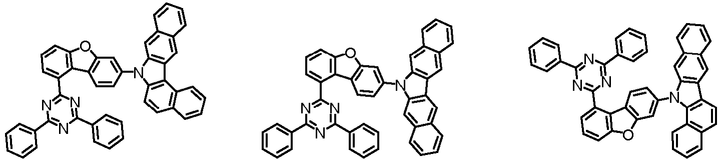

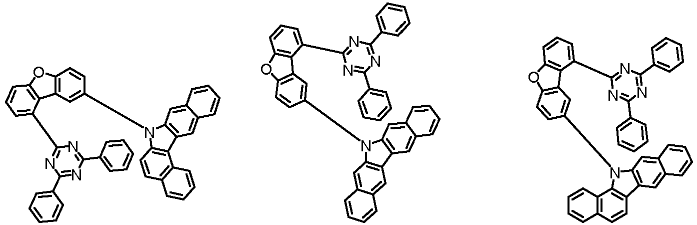

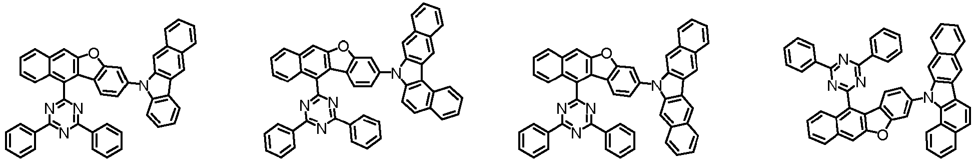

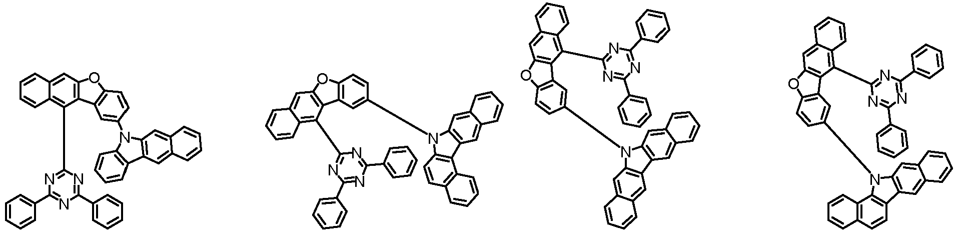

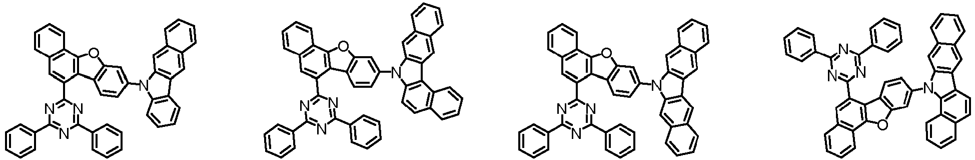

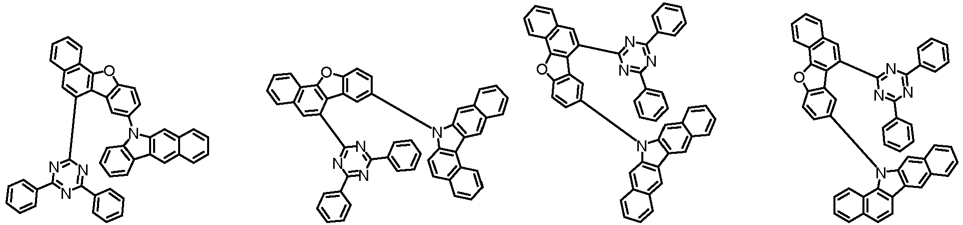

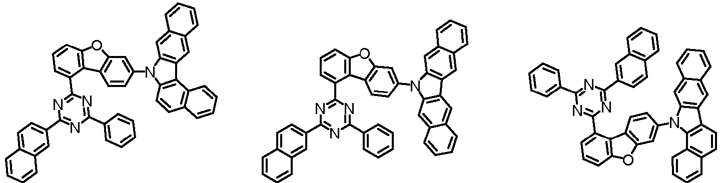

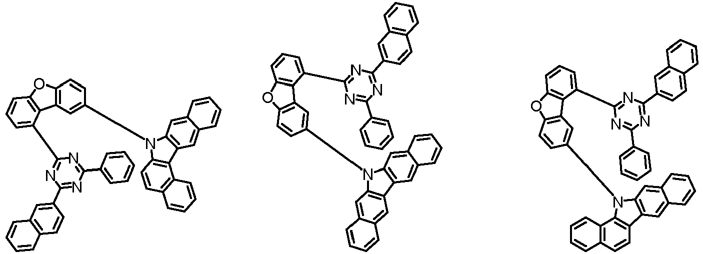

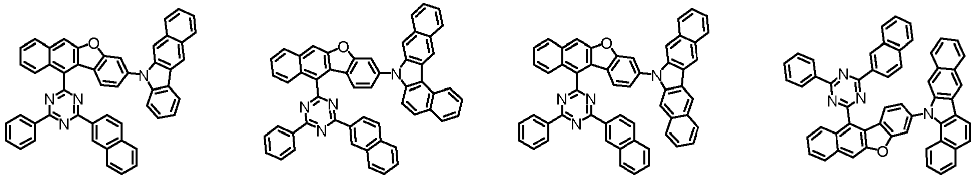

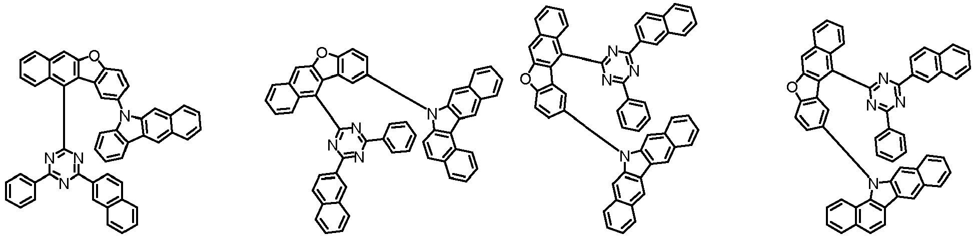

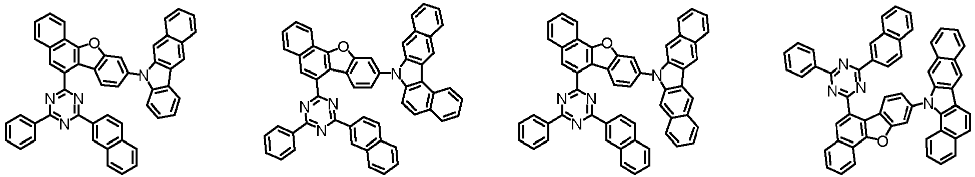

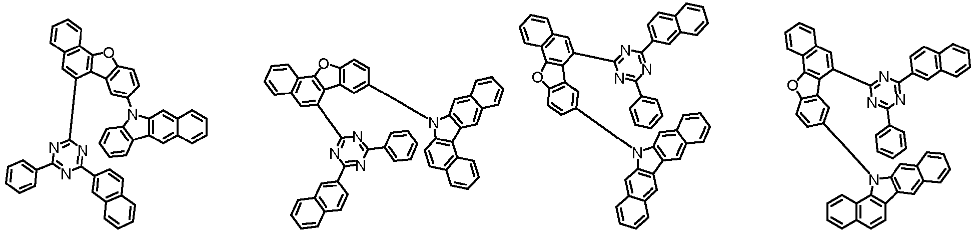

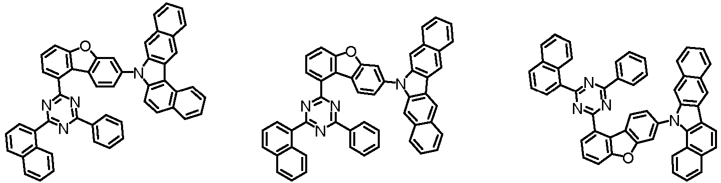

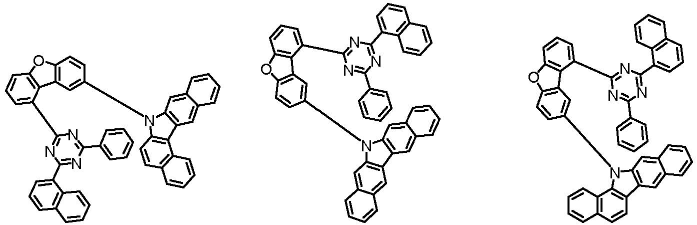

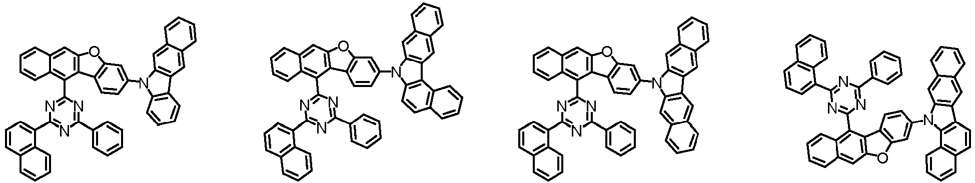

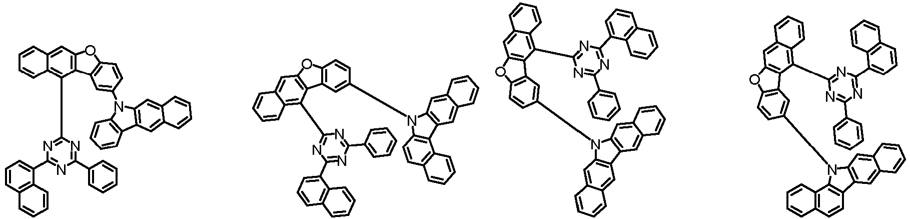

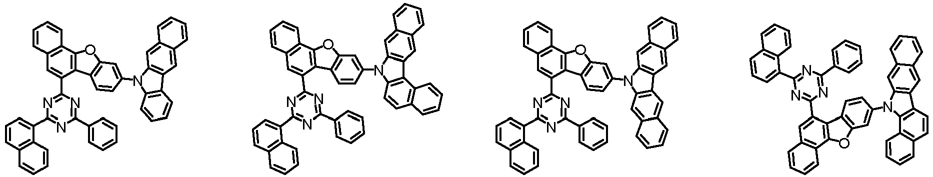

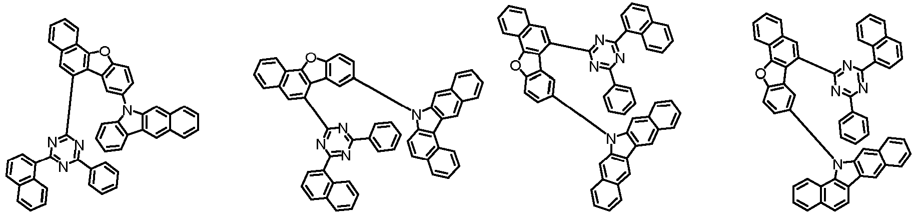

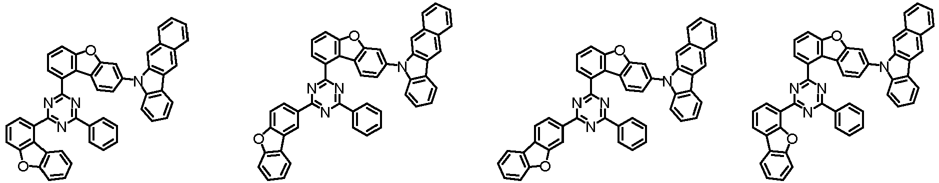

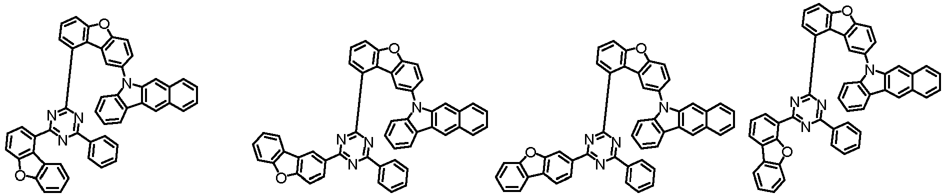

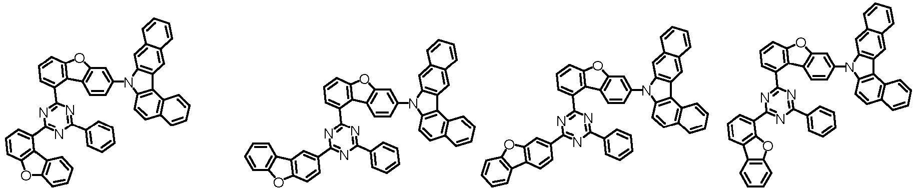

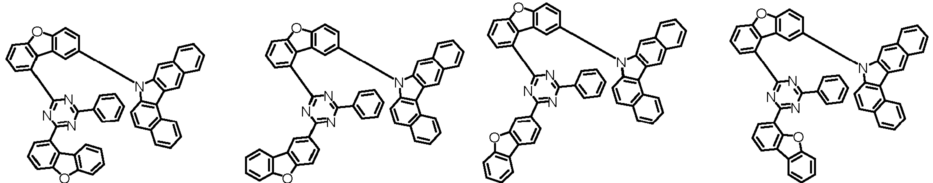

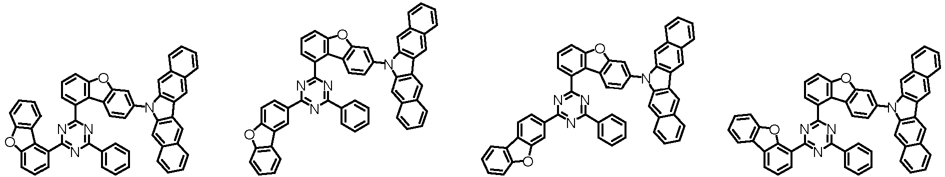

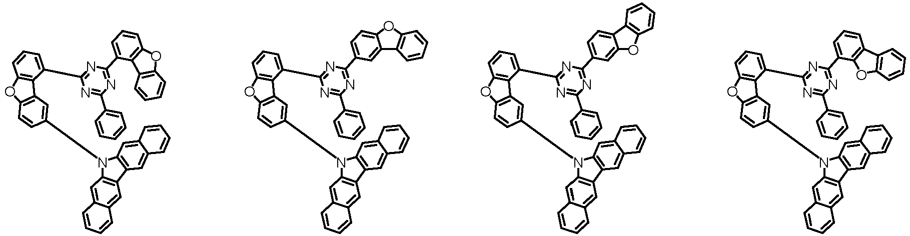

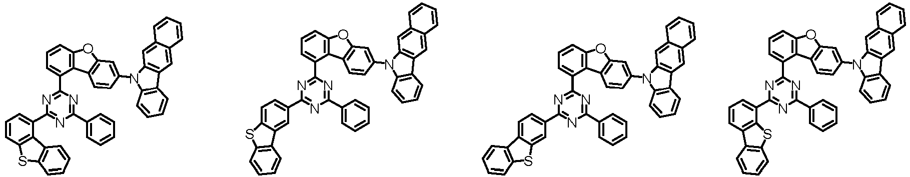

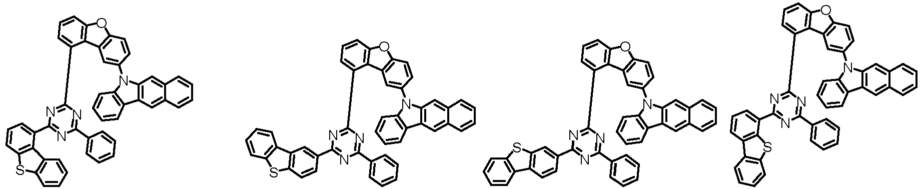

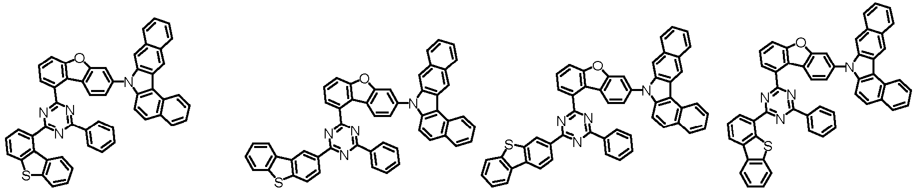

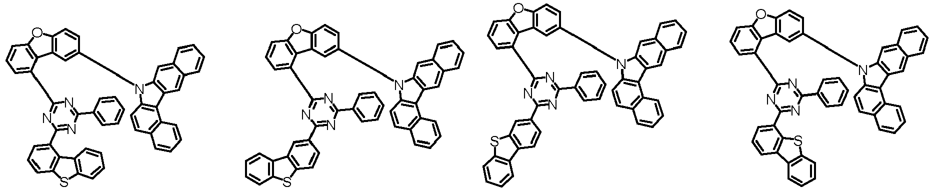

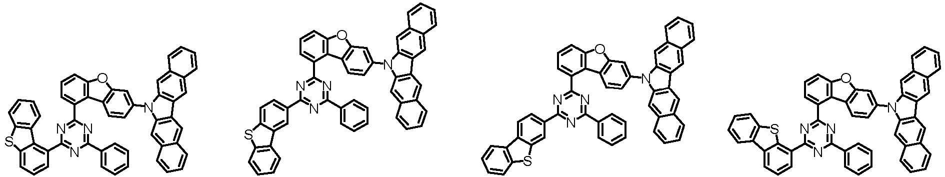

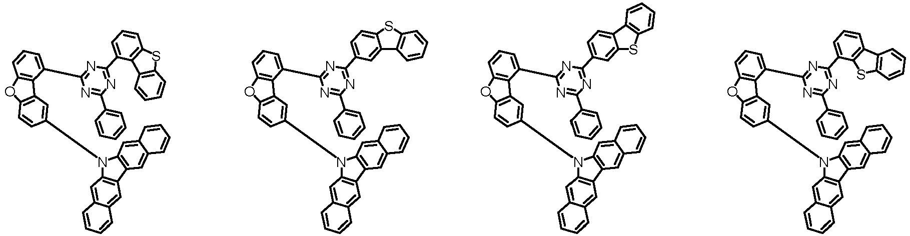

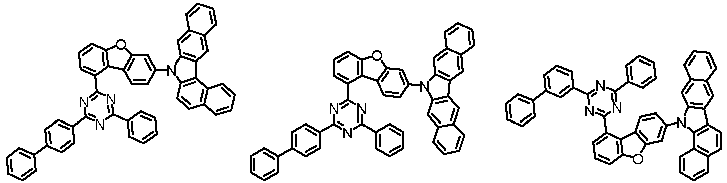

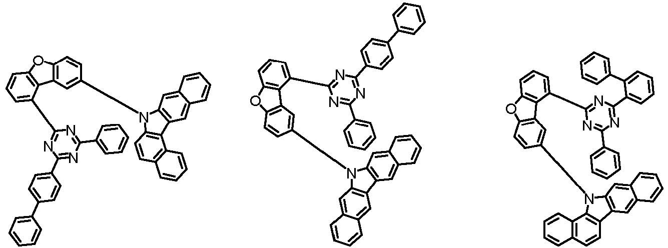

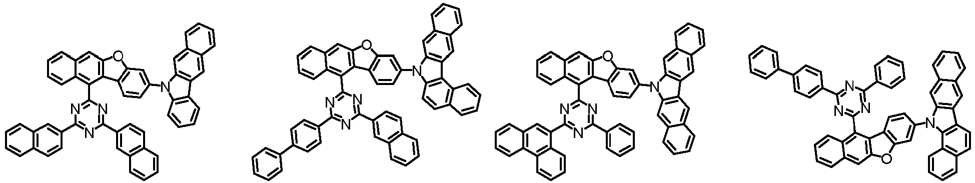

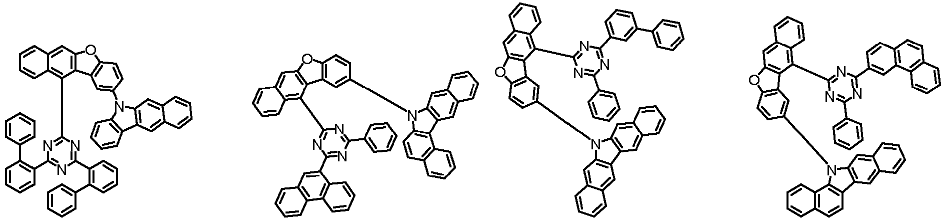

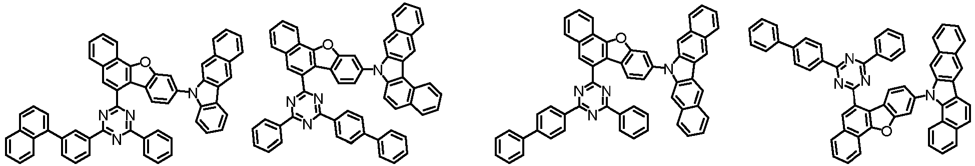

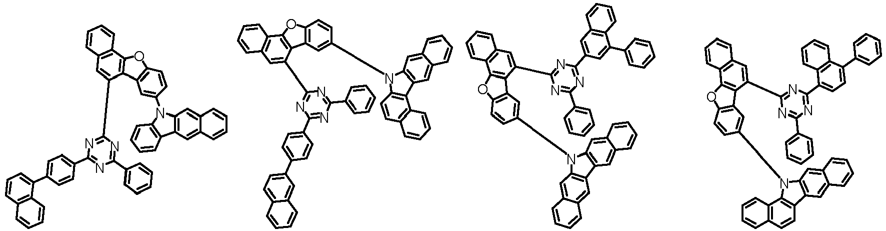

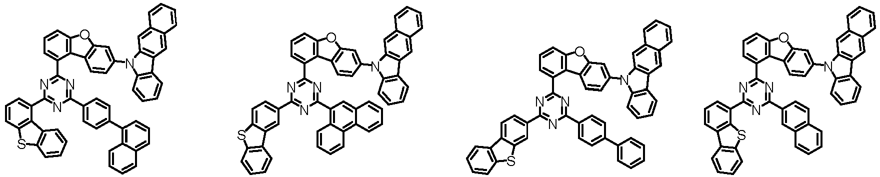

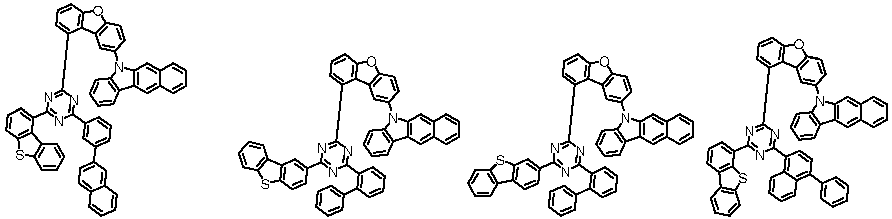

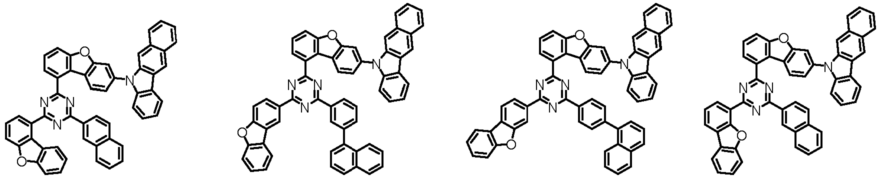

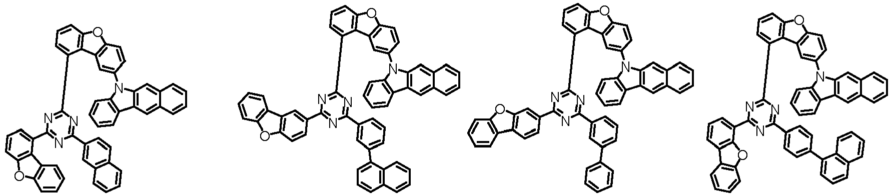

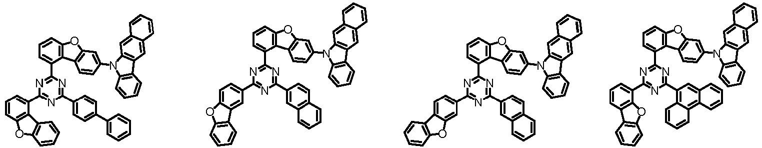

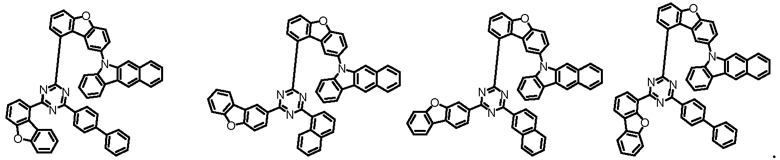

The compound represented by Chemical Formula 1 may be prepared by the method as shown in Reaction Scheme 1 or 2 below. 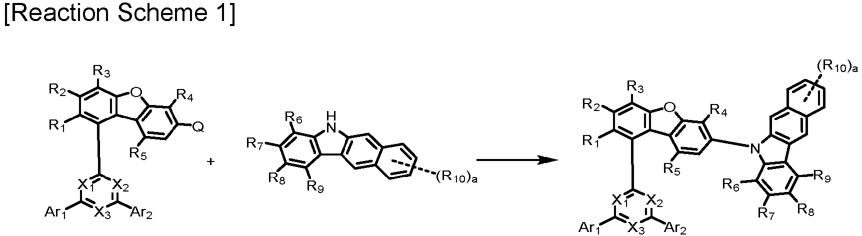 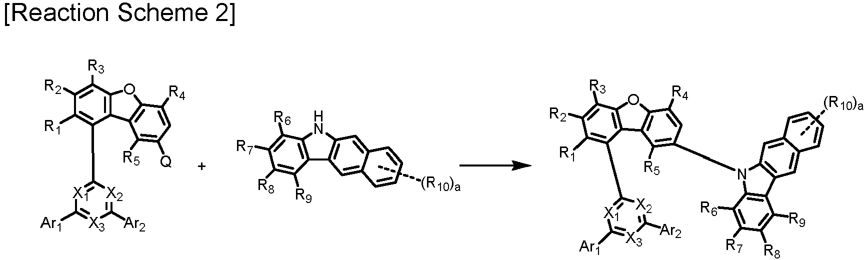 in the Reaction Schemes 1 and 2, the remaining substituents excluding Q are the same as defined above, and Q is halogen, more preferably bromo, or chloro. The above reaction is a Suzuki-coupling reaction, which is preferably carried out in the presence of a palladium catalyst and a base, and a reactive group for the Suzuki-coupling reaction can be modified as known in the art. The above preparation method will be more specifically described in the Preparation Examples described hereinafter.

Further, as the host material, a compound represented by Chemical Formula 1 and a compound represented by Chemical Formula 2 may be used together.

The compound represented by Chemical Formula 2 may be a compound represented by Chemical Formula 2-1. 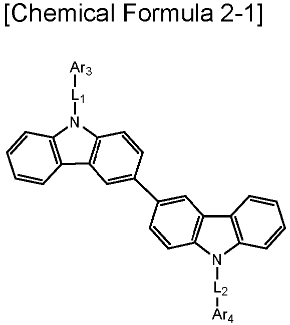 in the Chemical Formula 2-1,
Ar₃, Ar₄, L₁ and L₂ are as defined above.

In Chemical Formula 2, Ar₃ and Ar₄ may be each independently any one selected from the group consisting of:

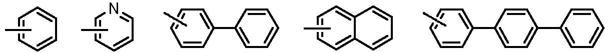

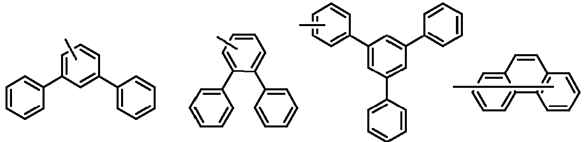

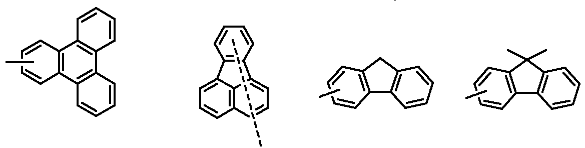

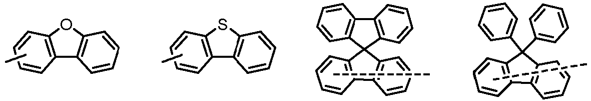

In Chemical Formula 2, L₁ and L₂ may be each independently a single bond or any one selected from the group consisting of:

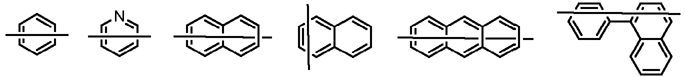

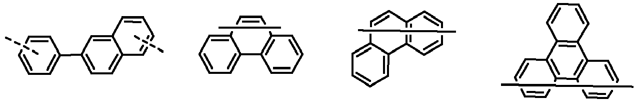

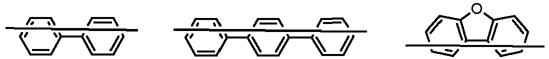

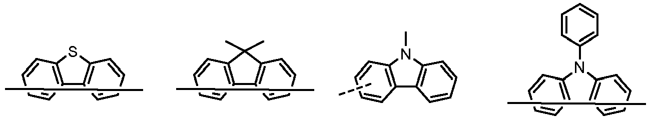

In Chemical Formula 2, R₁₁ to R₁₄ may be hydrogen.

The compound represented by Chemical Formula 2 may be selected from the group consisting of the following compounds.

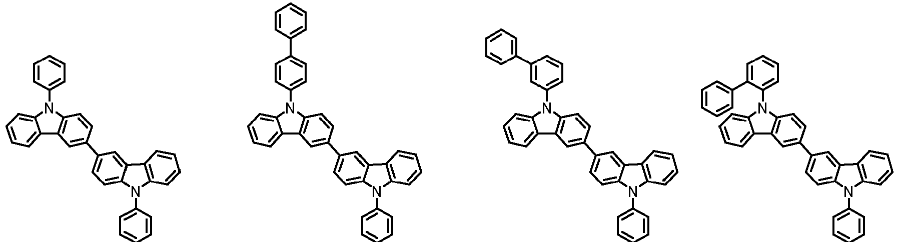

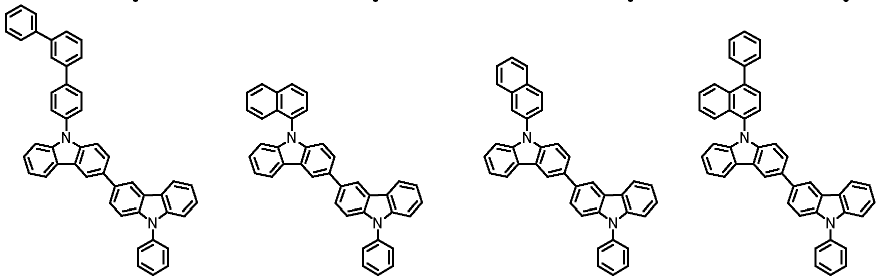

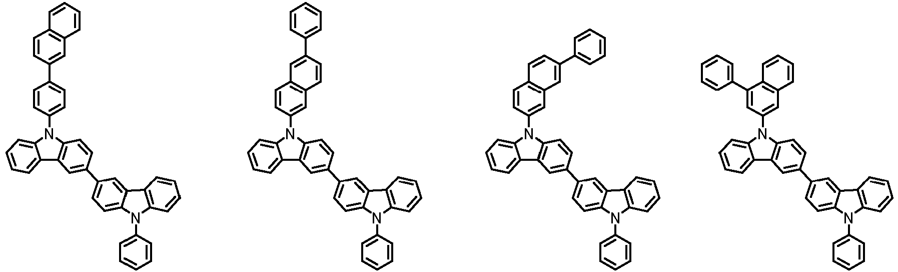

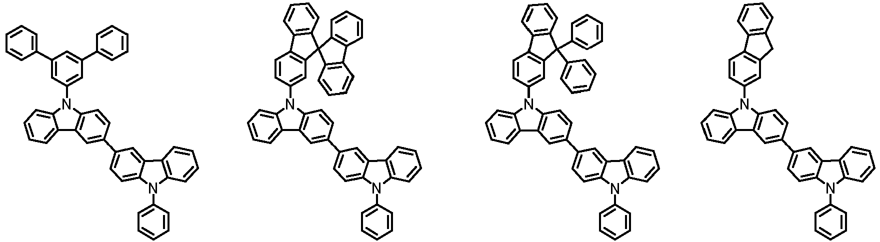

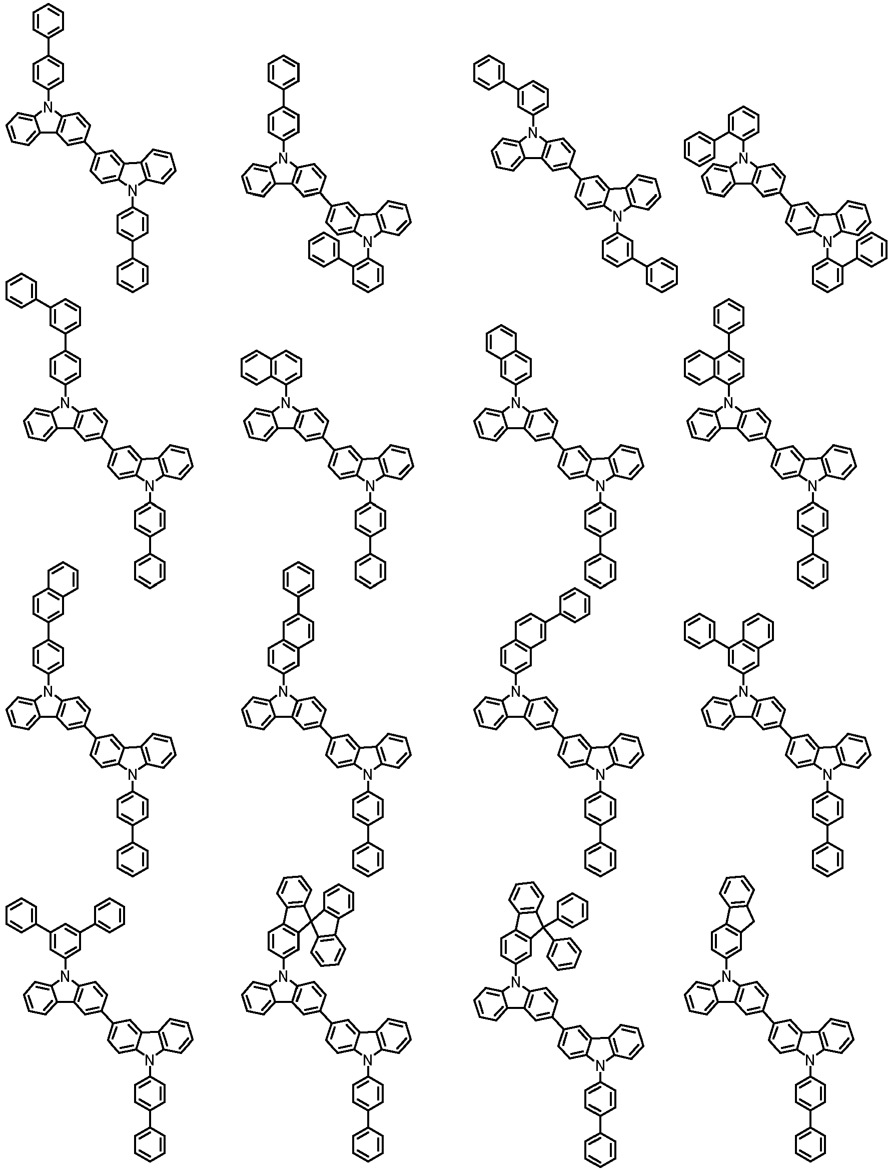

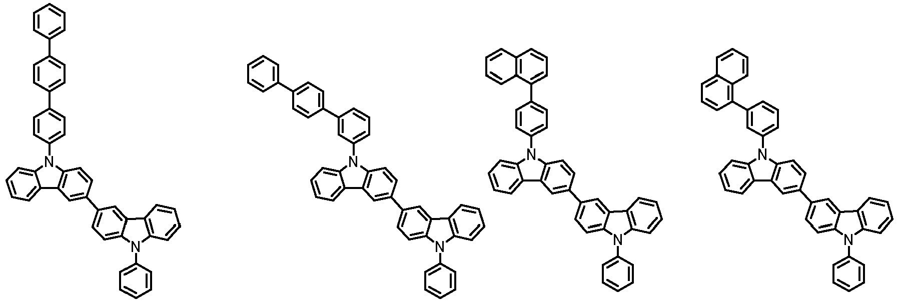

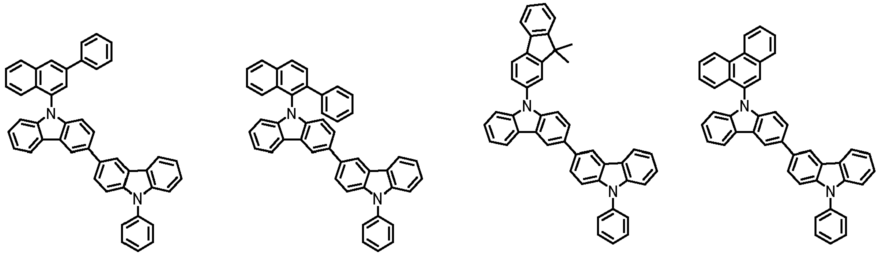

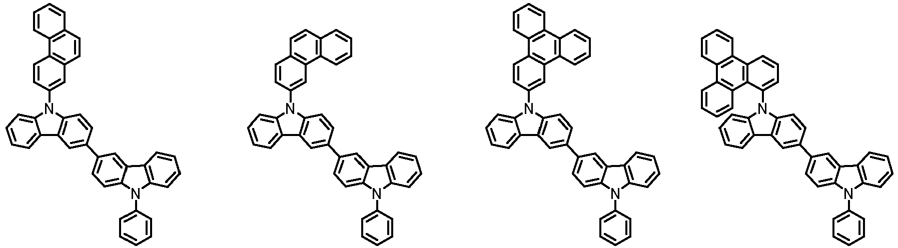

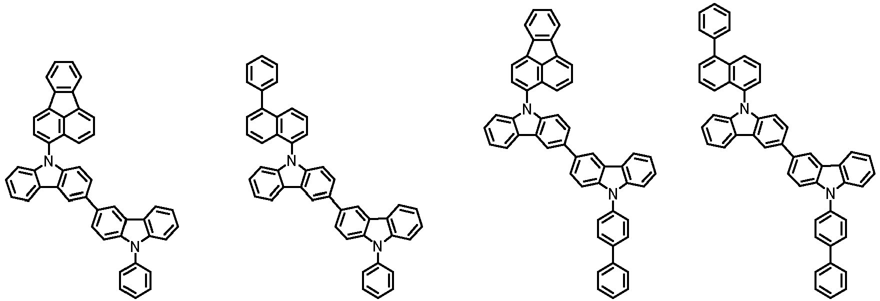

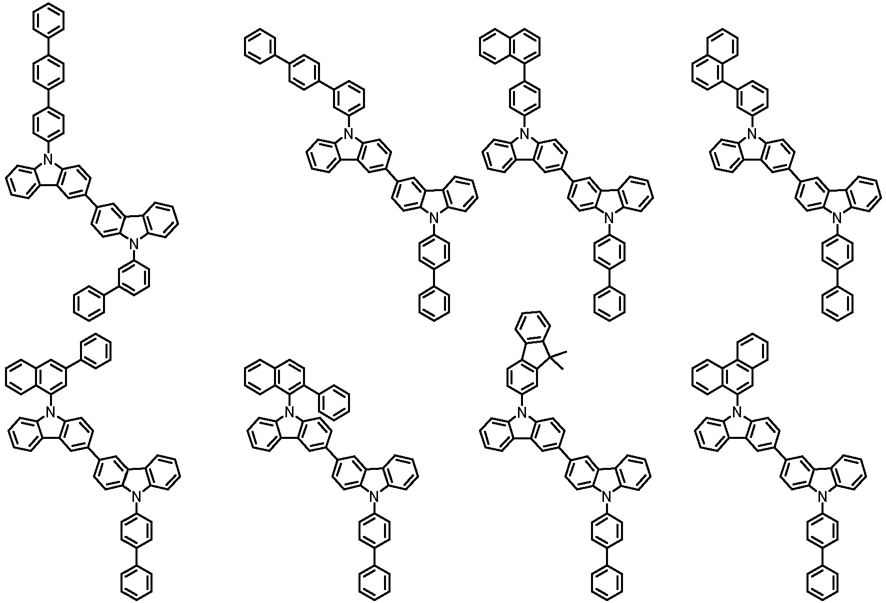

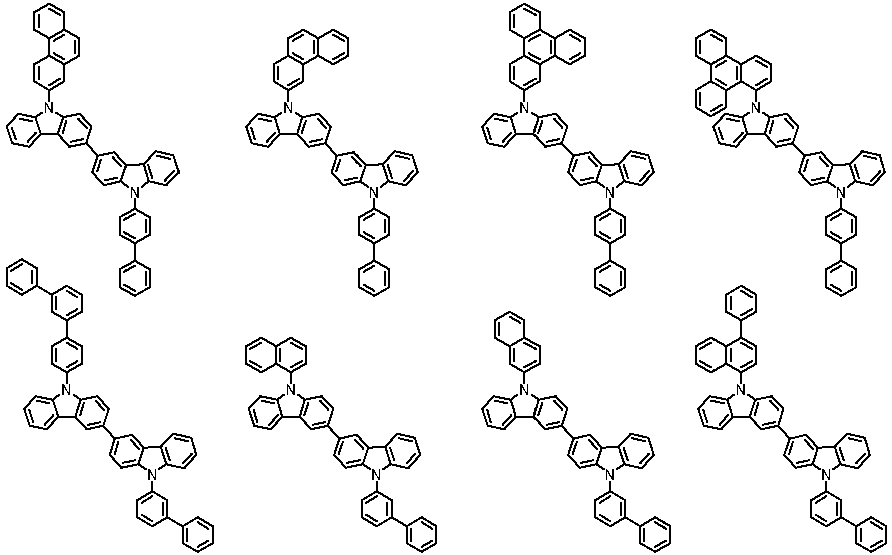

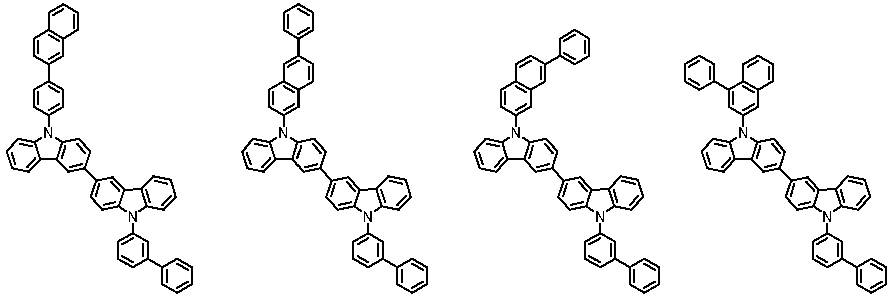

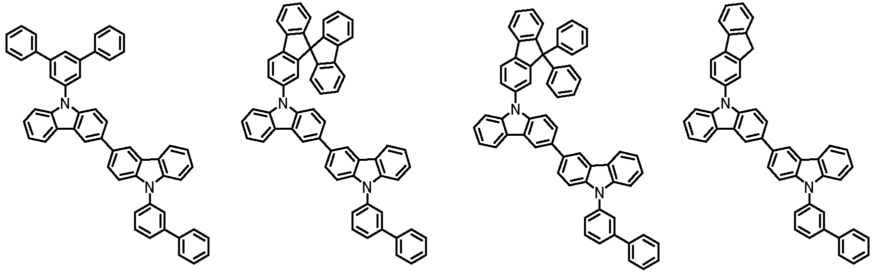

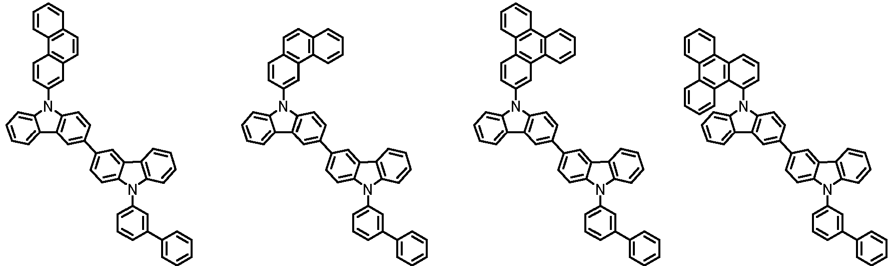

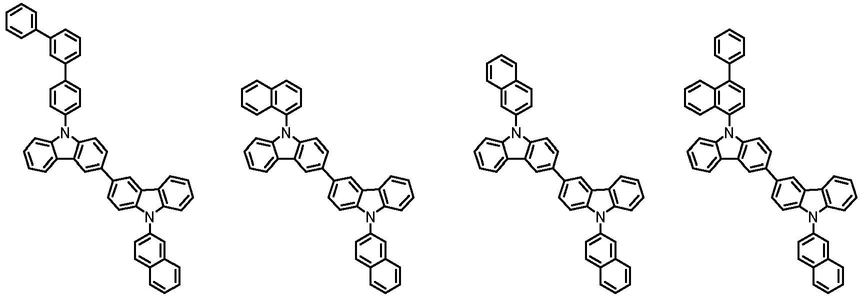

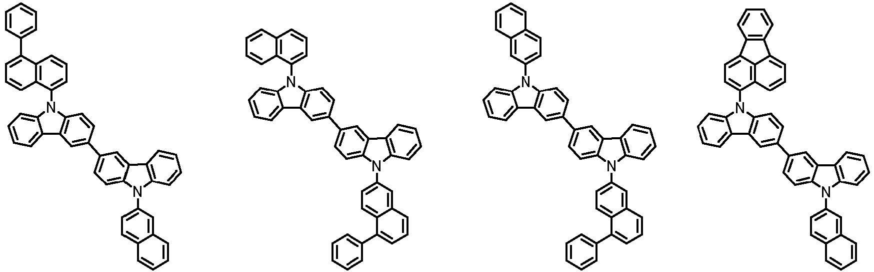

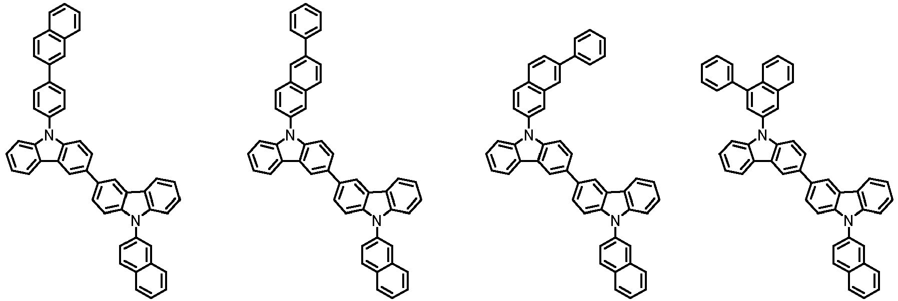

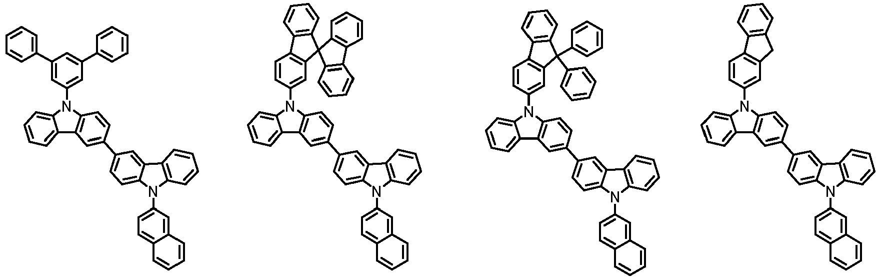

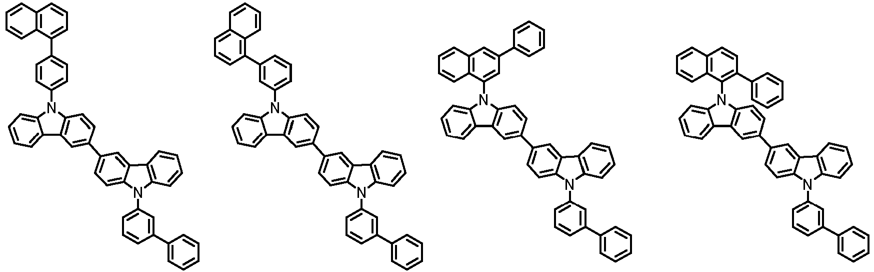

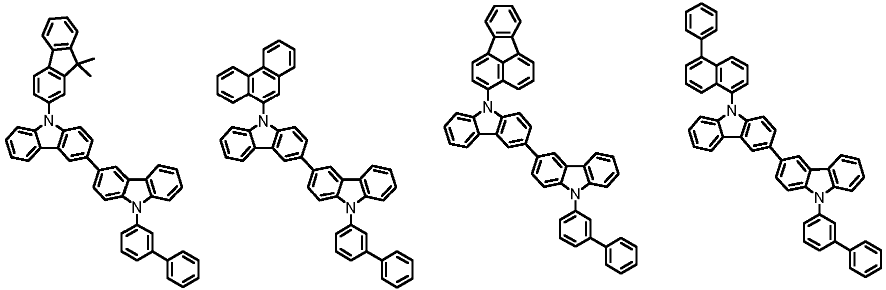

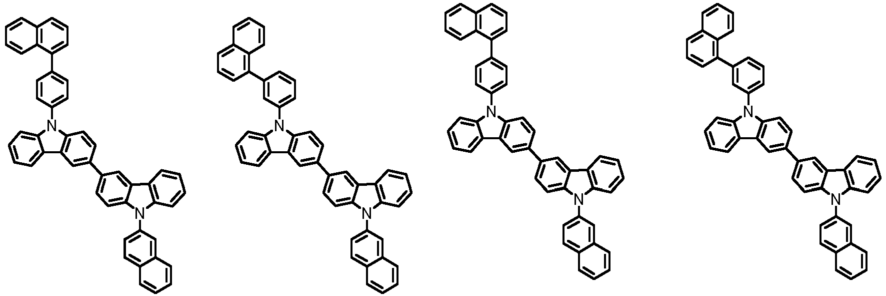

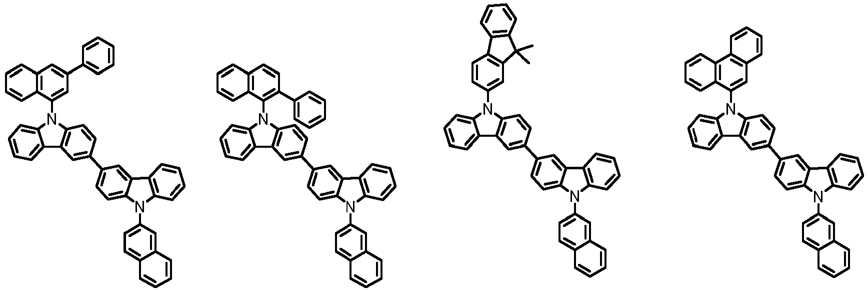

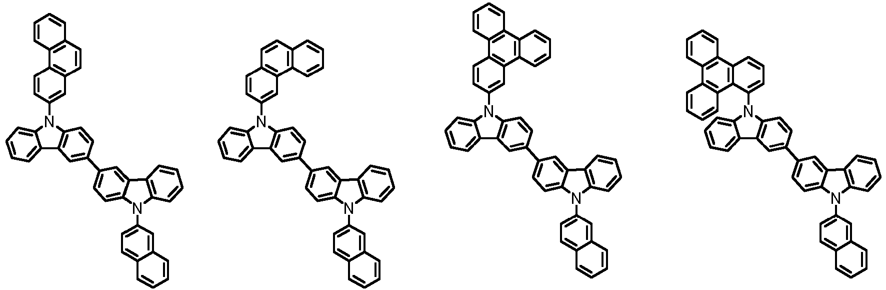

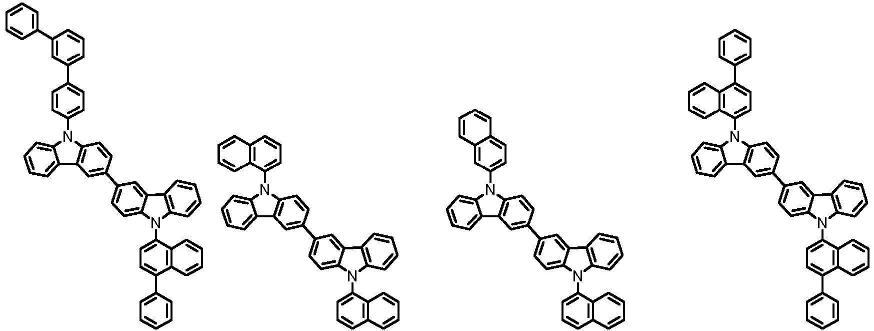

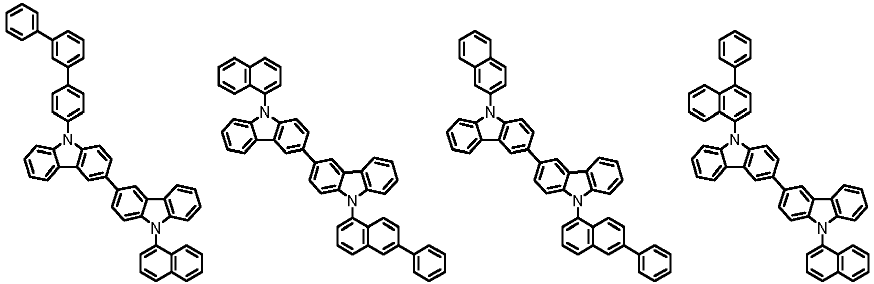

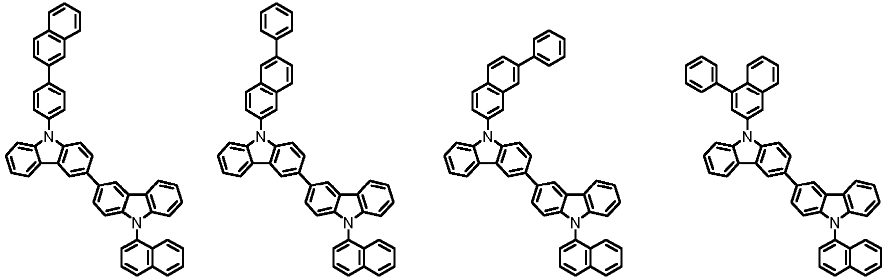

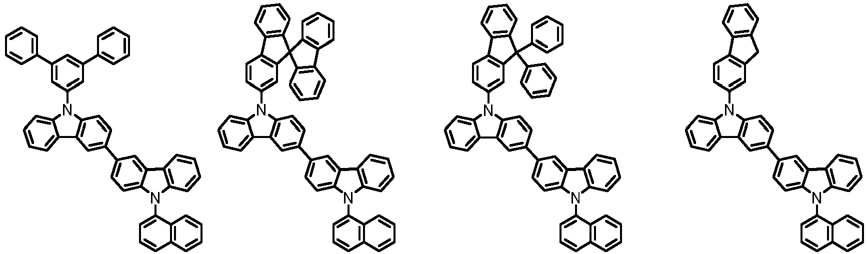

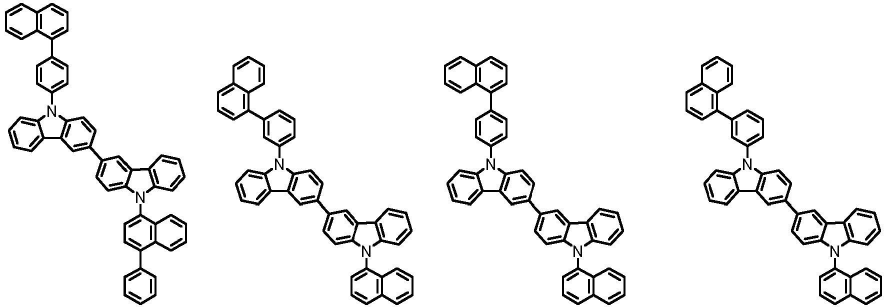

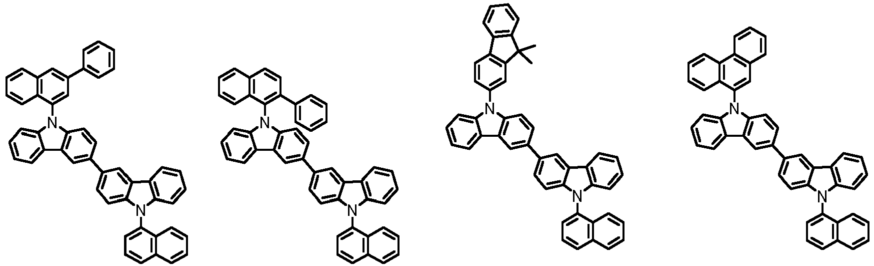

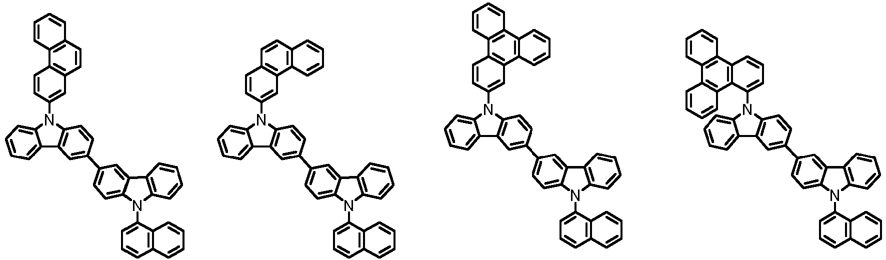

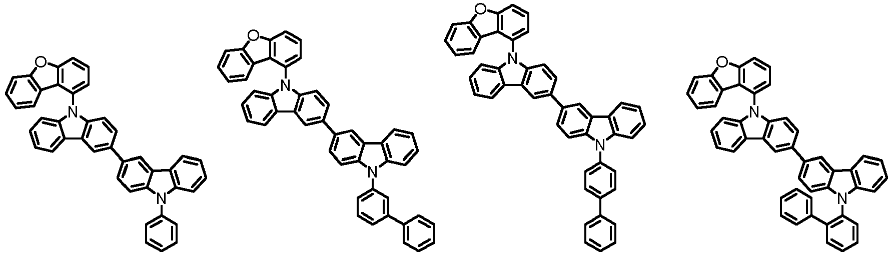

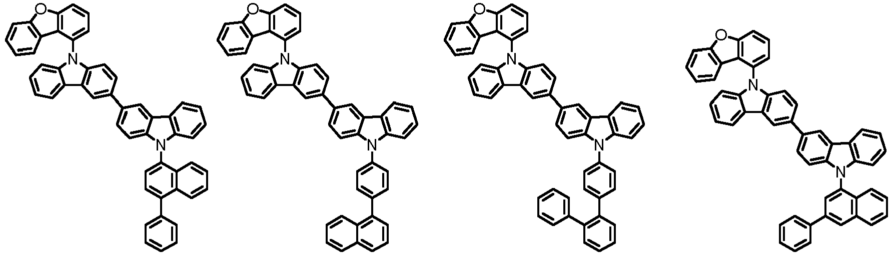

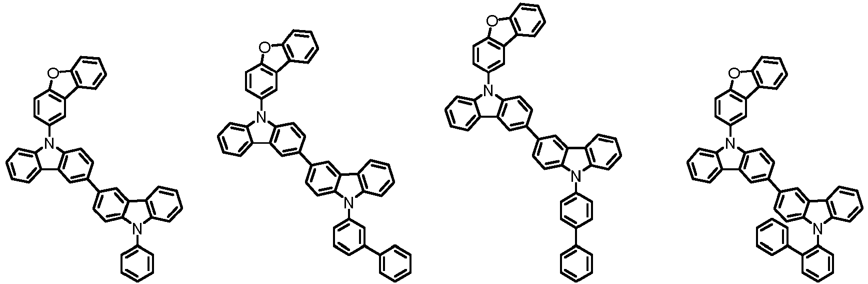

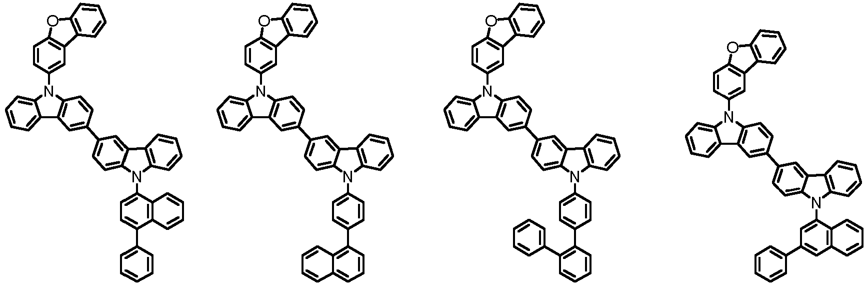

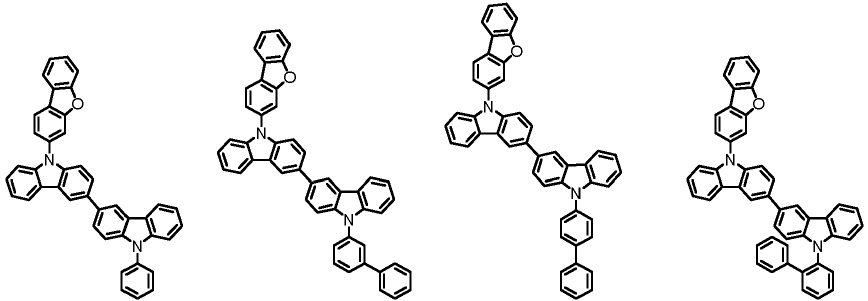

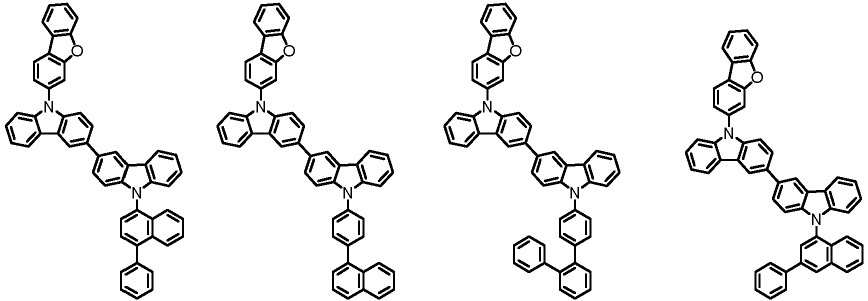

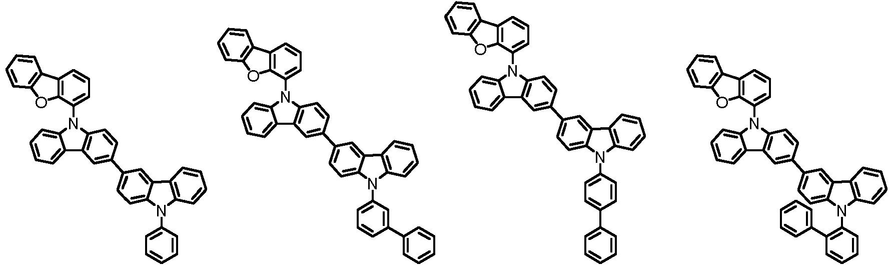

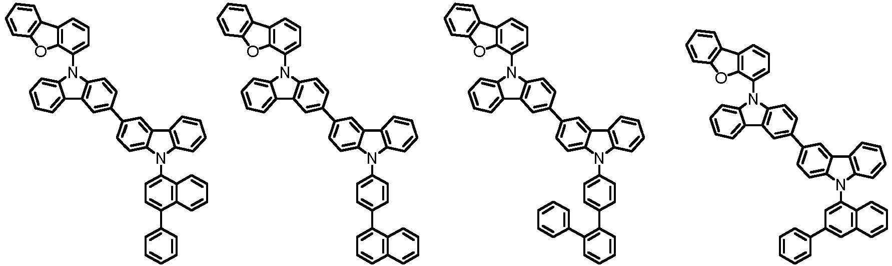

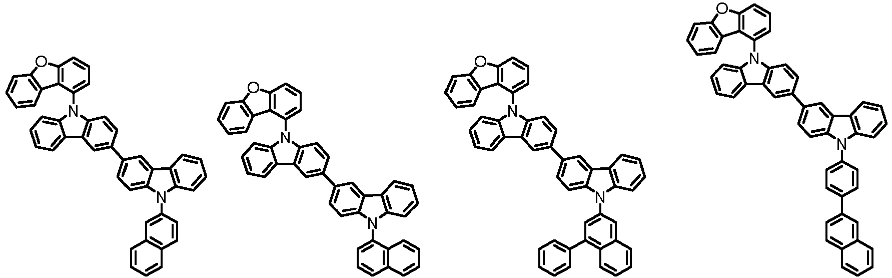

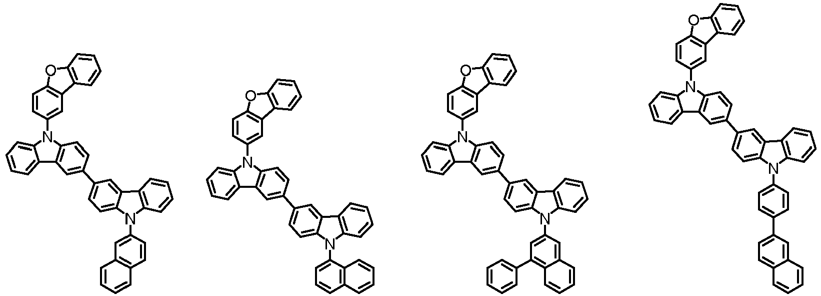

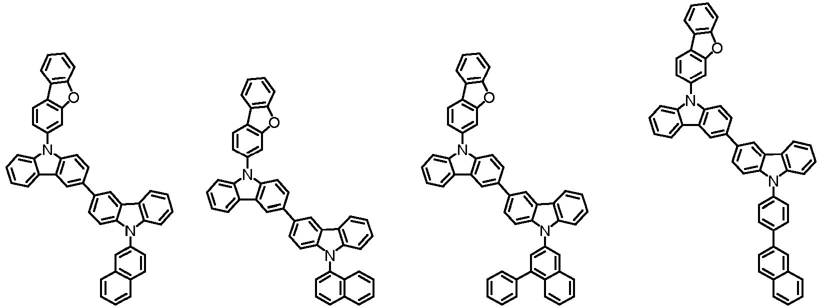

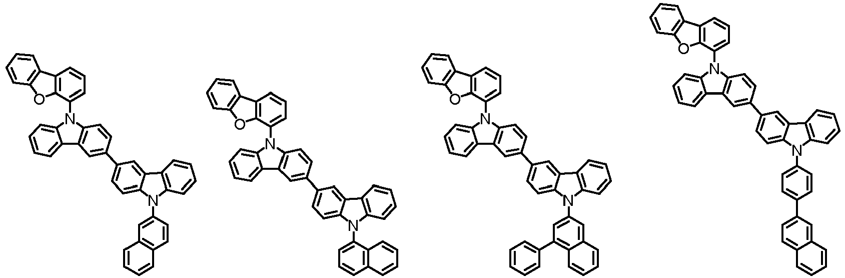

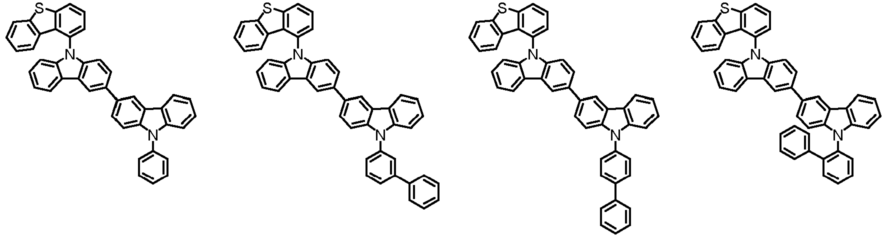

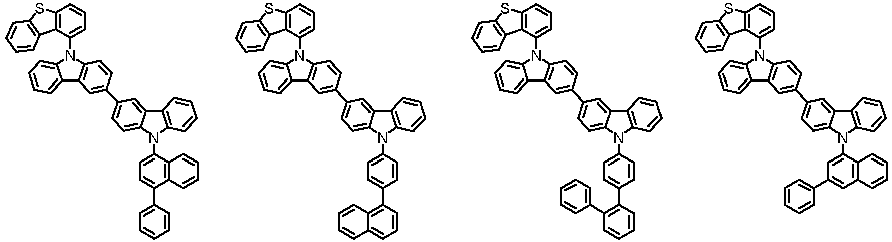

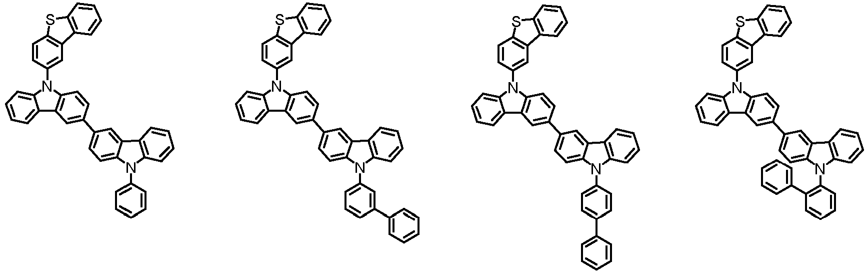

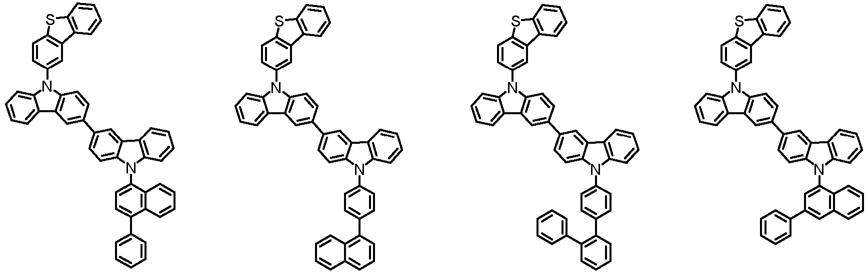

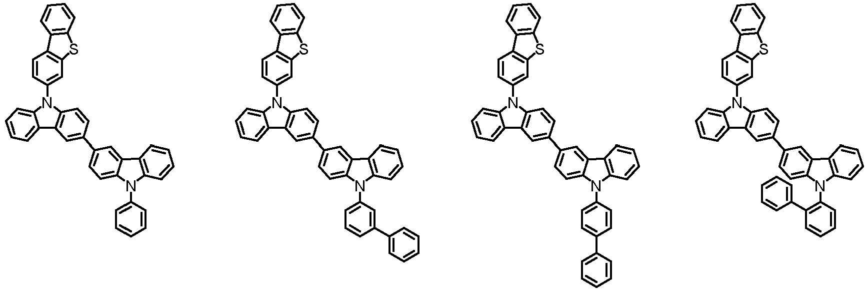

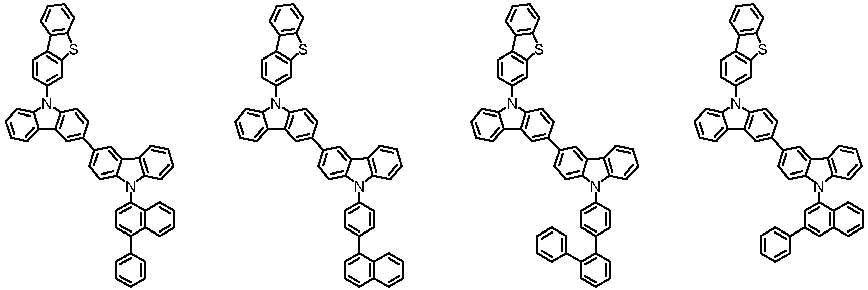

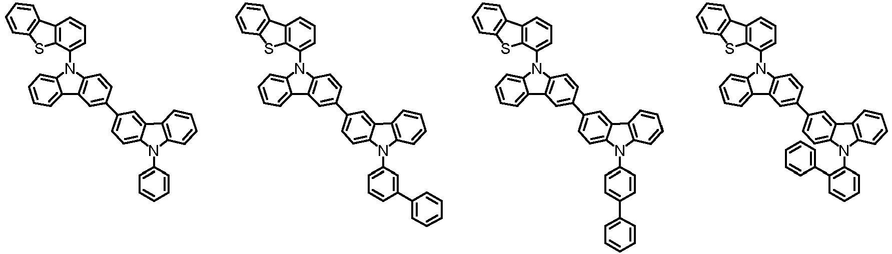

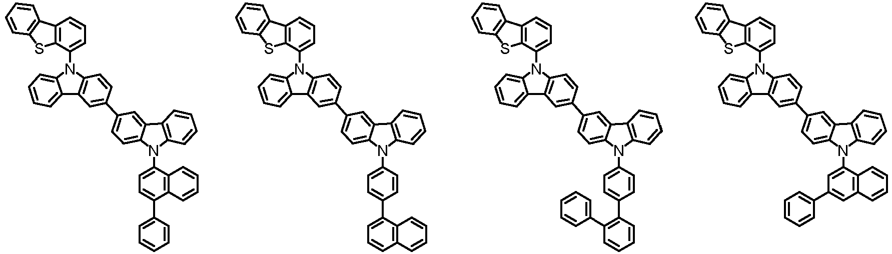

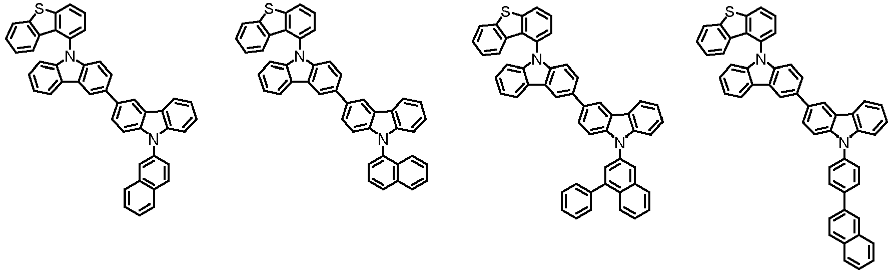

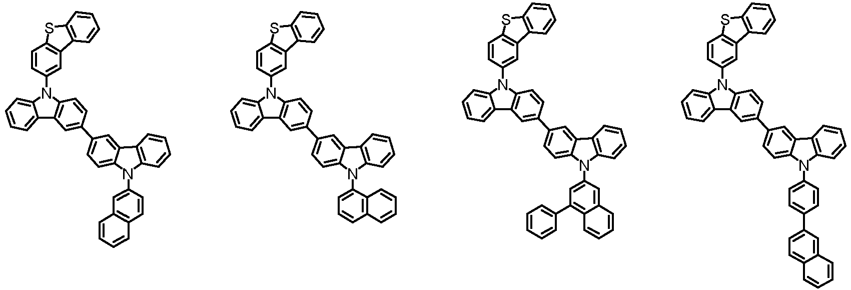

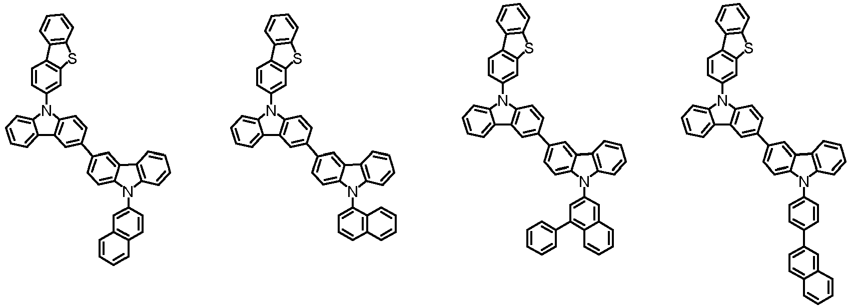

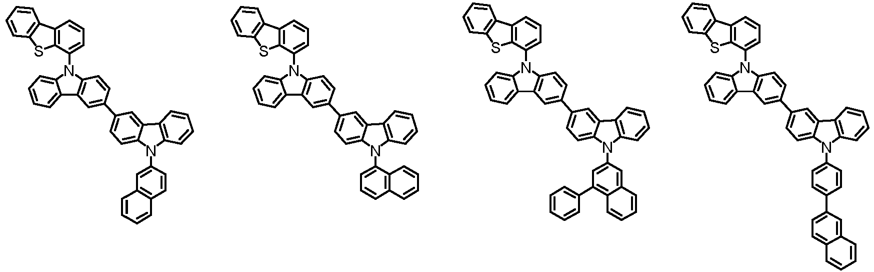

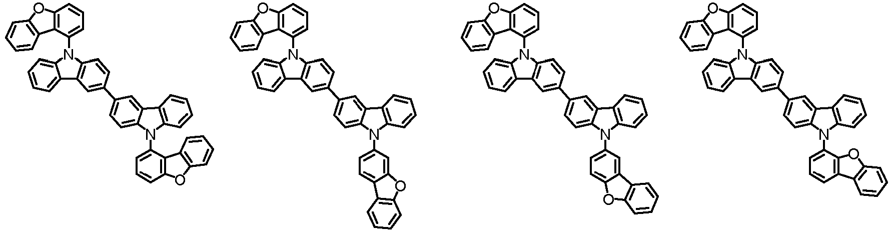

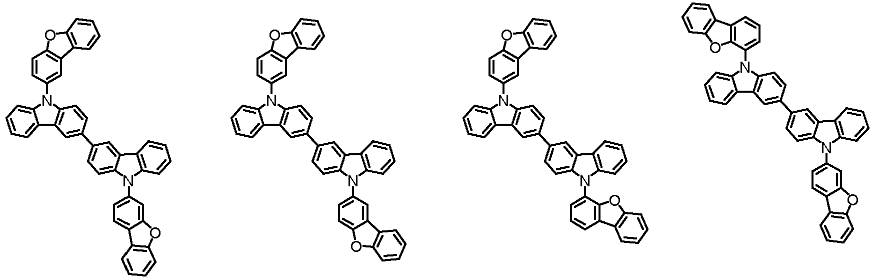

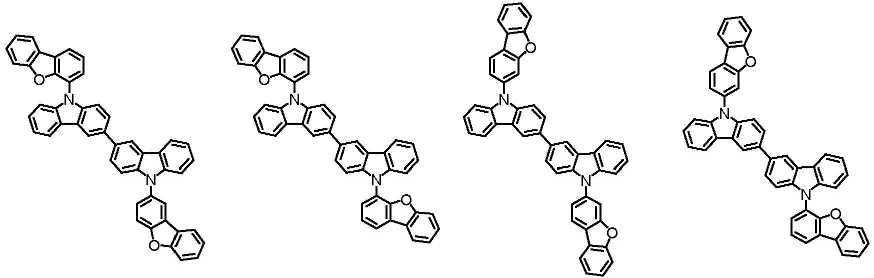

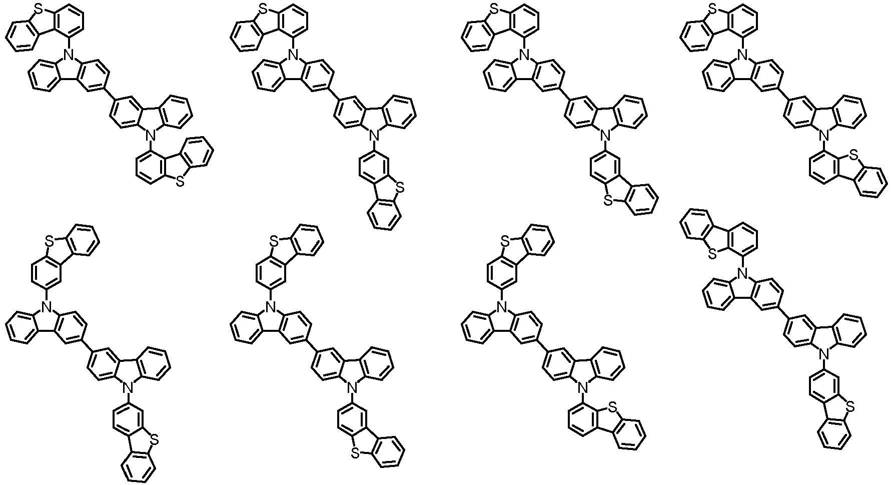

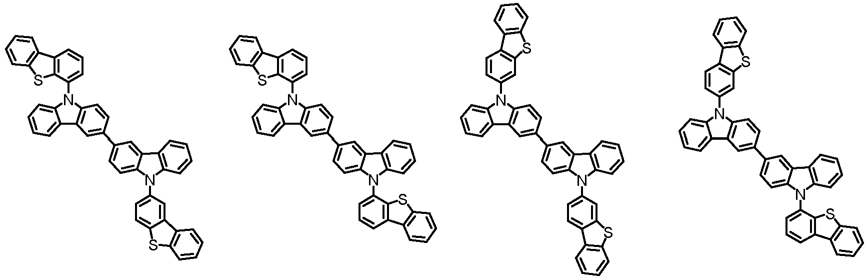

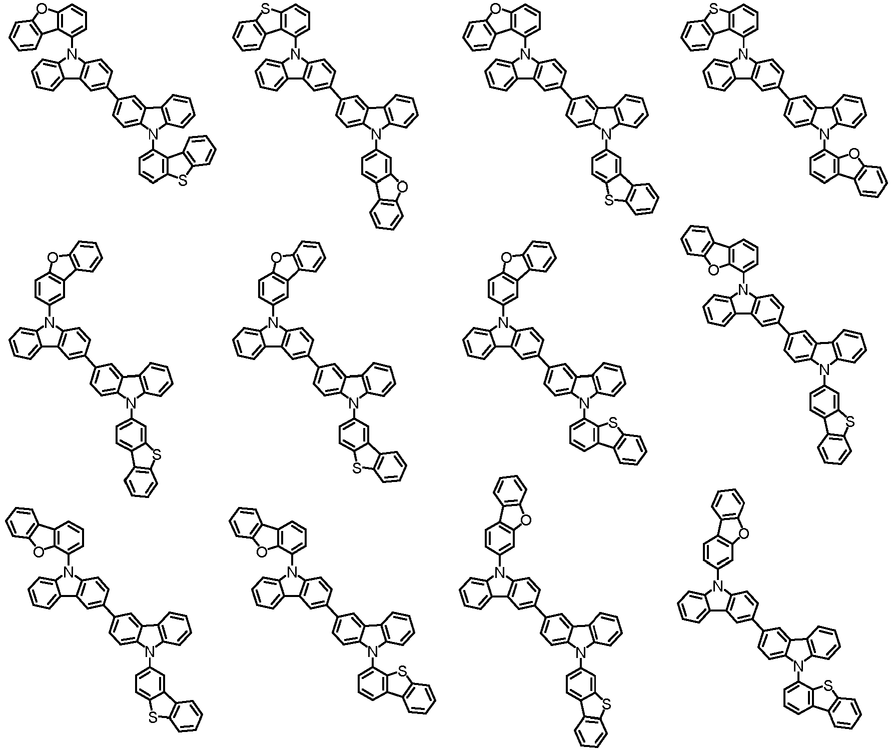

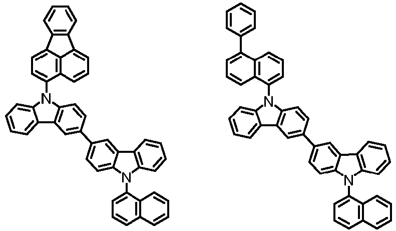

The compound represented by Chemical Formula 2 may be prepared by the method as shown in Reaction Scheme 3 below. The preparation method will be more specifically described in the Preparation Examples described hereinafter. 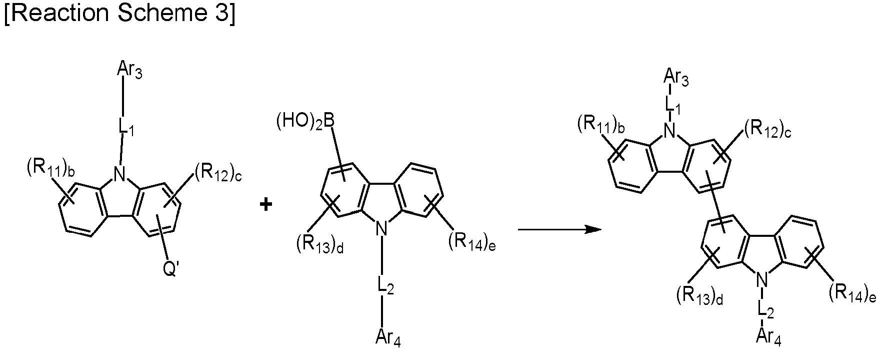 in the Reaction Scheme 3, the remaining substituents excluding Q' are the same as defined above, and Q' is halogen, more preferably bromo, or chloro. **The** above reaction is a Suzuki-coupling reaction, which is preferably carried out in the presence of a palladium catalyst and a base, and a reactive group for the Suzuki-coupling reaction can be modified as known in the art. **The** above preparation method will be more specifically described in the Preparation Examples described hereinafter.

**The** light emitting layer may further include a host material known in the technical field to which the present disclosure pertains, in addition to the compound represented by Chemical Formula 1 and the compound represented by Chemical Formula 2. Specific examples of such host materials include fused aromatic ring derivatives or heteroring-containing compounds or the like. Specifically, the fused aromatic ring derivative includes anthracene derivatives, pyrene derivatives, naphthalene derivatives, pentacene derivatives, phenanthrene compounds, fluoranthene compounds and the like, and the heteroring-containing compound includes carbazole derivatives, dibenzofuran derivatives, ladder-type furan compounds, pyrimidine derivatives and the like, but the material is not limited thereto.

Meanwhile, the light emitting layer may include a dopant material. **The** dopant material includes aromatic amine derivatives, styrylamine compounds, boron complexes, fluoranthene compounds, metal complexes and the like. Specifically, the aromatic amine derivative is a fused aromatic ring derivative having a substituted or unsubstituted arylamino group and includes pyrene, anthracene, chrysene, peryflanthene and the like, which have an arylamino group, and the styrylamine compound is a compound in which substituted or unsubstituted arylamine is substituted with at least one arylvinyl group, and one, two or more substituents selected from the group consisting of an aryl group, a silyl group, an alkyl group, a cycloalkyl group and an arylamino group are substituted or unsubstituted. Specifically, styrylamine, styryldiamine, styryltriamine, styryltetramine or the like is included, but the styrylamine compound is not limited thereto. **In** addition, the metal complex includes iridium complexes, platinum complexes or the like, but is not limited thereto.

For example, the dopant may be any one selected from compounds represented by the following Dp-1 to Dp-38.

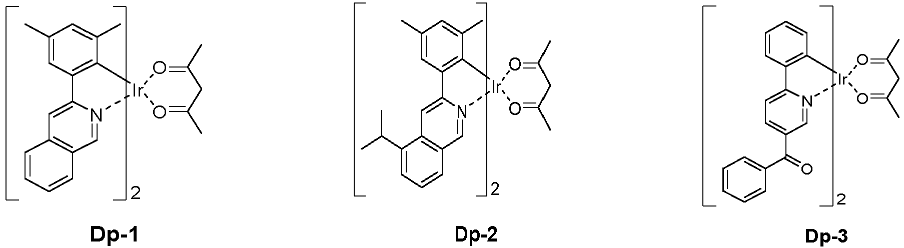

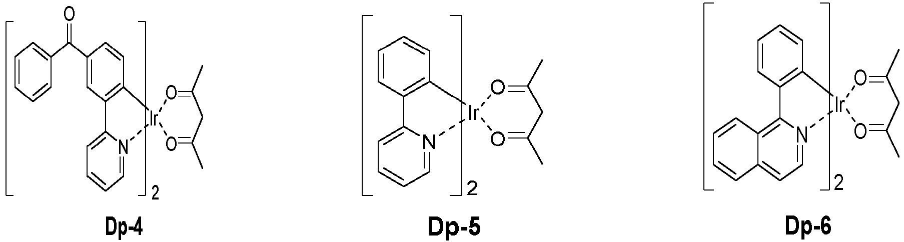

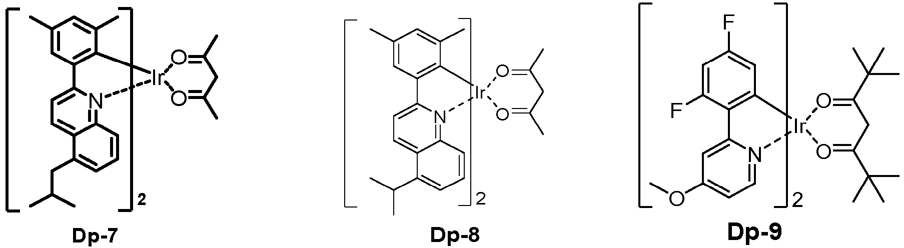

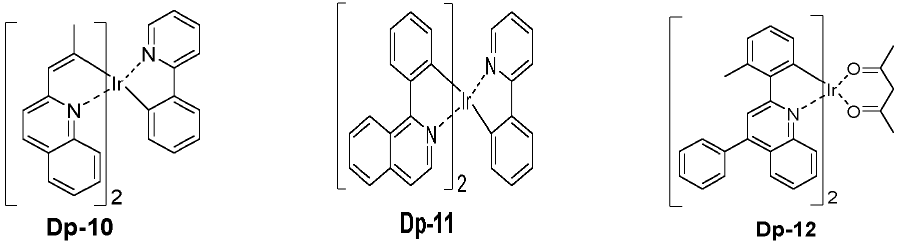

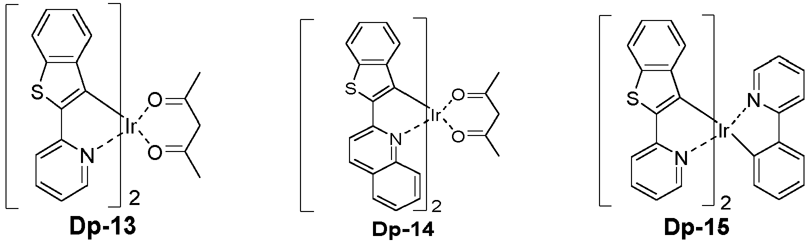

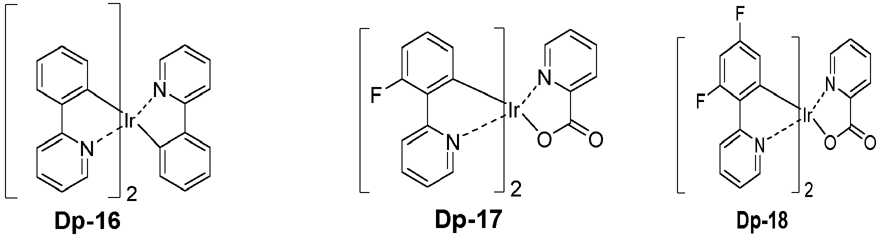

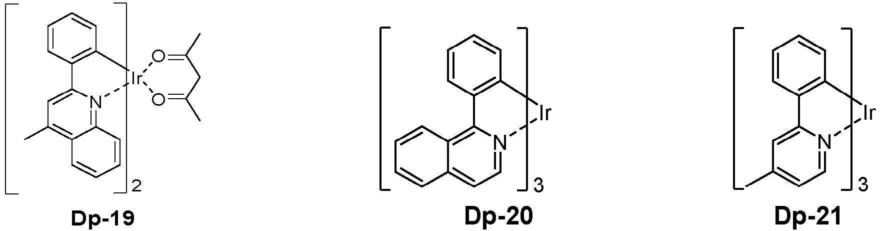

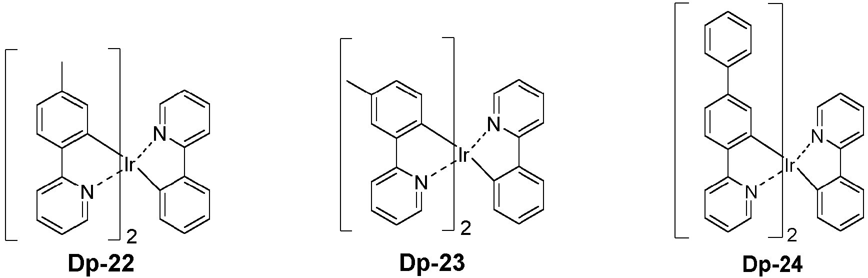

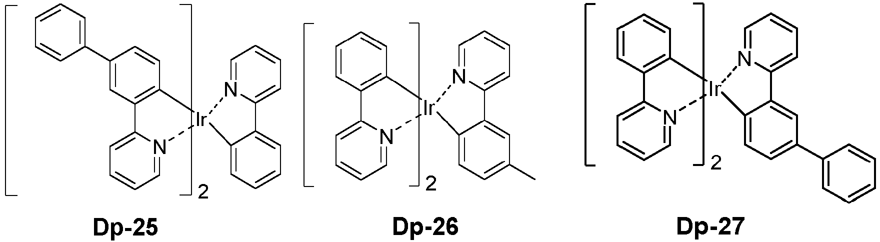

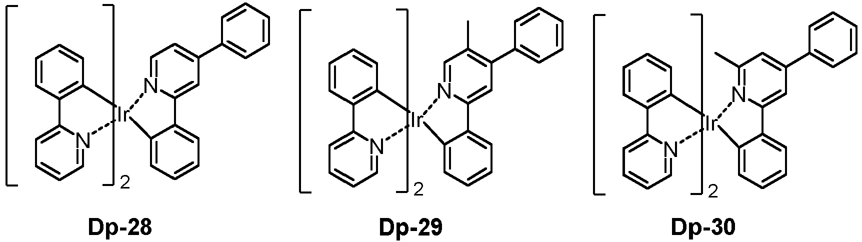

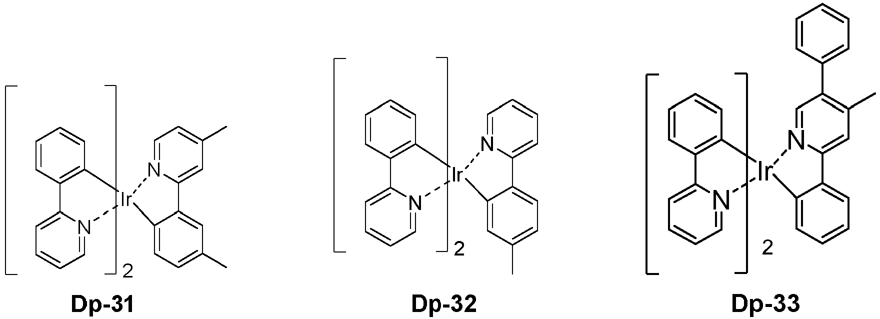

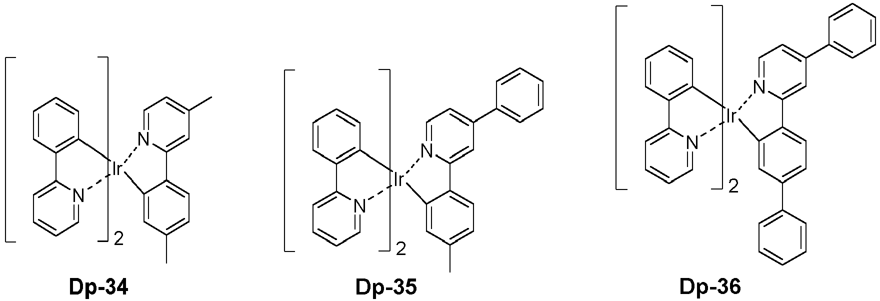

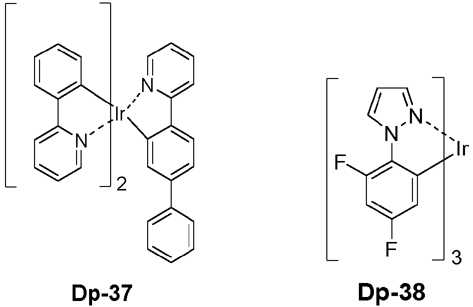

### Hole Transport Layer

The hole transport layer is a layer that receives holes from an anode or a hole injection layer formed on the anode and transports the holes to the light emitting layer. The hole transport material is suitably a material having large mobility to the holes, which may receive holes from the anode or the hole injection layer and transfer the holes to the light emitting layer.

Specific examples thereof include an arylamine-based organic material, a conductive polymer, a block copolymer in which a conjugate portion and a non-conjugate portion are present together, and the like, but are not limited thereto.

### Hole Regulating Layer

The hole regulating layer refers to a layer that serves to regulate a movement of holes according to the energy level of the light emitting layer in the organic light emitting device.

### Electron Transport Layer

The electron transport layer is layer which receives electrons from an electron injection layer and transports the electrons to a light emitting layer, and the electron transport material is suitably a material which may receive electrons well from a cathode and transfer the electrons to a light emitting layer, and has a large mobility for electrons. Specific examples of the electron transport material include: a pyridine derivative; a pyrimidine derivative; triazole derivative; an Al complex of 8-hydroxyquinoline; a complex including Alq₃; an organic radical compound; a hydroxyflavone-metal complex, and the like, but are not limited thereto. The electron transport layer may be used with any desired cathode material, as used according to the related art. In particular, appropriate examples of the cathode material are a typical material which has a low work function, followed by an aluminum layer or a silver layer. Specific examples thereof include cesium, barium, calcium, ytterbium, and samarium, in each case followed by an aluminum layer or a silver layer.

### Electron Injection Layer

The organic light emitting device according to the present disclosure may include an electron injection layer between the electron transport layer and the cathode, if necessary. The electron injection layer is a layer which injects electrons from a cathode, and is preferably a compound which has a capability of transporting electrons, has an effect of injecting electrons from a cathode and an excellent effect of injecting electrons into a light emitting layer or a light emitting material, prevents excitons produced from the light emitting layer from moving to a hole injection layer, and is also excellent in the ability to form a thin film. Specific examples thereof include fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, a metal complex compound, a nitrogen-containing 5-membered ring derivative, and the like, but are not limited thereto.

Examples of the metal complex compound include 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, bis(2-methyl-8-quinolinato)(2-naphtholato)gallium, and the like, but are not limited thereto.

### Organic Light Emitting Device

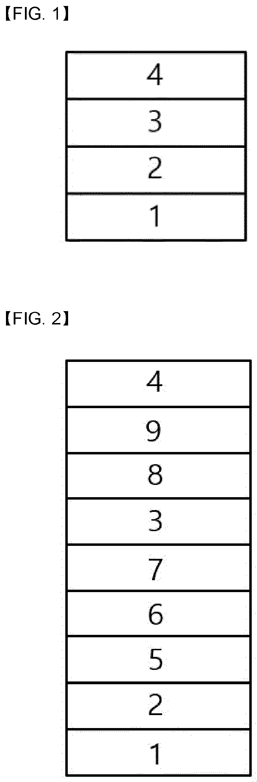

The structure of the organic light emitting device according to one embodiment is illustrated in FIG. 1 and FIG. 2. FIG. 1 shows an example of an organic light emitting device comprising a substrate 1, an anode 2, a light emitting layer 3, and a cathode 4. In such a structure, the compounds represented by Chemical Formula 1 and Chemical Formula 2 may be included in the light emitting layer.

FIG. 2 shows an example of an organic light emitting device comprising a substrate 1, an anode 2, a hole injection layer 5, a hole transport layer 6, an electron inhibition layer 7, a light emitting layer 3, a hole blocking layer 8, an electron injection and transport layer 9, and a cathode 4. In such a structure, the compounds represented by Chemical Formula 1 and Chemical Formula 2 may be included in at least one layer of the hole injection layer, the hole transport layer, the electron inhibition layer, the light emitting layer, the hole blocking layer and the electron injection and transport layer, and the light emitting layer may include two or more host materials. In this case, the two or more host materials may include the compounds represented by Chemical Formulae 1 and 2.

The organic light emitting device according to the present disclosure may be manufactured by sequentially laminating the above-mentioned components. In this case, the organic light emitting device may be manufactured may be manufactured by depositing a metal, metal oxides having conductivity, or an alloy thereof on the substrate using a PVD (physical vapor deposition) method such as a sputtering method or an e-beam evaporation method to form an anode, forming the above-mentioned respective layers thereon, and then depositing a material that can be used as the cathode thereon. In addition to such a method, the organic light emitting device may be manufactured by sequentially depositing a cathode material, an organic material layer and an anode material on a substrate. Further, the light emitting layer may be formed using the host and the dopant by a solution coating method as well as a vacuum deposition method. Herein, the solution coating method means a spin coating, a dip coating, a doctor blading, an inkjet printing, a screen printing, a spray method, a roll coating, or the like, but is not limited thereto.

In addition to such a method, the organic light emitting device may be manufactured by sequentially depositing a cathode material, an organic material layer and an anode material on a substrate (International Publication WO2003/012890). However, the manufacturing method is not limited thereto.

Meanwhile, the organic light emitting device according to the present disclosure may be a front side emission type, a backside emission type, or a double-sided emission type according to the used material.

The preparation of the organic light emitting device will be described in detail in the following examples. However, these examples are presented for illustrative purposes only, and are not intended to limit the scope of the present disclosure.

### Preparation Example 1-1: Preparation of Intermediate a

### 1) Preparation of Intermediate a-1

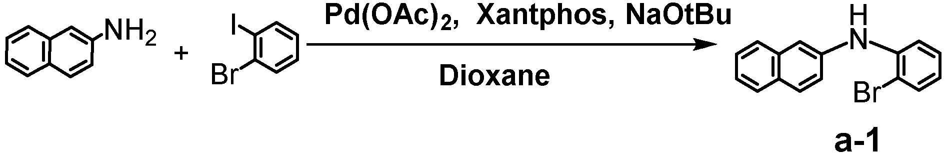

Naphthalen-2-amine (300.0 g, 1.0 eq), 1-bromo-2-iodobenzene (592.7 g, 1.0 eq), sodium tert-butoxide (NaOtBu, 302.0 g, 1.5 eq), palladium acetate (Pd(OAc)₂, 4.70 g, 0.01 eq), xantphos (12.12 g, 0.01 eq) were dissolved in 1,4-dioxane (5L), and the mixture was refluxed and stirred. When the reaction was terminated after 3 hours, the solvent was removed under reduced pressure. Then, the reaction mixture was completely dissolved in ethyl acetate, washed with water, and then approximately 70% of the solvent was removed under reduced pressure again. Under reflux again, crystals were dropped while adding hexane thereto, and the result was cooled and then filtered. This was subjected to column chromatography to give Intermediate a-1. (443.5 g, yield: 71%, [M+H]⁺ = 299)

### 2) Preparation of Intermediate a (5H-benzo [b] carbazole)

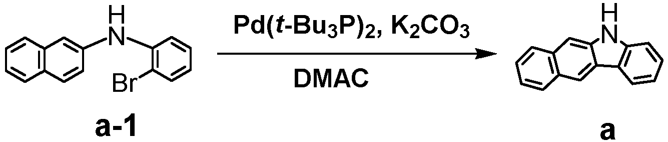

Intermediate a-1 (443.5 g, 1.0 eq), bis(tri-tert-butylphosphine)palladium (0) (Pd(t-BusP)₂, 8.56 g, 0.01 eq) and potassium carbonate (K₂CO₃, 463.2 g, 2.00 eq) were added to diethylacetamide (4L ), and the mixture was refluxed and stirred. After 3 hours, the reaction solution was poured into water, crystals were dropped, and filtered. The filtered solid was completely dissolved in 1,2-dichlorobenzene, washed with water, and the solution in which the product was dissolved was concentrated under reduced pressure, and crystals were dropped, and the result was cooled and then filtered. This was purified by column chromatography to give Intermediate a (5H-benzo[b]carbazole). (174.8 g, yield: 48%, [M+H]⁺ = 218)

### Preparation Example 1-2: Preparation of Intermediate b

The following Intermediate b (7H-dibenzo[b,g]carbazole) was obtained in the same manner as in Preparation Example 1-1, except that 1-bromo-2-iodonaphthalene was used instead of 1-bromo-2-iodobenzene.

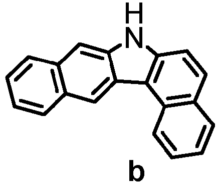

### Preparation Example 1-3: Preparation of Intermediate c

The following Intermediate c (6H-dibenzo[b,h]carbazole) was obtained in the same manner as in Preparation Example 1-1, except that 2,3-dibromonaphthalene was used instead of 1-bromo-2-iodobenzene.

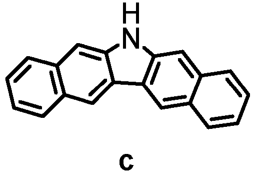

### Preparation Example 1-4: Preparation of Intermediate d

The following Intermediate d (13H-dibenzo[a,h]carbazole) was obtained in the same manner as in Preparation Example 1, except that 2-bromo-1-iodonaphthalene was used instead of 1-bromo-2-iodobenzene.

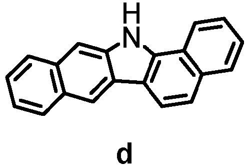

### Preparation Example 1-5: Preparation of Intermediate e

### 1) Preparation of Intermediate e-2

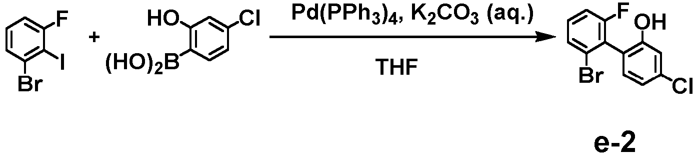

1-Bromo-3-fluoro-2-iodobenzene (200.0 g, 1.0 eq), (4-chloro-2-hydroxyphenyl)boronic acid (82.3 g, 1.0 eq), potassium carbonate (K₂CO₃, 164.6 g, 2.0 eq) and tetrakis(triphenylphosphine)palladium(0) (Pd(PPh₃)₄, 13.77 g, 0.02 eq) were dissolved in tetrahydrofuran (THF, 3L), and the mixture was refluxed and stirred. When the reaction was terminated after 2 hours, the solvent was removed under reduced pressure. Then, the reaction mixture was completely dissolved in ethyl acetate, washed with water, and then approximately 80% of the solvent was removed under reduced pressure again. Under reflux again, crystals were dropped while adding hexane thereto, and the result was cooled and then filtered. This was subjected to column chromatography to give Intermediate e-2. (129.5 g, yield: 72%, [M+H]⁺ = 300)

### 2) Preparation of Intermediate e-1

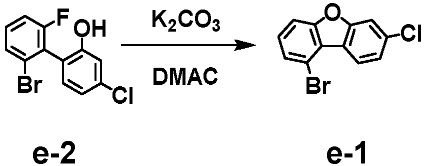

Intermediate e-2 (129.5 g, 1.0 eq) and potassium carbonate (K₂CO₃, 118.5 g, 2.00 eq) were added to diethylacetamide (2L), and the mixture was refluxed and stirred. After 1 hour, the reaction solution was poured into water, crystals were dropped and filtered. The filtered solid was completely dissolved in ethyl acetate, washed with water, and then approximately 70% of the solvent was removed under reduced pressure again. Under reflux again, crystals were dropped while adding hexane thereto, and the result was cooled and then filtered. This was subjected to column chromatography to give Intermediate e-1. (101.6 g, yield: 84%, [M+H]⁺ = 280)

### 3) Preparation of intermediate e

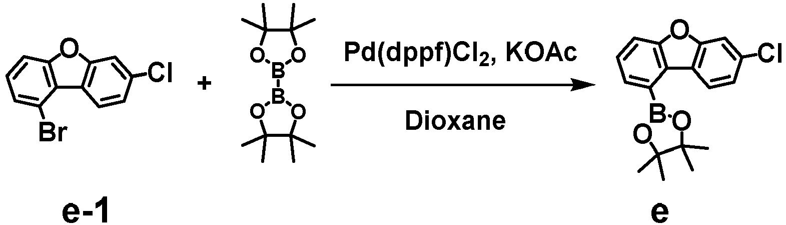

Intermediate e-1 (101.6 g, 1.0 eq), bis(pinacolato)diboron (119.1 g, 1.3 eq), 1,1-bis(diphenylphosphino) ferrocene-palladium (II) dichloride (Pd (dppf)Cl₂, 5.28 g, 0.02 eq) and potassium acetate (KOAc, 40.4 g, 2.00 eq) were added to dioxane (2L), and the mixture was refluxed and stirred. When the reaction was terminated after 3 hours, the solvent was removed under reduced pressure. The filtered solid was completely dissolved in chloroform (CHCl₃), washed with water, and the solution in which the product was dissolved was concentrated under reduced pressure to remove approximately 90% of the solvent. Under reflux again, crystals were dropped while adding ethanol thereto, and the result was cooled and then filtered to give Intermediate e. (103.1 g, yield: 87%, [M+H]⁺ = 329)

### Preparation Example 1-6: Preparation of Intermediate f

The following Intermediate f was obtained in the same manner as in Preparation Example 1-5, except that (5-chloro-2-hydroxyphenyl)boronic acid was used instead of (4-chloro-2-hydroxyphenyl)boronic acid.

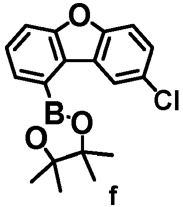

### Preparation Example 1-7: Preparation of Intermediate g

The following Intermediate g was obtained in the same manner as in Preparation Example 1-5, except that 3-bromo-1-fluoro-2-iodonaphthalene was used instead of 1-bromo-3-fluoro-2-iodobenzene.

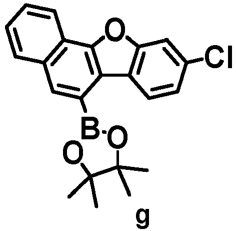

### Preparation Example 1-8: Preparation of Intermediate h

The following Intermediate h was obtained in the same manner as in Preparation Example 1-7, except that (4-chloro-2-hydroxyphenyl)boronic acid was used instead of (4-chloro-2-hydroxyphenyl)boronic acid.

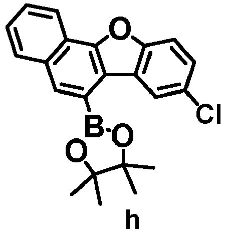

### Preparation Example 1-9: Preparation of Intermediate i

The following Intermediate i was obtained in the same manner as in Preparation Example 1-5, except that 1-bromo-3-fluoro-2-iodonaphthalene was used instead of 1-bromo-3-fluoro-2-iodobenzene.

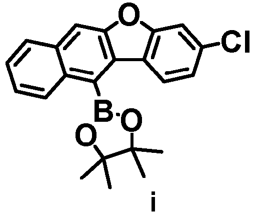

### Preparation Example 1-10: Preparation of Intermediate j

The following Intermediate j was obtained in the same manner as in Preparation Example 1-9, except that (5-chloro-2-hydroxyphenyl)boronic acid was used instead of (4-chloro-2-hydroxyphenyl)boronic acid.

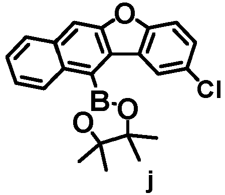

### Preparation Example 2-1: Preparation of Compound 1, not in accordance with the claimed invention.

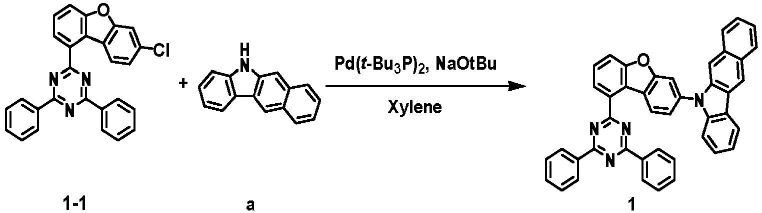

Intermediate 1-1 (20.0 g, 46.2 mmol), Intermediate a (10.0 g, 46.2 mmol), and sodium tert-butoxide (8.9 g, 92.4 mmol) were added to xylene (400 ml) under a nitrogen atmosphere, and the mixture was stirred and refluxed. Then, bis(tri-tert-butylphosphine)palladium(0) (0.5 g, 0.9 mmol) was added thereto. When the reaction was terminated after 2 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 1. (14.7 g, yield: 52%, MS: [M+H]⁺ = 615)

### Preparation Example 2-2: Preparation of Compound 2, not in accordance with the claimed invention

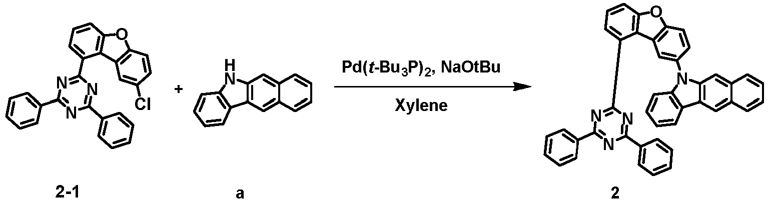

Intermediate 2-1 (20.0 g, 46.2 mmol), Intermediate a (10.0 g, 46.2 mmol) and sodium tert-butoxide (8.9 g, 92.4 mmol) were added to xylene (400 ml) under a nitrogen atmosphere, and the mixture was stirred and refluxed. Then, bis(tri-tert-butylphosphine)palladium(0) (0.5 g, 0.9 mmol) was added thereto. When the reaction was terminated after 3 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 2. (19.3 g, yield: 68%, MS: [M+H]⁺ = 615)

### Preparation Example 2-3: Preparation of Compound 3

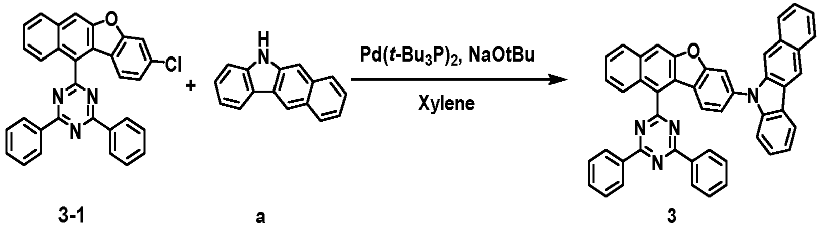

Intermediate 3-1 (20.0 g, 41.4 mmol), Intermediate a (9 g, 41.4 mmol) and sodium tert-butoxide (8 g, 82.8 mmol) were added to xylene (400 ml) under a nitrogen atmosphere, and the mixture was stirred and refluxed. Then, bis(tri-tert-butylphosphine)palladium(0) (0.4 g, 0.8 mmol) was added thereto. When the reaction was terminated after 3 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 3. (16.8 g, yield: 61%, MS: [M+H]⁺ = 665)

### Preparation Example 2-4: Preparation of Compound 4

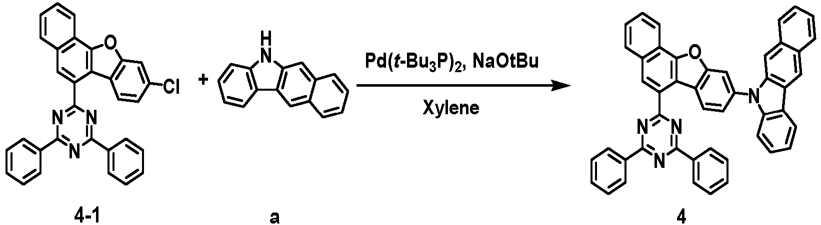

Intermediate 4-1 (20.0 g, 41.4 mmol), Intermediate a (9 g, 41.4 mmol) and sodium tert-butoxide (8 g, 82.8 mmol) were added to xylene (400 ml) under a nitrogen atmosphere, and the mixture was stirred and refluxed. Then, bis(tri-tert-butylphosphine)palladium(0) (0.4 g, 0.8 mmol) was added thereto. When the reaction was terminated after 2 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 4. (16.2 g, yield: 59%, MS: [M+H]⁺ = 665)

### Preparation Example 2-5: Preparation of Compound 5

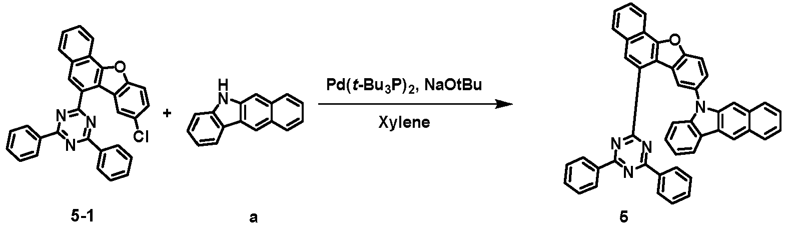

Intermediate 5-1 (20.0 g, 41.4 mmol), Intermediate a (9 g, 41.4 mmol) and sodium tert-butoxide (8 g, 82.8 mmol) were added to xylene (400 ml) under a nitrogen atmosphere, and the mixture was stirred and refluxed. Then, bis(tri-tert-butylphosphine)palladium(0) (0.4 g, 0.8 mmol) was added thereto. When the reaction was terminated after 2 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 5. (13.7 g, yield: 50%, MS: [M+H]⁺ = 665)

### Preparation Example 2-6: Preparation of Compound 6

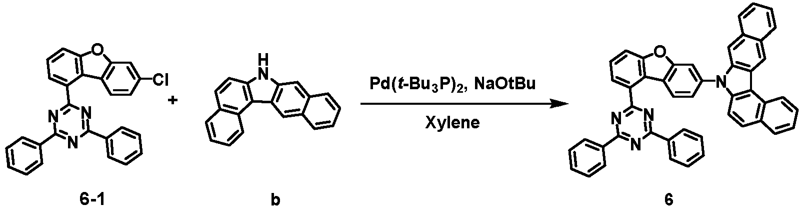

Intermediate 6-1 (20.0 g, 36.4 mmol), Intermediate b (9.7 g, 36.4 mmol) and sodium tert-butoxide (7 g, 72.8 mmol) were added to xylene (400 ml) under a nitrogen atmosphere, and the mixture was stirred and refluxed. Then, bis(tri-tert-butylphosphine)palladium(0) (0.4 g, 0.7 mmol) was added thereto. When the reaction was terminated after 3 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 6. (14.8 g, yield: 61%, MS: [M+H]⁺ = 665)

### Preparation Example 2-7: Preparation of Compound 7

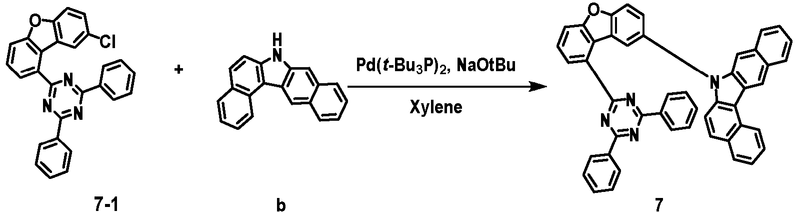

Intermediate 7-1 (20.0 g, 46.2 mmol), Intermediate b (12.3 g, 46.2 mmol) and sodium tert-butoxide (8.9 g, 92.4 mmol) were added to xylene (400 ml) under a nitrogen atmosphere, and the mixture was stirred and refluxed. Then, bis(tri-tert-butylphosphine)palladium(0) (0.5 g, 0.9 mmol) was added thereto. When the reaction was terminated after 2 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 7. (18.4 g, yield: 60%, MS: [M+H]⁺ = 665)

### Preparation Example 2-8: Preparation of Compound 8

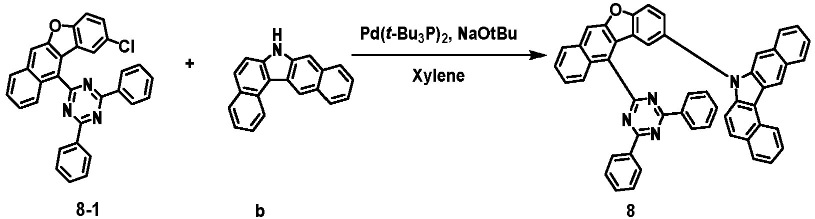

Intermediate 8-1 (20.0 g, 41.4 mmol), Intermediate b (11.1 g, 41.4 mmol) and sodium tert-butoxide (8 g, 82.8 mmol) were added to xylene (400 ml) under a nitrogen atmosphere, and the mixture was stirred and refluxed. Then, bis(tri-tert-butylphosphine)palladium(0) (0.4 g, 0.8 mmol) was added thereto. When the reaction was terminated after 2 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 8. (14.8 g, yield: 50%, MS: [M+H]⁺ = 715)

### Preparation Example 2-9: Preparation of Compound 9

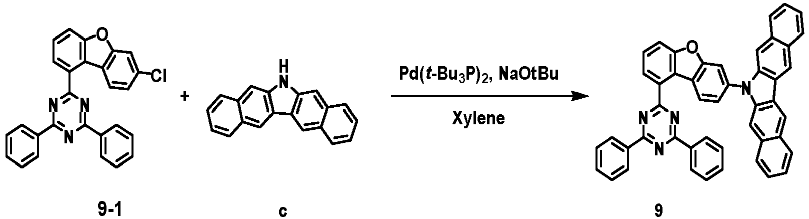

Intermediate 9-1 (20.0 g, 46.2 mmol), Intermediate c (12.3 g, 46.2 mmol) and sodium tert-butoxide (8.9 g, 92.4 mmol) were added to xylene (400 ml) under a nitrogen atmosphere, and the mixture was stirred and refluxed. Then, bis(tri-tert-butylphosphine)palladium(0) (0.5 g, 0.9 mmol) was added thereto. When the reaction was terminated after 2 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 9. (17.8 g, yield: 58%, MS: [M+H]⁺ = 665)

### Preparation Example 2-10: Preparation of Compound 10

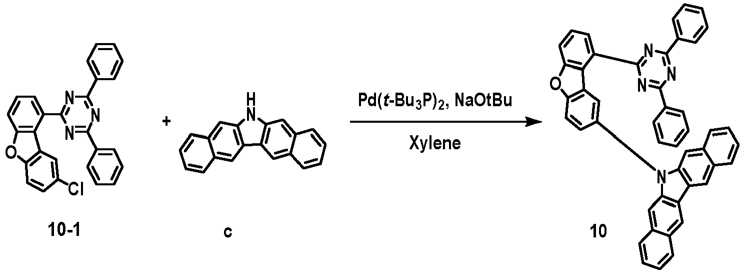

Intermediate 10-1 (20.0 g, 46.2 mmol), Intermediate c (12.3 g, 46.2 mmol) and sodium tert-butoxide (8.9 g, 92.4 mmol) were added to xylene (400 ml) under a nitrogen atmosphere, and the mixture was stirred and refluxed. Then, bis(tri-tert-butylphosphine)palladium(0) (0.5 g, 0.9 mmol) was added thereto. When the reaction was terminated after 2 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 10. (15.3 g, yield: 50%, MS: [M+H]⁺ = 665)

### Preparation Example 2-11: Preparation of Compound 11

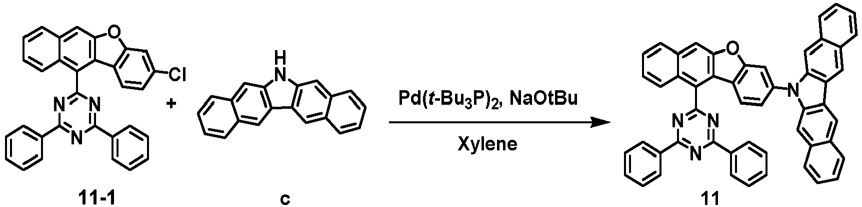

Intermediate 11-1 (20.0 g, 41.4 mmol), Intermediate c (11.1 g, 41.4 mmol) and sodium tert-butoxide (8 g, 82.8 mmol) were added to xylene (400 ml) under a nitrogen atmosphere, and the mixture was stirred and refluxed. Then, bis(tri-tert-butylphosphine)palladium(0) (0.4 g, 0.8 mmol) was added thereto. When the reaction was terminated after 3 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 11. (16.9 g, yield: 57%, MS: [M+H]⁺ = 715)

### Preparation Example 2-12: Preparation of Compound 12

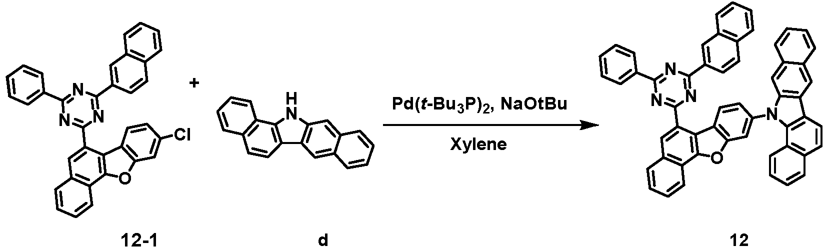

Intermediate 12-1 (20.0 g, 37.5 mmol), Intermediate d (10.0 g, 37.5 mmol) and sodium tert-butoxide (7.2 g, 75 mmol) were added to xylene (400 ml) under a nitrogen atmosphere, and the mixture was stirred and refluxed. Then, bis(tri-tert-butylphosphine)palladium(0) (0.4 g, 0.8 mmol) was added thereto. When the reaction was terminated after 3 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 12. (15.2 g, yield: 53%, MS: [M+H]⁺ = 765)

### Preparation Example 2-13: Preparation of Compound 13

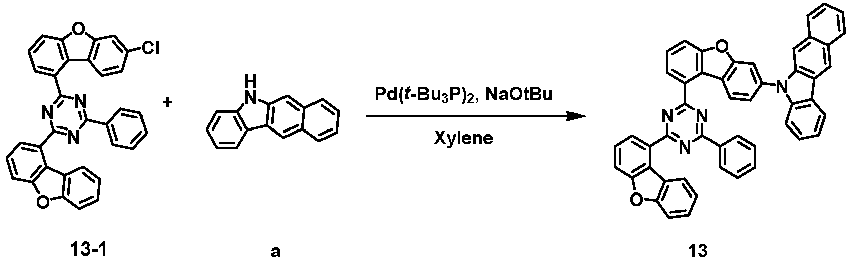

Intermediate 13-1 (20.0 g, 40.9 mmol), Intermediate a (8.9 g, 40.9 mmol) and sodium tert-butoxide (7.9 g, 81.7 mmol) were added to xylene (400 ml) under a nitrogen atmosphere, and the mixture was stirred and refluxed. Then, bis(tri-tert-butylphosphine)palladium(0) (0.4 g, 0.8 mmol) was added thereto. When the reaction was terminated after 3 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 13. (15 g, yield: 52%, MS: [M+H]⁺ = 706)

### Preparation Example 2-14: Preparation of Compound 14, not in accordance with the claimed invention.

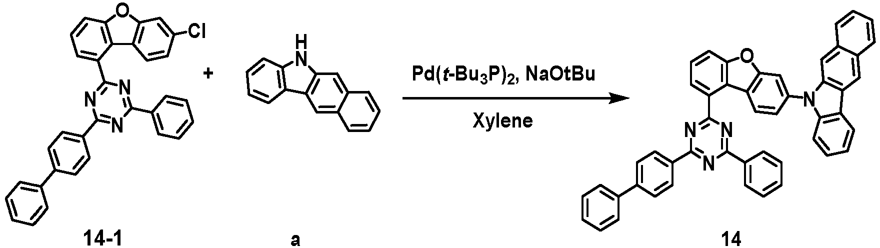

Intermediate 14-1 (20.0 g, 42.1 mmol), Intermediate a (9.1 g, 42.1 mmol) and sodium tert-butoxide (8.1 g, 84.1 mmol) were added to xylene (400 ml) under a nitrogen atmosphere, and the mixture was stirred and refluxed. Then, bis(tri-tert-butylphosphine)palladium(0) (0.4 g, 0.8 mmol) was added thereto. When the reaction was terminated after 2 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 14. (20 g, yield: 69%, MS: [M+H]⁺ = 692)

### Preparation Example 2-15: Preparation of Compound 15

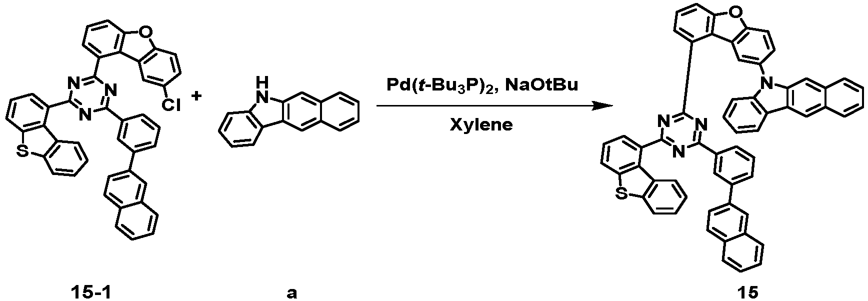

Intermediate 15-1 (20.0 g, 31.7 mmol), Intermediate a (6.9 g, 31.7 mmol) and sodium tert-butoxide (6.1 g, 63.3 mmol) were added to xylene (400 ml) under a nitrogen atmosphere, and the mixture was stirred and refluxed. Then, bis(tri-tert-butylphosphine)palladium(0) (0.3 g, 0.6 mmol) was added thereto. When the reaction was terminated after 3 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 15. (18.5 g, yield: 69%, MS: [M+H]⁺ = 848)

### Preparation Example 2-16: Preparation of Compound 16

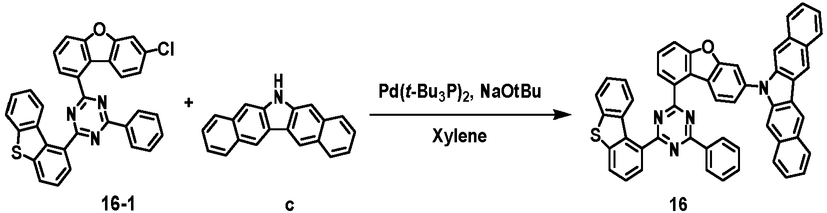

Intermediate 16-1 (20.0 g, 39.6 mmol), Intermediate c (10.6 g, 39.6 mmol) and sodium tert-butoxide (7.6 g, 79.1 mmol) were added to xylene (400 ml) under a nitrogen atmosphere, and the mixture was stirred and refluxed. Then, bis(tri-tert-butylphosphine)palladium(0) (0.4 g, 0.8 mmol) was added thereto. When the reaction was terminated after 2 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 16. (20.4 g, yield: 67%, MS: [M+H]⁺ = 772)

### Preparation Example 2-17: Preparation of Compound 17

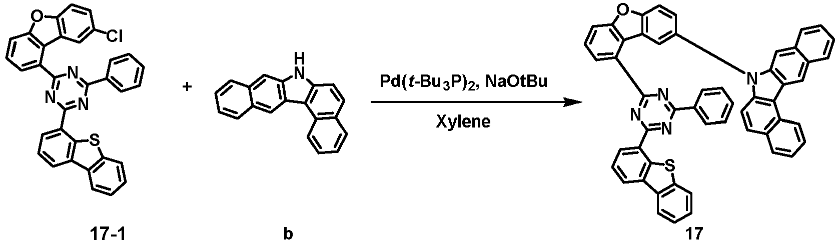

Intermediate 17-1 (20.0 g, 39.6 mmol), Intermediate b (10.6 g, 39.6 mmol) and sodium tert-butoxide (7.6 g, 79.1 mmol) were added to xylene (400 ml) under a nitrogen atmosphere, and the mixture was stirred and refluxed. Then, bis(tri-tert-butylphosphine)palladium(0) (0.4 g, 0.8 mmol) was added thereto. When the reaction was terminated after 3 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 17. (21.3 g, yield: 70%, MS: [M+H]⁺ = 772)

### Preparation Example 2-18: Preparation of Compound 18

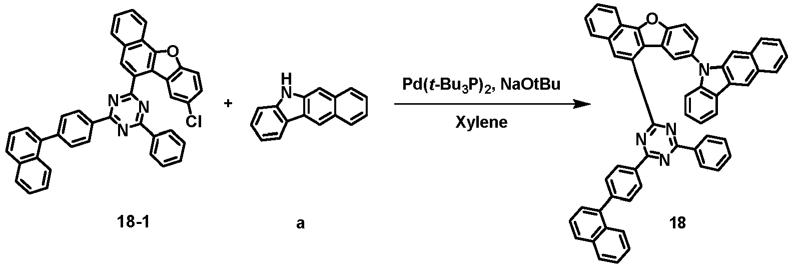

Intermediate 18-1 (20.0 g, 34.7 mmol), Intermediate a (7.5 g, 34.7 mmol) and sodium tert-butoxide (6.7 g, 69.5 mmol) were added to xylene (400 ml) under a nitrogen atmosphere, and the mixture was stirred and refluxed. Then, bis(tri-tert-butylphosphine)palladium(0) (0.4 g, 0.7 mmol) was added thereto. When the reaction was terminated after 2 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 18. (17.6 g, yield: 64%, MS: [M+H]⁺ = 792)

### Preparation Example 2-19: Preparation of Compound 19

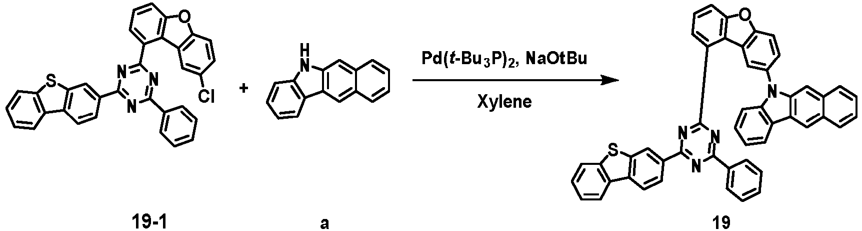

Intermediate 19-1 (20.0 g, 39.6 mmol), Intermediate a (8.6 g, 39.6 mmol) and sodium tert-butoxide (7.6 g, 79.1 mmol) were added to xylene (400 ml) under a nitrogen atmosphere, and the mixture was stirred and refluxed. Then, bis(tri-tert-butylphosphine)palladium(0) (0.4 g, 0.8 mmol) was added thereto. When the reaction was terminated after 2 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 19. (17.4 g, yield: 61%, MS: [M+H]⁺ = 722)

### Preparation Example 2-20: Preparation of Compound 20

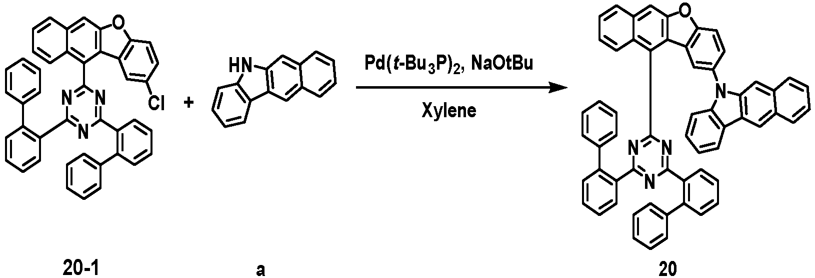

Intermediate 20-1 (20.0 g, 33.2 mmol), Intermediate a (7.2 g, 33.2 mmol) and sodium tert-butoxide (6.4 g, 66.5 mmol) were added to xylene (400 ml) under a nitrogen atmosphere, and the mixture was stirred and refluxed. Then, bis(tri-tert-butylphosphine)palladium(0) (0.3 g, 0.7 mmol) was added thereto. When the reaction was terminated after 3 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 20. (18.2 g, yield: 67%, MS: [M+H]⁺ = 818)

### Preparation Example 2-21: Preparation of Compound 21

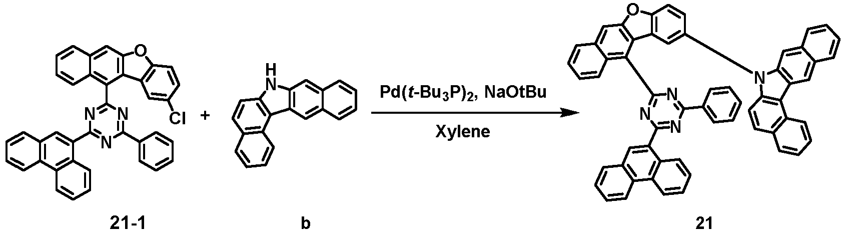

Intermediate 21-1 (20.0 g, 36.4 mmol), Intermediate b (9.7 g, 36.4 mmol) and sodium tert-butoxide (7 g, 72.8 mmol) were added to xylene (400 ml) under a nitrogen atmosphere, and the mixture was stirred and refluxed. Then, bis(tri-tert-butylphosphine)palladium(0) (0.4 g, 0.7 mmol) was added thereto. When the reaction was terminated after 3 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 21. (18.1 g, yield: 61%, MS: [M+H]⁺ = 816)

### Preparation Example 2-22: Preparation of Compound 22

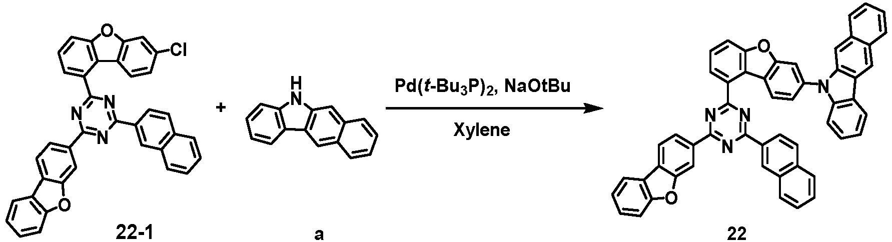

Intermediate 22-1 (20.0 g, 37.1 mmol), Intermediate a (8.1 g, 37.1 mmol) and sodium tert-butoxide (7.1 g, 74.1 mmol) were added to xylene (400 ml) under a nitrogen atmosphere, and the mixture was stirred and refluxed. Then, bis(tri-tert-butylphosphine)palladium(0) (0.4 g, 0.7 mmol) was added thereto. When the reaction was terminated after 2 hours, the reaction mixture was cooled to room temperature and the solvent was removed under reduced pressure. Then, the compound was completely dissolved again in chloroform, washed twice with water, and then the organic layer was separated, treated with anhydrous magnesium sulfate, then filtered, and the filtrate was distilled under reduced pressure. The concentrated compound was purified by silica gel column chromatography to give Compound 22. (19 g, yield: 68%, MS: [M+H]⁺ = 756)

### Preparation Example 3-1: Preparation of Compound 2-1

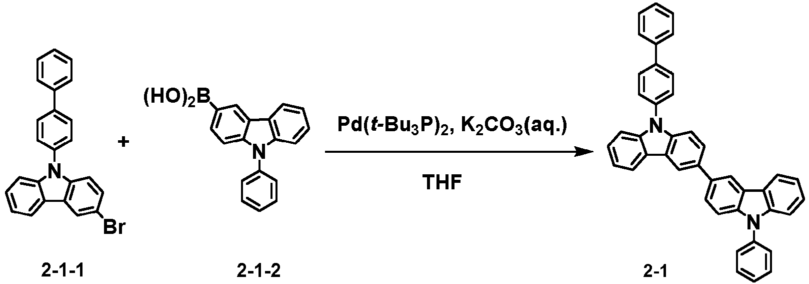

Intermediate 2-1-1 (10.0 g, 25.2 mmol) and Intermediate 2-1-2 (8 g, 27.7 mmol) were added to tetrahydrofuran (THF, 200 ml) under a nitrogen atmosphere, stirred, and potassium carbonate (13.9 g, 100.7 mmol) was dissolved in water and added thereto. The mixture was sufficiently stirred and refluxed, and then bis(tri-tert-butylphosphine)palladium(0) (0.1 g, 0.3 mmol) was added. After the reaction for 3 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-1. (9 g, yield: 64%, MS: [M+H]⁺ = 561)

### Preparation Example 3-2: Preparation of Compound 2-2

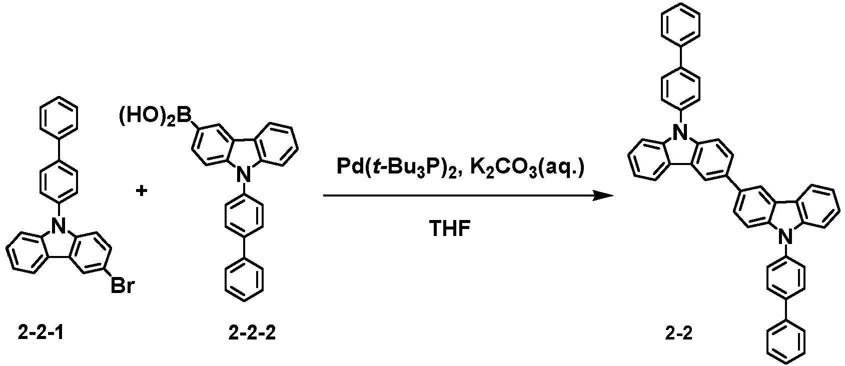

Intermediate 2-2-1 (10.0 g, 25.2 mmol) and Intermediate 2-2-2 (8 g, 27.7 mmol) were added to THF (200 ml) under a nitrogen atmosphere, stirred, and potassium carbonate (13.9 g, 100.7 mmol) was dissolved in water and added thereto. The mixture was sufficiently stirred and refluxed, and then bis(tri-tert-butylphosphine)palladium(0) (0.1 g, 0.3 mmol) was added. After the reaction for 4 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-2. (10.6 g, yield: 66%, MS: [M+H]⁺ = 637)

### Preparation Example 3-3: Preparation of Compound 2-3

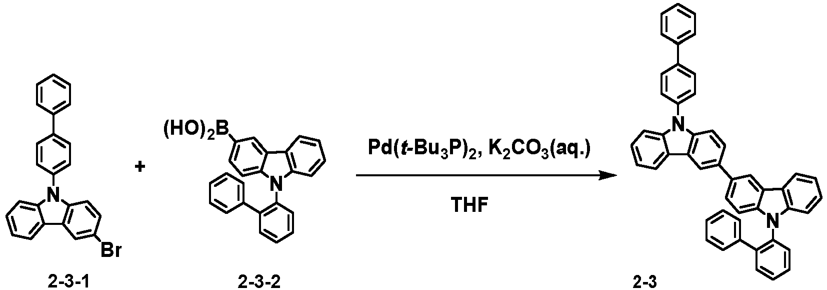

Intermediate 2-3-1 (10.0 g, 25.2 mmol) and Intermediate 2-3-2 (10.1 g, 27.7 mmol) were added to **THF** (200 ml) under a nitrogen atmosphere, stirred, and potassium carbonate (13.9 g, 100.7 mmol) was dissolved in water and added thereto. The mixture was sufficiently stirred and refluxed, and then bis(tri-tert-butylphosphine)palladium(0) (0.1 g, 0.3 mmol) was added. After the reaction for 4 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-3. (9 g, yield: 56%, MS: [M+H]⁺ = 637)

### Preparation Example 3-4: Preparation of Compound 2-4

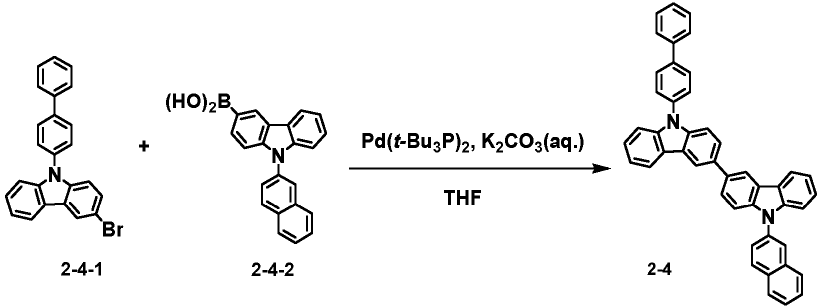

Intermediate 2-4-1 (10.0 g, 25.2 mmol) and Intermediate 2-4-2 (9.3 g, 27.7 mmol) were added to THF (200 ml) under a nitrogen atmosphere, stirred, and potassium carbonate (13.9 g, 100.7 mmol) was dissolved in water and added thereto. The mixture was sufficiently stirred and refluxed, and then bis(tri-tert-butylphosphine)palladium(0) (0.1 g, 0.3 mmol) was added. After the reaction for 2 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-4. (7.8 g, yield: 51%, MS: [M+H]⁺ = 611)

### Preparation Example 3-5: Preparation of Compound 2-5

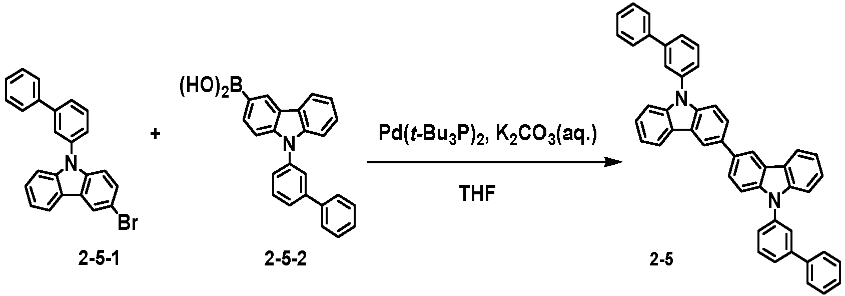

Intermediate 2-5-1 (10.0 g, 25.2 mmol) and Intermediate 2-5-2 (10.1 g, 27.7 mmol) were added to THF (200 ml) under a nitrogen atmosphere, stirred, and potassium carbonate (13.9 g, 100.7 mmol) was dissolved in water and added thereto. The mixture was sufficiently stirred and refluxed, and then bis(tri-tert-butylphosphine)palladium(0) (0.1 g, 0.3 mmol) was added. After the reaction for 4 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-5. (10.4 g, yield: 65%, MS: [M+H]⁺ = 637)

### Preparation Example 3-6: Preparation of Compound 2-6

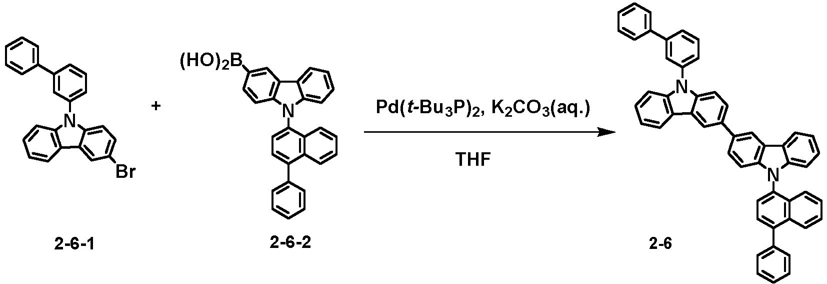

Intermediate 2-6-1 (10.0 g, 25.2 mmol) and Intermediate 2-6-2 (11.4 g, 27.7 mmol) were added to THF (200 ml) under a nitrogen atmosphere, stirred, and potassium carbonate (13.9 g, 100.7 mmol) was dissolved in water and added thereto. The mixture was sufficiently stirred and refluxed, and then bis(tri-tert-butylphosphine)palladium(0) (0.1 g, 0.3 mmol) was added. After the reaction for 2 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-6. (10.5 g, yield: 61%, MS: [M+H]⁺ = 687)

### Preparation Example 3-7: Preparation of Compound 2-7

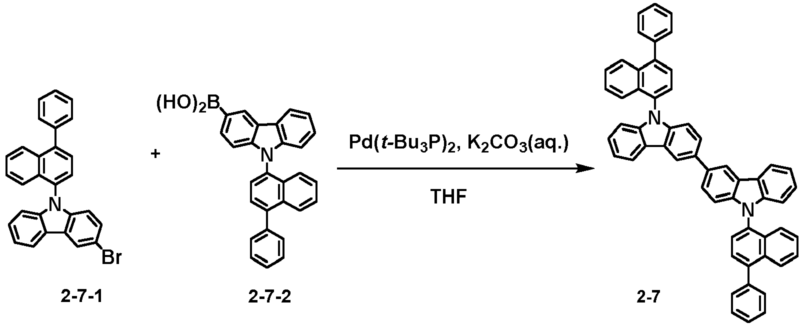

Intermediate 2-7-1 (10.0 g, 22.4 mmol) and Intermediate 2-7-2 (10.2 g, 24.6 mmol) were added to THF (200 ml) under a nitrogen atmosphere, stirred, and potassium carbonate (12.4 g, 89.5 mmol) was dissolved in water and added thereto. The mixture was sufficiently stirred and refluxed, and then bis(tri-tert-butylphosphine)palladium(0) (0.1 g, 0.2 mmol) was added. After the reaction for 3 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-7. (11 g, yield: 67%, MS: [M+H]⁺ = 737)

### Preparation Example 3-8: Preparation of Compound 2-8

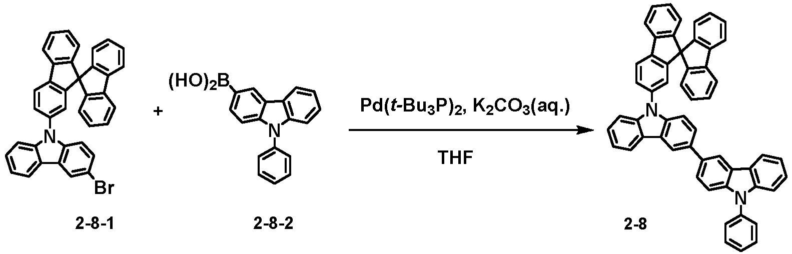

Intermediate 2-8-1 (10.0 g, 17.9 mmol) and Intermediate 2-8-2 (5.6 g, 19.7 mmol) were added to THF (200 ml) under a nitrogen atmosphere, stirred, and potassium carbonate (9.9 g, 71.5 mmol) was dissolved in water and added thereto. The mixture was sufficiently stirred and refluxed, and then bis(tri-tert-butylphosphine)palladium(0) (0.1 g, 0.2 mmol) was added. After the reaction for 3 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-8. (7.8 g, yield: 60%, MS: [M+H]⁺ = 723)

### Preparation Example 3-9: Preparation of Compound 2-9

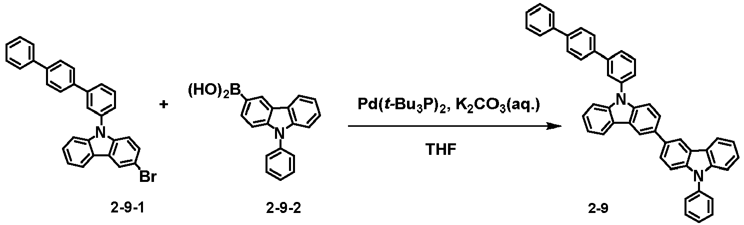

Intermediate 2-9-1 (10.0 g, 21.1 mmol) and Intermediate 2-9-2 (6.7 g, 23.3 mmol) were added to THF (200 ml) under a nitrogen atmosphere, stirred, and potassium carbonate (11.7 g, 84.6 mmol) was dissolved in water and added thereto. The mixture was sufficiently stirred and refluxed, and then bis(tri-tert-butylphosphine)palladium(0) (0.1 g, 0.2 mmol) was added. After the reaction for 3 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-9. (7.4 g, yield: 55%, MS: [M+H]⁺ = 637)

### Preparation Example 3-10: Preparation of Compound 2-10

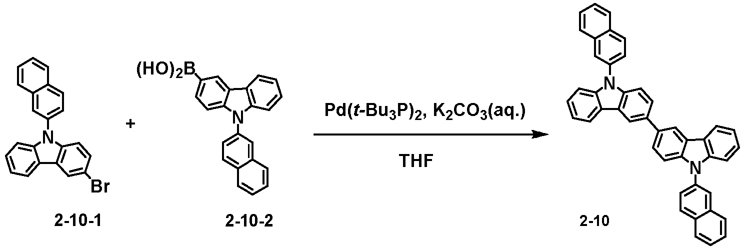

Intermediate 2-10-1 (10.0 g, 27 mmol) and Intermediate 2-10-2 (10.0 g, 29.6 mmol) were added to THF (200 ml) under a nitrogen atmosphere, stirred, and potassium carbonate (14.9 g, 107.8 mmol) was dissolved in water and added thereto. The mixture was sufficiently stirred and refluxed, and then bis(tri-tert-butylphosphine)palladium(0) (0.1 g, 0.3 mmol) was added. After the reaction for 2 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-10. (11 g, yield: 70%, MS: [M+H]⁺ = 585)

### Preparation Example 3-11: Preparation of Compound 2-11

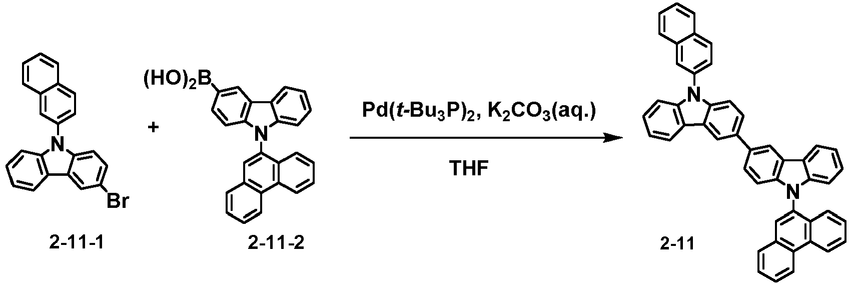

Intermediate 2-11-1 (10.0 g, 27 mmol) and Intermediate 2-11-2 (11.5 g, 29.6 mmol) were added to THF (200 ml) under a nitrogen atmosphere, stirred, and potassium carbonate (14.9 g, 107.8 mmol) was dissolved in water and added thereto. The mixture was sufficiently stirred and refluxed, and then bis(tri-tert-butylphosphine)palladium(0) (0.1 g, 0.3 mmol) was added. After the reaction for 2 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-11. (11.5 g, yield: 67%, MS: [M+H]⁺ = 635)

### Preparation Example 3-12: Preparation of Compound 2-12

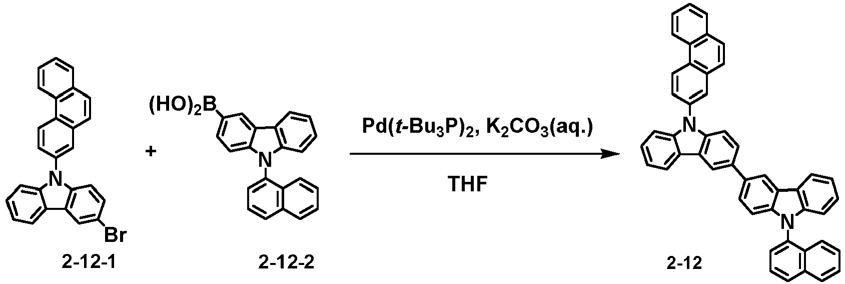

Intermediate 2-12-1 (10.0 g, 23.8 mmol) and Intermediate 2-12-2 (8.8 g, 26.1 mmol) were added to THF (200 ml) under a nitrogen atmosphere, stirred, and potassium carbonate (13.1 g, 95 mmol) was dissolved in water and added thereto. The mixture was sufficiently stirred and refluxed, and then bis(tri-tert-butylphosphine)palladium(0) (0.1 g, 0.2 mmol) was added. After the reaction for 3 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-12. (10.4 g, yield: 69%, MS: [M+H]⁺ = 635)

### Preparation Example 3-13: Preparation of Compound 2-13

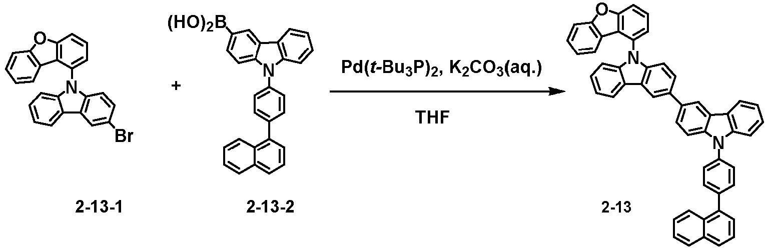

Intermediate 2-13-1 (10.0 g, 24.3 mmol) and Intermediate 2-13-2 (11.1 g, 26.8 mmol) were added to THF (200 ml) under a nitrogen atmosphere, stirred, and potassium carbonate (13.5 g, 97.3 mmol) was dissolved in water and added thereto. The mixture was sufficiently stirred and refluxed, and then bis(tri-tert-butylphosphine)palladium(0) (0.1 g, 0.2 mmol) was added. After the reaction for 2 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-13. (9 g, yield: 53%, MS: [M+H]⁺ = 701)

### Preparation Example 3-14: Preparation of Compound 2-14

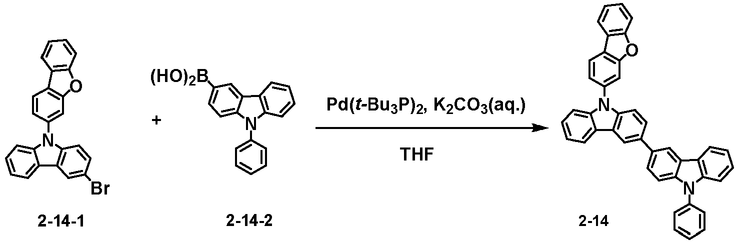

Intermediate 2-14-1 (10.0 g, 24.3 mmol) and Intermediate 2-14-2 (7.7 g, 26.8 mmol) were added to THF (200 ml) under a nitrogen atmosphere, stirred, and potassium carbonate (13.5 g, 97.3 mmol) was dissolved in water and added thereto. The mixture was sufficiently stirred and refluxed, and then bis(tri-tert-butylphosphine)palladium(0) (0.1 g, 0.2 mmol) was added. After the reaction for 3 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-14. (8.9 g, yield: 64%, MS: [M+H]⁺ = 575)

### Preparation Example 3-15: Preparation of Compound 2-15

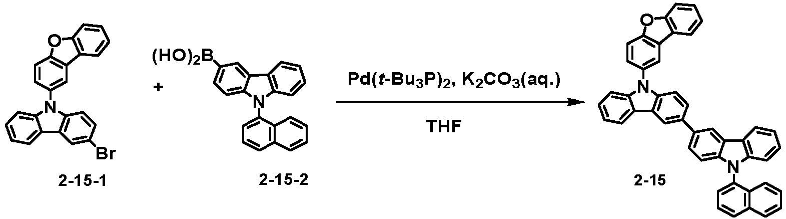

Intermediate 2-15-1 (10.0 g, 24.3 mmol) and Intermediate 2-15-2 (9 g, 26.8 mmol) were added to THF (200 ml) under a nitrogen atmosphere, stirred, and potassium carbonate (13.5 g, 97.3 mmol) was dissolved in water and added thereto. The mixture was sufficiently stirred and refluxed, and then bis(tri-tert-butylphosphine)palladium(0) (0.1 g, 0.2 mmol) was added. After the reaction for 4 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-15. (8.4 g, yield: 55%, MS: [M+H]⁺ = 625)

### Preparation Example 3-16: Preparation of Compound 2-16

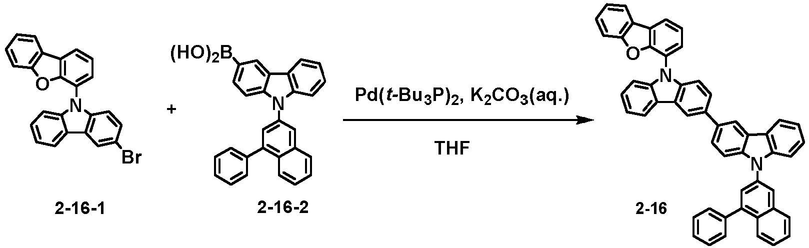

Intermediate 2-16-1 (10.0 g, 24.3 mmol) and Intermediate 2-16-2 (11.1 g, 26.8 mmol) were added to THF (200 ml) under a nitrogen atmosphere, stirred, and potassium carbonate (13.5 g, 97.3 mmol) was dissolved in water and added thereto. The mixture was sufficiently stirred and refluxed, and then bis(tri-tert-butylphosphine)palladium(0) (0.1 g, 0.2 mmol) was added. After the reaction for 2 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-16. (11.2 g, yield: 66%, MS: [M+H]⁺ = 701)

### Preparation Example 3-17: Preparation of Compound 2-17

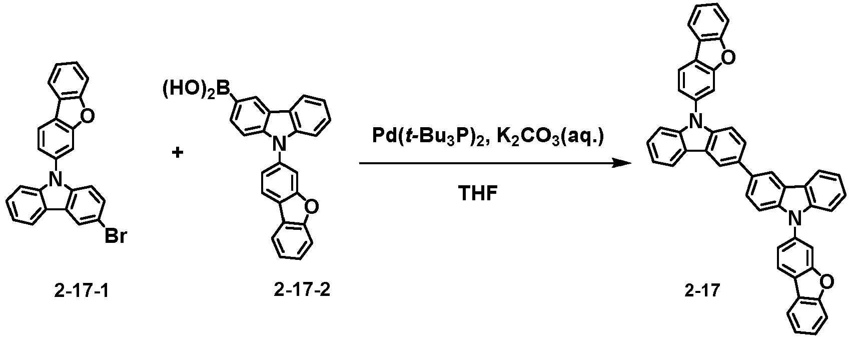

Intermediate 2-17-1 (10.0 g, 24.3 mmol) and Intermediate 2-17-2 (10.1 g, 26.8 mmol) were added to THF (200 ml) under a nitrogen atmosphere, stirred, and potassium carbonate (13.5 g, 97.3 mmol) was dissolved in water and added thereto. The mixture was sufficiently stirred and refluxed, and then bis(tri-tert-butylphosphine)palladium(0) (0.1 g, 0.2 mmol) was added. After the reaction for 3 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-17. (10.0 g, yield: 62%, MS: [M+H]⁺ = 665)

### Preparation Example 3-18: Preparation of Compound 2-18

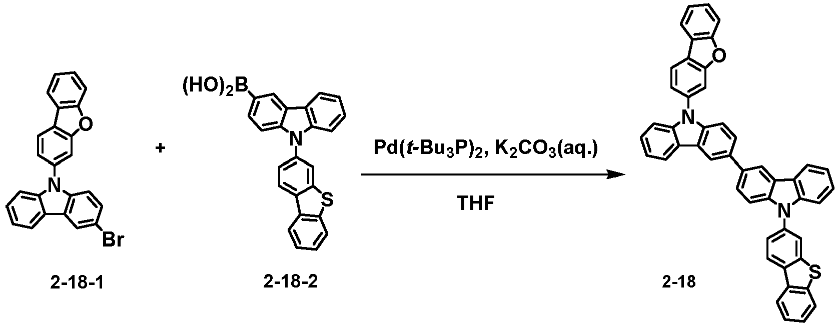

Intermediate 2-18-1 (10.0 g, 24.3 mmol) and Intermediate 2-18-2 (10.5 g, 26.8 mmol) were added to THF (200 ml) under a nitrogen atmosphere, stirred, and potassium carbonate (13.5 g, 97.3 mmol) was dissolved in water and added thereto. The mixture was sufficiently stirred and refluxed, and then bis(tri-tert-butylphosphine)palladium(0) (0.1 g, 0.2 mmol) was added. After the reaction for 2 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-18. (9.8 g, yield: 59%, MS: [M+H]⁺ = 681)

### Preparation Example 3-19: Preparation of Compound 2-19

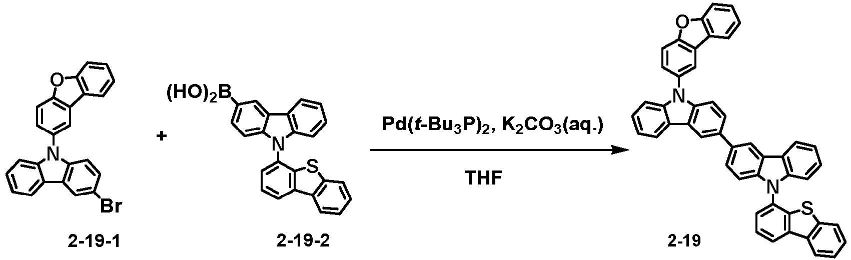

Intermediate 2-19-1 (10.0 g, 24.3 mmol) and Intermediate 2-19-2 (10.5 g, 26.8 mmol) were added to THF (200 ml) under a nitrogen atmosphere, stirred, and potassium carbonate (13.5 g, 97.3 mmol) was dissolved in water and added thereto. The mixture was sufficiently stirred and refluxed, and then bis(tri-tert-butylphosphine)palladium(0) (0.1 g, 0.2 mmol) was added. After the reaction for 3 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-19. (11.4 g, yield: 69%, MS: [M+H]⁺ = 681)

### Preparation Example 3-20: Preparation of Compound 2-20

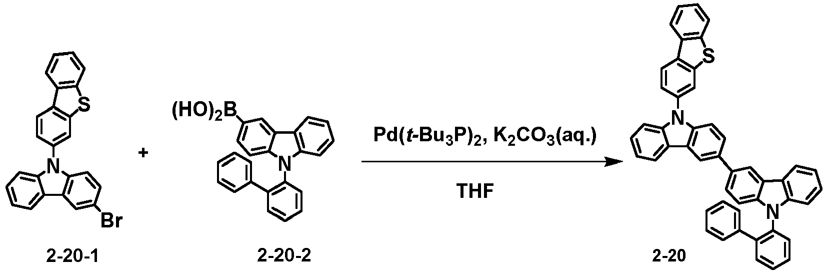

Intermediate 2-20-1 (10.0 g, 23.4 mmol) and Intermediate 2-20-2 (9.4 g, 25.8 mmol) were added to THF (200 ml) under a nitrogen atmosphere, stirred, and potassium carbonate (12.9 g, 93.7 mmol) was dissolved in water and added thereto. The mixture was sufficiently stirred and refluxed, and then bis(tri-tert-butylphosphine)palladium(0) (0.1 g, 0.2 mmol) was added. After the reaction for 2 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-20. (9 g, yield: 58%, MS: [M+H]⁺ = 667)

### Preparation Example 3-21: Preparation of Compound 2-21

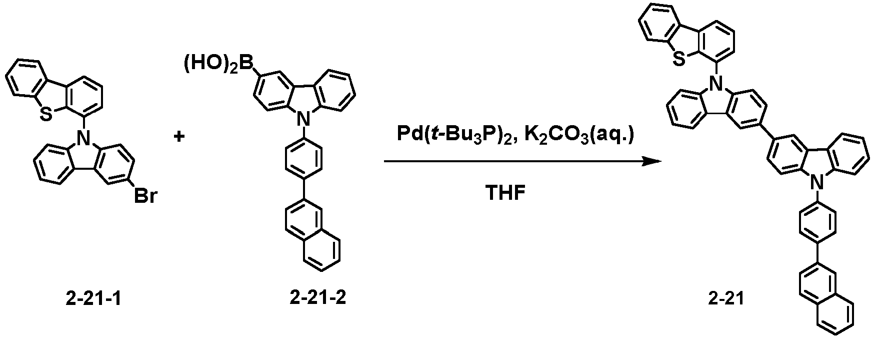

Intermediate 2-21-1 (10.0 g, 23.4 mmol) and Intermediate 2-21-2 (10.6 g, 25.8 mmol) were added to THF (200 ml) under a nitrogen atmosphere, stirred, and potassium carbonate (12.9 g, 93.7 mmol) was dissolved in water and added thereto. The mixture was sufficiently stirred and refluxed, and then bis(tri-tert-butylphosphine)palladium(0) (0.1 g, 0.2 mmol) was added. After the reaction for 4 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-21. (10.7 g, yield: 64%, MS: [M+H]⁺ = 717)

### Preparation Example 3-22: Preparation of Compound 2-22

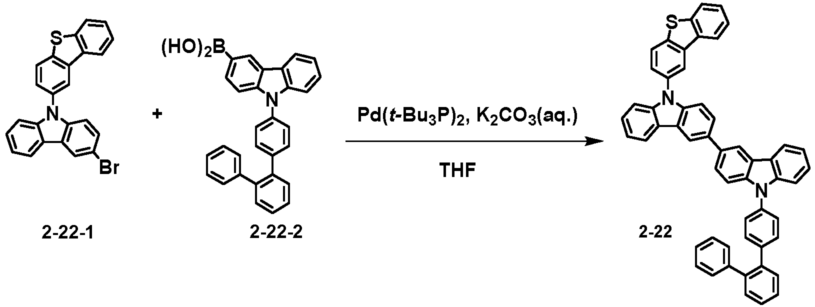

Intermediate 2-22-1 (10.0 g, 23.4 mmol) and Intermediate 2-22-2 (11.3 g, 25.8 mmol) were added to THF (200 ml) under a nitrogen atmosphere, stirred, and potassium carbonate (12.9 g, 93.7 mmol) was dissolved in water and added thereto. The mixture was sufficiently stirred and refluxed, and then bis(tri-tert-butylphosphine)palladium(0) (0.1 g, 0.2 mmol) was added. After the reaction for 3 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-22. (9 g, yield: 52%, MS: [M+H]⁺ = 743)

### Preparation Example 3-23: Preparation of Compound 2-23

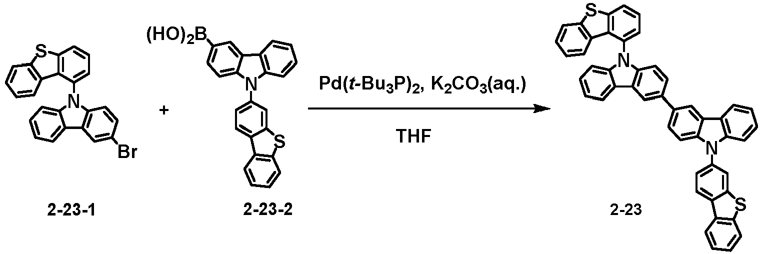

Intermediate 2-23-1 (10.0 g, 23.4 mmol) and Intermediate 2-23-2 (10.1 g, 25.8 mmol) were added to THF (200 ml) under a nitrogen atmosphere, stirred, and potassium carbonate (12.9 g, 93.7 mmol) was dissolved in water and added thereto. The mixture was sufficiently stirred and refluxed, and then bis(tri-tert-butylphosphine)palladium(0) (0.1 g, 0.2 mmol) was added. After the reaction for 4 hours, the reaction mixture was cooled to room temperature, the organic layer and the aqueous layer were separated and then the organic layer was distilled. This was again dissolved in chloroform and washed twice with water. The organic layer was then separated, anhydrous magnesium sulfate was added, stirred and then filtered. The filtrate was distilled under reduced pressure. The concentrated compound was purified by silica gel column chromatography to give Compound 2-23. (9.1 g, yield: 56%, MS: [M+H]⁺ = 697)

### Example 1

A glass substrate on which ITO (indium tin oxide) was coated as a thin film to a thickness of 1,000 Å was put into distilled water in which a detergent was dissolved, and ultrasonically cleaned. At this time, a product manufactured by Fischer Co. was used as the detergent, and as the distilled water, distilled water filtered twice using a filter manufactured by Millipore Co. was used. After the ITO was cleaned for 30 minutes, ultrasonic cleaning was repeated twice using distilled water for 10 minutes. After the cleaning with distilled water was completed, the substrate was ultrasonically cleaned with solvents of isopropyl alcohol, acetone, and methanol, dried, and then transferred to a plasma cleaner. In addition, the substrate was cleaned for 5 minutes using oxygen plasma and then transferred to a vacuum depositor.

A compound HI-1 below was formed in a thickness of 1150 Å on the ITO transparent electrode prepared above as a hole injection layer, wherein a compound A-1 below was p-doped at a concentration of 1.5%. A compound HT-1 below was vacuum-deposited on the hole injection layer to form a hole transport layer with a film thickness of 800 Å. Then, a compound EB-1 below was vacuum-deposited in a thickness of 150 Å on the hole transport layer to form an electron inhibition layer. Then, the compound 1 prepared in Preparation Example 2-1 and the compound 2-1 prepared in Preparation Example 3-1 as host materials were co-deposited at a weight ratio of 1: 1 on the EB-1 deposited film, and a dopant Dp-7 compound below was vacuum-deposited at a weight ratio of 98: 2 (host: dopant) to form a red light emitting layer with a thickness of 400 Å. A compound HB-1 below was vacuum-deposited in a film thickness of 30Å on the light emitting layer to form a hole blocking layer. Then, a compound ET-1 below and a compound LiQ below were vacuum deposited at a weight ratio of 2: 1 on the hole blocking layer to form an electron injection and transport layer with a thickness of 300 Å. Lithium fluoride (LiF) and aluminum were sequentially deposited to have a thickness of 12 Å and 1,000 Å, respectively, on the electron injection and transport layer, thereby forming a cathode.

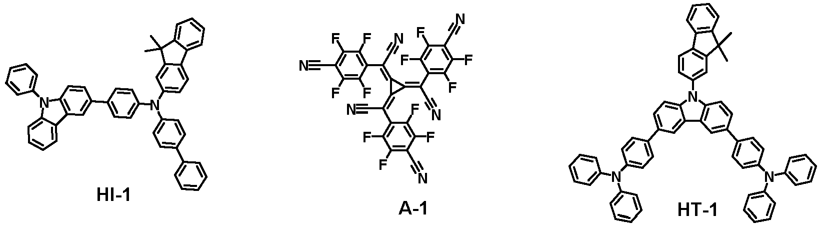

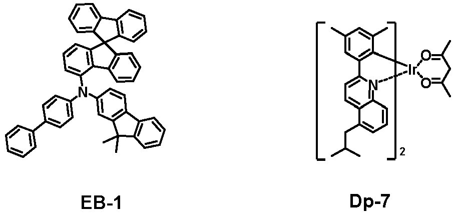

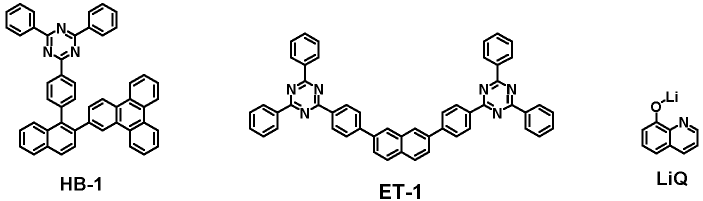

In the above-mentioned process, the vapor deposition rate of the organic material was maintained at 0.4 to 0.7 Å/sec, the deposition rate of lithium fluoride of the cathode was maintained at 0.3 Å/sec, the deposition rate of aluminum was maintained at 2 Å/sec, and the degree of vacuum during the deposition was maintained at 2x10⁻⁷ to 5x10⁻⁶ torr, thereby manufacturing the organic light emitting device.

### Examples 2 to 88 and Comparative Examples 1 to 56

An organic light emitting device was manufactured in the same manner as in Example 1, except that the first host and the second host described in Tables 1 to 5 respectively below were co-deposited in a ratio of 1:1 instead of Compound 1 and Compound 2-1 used in the organic light emitting device of Example 1.

At this time, the Compounds C-1 to C-14 used in Comparative Examples 1 to 56 are as follows.

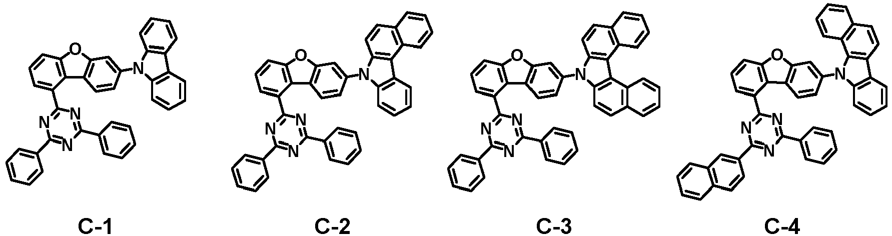

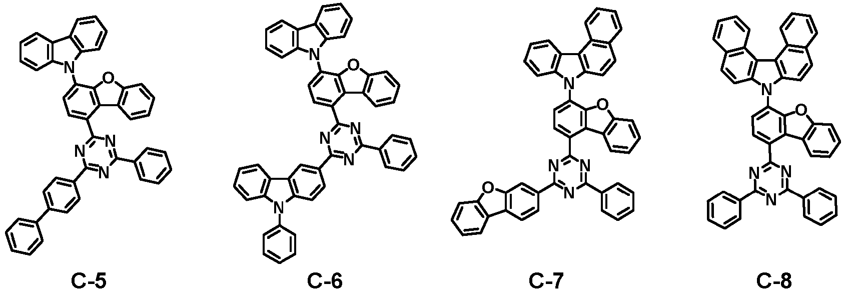

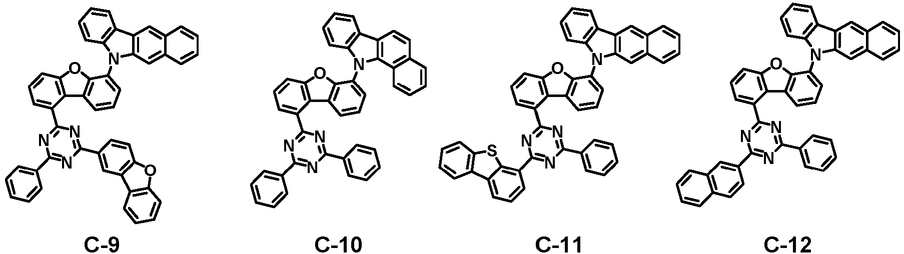

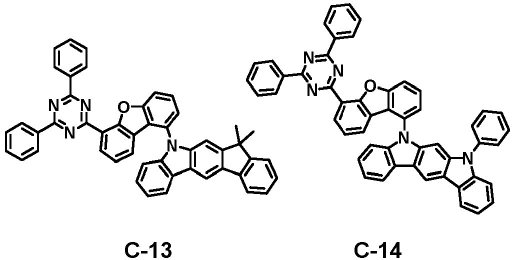

For the organic light emitting devices manufactured in the Examples and Comparative Examples, the voltage and efficiency were measured at a current density of 10 mA/cm², and the lifetime was measured at a current density of 50 ma/cm². The results are shown in Tables 1 to 5 below. At this time, T₉₅ means the time (hr) required for the luminance to be reduced to 95% of the initial luminance.

The following compounds in the examples in Table 1 do not form part of the present invention: compound 1, compound 2 and compound 14.

**[Table 1]**

| Category | First host | Second host | Driving voltage (V) | Efficiency (cd/A) | Lifetime T₉₅ (hr) | Light emitting color |
|---|---|---|---|---|---|---|
| Example 1 | Compound 1 | Compound 2-1 | 3.86 | 21.5 | 208 | Red |
| Example 2 | Compound 1 | Compound 2-2 | 3.88 | 21.2 | 213 | Red |
| Example 3 | Compound 1 | Compound 2-8 | 3.87 | 21.1 | 197 | Red |
| Example 4 | Compound 1 | Compound 2-19 | 3.86 | 21.4 | 213 | Red |
| Example 5 | Compound 2 | Compound 2-1 | 3.88 | 20.4 | 217 | Red |
| Example 6 | Compound 2 | Compound 2-2 | 3.81 | 21.3 | 214 | Red |
| Example 7 | Compound 2 | Compound 2-8 | 3.80 | 20.7 | 201 | Red |
| Example 8 | Compound 2 | Compound 2-19 | 3.82 | 20.5 | 210 | Red |
| Example 9 | Compound 3 | Compound 2-1 | 3.58 | 22.4 | 238 | Red |
| Example 10 | Compound 3 | Compound 2-2 | 3.52 | 22.8 | 242 | Red |
| Example 11 | Compound 3 | Compound 2-8 | 3.54 | 22.9 | 237 | Red |
| Example 12 | Compound 3 | Compound 2-19 | 3.56 | 23.4 | 251 | Red |
| Example 13 | Compound 4 | Compound 2-1 | 3.55 | 22.1 | 233 | Red |
| Example 14 | Compound 4 | Compound 2-2 | 3.50 | 23.3 | 239 | Red |
| Example 15 | Compound 4 | Compound 2-8 | 3.54 | 22.4 | 247 | Red |
| Example 16 | Compound 4 | Compound 2-19 | 3.53 | 22.5 | 231 | Red |
| Example 17 | Compound 5 | Compound 2-1 | 3.55 | 24.1 | 233 | Red |
| Example 18 | Compound 5 | Compound 2-2 | 3.51 | 24.3 | 247 | Red |
| Example 19 | Compound 5 | Compound 2-8 | 3.50 | 23.8 | 252 | Red |
| Example 20 | Compound 5 | Compound 2-19 | 3.58 | 23.6 | 243 | Red |
| Example 21 | Compound 6 | Compound 2-1 | 3.54 | 21.1 | 246 | Red |
| Example 22 | Compound 6 | Compound 2-2 | 3.52 | 21.5 | 243 | Red |
| Example 23 | Compound 6 | Compound 2-8 | 3.51 | 20.2 | 247 | Red |
| Example 24 | Compound 6 | Compound 2-19 | 3.54 | 21.1 | 240 | Red |
| Example 25 | Compound 7 | Compound 2-1 | 3.69 | 20.3 | 221 | Red |
| Example 26 | Compound 7 | Compound 2-2 | 3.62 | 21.2 | 223 | Red |
| Example 27 | Compound 7 | Compound 2-8 | 3.61 | 21.7 | 213 | Red |
| Example 28 | Compound 7 | Compound 2-19 | 3.65 | 20.8 | 224 | Red |

**[Table 2]**

| Category | First host | Second host | Driving voltage (V) | Efficiency (cd/A) | Lifetime T₉₅ (hr) | Light emitting color |
|---|---|---|---|---|---|---|
| Example 29 | Compound 8 | Compound 2-5 | 3.52 | 22.9 | 241 | Red |
| Example 30 | Compound 8 | Compound 2-6 | 3.55 | 23.1 | 256 | Red |
| Example 31 | Compound 8 | Compound 2-13 | 3.52 | 22.9 | 240 | Red |
| Example 32 | Compound 8 | Compound 2-20 | 3.56 | 23.4 | 259 | Red |
| Example 33 | Compound 9 | Compound 2-5 | 3.60 | 22.7 | 217 | Red |
| Example 34 | Compound 9 | Compound 2-6 | 3.61 | 23.5 | 219 | Red |
| Example 35 | Compound 9 | Compound 2-13 | 3.64 | 22.3 | 211 | Red |
| Example 36 | Compound 9 | Compound 2-20 | 3.63 | 21.7 | 208 | Red |
| Example 37 | Compound 10 | Compound 2-5 | 3.64 | 21.1 | 211 | Red |
| Example 38 | Compound 10 | Compound 2-6 | 3.61 | 20.8 | 203 | Red |
| Example 39 | Compound 10 | Compound 2-13 | 3.67 | 20.7 | 198 | Red |
| Example 40 | Compound 10 | Compound 2-20 | 3.60 | 21.3 | 204 | Red |
| Example 41 | Compound 11 | Compound 2-5 | 3.52 | 22.9 | 251 | Red |
| Example 42 | Compound 11 | Compound 2-6 | 3.54 | 23.3 | 257 | Red |
| Example 43 | Compound 11 | Compound 2-13 | 3.50 | 22.9 | 246 | Red |
| Example 44 | Compound 11 | Compound 2-20 | 3.57 | 22.4 | 260 | Red |
| Example 45 | Compound 12 | Compound 2-5 | 3.56 | 23.1 | 238 | Red |
| Example 46 | Compound 12 | Compound 2-6 | 3.54 | 23.3 | 221 | Red |
| Example 47 | Compound 12 | Compound 2-13 | 3.60 | 22.9 | 229 | Red |
| Example 48 | Compound 12 | Compound 2-20 | 3.55 | 22.4 | 231 | Red |
| Example 49 | Compound 13 | Compound 2-5 | 3.81 | 21.3 | 191 | Red |
| Example 50 | Compound 13 | Compound 2-6 | 3.80 | 20.8 | 194 | Red |
| Example 51 | Compound 13 | Compound 2-13 | 3.84 | 21.0 | 190 | Red |
| Example 52 | Compound 13 | Compound 2-20 | 3.83 | 21.5 | 198 | Red |
| Example 53 | Compound 14 | Compound 2-5 | 3.86 | 20.9 | 194 | Red |
| Example 54 | Compound 14 | Compound 2-6 | 3.88 | 21.1 | 196 | Red |
| Example 55 | Compound 14 | Compound 2-13 | 3.86 | 20.7 | 190 | Red |
| Example 56 | Compound 14 | Compound 2-20 | 3.85 | 20.9 | 195 | Red |

**[Table 3]**

| Category | First host | Second host | Driving voltage (V) | Efficiency (cd/A) | Lifetime T₉₅ (hr) | Light emitting color |
|---|---|---|---|---|---|---|
| Example 57 | Compound 15 | Compound 2-5 | 3.91 | 20.0 | 198 | Red |
| Example 58 | Compound 15 | Compound 2-6 | 3.90 | 20.5 | 197 | Red |
| Example 59 | Compound 15 | Compound 2-13 | 3.88 | 20.3 | 203 | Red |
| Example 60 | Compound 15 | Compound 2-20 | 3.90 | 20.7 | 195 | Red |
| Example 61 | Compound 16 | Compound 2-3 | 3.60 | 21.8 | 200 | Red |
| Example 62 | Compound 16 | Compound 2-4 | 3.61 | 21.1 | 191 | Red |
| Example 63 | Compound 16 | Compound 2-14 | 3.64 | 21.4 | 198 | Red |
| Example 64 | Compound 16 | Compound 2-21 | 3.62 | 21.2 | 205 | Red |
| Example 65 | Compound 17 | Compound 2-3 | 3.67 | 21.8 | 208 | Red |
| Example 66 | Compound 17 | Compound 2-4 | 3.68 | 21.1 | 203 | Red |
| Example 67 | Compound 17 | Compound 2-14 | 3.71 | 21.4 | 198 | Red |
| Example 68 | Compound 17 | Compound 2-21 | 3.69 | 21.2 | 210 | Red |
| Example 69 | Compound 18 | Compound 2-3 | 3.59 | 22.3 | 234 | Red |
| Example 70 | Compound 18 | Compound 2-4 | 3.57 | 22.5 | 221 | Red |
| Example 71 | Compound 18 | Compound 2-14 | 3.58 | 22.2 | 224 | Red |
| Example 72 | Compound 18 | Compound 2-21 | 3.57 | 22.0 | 228 | Red |
| Example 73 | Compound 19 | Compound 2-3 | 3.87 | 20.7 | 195 | Red |
| Example 74 | Compound 19 | Compound 2-4 | 3.88 | 20.1 | 183 | Red |
| Example 75 | Compound 19 | Compound 2-14 | 3.88 | 20.6 | 201 | Red |
| Example 76 | Compound 19 | Compound 2-21 | 3.91 | 19.8 | 199 | Red |
| Example 77 | Compound 20 | Compound 2-3 | 3.57 | 22.1 | 205 | Red |
| Example 78 | Compound 20 | Compound 2-4 | 3.60 | 21.9 | 191 | Red |
| Example 79 | Compound 20 | Compound 2-14 | 3.59 | 21.8 | 208 | Red |
| Example 80 | Compound 20 | Compound 2-21 | 3.61 | 22.0 | 214 | Red |
| Example 81 | Compound 21 | Compound 2-3 | 3.51 | 22.9 | 257 | Red |
| Example 82 | Compound 21 | Compound 2-4 | 3.53 | 23.1 | 242 | Red |
| Example 83 | Compound 21 | Compound 2-14 | 3.52 | 23.4 | 263 | Red |
| Example 84 | Compound 21 | Compound 2-21 | 3.55 | 23.8 | 254 | Red |
| Example 85 | Compound 22 | Compound 2-3 | 3.78 | 20.1 | 210 | Red |
| Example 86 | Compound 22 | Compound 2-4 | 3.82 | 20.4 | 204 | Red |
| Example 87 | Compound 22 | Compound 2-14 | 3.86 | 20.6 | 208 | Red |
| Example 88 | Compound 22 | Compound 2-21 | 3.83 | 20.2 | 200 | Red |

**[Table 4]**

| Category | First host | Second host | Driving voltage (V) | Efficiency (cd/A) | Lifetime T95 (hr) | Light emitting color |
|---|---|---|---|---|---|---|
| Comparative Example 1 | Compound C-1 | Compound 2-1 | 4.25 | 14.1 | 131 | Red |
| Comparative Example 2 | Compound C-1 | Compound 2-2 | 4.24 | 14.3 | 133 | Red |
| Comparative Example 3 | Compound C-1 | Compound 2-8 | 4.22 | 15.8 | 137 | Red |
| Comparative Example 4 | Compound C-1 | Compound 2-19 | 4.23 | 15.5 | 132 | Red |
| Comparative Example 5 | Compound C-2 | Compound 2-1 | 4.20 | 15.0 | 164 | Red |
| Comparative Example 6 | Compound C-2 | Compound 2-2 | 4.22 | 15.7 | 163 | Red |
| Comparative Example 7 | Compound C-2 | Compound 2-8 | 4.25 | 15.2 | 174 | Red |
| Comparative Example 8 | Compound C-2 | Compound 2-19 | 4.21 | 15.4 | 167 | Red |
| Comparative Example 9 | Compound C-3 | Compound 2-1 | 4.21 | 16.2 | 158 | Red |
| Comparative Example 10 | Compound C-3 | Compound 2-2 | 4.23 | 15.8 | 167 | Red |
| Comparative Example 11 | Compound C-3 | Compound 2-8 | 4.22 | 15.4 | 151 | Red |
| Comparative Example 12 | Compound C-3 | Compound 2-19 | 4.08 | 15.0 | 154 | Red |
| Comparative Example 13 | Compound C-4 | Compound 2-1 | 4.05 | 15.8 | 105 | Red |
| Comparative Example 14 | Compound C-4 | Compound 2-2 | 4.04 | 15.5 | 103 | Red |
| Comparative Example 15 | Compound C-4 | Compound 2-8 | 4.17 | 15.2 | 111 | Red |
| Comparative Example 16 | Compound C-4 | Compound 2-19 | 4.10 | 14.3 | 109 | Red |
| Comparative Example 17 | Compound C-5 | Compound 2-9 | 4.23 | 15.0 | 128 | Red |
| Comparative Example 18 | Compound C-5 | Compound 2-10 | 4.10 | 13.8 | 123 | Red |
| Comparative Example 19 | Compound C-5 | Compound 2-12 | 4.17 | 15.1 | 120 | Red |
| Comparative Example 20 | Compound C-5 | Compound 2-16 | 4.12 | 14.5 | 111 | Red |
| Comparative Example 21 | Compound C-6 | Compound 2-9 | 4.20 | 15.1 | 116 | Red |
| Comparative Example 22 | Compound C-6 | Compound 2-10 | 4.25 | 15.4 | 120 | Red |
| Comparative Example 23 | Compound C-6 | Compound 2-12 | 4.21 | 15.3 | 127 | Red |
| Comparative Example 24 | Compound C-6 | Compound 2-16 | 4.18 | 14.0 | 104 | Red |
| Comparative Example 25 | Compound C-7 | Compound 2-5 | 4.12 | 13.6 | 139 | Red |
| Comparative Example 26 | Compound C-7 | Compound 2-6 | 4.25 | 14.1 | 131 | Red |
| Comparative Example 27 | Compound C-7 | Compound 2-13 | 4.13 | 15.5 | 135 | Red |
| Comparative Example 28 | Compound C-7 | Compound 2-20 | 4.17 | 13.4 | 128 | Red |

**[Table 5]**

| Category | First host | Second host | Driving voltage (V) | Efficiency (cd/A) | Lifetime T95 (hr) | Light emitting color |
|---|---|---|---|---|---|---|
| Comparative Example 29 | Compound C-8 | Compound 2-5 | 4.05 | 14.0 | 120 | Red |
| Comparative Example 30 | Compound C-8 | Compound 2-6 | 4.03 | 13.4 | 128 | Red |
| Comparative Example 31 | Compound C-8 | Compound 2-13 | 4.08 | 13.1 | 121 | Red |
| Comparative Example 32 | Compound C-8 | Compound 2-20 | 4.09 | 14.2 | 119 | Red |
| Comparative Example 33 | Compound C-9 | Compound 2-5 | 4.11 | 13.5 | 117 | Red |
| Comparative Example 34 | Compound C-9 | Compound 2-6 | 4.13 | 15.9 | 119 | Red |
| Comparative Example 35 | Compound C-9 | Compound 2-13 | 4.15 | 15.3 | 117 | Red |
| Comparative Example 36 | Compound C-9 | Compound 2-20 | 4.14 | 14.5 | 110 | Red |
| Comparative Example 37 | Compound C-10 | Compound 2-5 | 4.06 | 15.3 | 127 | Red |
| Comparative Example 38 | Compound C-10 | Compound 2-6 | 4.09 | 16.0 | 131 | Red |
| Comparative Example 39 | Compound C-10 | Compound 2-13 | 4.05 | 16.4 | 128 | Red |
| Comparative Example 40 | Compound C-10 | Compound 2-20 | 4.02 | 16.1 | 120 | Red |
| Comparative Example 41 | Compound C-11 | Compound 2-5 | 4.11 | 16.0 | 121 | Red |
| Comparative Example 42 | Compound C-11 | Compound 2-6 | 4.18 | 16.1 | 127 | Red |
| Comparative Example 43 | Compound C-11 | Compound 2-13 | 4.21 | 17.3 | 138 | Red |
| Comparative Example 44 | Compound C-11 | Compound 2-20 | 4.18 | 16.0 | 121 | Red |
| Comparative Example 45 | Compound C-12 | Compound 2-3 | 4.10 | 16.2 | 131 | Red |
| Comparative Example 46 | Compound C-12 | Compound 2-4 | 4.11 | 15.5 | 129 | Red |
| Comparative Example 47 | Compound C-12 | Compound 2-14 | 4.10 | 16.8 | 124 | Red |
| Comparative Example 48 | Compound C-12 | Compound 2-21 | 4.14 | 15.9 | 136 | Red |
| Comparative Example 49 | Compound C-13 | Compound 2-3 | 4.25 | 15.8 | 105 | Red |
| Comparative Example 50 | Compound C-13 | Compound 2-4 | 4.24 | 15.5 | 103 | Red |
| Comparative Example 51 | Compound C-13 | Compound 2-14 | 4.27 | 15.2 | 101 | Red |
| Comparative Example 52 | Compound C-13 | Compound 2-21 | 4.20 | 14.3 | 118 | Red |
| Comparative Example 53 | Compound C-14 | Compound 2-3 | 4.23 | 15.0 | 108 | Red |
| Comparative Example 54 | Compound C-14 | Compound 2-4 | 4.20 | 13.8 | 93 | Red |
| Comparative Example 55 | Compound C-14 | Compound 2-14 | 4.27 | 15.1 | 100 | Red |
| Comparative Example 56 | Compound C-14 | Compound 2-21 | 4.22 | 14.5 | 101 | Red |

Referring to Tables 1 to 5 above, it was confirmed that Example 1 uses the EB-1 as the electron inhibition layer, and the compound of Chemical Formula 1 and the compound of Chemical Formula 2 as the red light emitting layer, and Dp-7 as the dopant, and thereby exhibits low driving voltage and high efficiency and lifetime, as compared with the organic light emitting devices of the Comparative Examples. From this, it can be predicted that when the combination of the compound of Chemical Formula 1 which is the first host and the compound of Formula 2 which is the second host is used, energy transfer to the red dopant in the red light emitting layer is performed well and thus, the efficiency and lifetime of the organic light emitting device are effectively increased. Furthermore, it can be predicted that the Examples have higher stability to electrons and holes as compared with the Comparative Examples. In addition, it can be predicted that as the amount of holes increases depending on the use of the second host, electrons and holes in the red light emitting layer maintain a more stable balance and thereby, the efficiency and the lifetime are further increased. That is, it was confirmed that when the compound of Chemical Formula 1 and the compound of Chemical Formula 2 were vapor-deposited and used as a host of the red light emitting layer, the driving voltage, light emitting efficiency and lifetime characteristics of the organic light emitting device could be improved.

**EXPLANATION OF SIGN**

| | | | |
|---|---|---|---|
| 1: | substrate | 2: | anode |
| 3: | light emitting layer | 4: | cathode |
| 5: | hole injection layer | 6: | hole transport layer |
| 7: | electron inhibition layer | 8. | hole blocking layer |
| 9: | electron injection and transport layer | | |

## Claims

1. An organic light emitting device comprising:
an anode (2); a cathode (4) that is provided opposite to the anode; and one or more organic material layers that are provided between the anode and the cathode,
wherein the organic material layer includes any one selected from compounds represented by the following Chemical Formulas 1-B and 1-C and a compound represented by the following Chemical Formula 2: 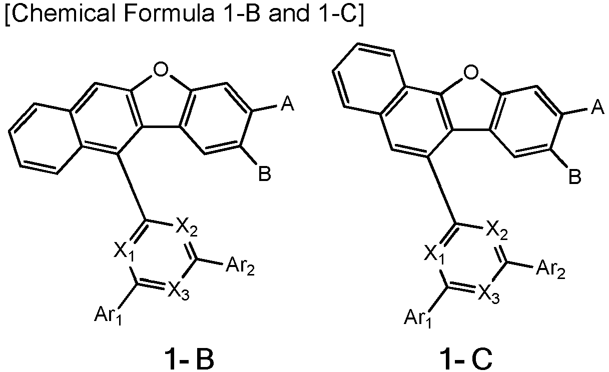
in the Chemical Formula 1-B and 1-C,
X₁ to X₃ are each independently N or CR₅', provided that at least one of X₁ to X₃ is N,
Ar₁ and Ar₂ are each independently a substituted or unsubstituted C₆₋₆₀ aryl;
or a substituted or unsubstituted C₂₋₆₀ heteroaryl containing at least one of O, N, Si and S,
R₅' is hydrogen; deuterium; halogen; hydroxy; nitrile; nitro; amino; a substituted or unsubstituted C₂₋₆₀ alkyl; a substituted or unsubstituted C₂₋₆₀ alkoxy; a substituted or unsubstituted C₂₋₆₀ alkenyl; a substituted or unsubstituted C₆₋₆₀ aryl; or a substituted or unsubstituted C₂₋₆₀ heteroaryl containing at least one of O, N, Si and S,
one of A and B is a substituent represented by the following Chemical Formula 1-1, and the other is hydrogen or deuterium, 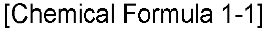 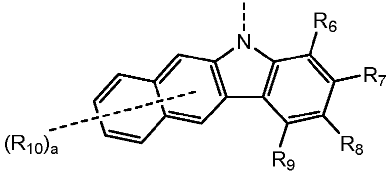
in the Chemical Formula 1-1,
R₆ to R₁₀ are each independently hydrogen; deuterium; halogen; hydroxy; nitrile; nitro; amino; a substituted or unsubstituted C₂₋₆₀ alkyl; a substituted or unsubstituted C₂₋₆₀ alkoxy; a substituted or unsubstituted C₂₋₆₀ alkenyl; a substituted or unsubstituted C₆₋₆₀ aryl; a substituted or unsubstituted C₂₋₆₀ heteroaryl containing at least one of O, N, Si and S, or R₆ to R₉ combine with an adjacent group to form a condensed ring,
a is an integer of 1 to 6, 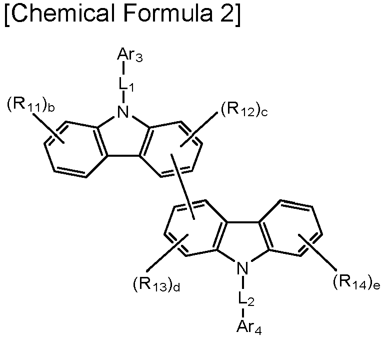
in the Chemical Formula 2,
Ar₃ and Ar₄ are each independently a substituted or unsubstituted C₆₋₆₀ aryl; or a substituted or unsubstituted C₂₋₆₀ heteroaryl containing at least one of O, N, Si and S,
L₁ and L₂ are each independently a single bond; or a substituted or unsubstituted C₆₋₆₀ arylene,
R₁₁ to R₁₄ are each independently hydrogen; deuterium; halogen; hydroxy; nitrile; nitro; amino; a substituted or unsubstituted C₂₋₆₀ alkyl; a substituted or unsubstituted C₂₋₆₀ alkoxy; a substituted or unsubstituted C₂₋₆₀ alkenyl; a substituted or unsubstituted C₆₋₆₀ aryl; a substituted or unsubstituted C₂₋₆₀ heteroaryl containing at least one of O, N, Si and S,
b and e are each independently an integer of 1 to 4, and
c and d are each independently an integer of 1 to 3.

2. An organic light emitting device comprising:
an anode; a cathode that is provided opposite to the anode; and one or more organic material layers that are provided between the anode and the cathode,
wherein the organic material layer includes a compound represented by the following Chemical Formula 1-A and a compound represented by the following Chemical Formula 2: 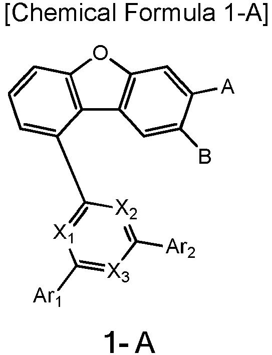
in the Chemical Formula 1-A,
X₁ to X₃ are each independently N or CR₅', provided that at least one of X₁ to X₃ is N,
Ar₁ and Ar₂ are each independently a substituted or unsubstituted C₆₋₆₀ aryl; or a substituted or unsubstituted C₂₋₆₀ heteroaryl containing at least one of O, N, Si and S,
R₅' is hydrogen; deuterium; halogen; hydroxy; nitrile; nitro; amino; a substituted or unsubstituted C₂₋₆₀ alkyl; a substituted or unsubstituted C₂₋₆₀ alkoxy; a substituted or unsubstituted C₂₋₆₀ alkenyl; a substituted or unsubstituted C₆₋₆₀ aryl; or a substituted or unsubstituted C₂₋₆₀ heteroaryl containing at least one of O, N, Si and S,
one of A and B is a substituent selected from the group consisting of the following substituents, and the other is hydrogen or deuterium, 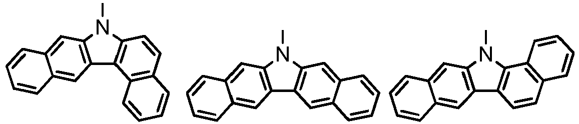 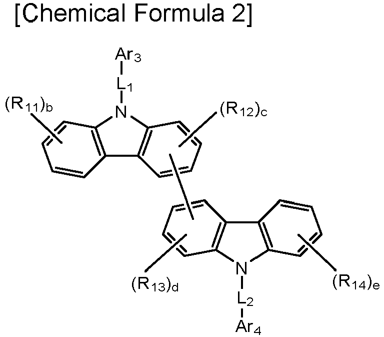
in the Chemical Formula 2,
Ar₃ and Ar₄ are each independently a substituted or unsubstituted C₆₋₆₀ aryl; or a substituted or unsubstituted C₂₋₆₀ heteroaryl containing at least one of O, N, Si and S,
L₁ and L₂ are each independently a single bond; or a substituted or unsubstituted C₆₋₆₀ arylene,
R₁₁ to R₁₄ are each independently hydrogen; deuterium; halogen; hydroxy; nitrile; nitro; amino; a substituted or unsubstituted C₂₋₆₀ alkyl; a substituted or unsubstituted C₂₋₆₀ alkoxy; a substituted or unsubstituted C₂₋₆₀ alkenyl; a substituted or unsubstituted C₆₋₆₀ aryl; a substituted or unsubstituted C₂₋₆₀ heteroaryl containing at least one of O, N, Si and S,
b and e are each independently an integer of 1 to 4, and
c and d are each independently an integer of 1 to 3.

3. The organic light emitting device according to claim 1 or 2,
wherein X₁ to X₃ all are N.

4. The organic light emitting device according to claim 1 or 2,
wherein Ar₁ and Ar₂ are each independently any one selected from the group consisting of:

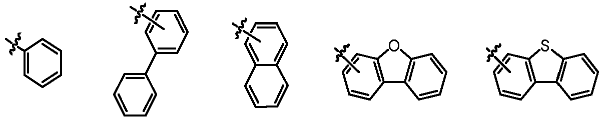

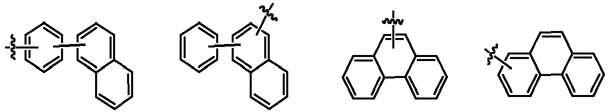

5. The organic light emitting device according to claim 1,
wherein the Chemical Formula 1-1 is selected from the group consisting of the following substituents:

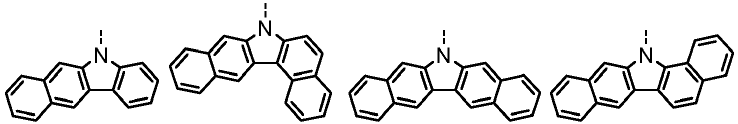

6. An organic light emitting device comprising:
an anode; a cathode that is provided opposite to the anode; and one or more organic material layers that are provided between the anode and the cathode,
wherein the organic material layer includes a compound represented by the following Chemical Formula 2 and any one compound selected from the group (G) consisting of the following compounds: 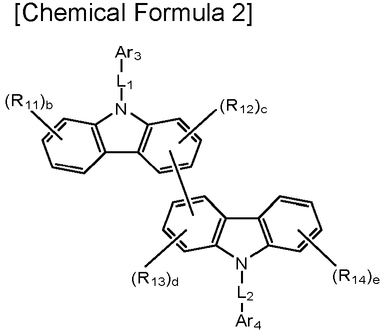
in the Chemical Formula 2,
Ar₃ and Ar₄ are each independently a substituted or unsubstituted C₆₋₆₀ aryl; or a substituted or unsubstituted C₂₋₆₀ heteroaryl containing at least one of O, N, Si and S,
L₁ and L₂ are each independently a single bond; or a substituted or unsubstituted C₆₋₆₀ arylene,
R₁₁ to R₁₄ are each independently hydrogen; deuterium; halogen; hydroxy; nitrile; nitro; amino; a substituted or unsubstituted C₂₋₆₀ alkyl; a substituted or unsubstituted C₂₋₆₀ alkoxy; a substituted or unsubstituted C₂₋₆₀ alkenyl; a substituted or unsubstituted C₆₋₆₀ aryl; a substituted or unsubstituted C₂₋₆₀ heteroaryl containing at least one of O, N, Si and S,
b and e are each independently an integer of 1 to 4, and
c and d are each independently an integer of 1 to 3,

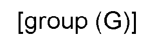

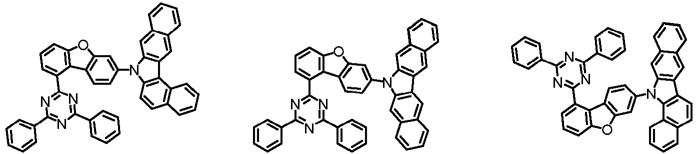

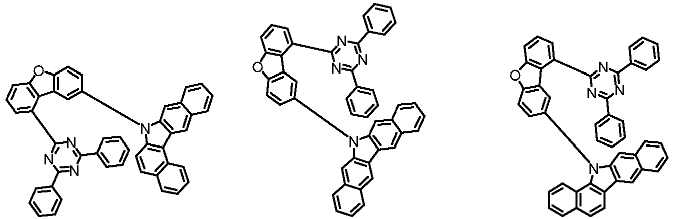

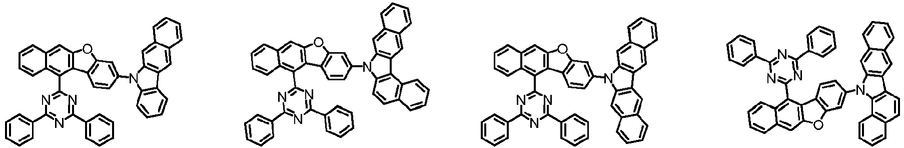

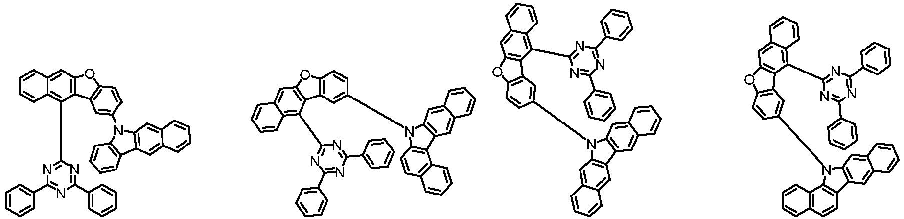

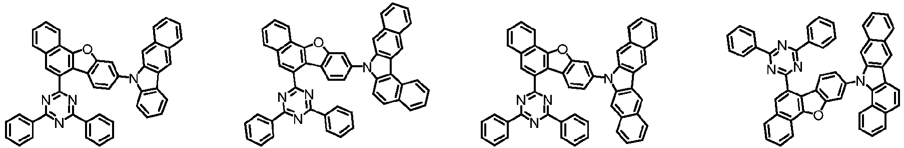

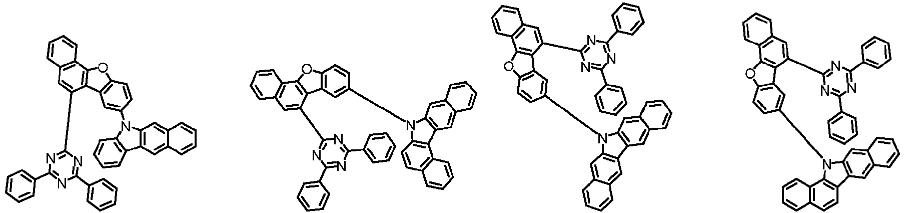

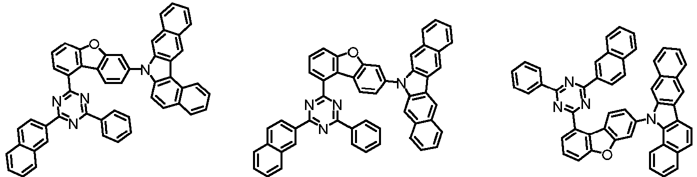

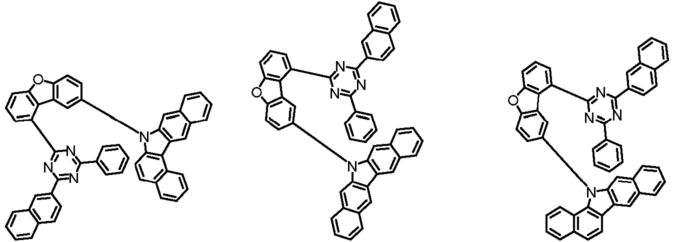

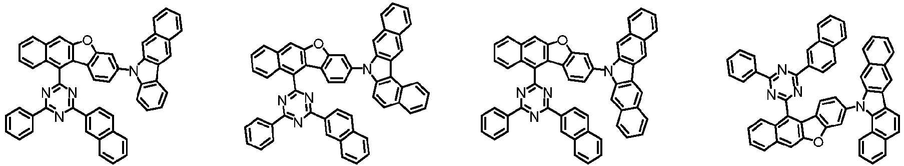

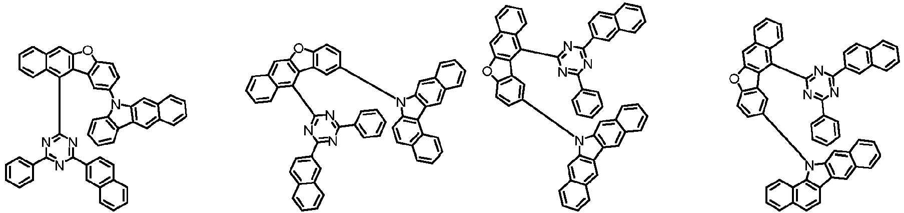

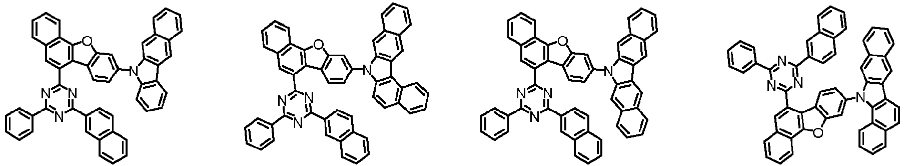

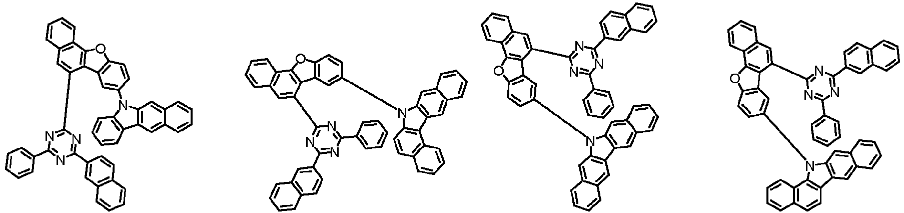

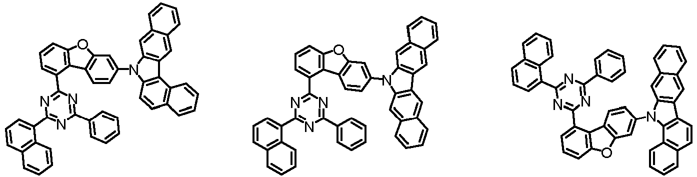

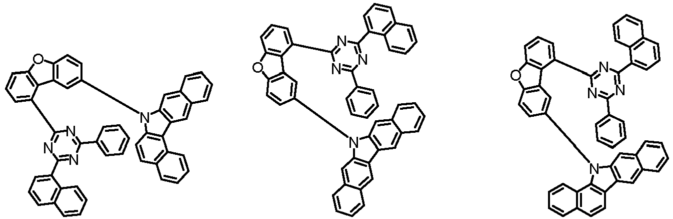

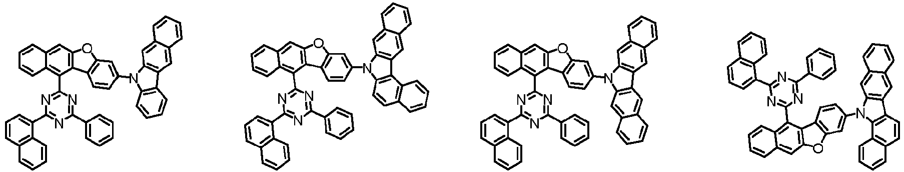

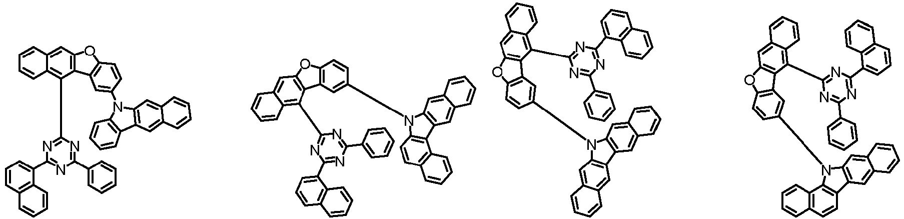

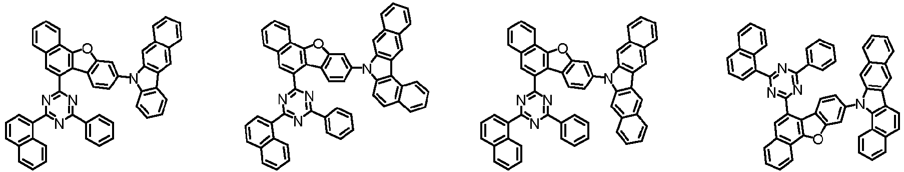

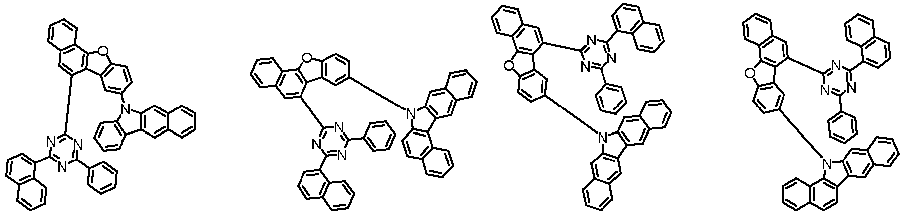

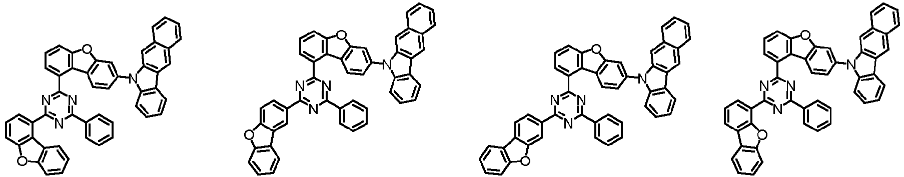

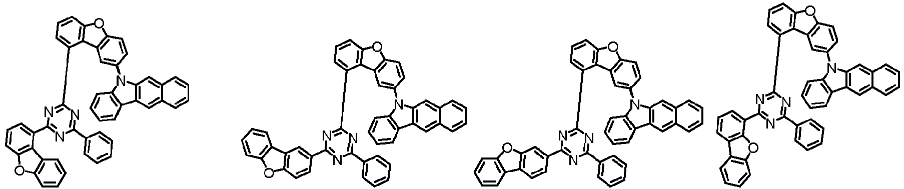

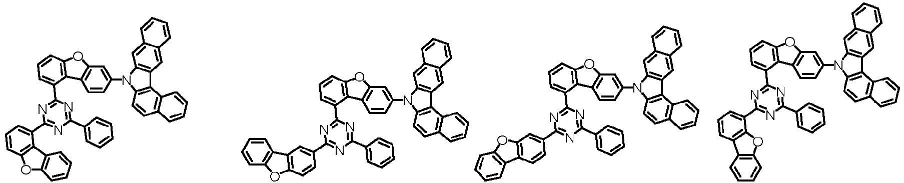

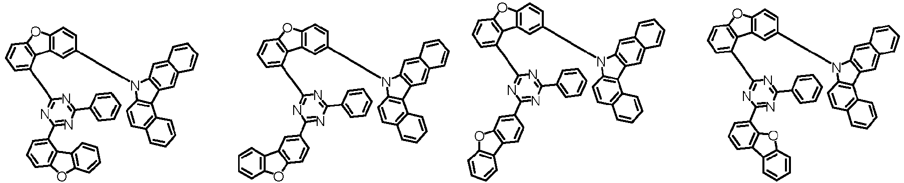

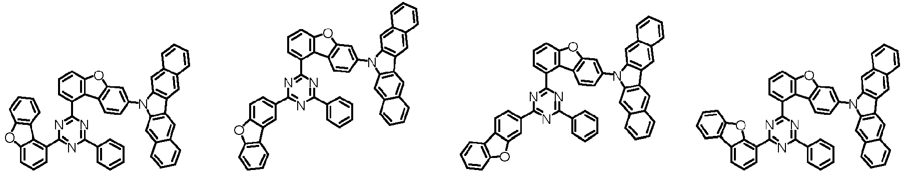

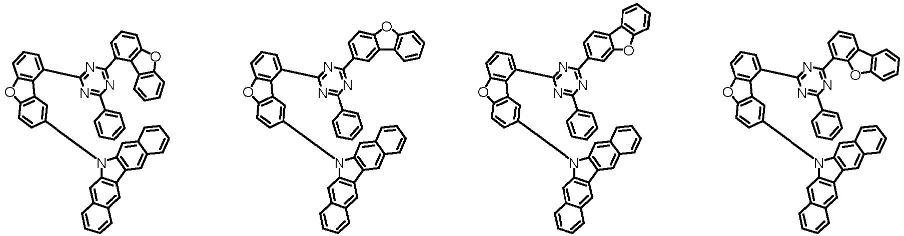

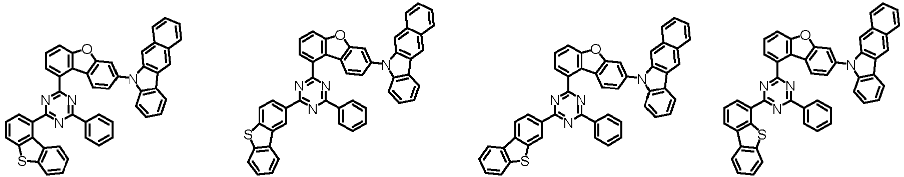

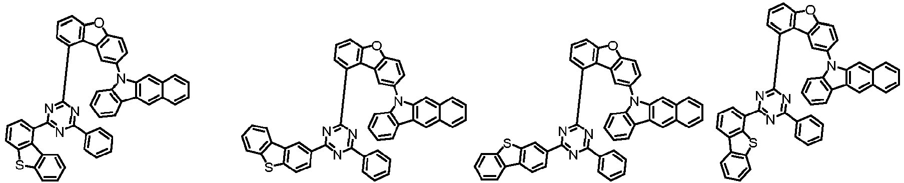

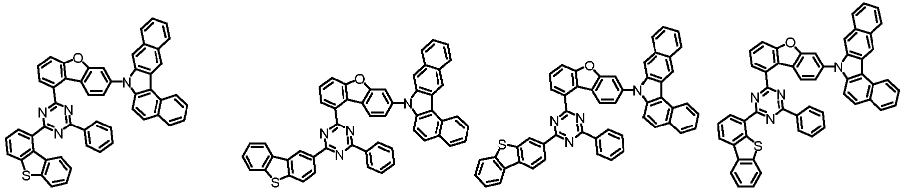

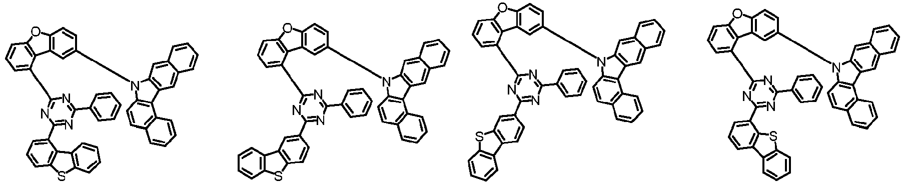

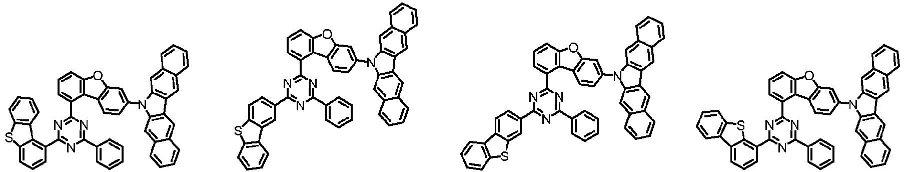

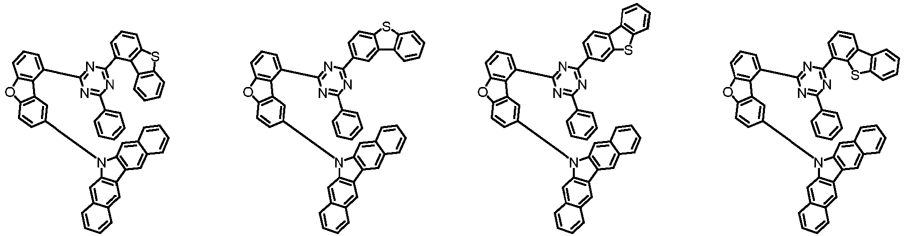

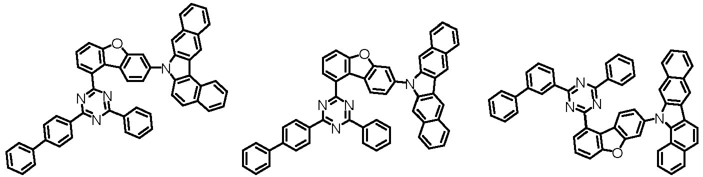

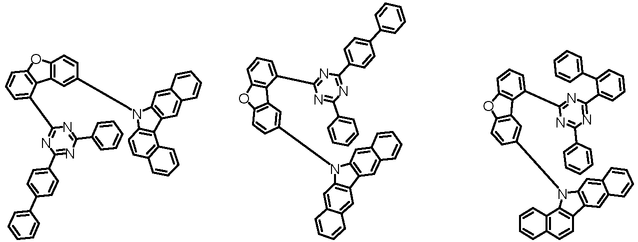

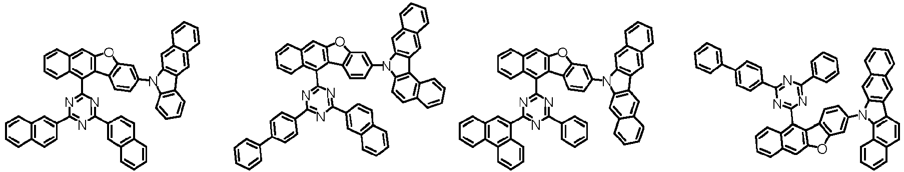

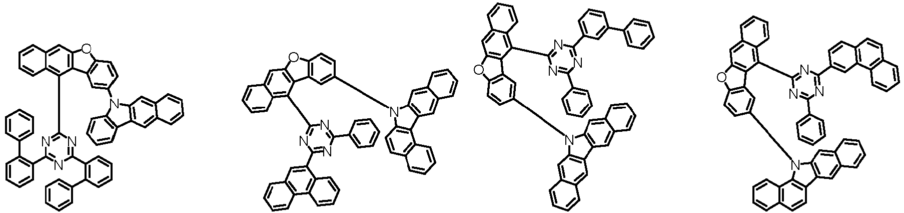

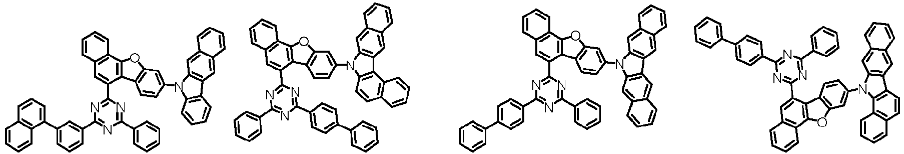

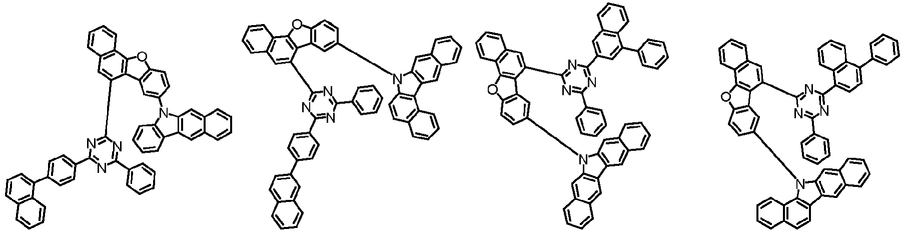

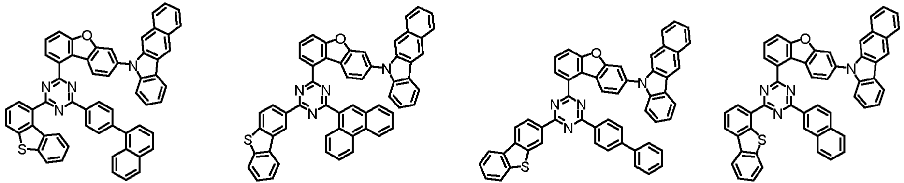

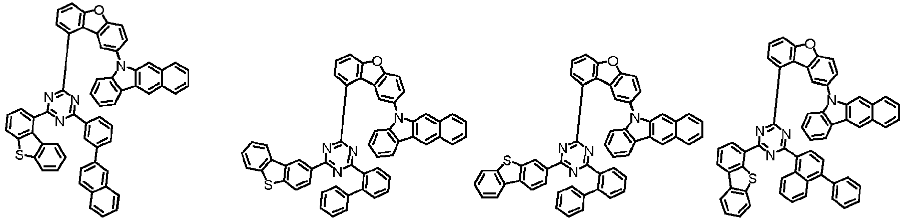

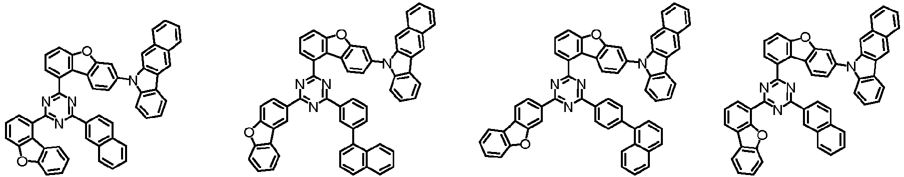

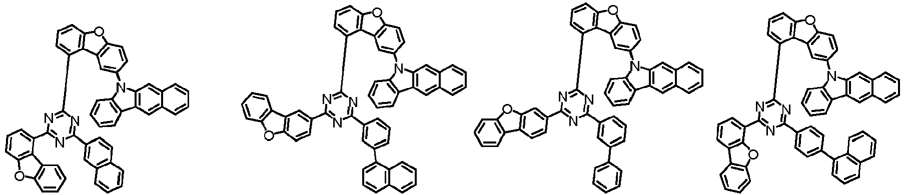

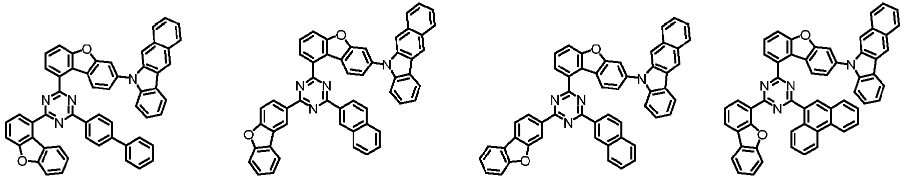

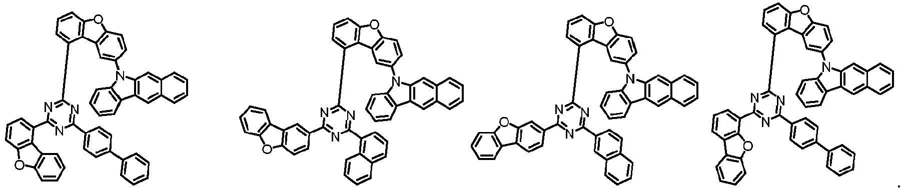

7. The organic light emitting device according to claim 1 or 2,
wherein the compound represented by Chemical Formula 2 is a compound represented by Chemical Formula 2-1: 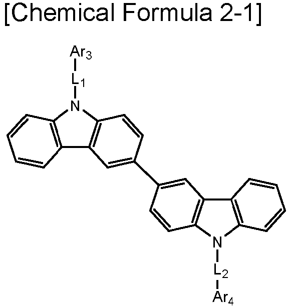
in Chemical Formula 2-1,
Ar₃, Ar₄, L₁ and L₂ are as defined in claim 1 or 2.

8. The organic light emitting device according to claim 1 or 2,
wherein Ar₃ and Ar₄ are each independently any one selected from the group consisting of:

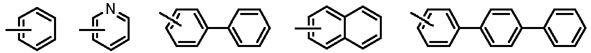

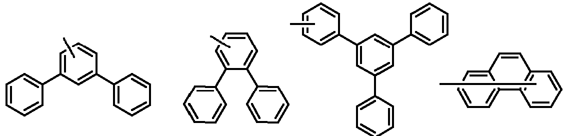

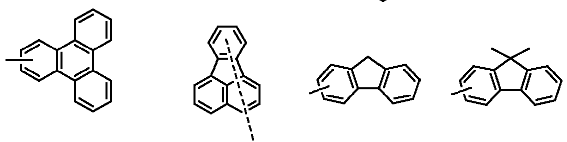

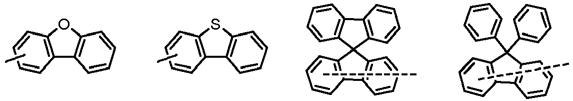

9. The organic light emitting device according to claim 1 or 2,
wherein L₁ and L₂ are each independently a single bond or any one selected from the group consisting of:

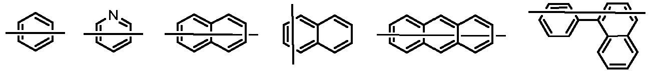

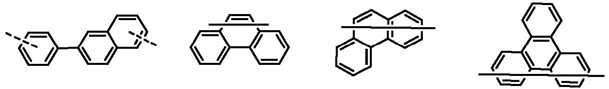

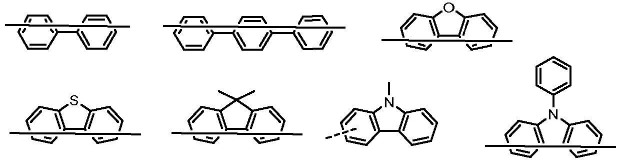

10. The organic light emitting device according to claim 1 or 2,
wherein R₁₁ to R₁₄ are hydrogen.

11. The organic light emitting device according to claim 1 or 2,
wherein the compound represented by Chemical Formula 2 is selected from the group consisting of the following compounds:

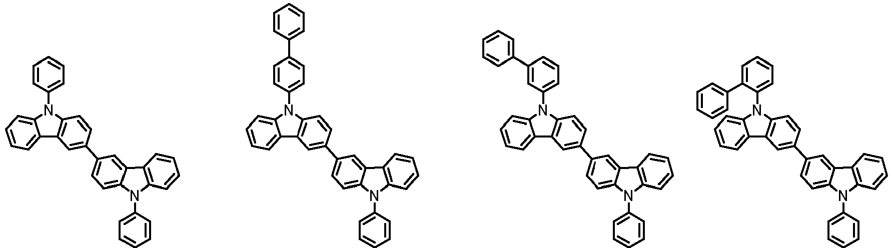

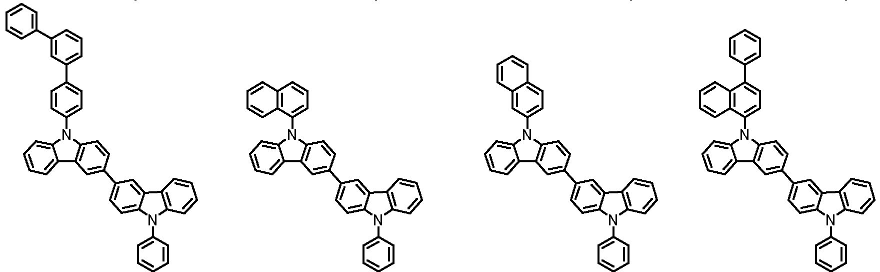

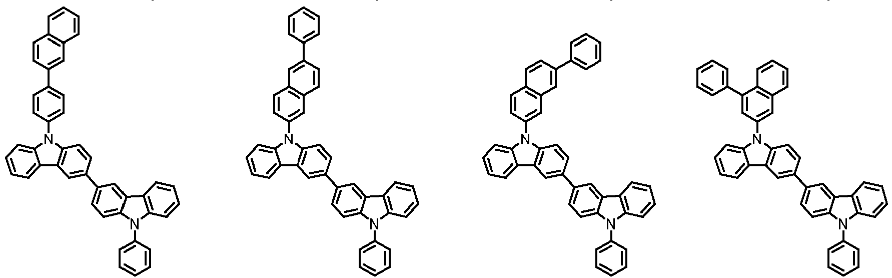

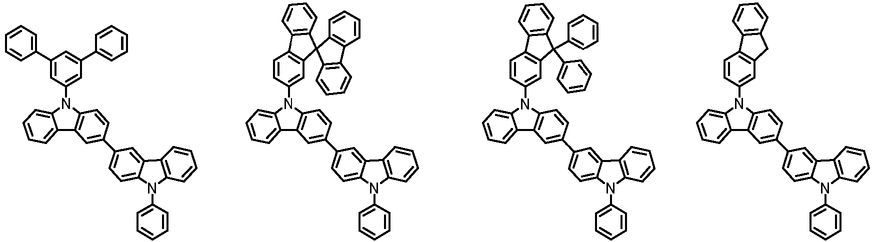

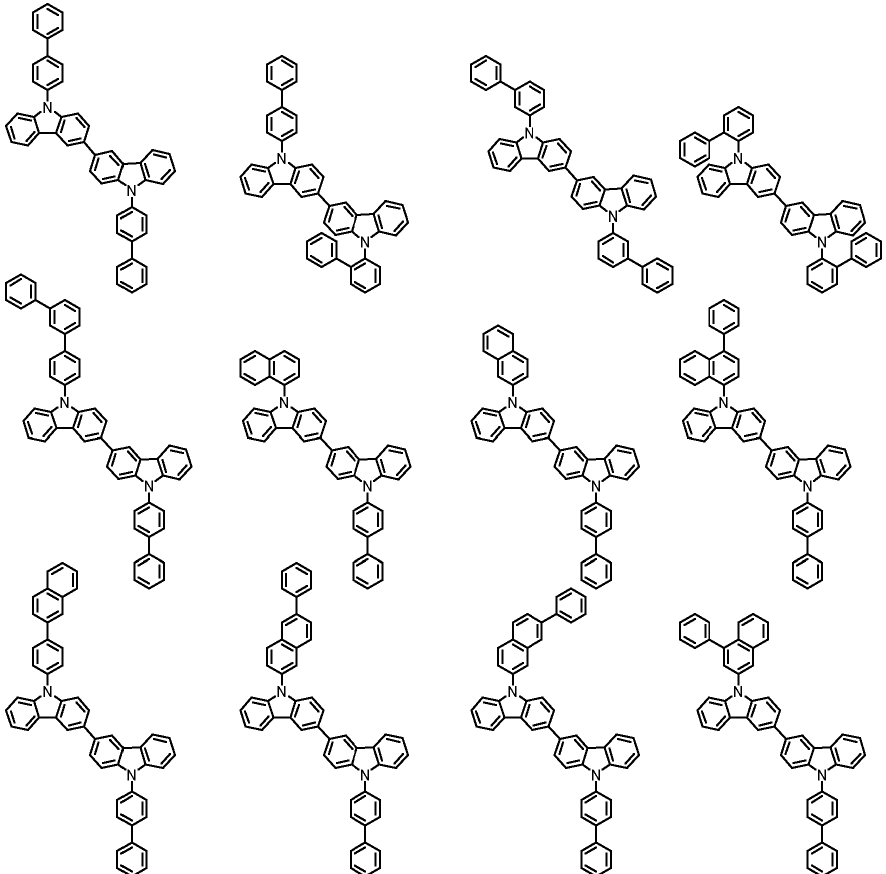

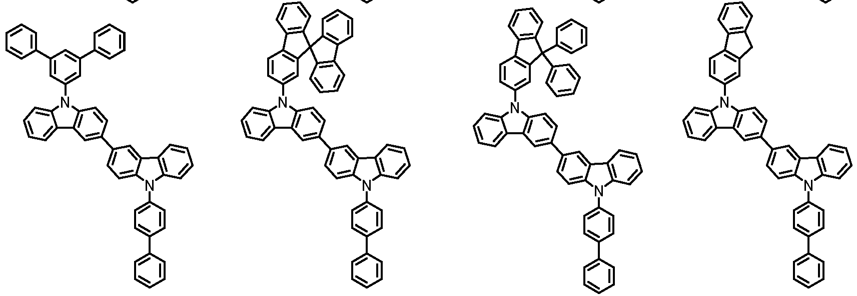

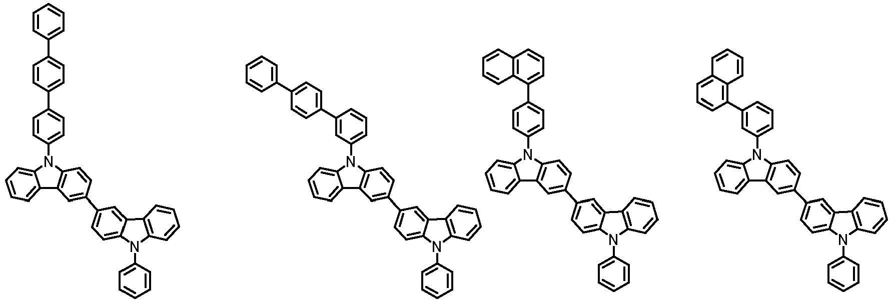

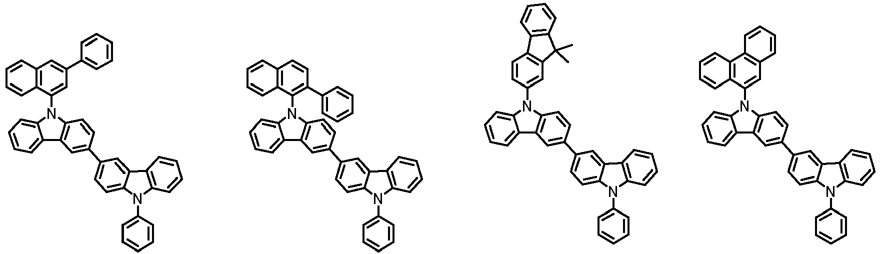

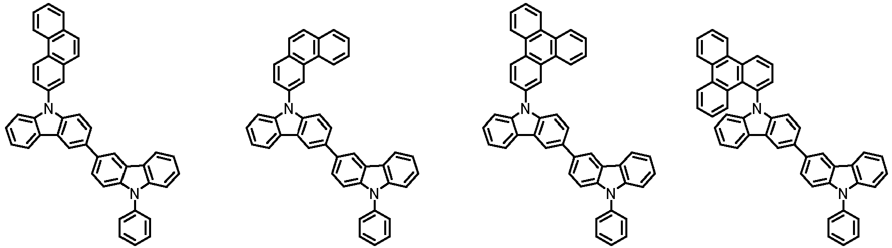

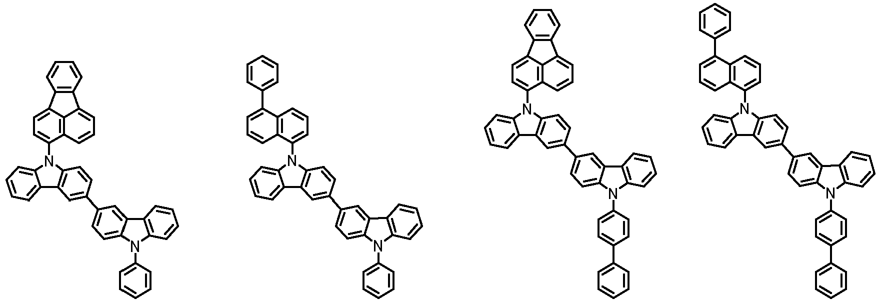

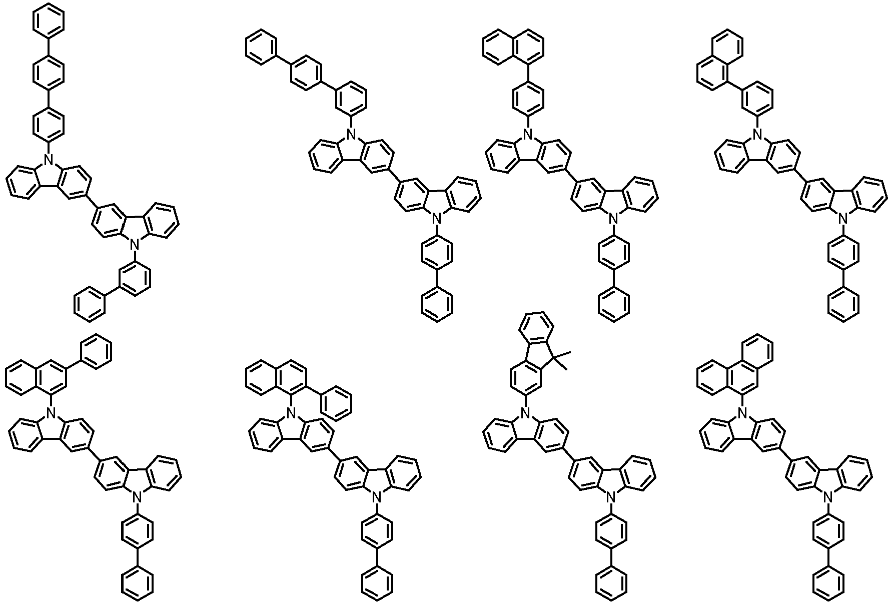

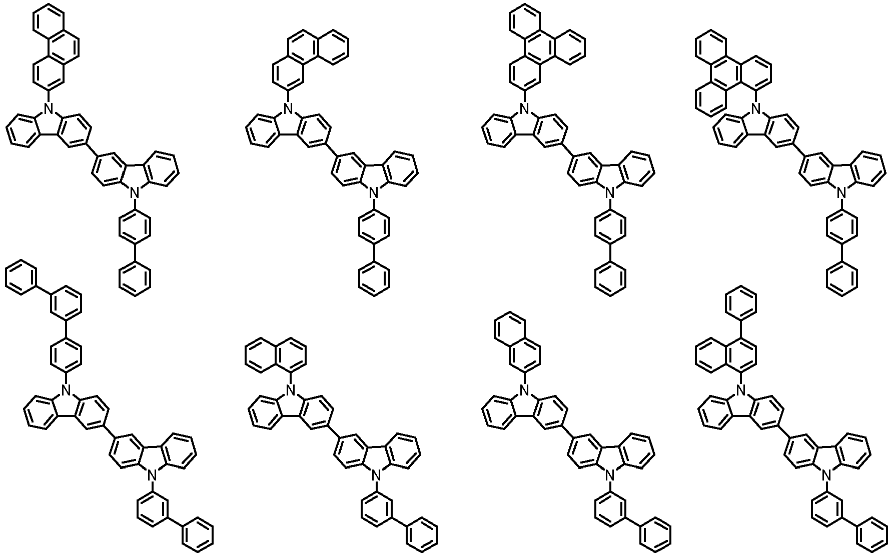

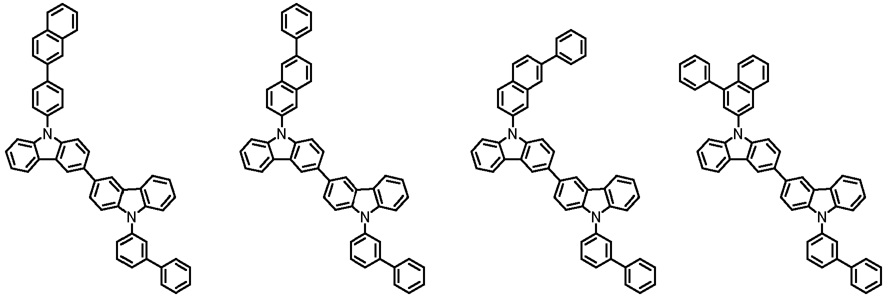

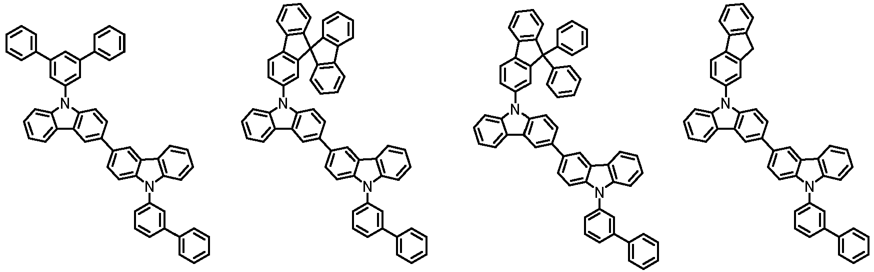

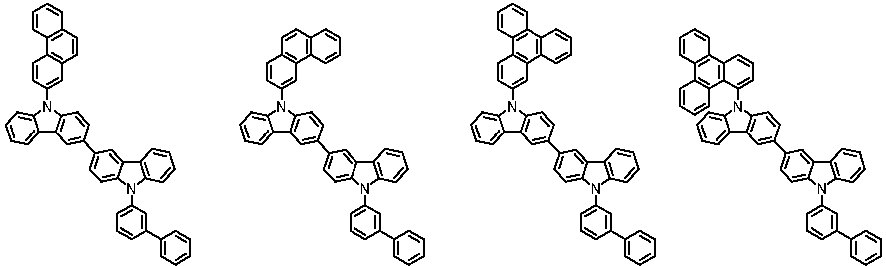

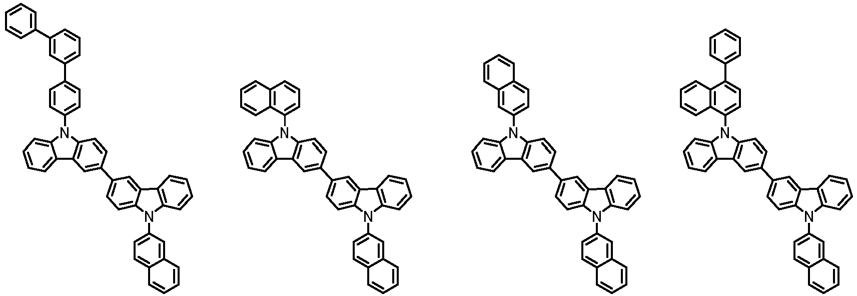

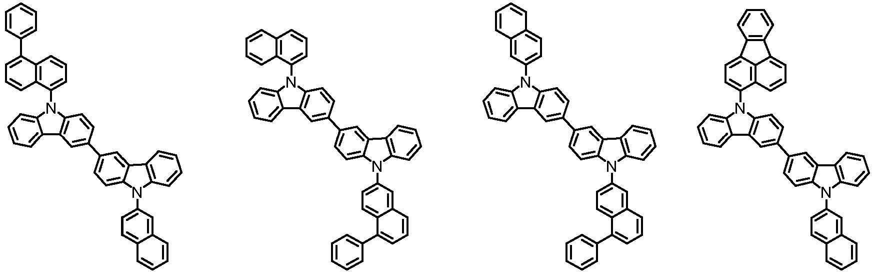

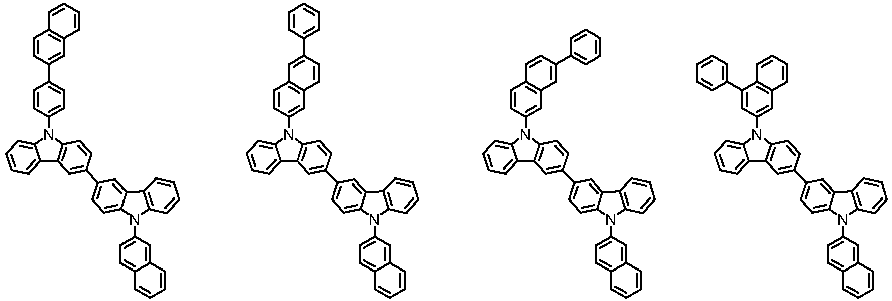

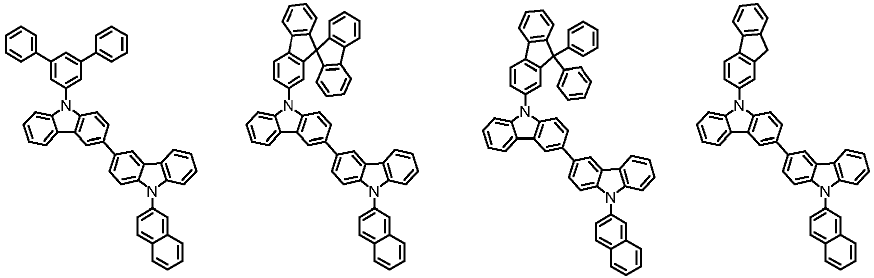

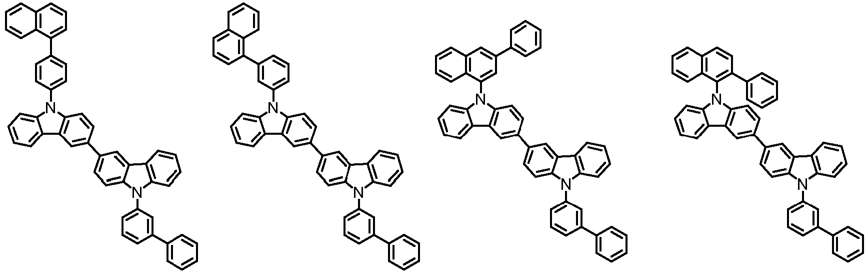

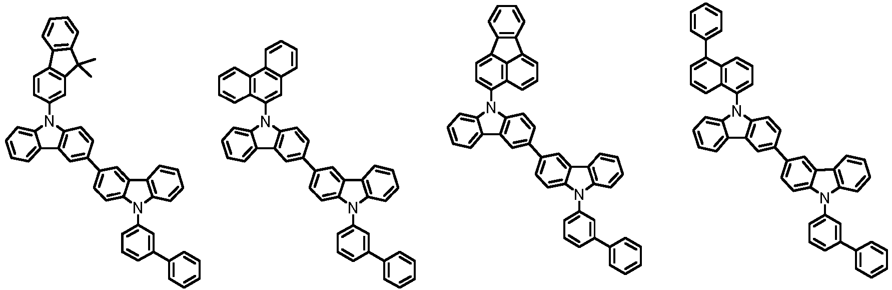

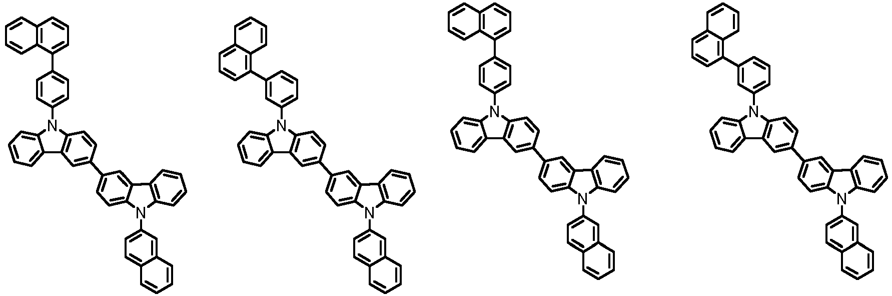

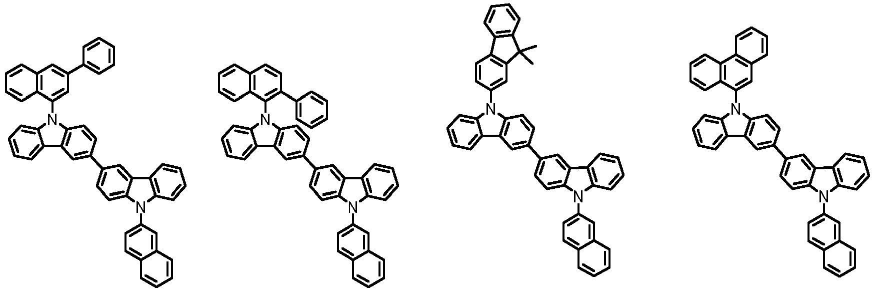

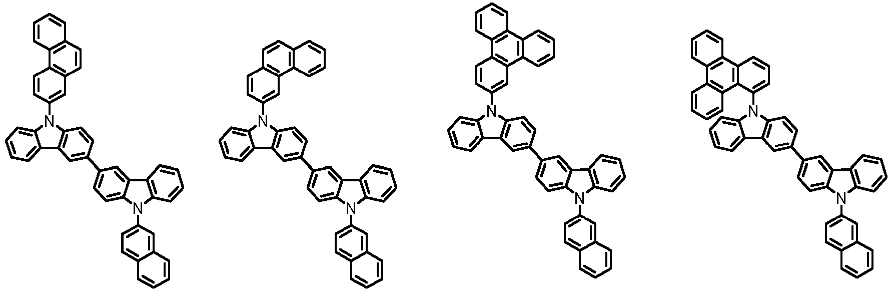

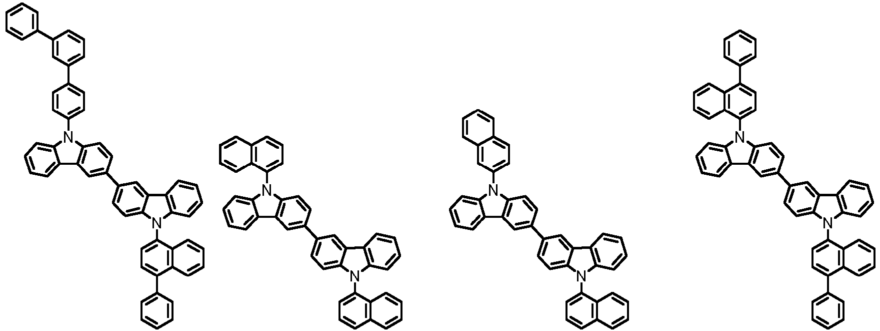

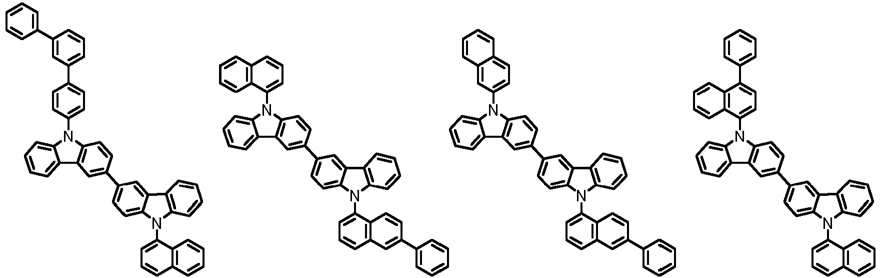

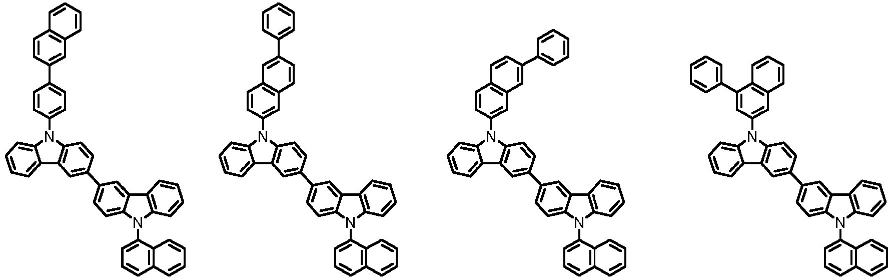

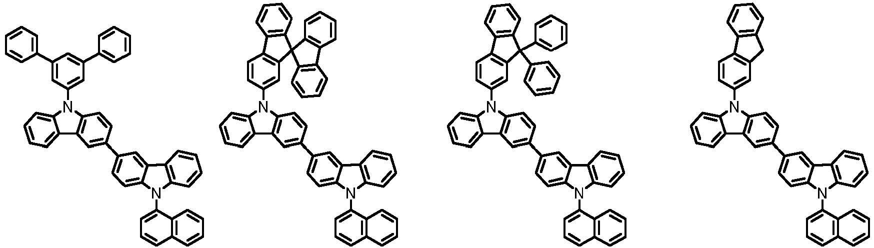

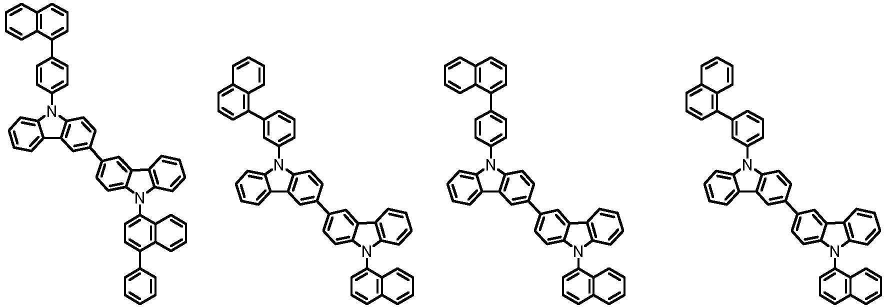

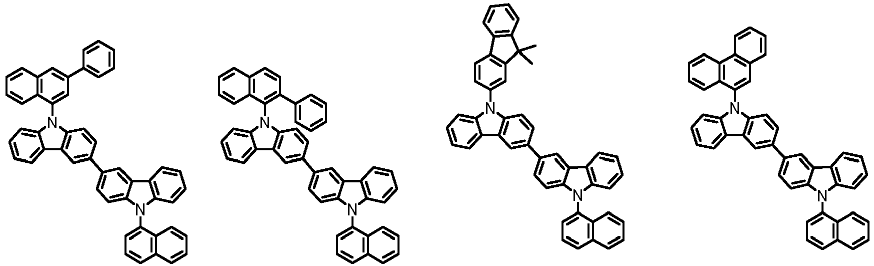

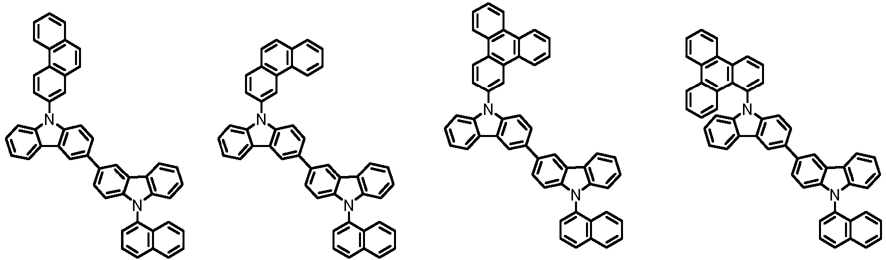

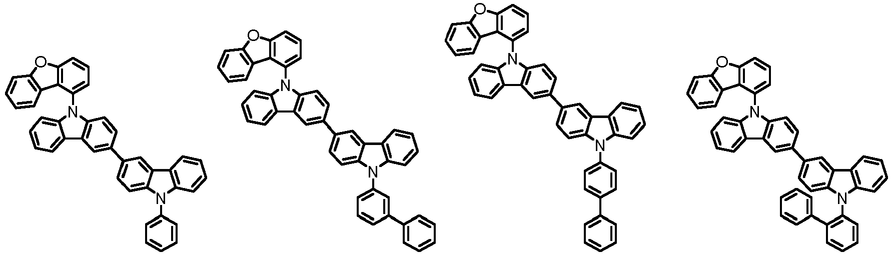

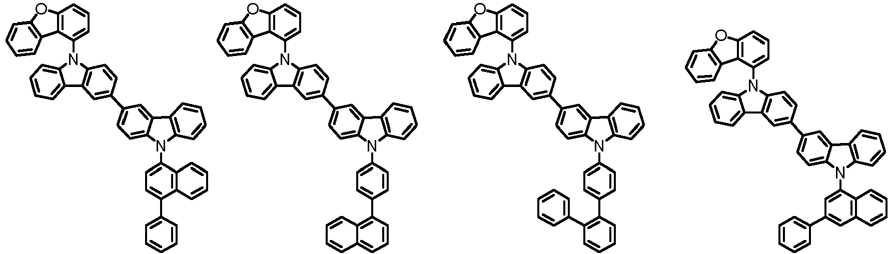

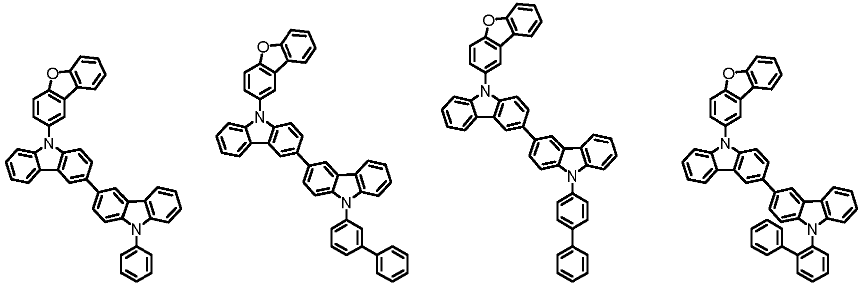

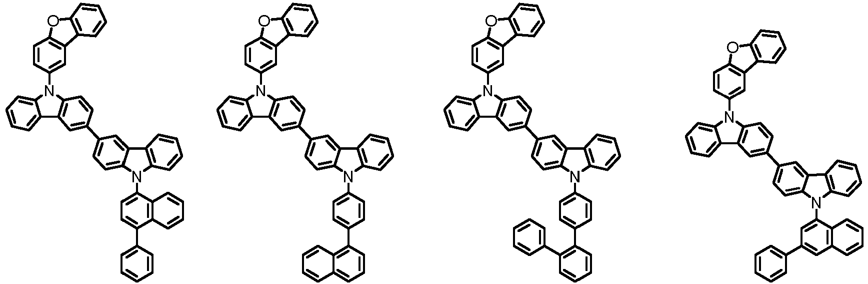

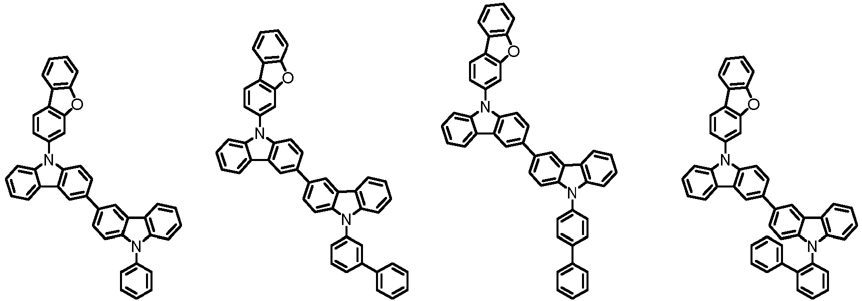

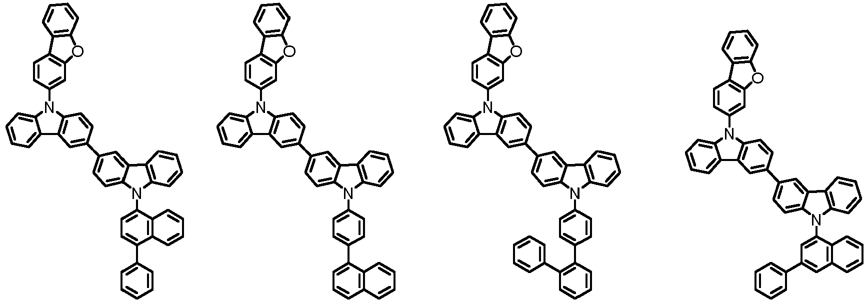

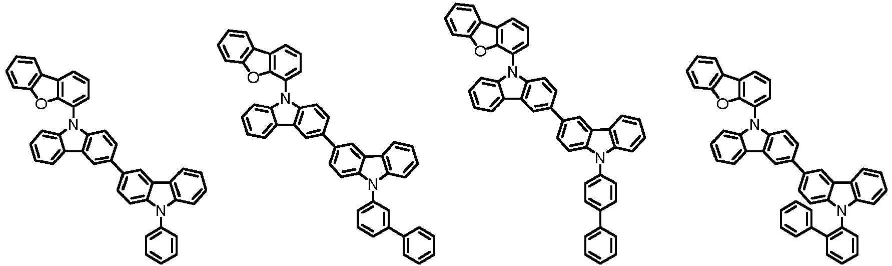

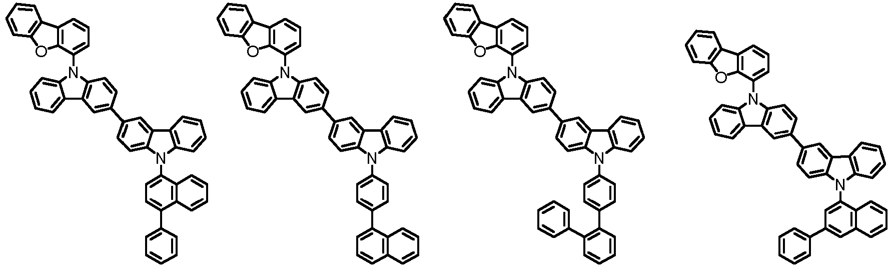

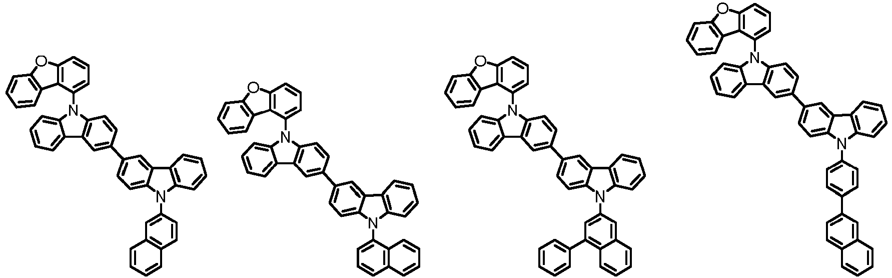

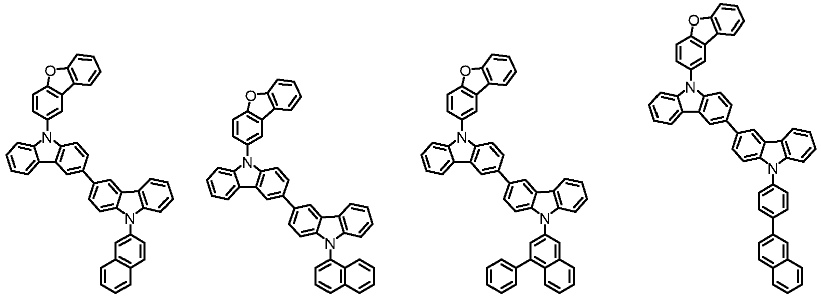

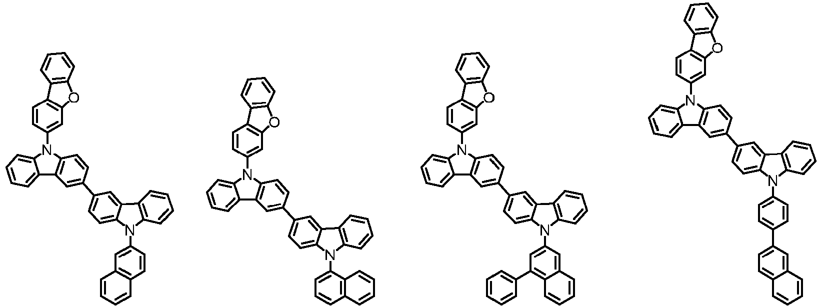

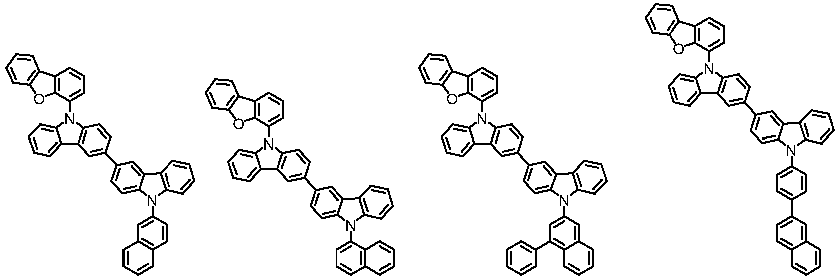

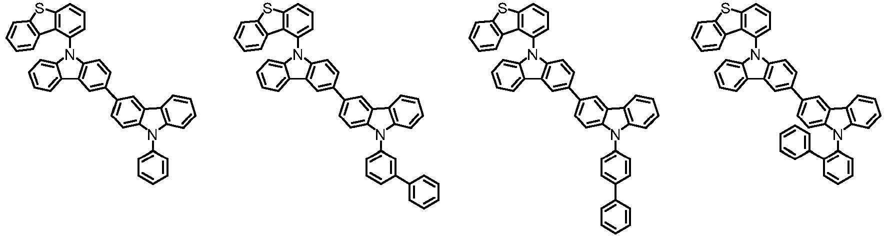

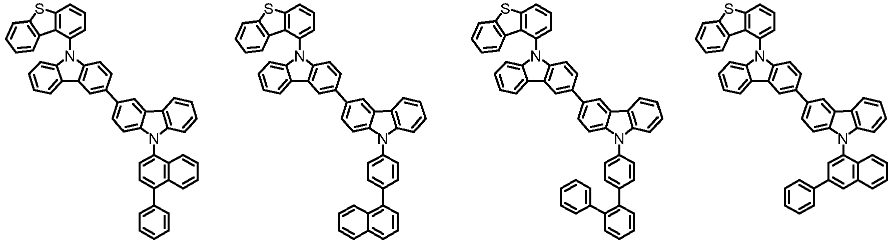

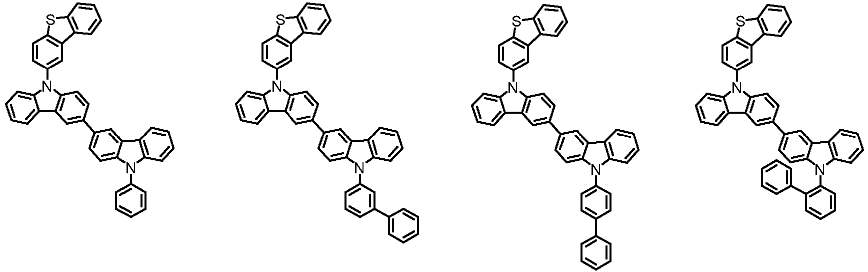

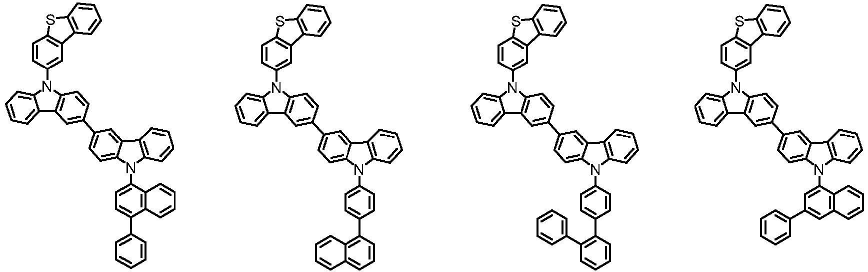

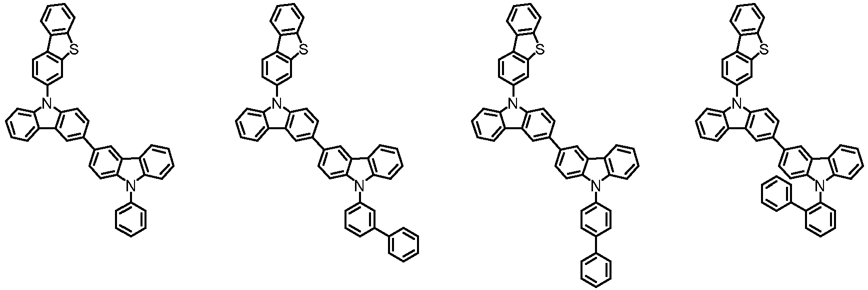

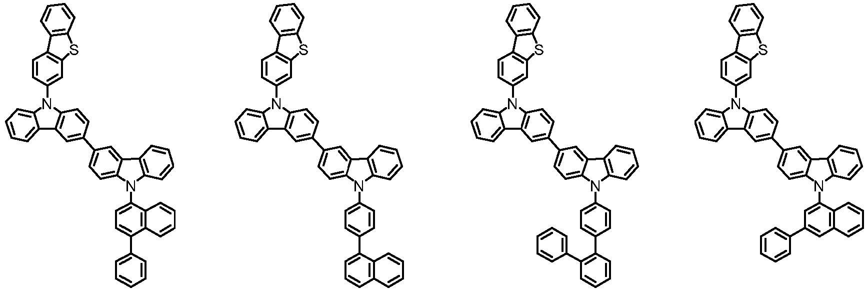

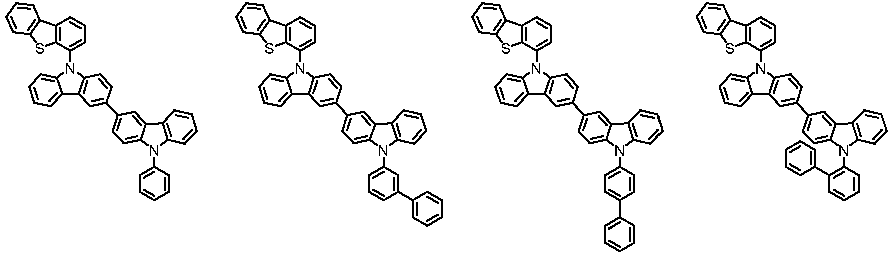

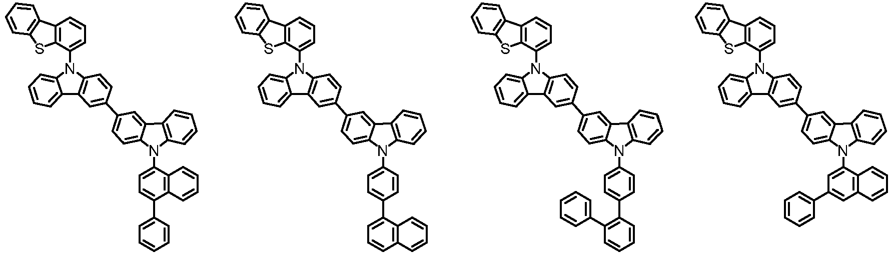

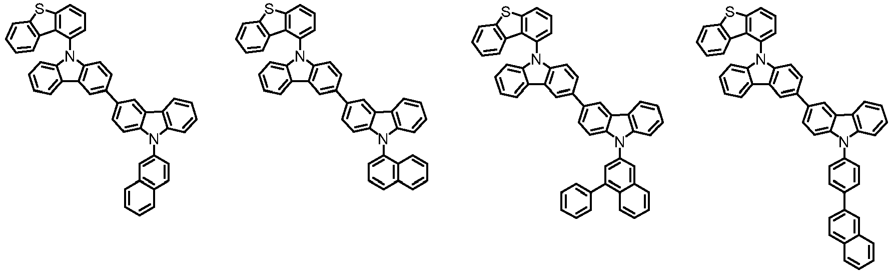

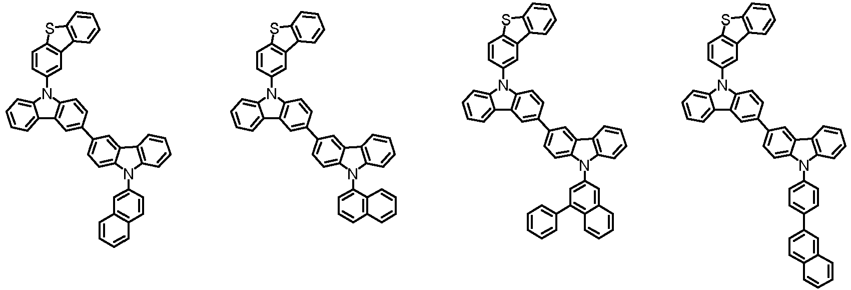

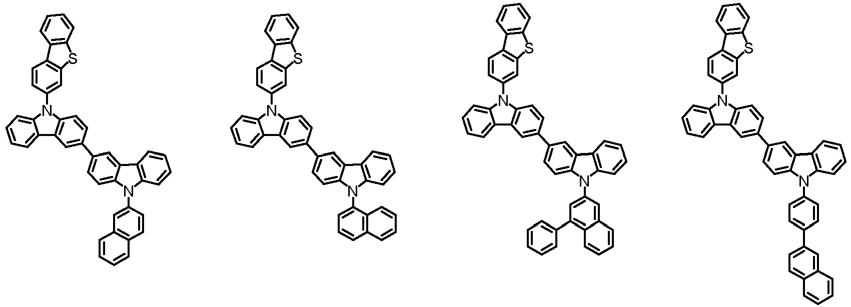

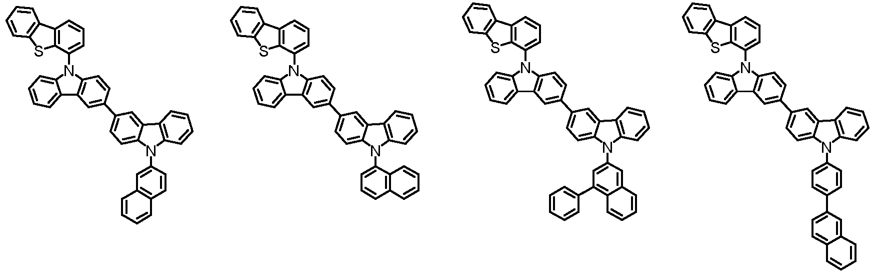

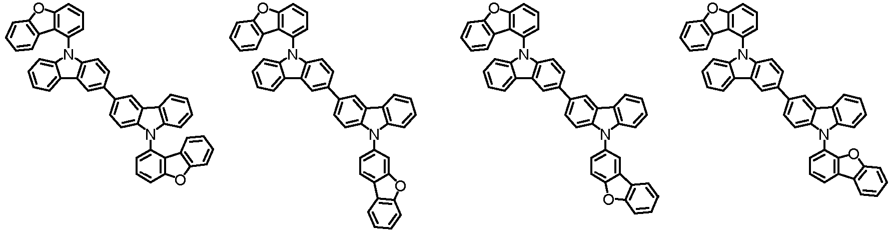

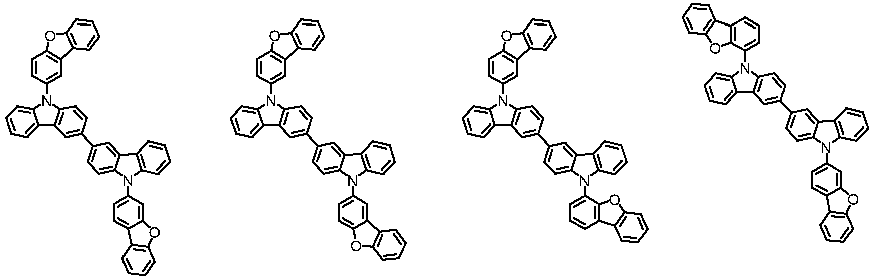

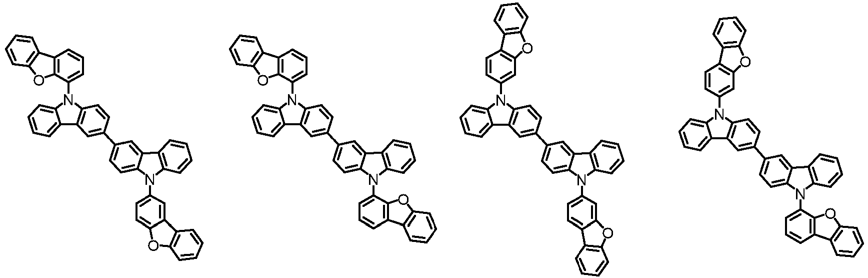

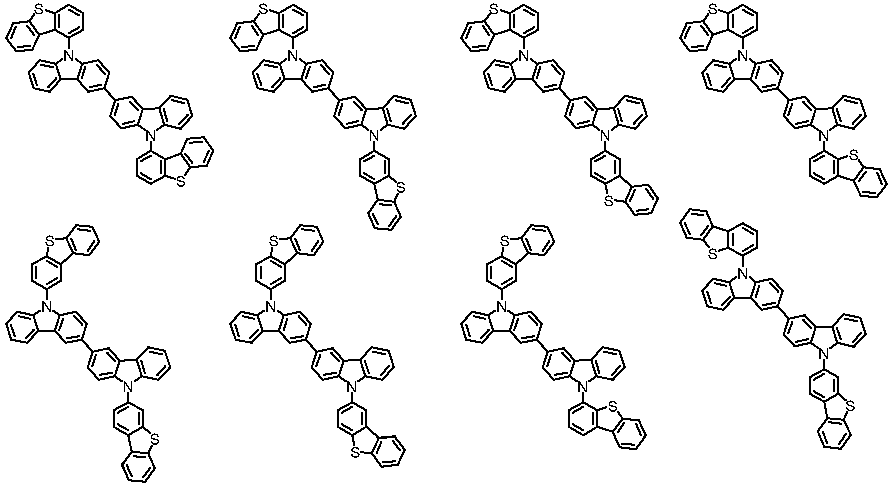

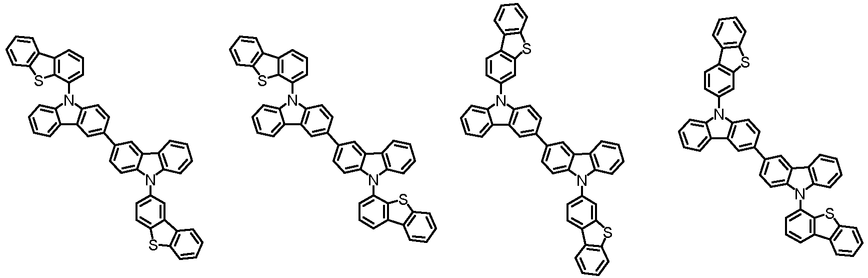

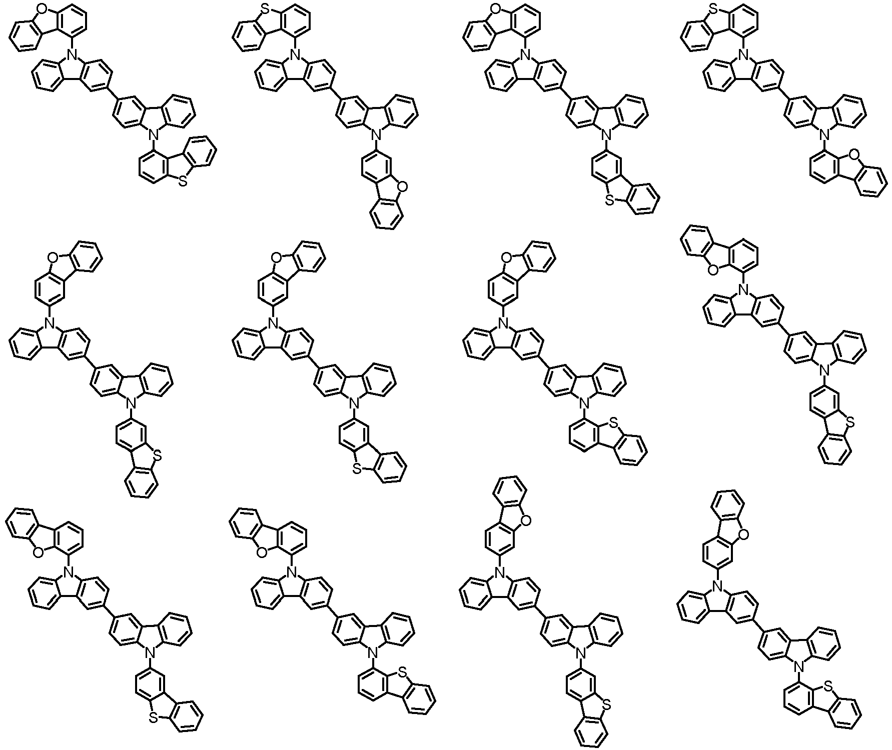

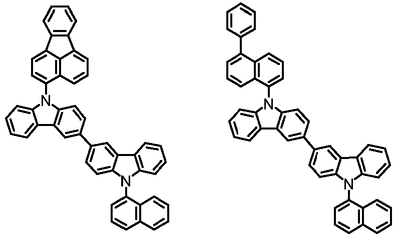

12. The organic light emitting device according to claim 1 or 2,
wherein the organic material layer including any one selected from the compounds represented by Chemical Formulas 1-A, 1-B and 1-C and the compound represented by Chemical Formula 2 is a light emitting layer.

13. The organic light emitting device according to claim 12,
wherein any one selected from the compounds represented by Chemical Formulas 1-A, 1-B and 1-C and the compound represented by Chemical Formula 2 are host materials in the light emitting layer.

14. The organic light emitting device according to claim 12,
wherein the light emitting layer further comprises a dopant material.

## Patentansprüche

1. Organische lichtemittierende Vorrichtung, umfassend:
eine Anode (2); eine Kathode (4), die gegenüber der Anode vorgesehen ist; und eine oder mehrere Schichten aus organischem Material, die zwischen der Anode und der Kathode vorgesehen sind,
wobei die Schicht aus organischem Material eine Verbindung enthält, die ausgewählt ist aus den durch die folgenden chemischen Formeln 1-B und 1-C dargestellten Verbindungen, und eine durch die folgende chemische Formel 2 dargestellte Verbindung: 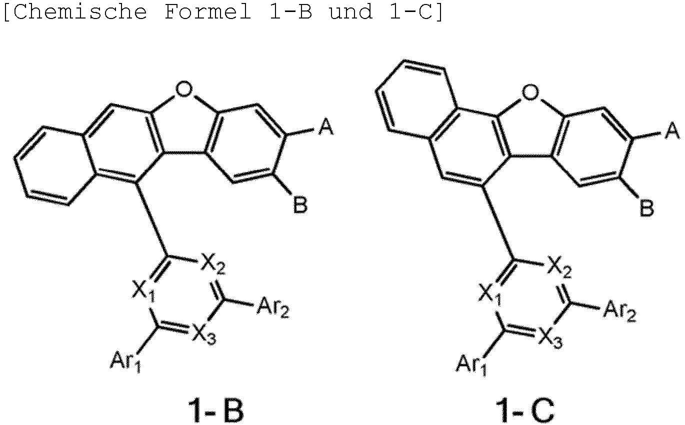
worin in den chemischen Formeln 1-B und 1-C,
X₁ bis X₃ jeweils unabhängig N oder CR₅' sind, mit der Maßgabe, dass mindestens eines von X₁ bis X₃ N ist,
Ar₁ und Ar₂ jeweils unabhängig ein substituiertes oder unsubstituiertes C₆₋₆₀-Aryl; oder ein substituiertes oder unsubstituiertes C₂₋₆₀-Heteroaryl, das mindestens eines von O, N, Si und S enthält, sind,
R₅' Wasserstoff; Deuterium; Halogen; Hydroxy; Nitril; Nitro; Amino; ein substituiertes oder unsubstituiertes C₂₋₆₀-Alkyl; ein substituiertes oder unsubstituiertes C₂₋₆₀-Alkoxy; ein substituiertes oder unsubstituiertes C₂₋₆₀-Alkenyl; ein substituiertes oder unsubstituiertes C₆₋₆₀-Aryl; oder ein substituiertes oder unsubstituiertes C₂₋₆₀-Heteroaryl, das mindestens eines von O, N, Si und S enthält, ist,
eines von A und B ein Substituent ist, der durch die folgende chemische Formel 1-1 dargestellt wird, und das andere Wasserstoff oder Deuterium ist, 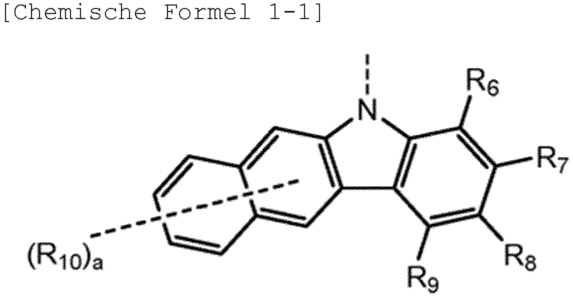
worin in der Chemischen Formel 1-1,
R₆ bis R₁₀ jeweils unabhängig Wasserstoff; Deuterium; Halogen; Hydroxy; Nitril; Nitro; Amino; ein substituiertes oder unsubstituiertes C₂₋₆₀-Alkyl; ein substituiertes oder unsubstituiertes C₂₋₆₀-Alkoxy; ein substituiertes oder unsubstituiertes C₂₋₆₀-Alkenyl; ein substituiertes oder unsubstituiertes C₆₋₆₀-Aryl; ein substituiertes oder unsubstituiertes C₂₋₆₀-Heteroaryl, das mindestens eines von O, N, Si und S enthält, sind, oder R₆ bis R₉ mit einer benachbarten Gruppe unter Bildung eines kondensierten Rings kombinieren,
a eine ganze Zahl von 1 bis 6 ist, 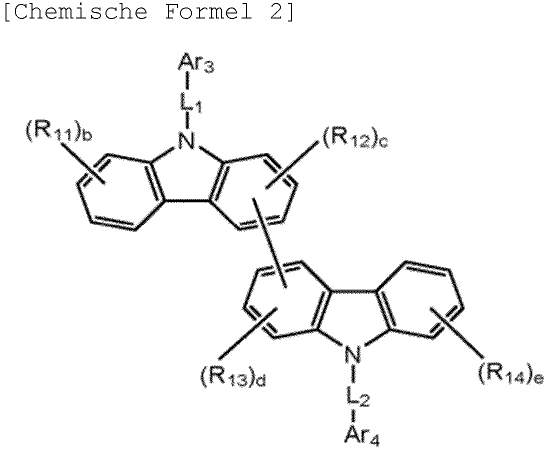
worin in der chemischen Formel 2,
Ar₃ und Ar₄ jeweils unabhängig ein substituiertes oder unsubstituiertes C₆₋₆₀-Aryl; oder ein substituiertes oder unsubstituiertes C₂₋₆₀-Heteroaryl, das mindestens eines von O, N, Si und S enthält, sind,
L₁ und L₂ jeweils unabhängig eine Einfachbindung; oder ein substituiertes oder unsubstituiertes C₆₋₆₀-Arylen sind,
R₁₁ bis R₁₄ jeweils unabhängig Wasserstoff; Deuterium; Halogen; Hydroxy; Nitril; Nitro; Amino; ein substituiertes oder unsubstituiertes C2-60-Alkyl; ein substituiertes oder unsubstituiertes C2-60-Alkoxy; ein substituiertes oder unsubstituiertes C2-60-Alkenyl; ein substituiertes oder unsubstituiertes C6-60-Aryl; ein substituiertes oder unsubstituiertes C2-60-Heteroaryl, das mindestens eines von O, N, Si und S enthält, sind,
b und e jeweils unabhängig eine ganze Zahl von 1 bis 4 sind, und
c und d jeweils unabhängig eine ganze Zahl von 1 bis 3 sind.

2. Organische lichtemittierende Vorrichtung, umfassend:
eine Anode; eine Kathode, die gegenüber der Anode vorgesehen ist; und eine oder mehrere Schichten aus organischem Material, die zwischen der Anode und der Kathode vorgesehen sind,
wobei die Schicht aus organischem Material eine durch die folgende chemische Formel 1-A dargestellte Verbindung, und eine durch die folgende chemische Formel 2 dargestellte Verbindung enthält: 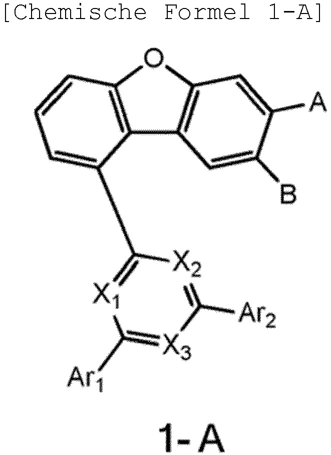
worin in der chemischen Formel 1-A,
X₁ bis X₃ jeweils unabhängig N oder CR₅' sind, mit der Maßgabe, dass mindestens eines von X₁ bis X₃ N ist,
Ar₁ und Ar₂ jeweils unabhängig ein substituiertes oder unsubstituiertes C₆₋₆₀-Aryl; oder ein substituiertes oder unsubstituiertes C₂₋₆₀-Heteroaryl, das mindestens eines von O, N, Si und S enthält, sind,
R₅' Wasserstoff; Deuterium; Halogen; Hydroxy; Nitril; Nitro; Amino; ein substituiertes oder unsubstituiertes C₂₋₆₀-Alkyl; ein substituiertes oder unsubstituiertes C₂₋₆₀-Alkoxy; ein substituiertes oder unsubstituiertes C₂₋₆₀-Alkenyl; ein substituiertes oder unsubstituiertes C₆₋₆₀-Aryl; oder ein substituiertes oder unsubstituiertes C₂₋₆₀-Heteroaryl, das mindestens eines von O, N, Si und S enthält, ist,
eines von A und B ein Substituent ist, der ausgewählt ist aus einer Gruppe bestehend aus den folgenden Substituenten, und der andere Wasserstoff oder Deuterium ist, 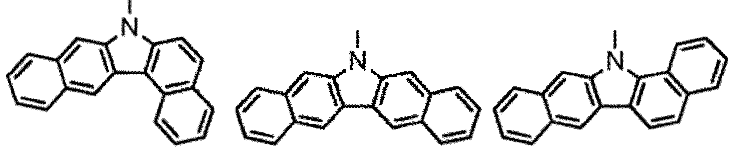 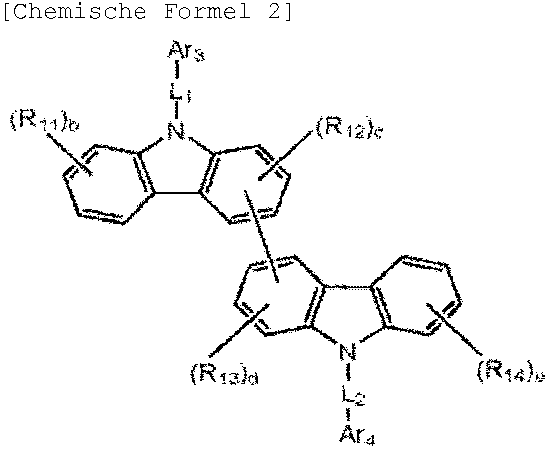
worin in der chemischen Formel 2,
Ar₃ und Ar₄ jeweils unabhängig ein substituiertes oder unsubstituiertes C₆₋₆₀-Aryl; oder ein substituiertes oder unsubstituiertes C₂₋₆₀-Heteroaryl, das mindestens eines von O, N, Si und S enthält, sind,
L₁ und L₂ jeweils unabhängig eine Einfachbindung; oder ein substituiertes oder unsubstituiertes C₆₋₆₀-Arylen sind,
R₁₁ bis R₁₄ jeweils unabhängig Wasserstoff; Deuterium; Halogen; Hydroxy; Nitril; Nitro; Amino; ein substituiertes oder unsubstituiertes C₂₋₆₀-Alkyl; ein substituiertes oder unsubstituiertes C₂₋₆₀-Alkoxy; ein substituiertes oder unsubstituiertes C₂₋₆₀-Alkenyl; ein substituiertes oder unsubstituiertes C₆₋₆₀-Aryl; ein substituiertes oder unsubstituiertes C₂₋₆₀-Heteroaryl, das mindestens eines von O, N, Si und S enthält, sind,
b und e jeweils unabhängig eine ganze Zahl von 1 bis 4 sind, und
c und d jeweils unabhängig eine ganze Zahl von 1 bis 3 sind.

3. Organische lichtemittierende Vorrichtung gemäß Anspruch 1 oder 2,
worin X₁ bis X₃ alle N sind.

4. Organische lichtemittierende Vorrichtung gemäß Anspruch 1 oder 2,
worin Ar₁ und Ar₂ jeweils unabhängig ausgewählt sind aus einer Gruppe bestehend aus:

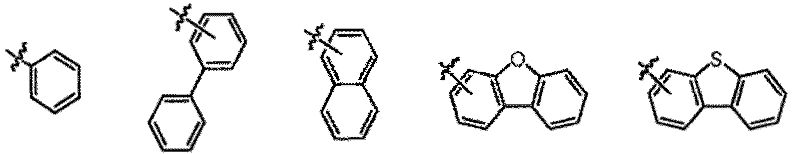

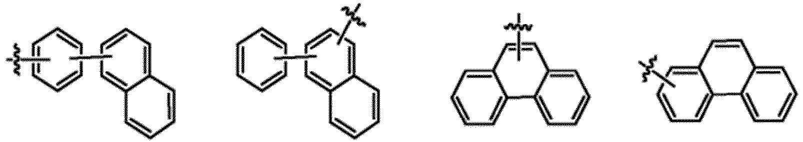

5. Organische lichtemittierende Vorrichtung gemäß Anspruch 1,
worin die chemische Formel 1-1 ausgewählt ist aus einer Gruppe bestehend aus den folgenden Substituenten:

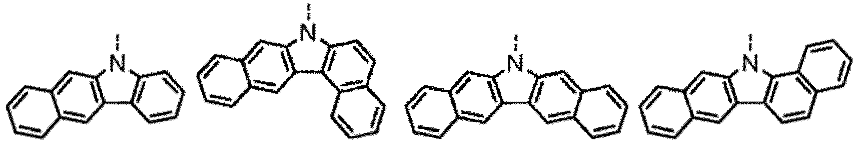

6. Organische lichtemittierende Vorrichtung, umfassend:
eine Anode; eine Kathode, die gegenüber der Anode vorgesehen ist; und eine oder mehrere Schichten aus organischem Material, die zwischen der Anode und der Kathode vorgesehen sind,
wobei die Schicht aus organischem Material eine durch die folgende chemische Formel 2 dargestellte Verbindung und eine beliebige Verbindung enthält, die ausgewählt ist aus der Gruppe (G), bestehend aus den folgenden Verbindungen: 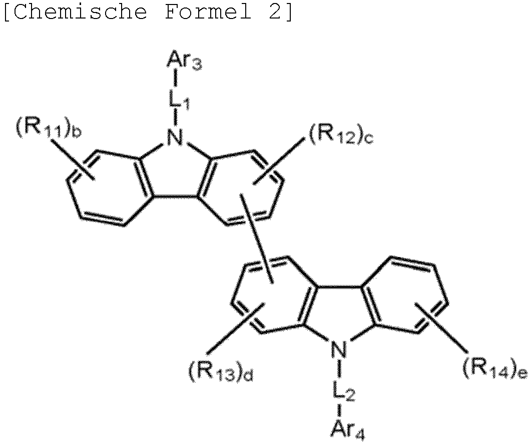
worin in der chemischen Formel 2,
Ar₃ und Ar₄ jeweils unabhängig ein substituiertes oder unsubstituiertes C₆₋₆₀-Aryl; oder ein substituiertes oder unsubstituiertes C₂₋₆₀-Heteroaryl, das mindestens eines von O, N, Si und S enthält, sind
L₁ und L₂ jeweils unabhängig eine Einfachbindung; oder ein substituiertes oder unsubstituiertes C₆₋₆₀-Arylen sind,
R₁₁ bis R₁₄ jeweils unabhängig Wasserstoff; Deuterium; Halogen; Hydroxy; Nitril; Nitro; Amino; ein substituiertes oder unsubstituiertes C₂₋₆₀-Alkyl; ein substituiertes oder unsubstituiertes C₂₋₆₀-Alkoxy; ein substituiertes oder unsubstituiertes C₂₋₆₀-Alkenyl; ein substituiertes oder unsubstituiertes C₆₋₆₀-Aryl; ein substituiertes oder unsubstituiertes C₂₋₆₀-Heteroaryl, das mindestens eines von O, N, Si und S enthält, sind,
b und e jeweils unabhängig eine ganze Zahl von 1 bis 4 sind, und
c und d jeweils unabhängig eine ganze Zahl von 1 bis 3 sind,

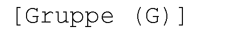

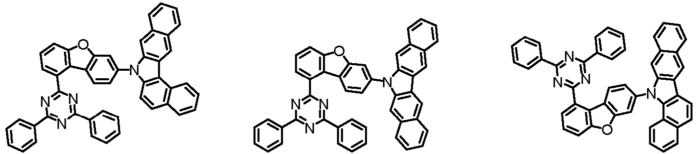

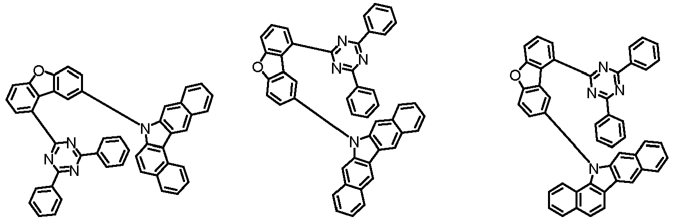

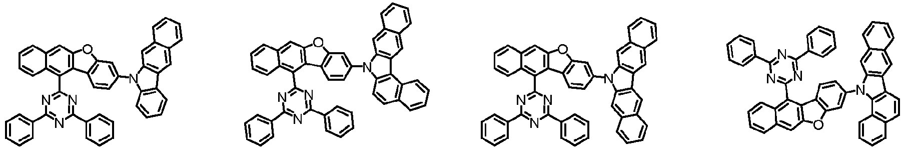

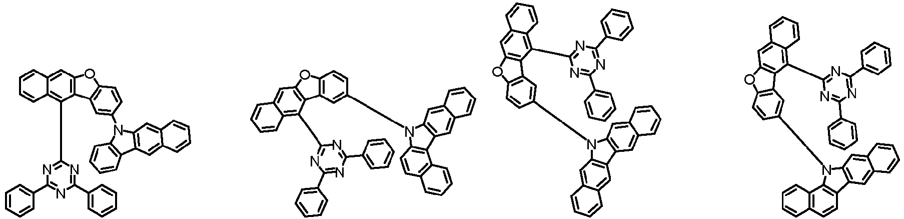

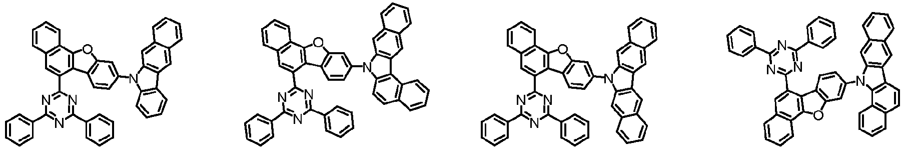

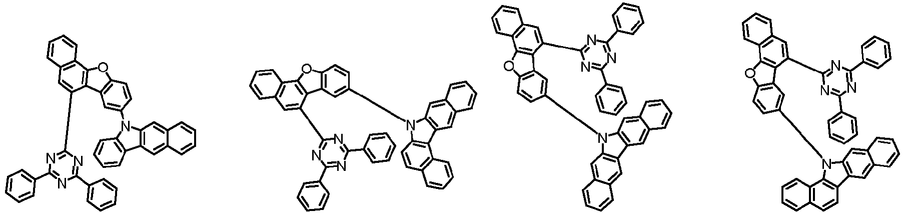

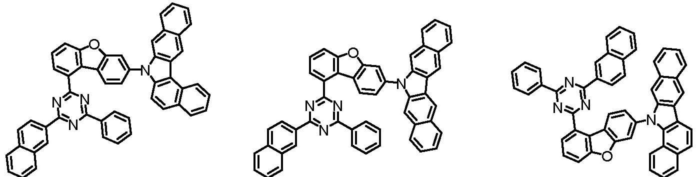

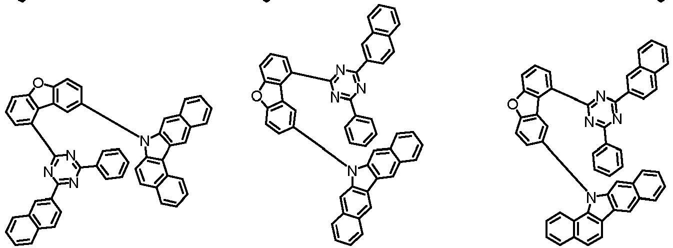

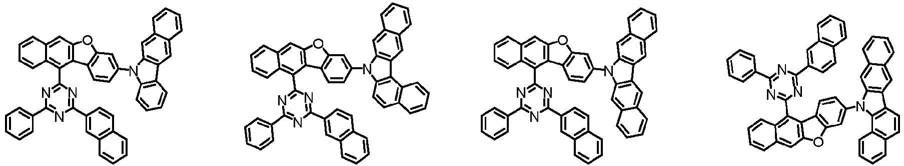

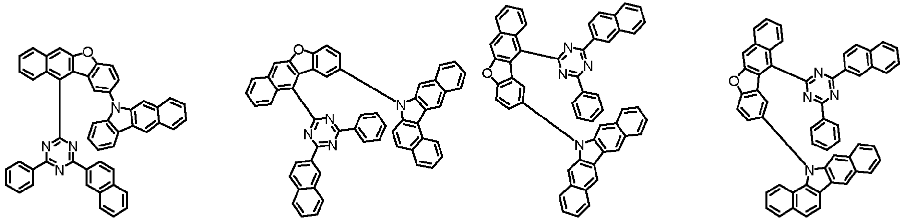

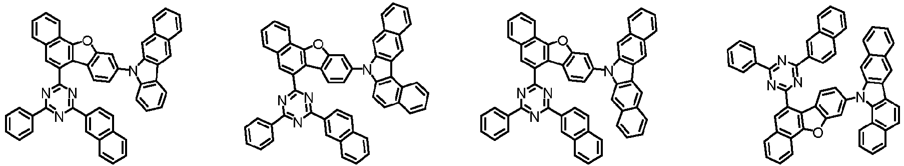

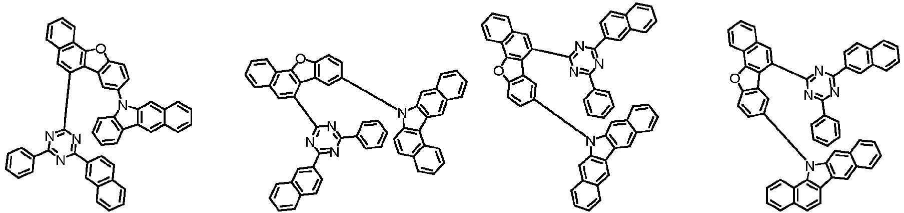

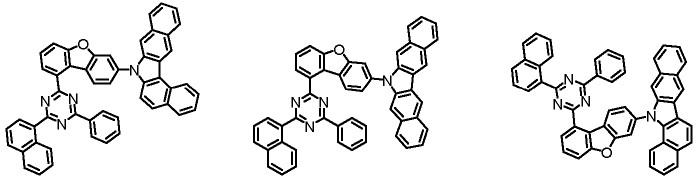

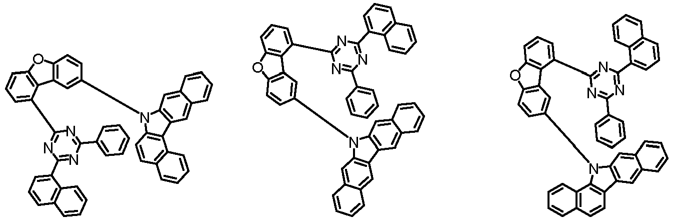

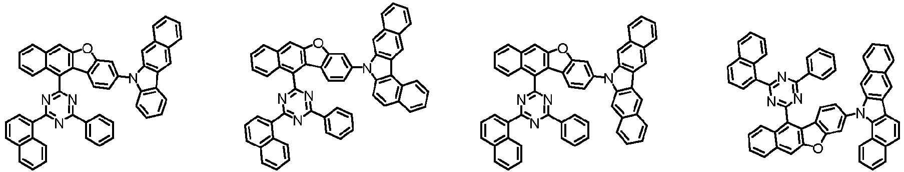

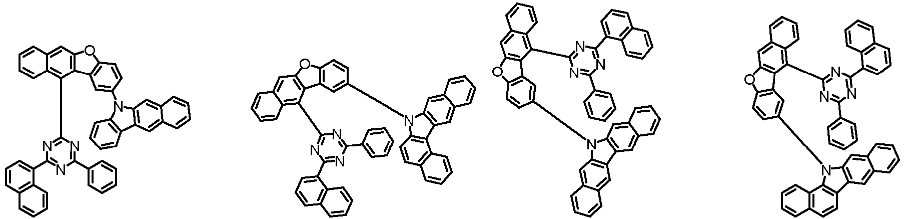

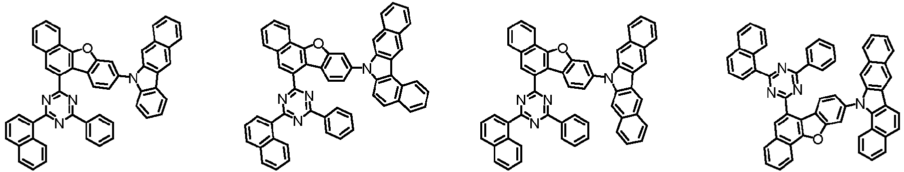

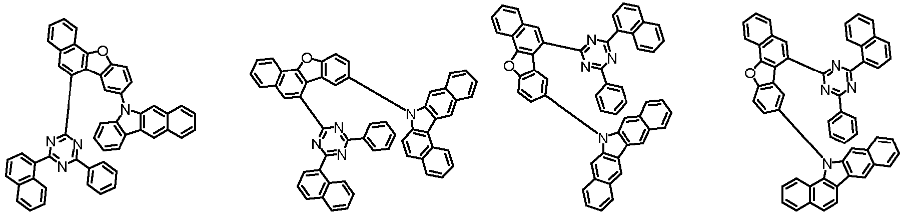

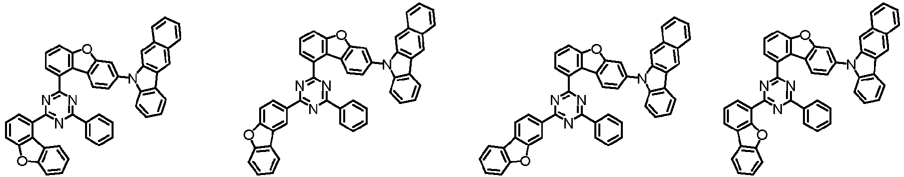

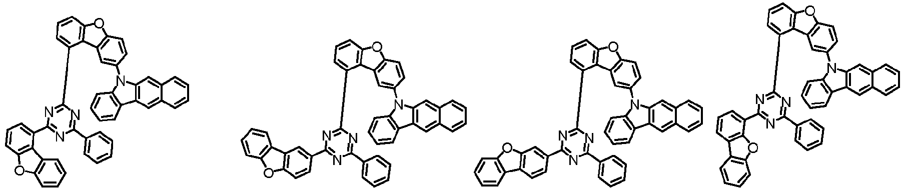

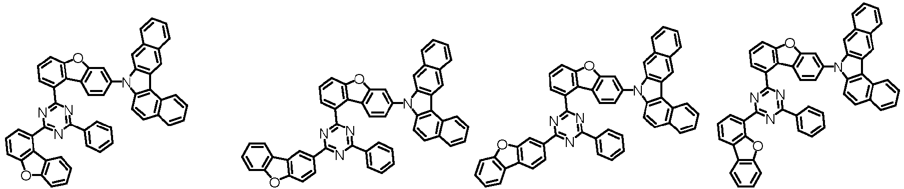

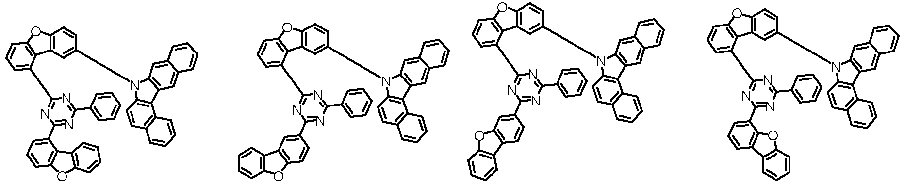

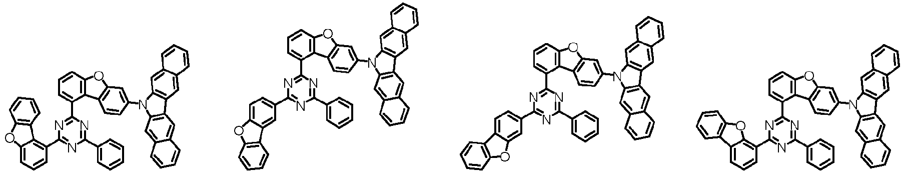

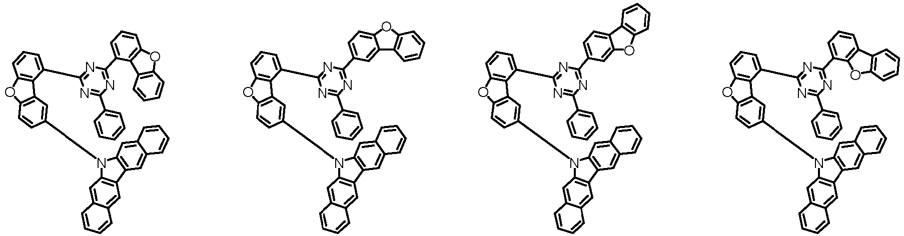

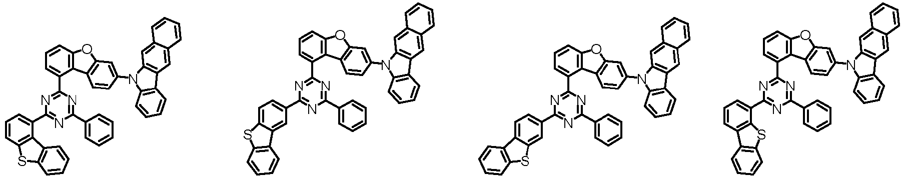

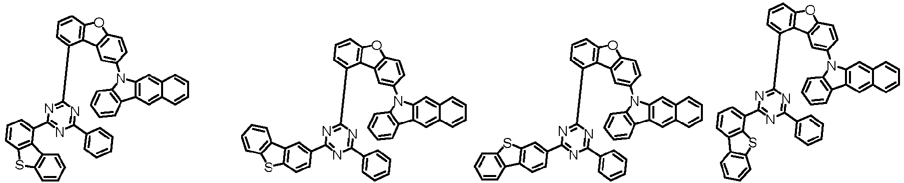

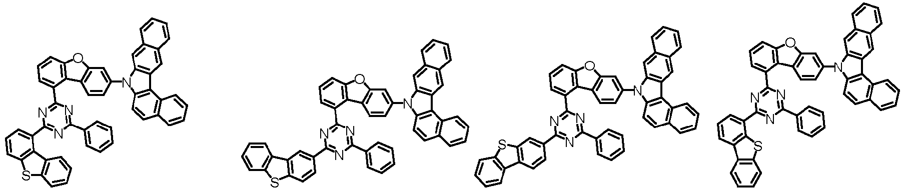

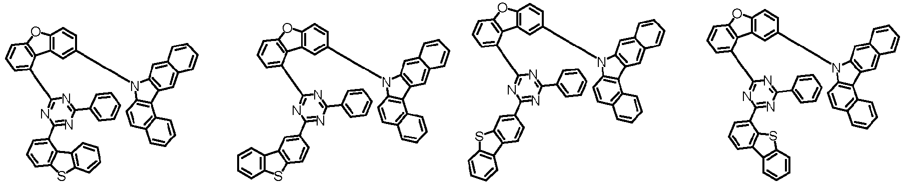

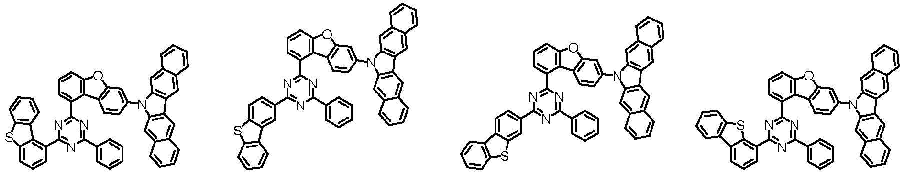

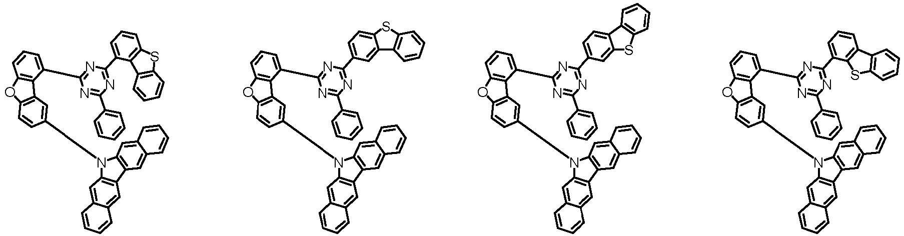

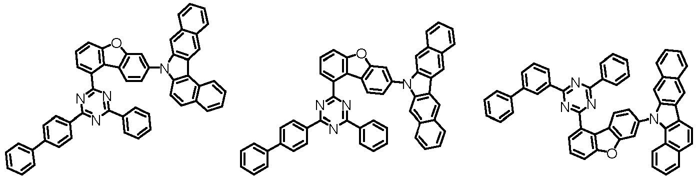

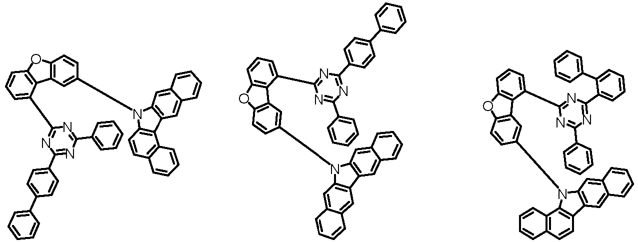

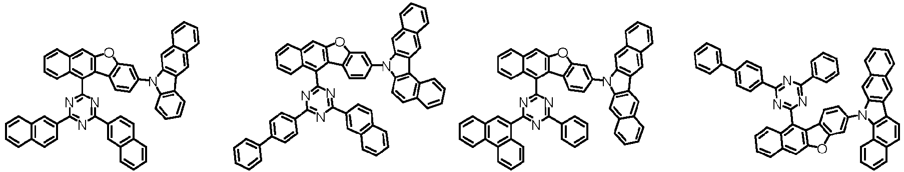

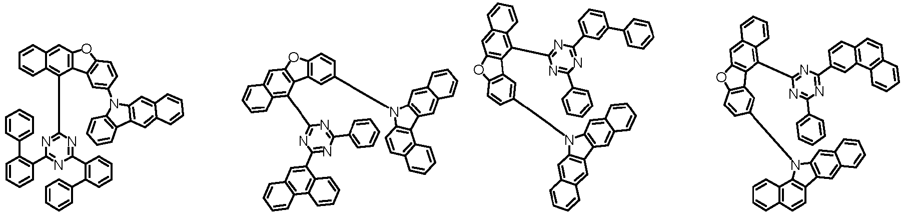

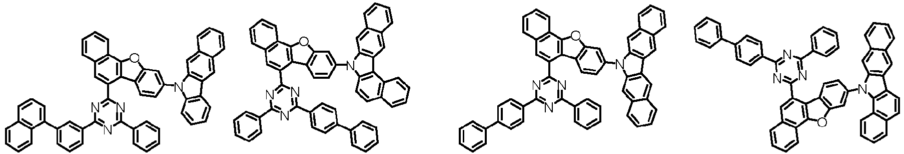

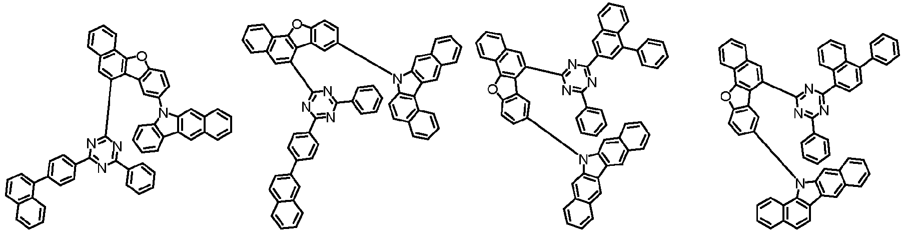

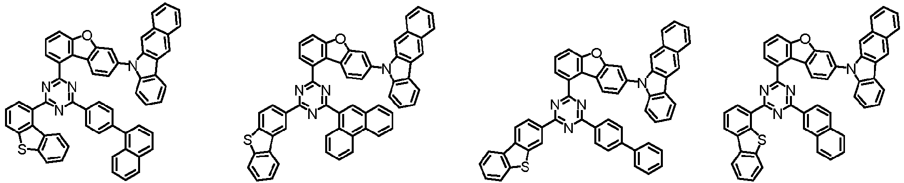

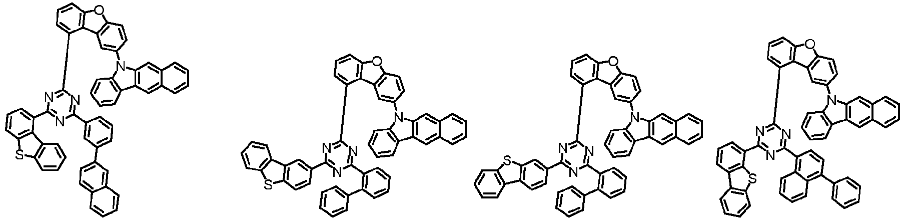

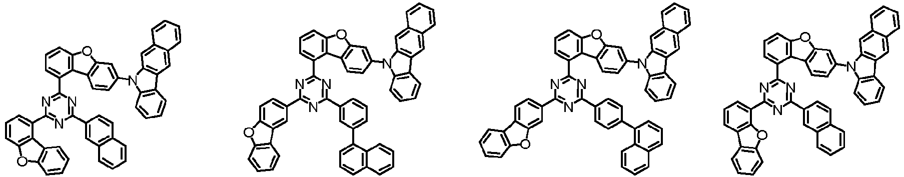

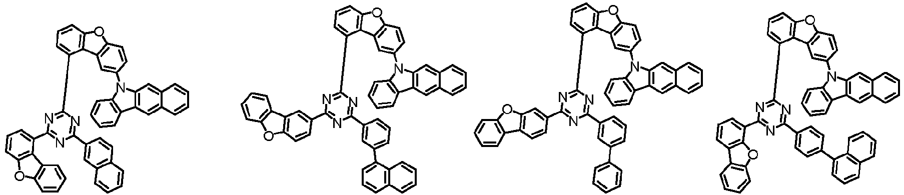

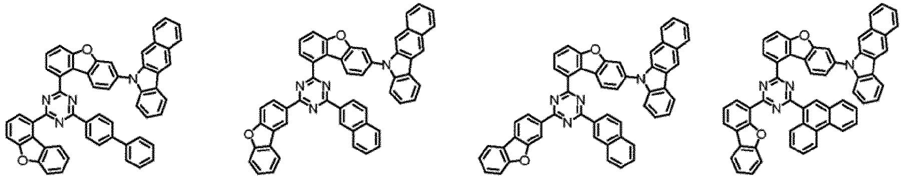

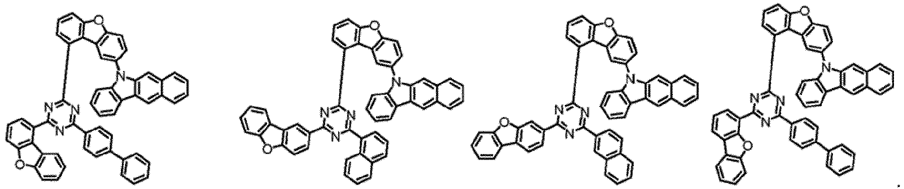

7. Organische lichtemittierende Vorrichtung gemäß Anspruch 1 oder 2,
wobei die durch die chemische Formel 2 dargestellte Verbindung eine durch die chemische Formel 2-1 dargestellte Verbindung ist: 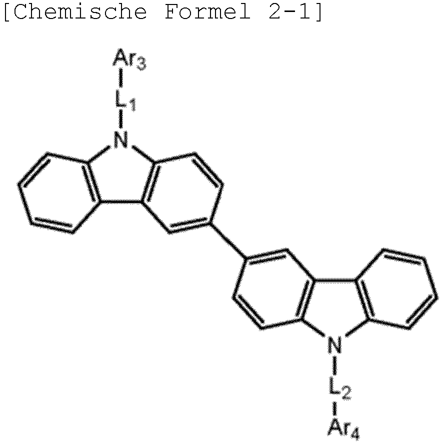
worin in der chemischen Formel 2-1,
Ar₃, Ar₄, L₁ und L₂ wie in Anspruch 1 oder 2 definiert sind.

8. Organische lichtemittierende Vorrichtung gemäß Anspruch 1 oder 2,
worin Ar₃ und Ar₄ jeweils unabhängig ausgewählt sind aus einer Gruppe bestehend aus:

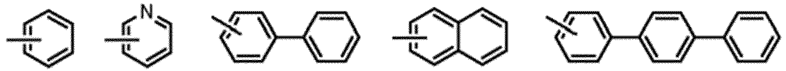

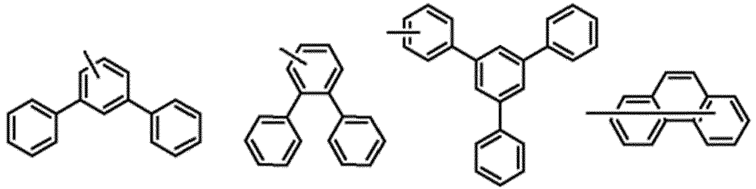

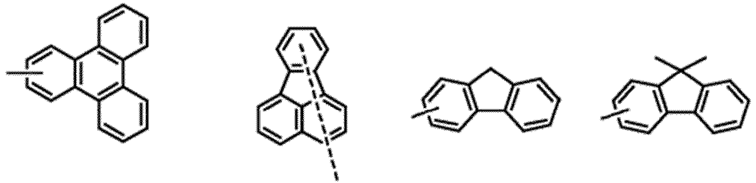

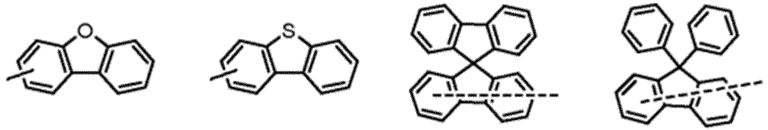

9. Organische lichtemittierende Vorrichtung gemäß Anspruch 1 oder 2,
worin L₁ und L₂ jeweils unabhängig eine Einfachbindung oder mindestens eine Verbindung sind, die ausgewählt ist aus einer Gruppe bestehend aus:

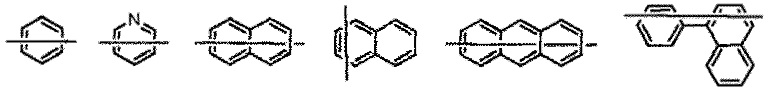

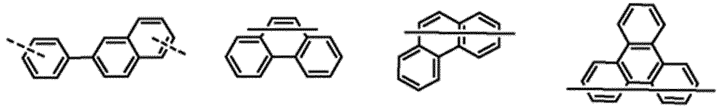

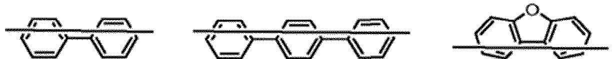

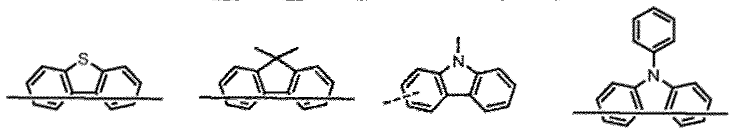

10. Organische lichtemittierende Vorrichtung gemäß Anspruch 1 oder 2,
worin R₁₁ bis R₁₄ Wasserstoff sind.

11. Organische lichtemittierende Vorrichtung gemäß Anspruch 1 oder 2,
wobei die durch die chemische Formel 2 dargestellte Verbindung ausgewählt ist aus einer Gruppe bestehend aus den folgenden Verbindungen:

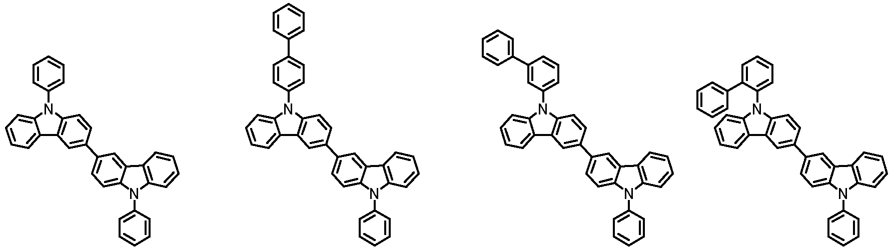

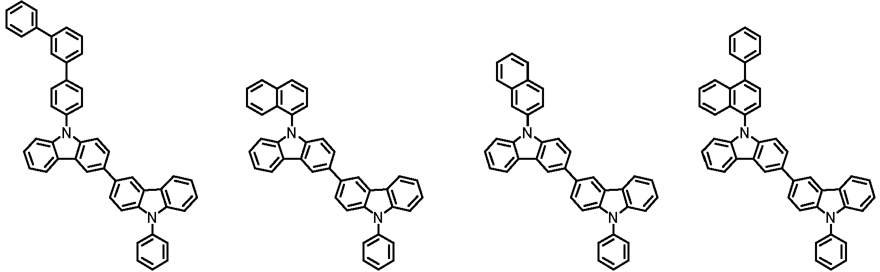

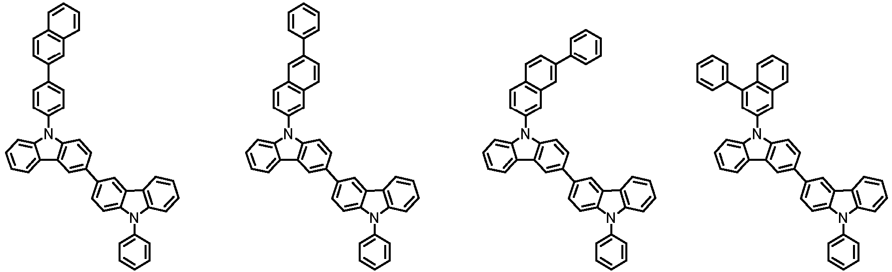

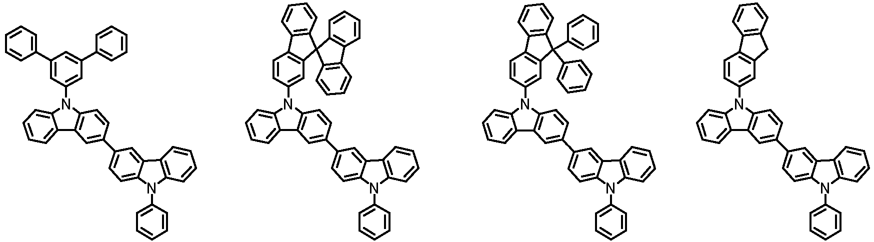

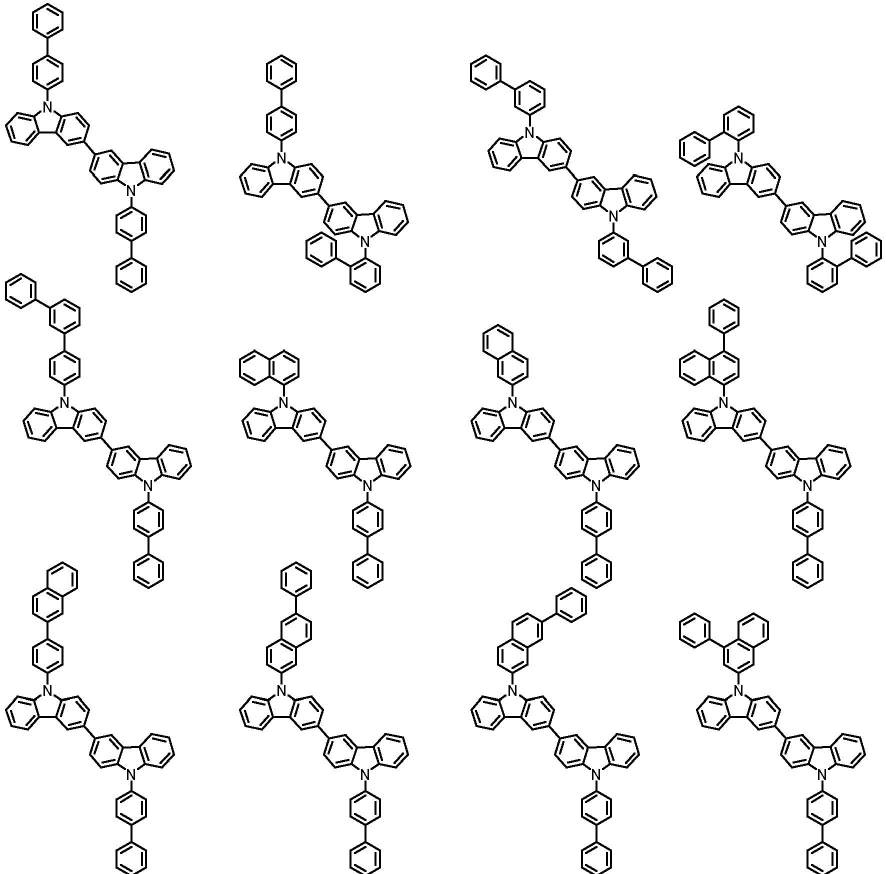

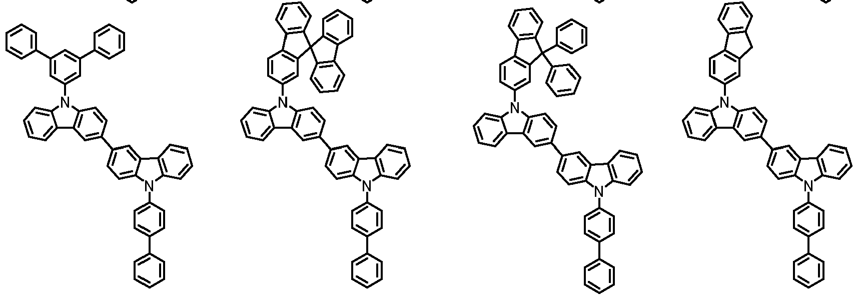

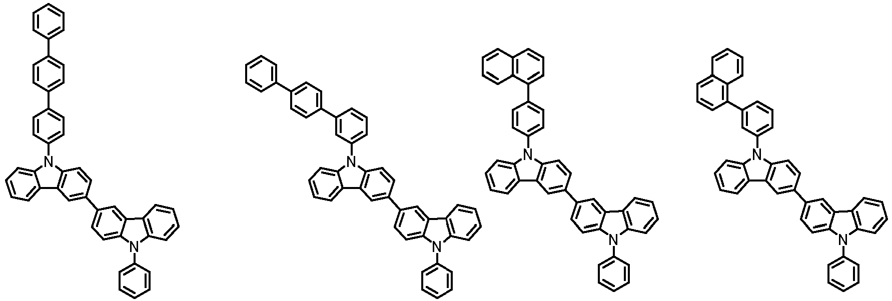

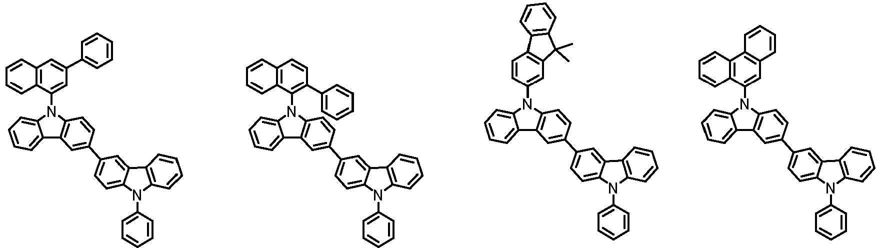

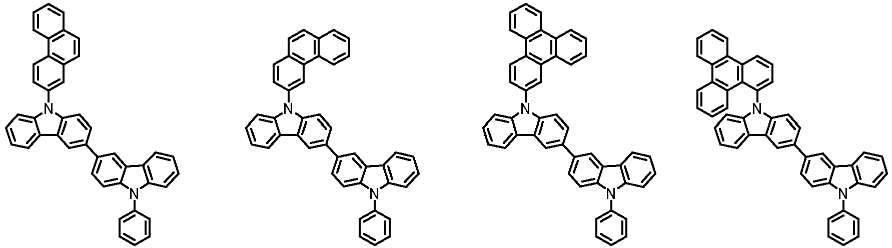

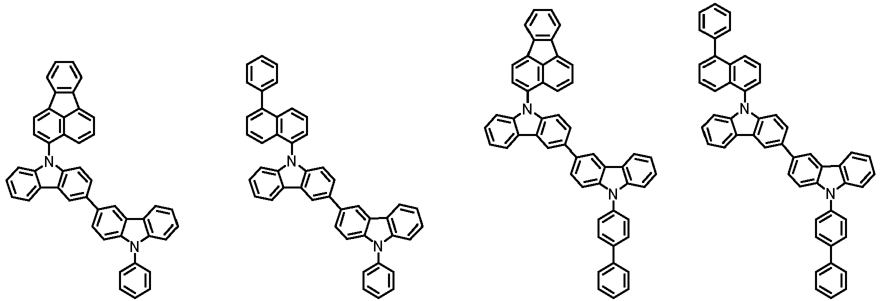

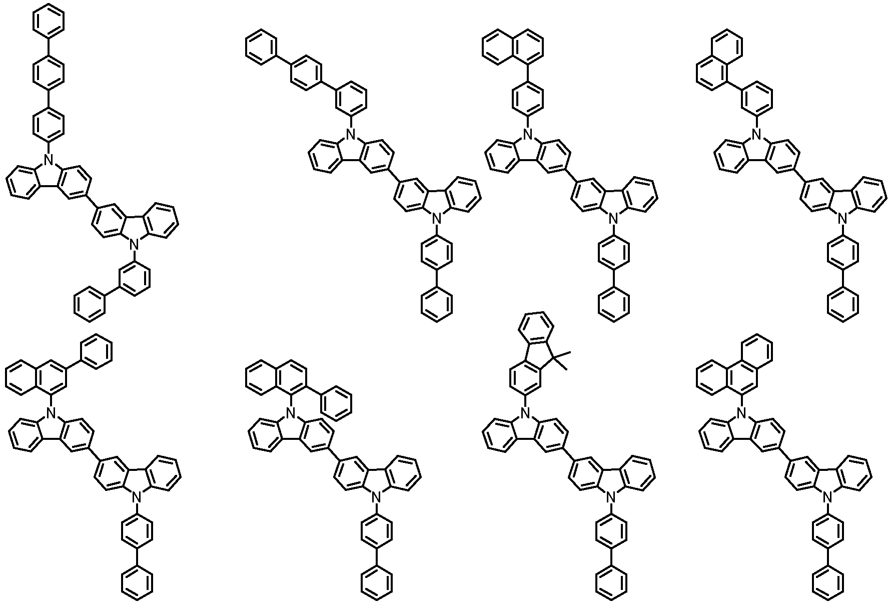

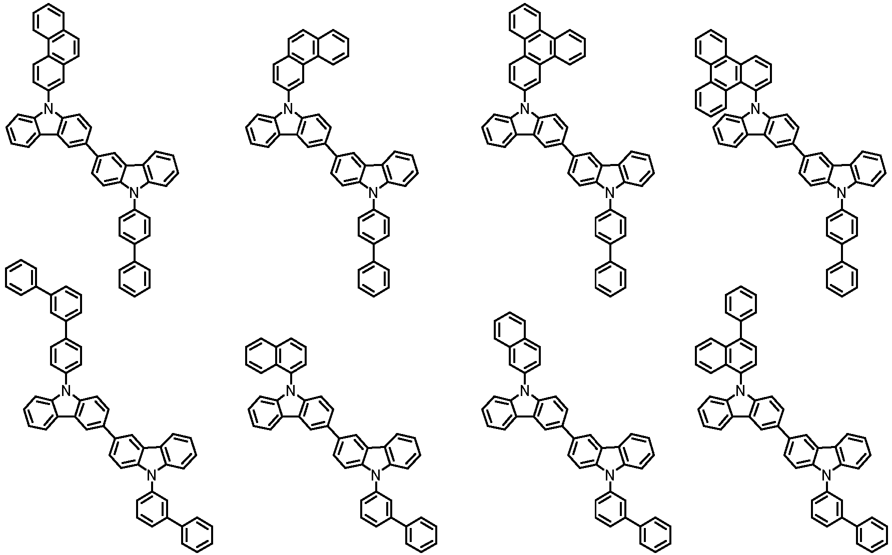

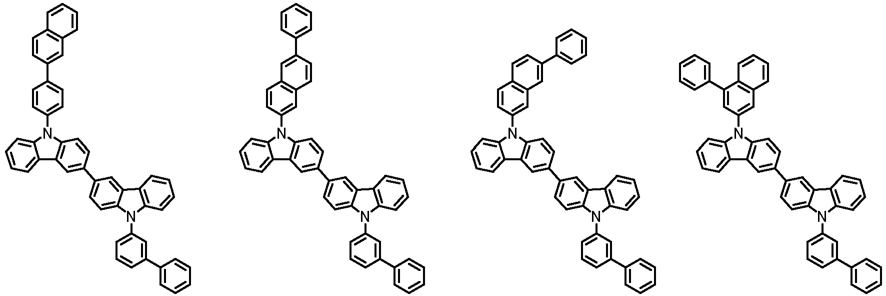

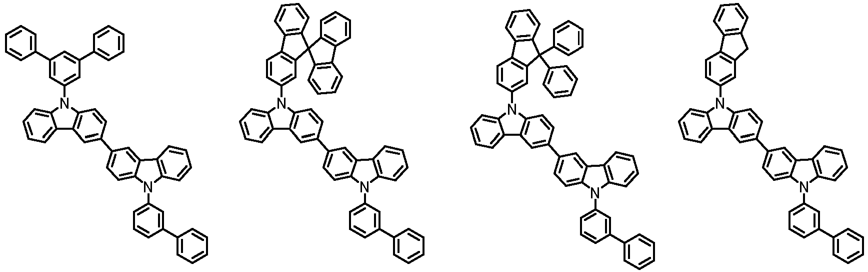

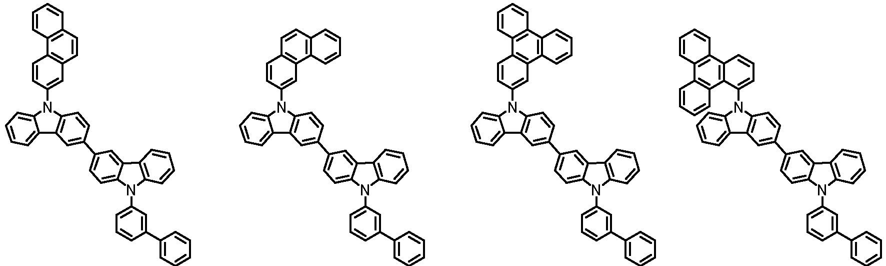

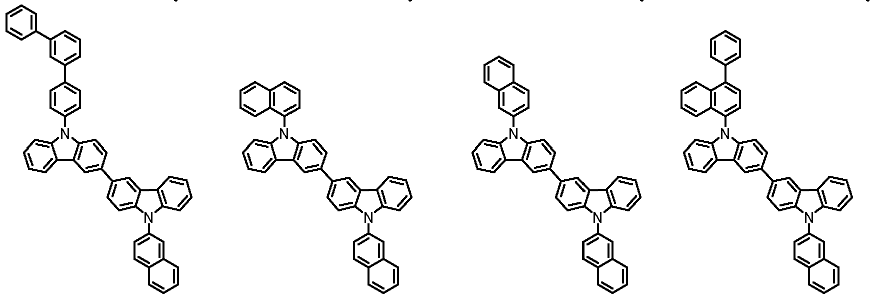

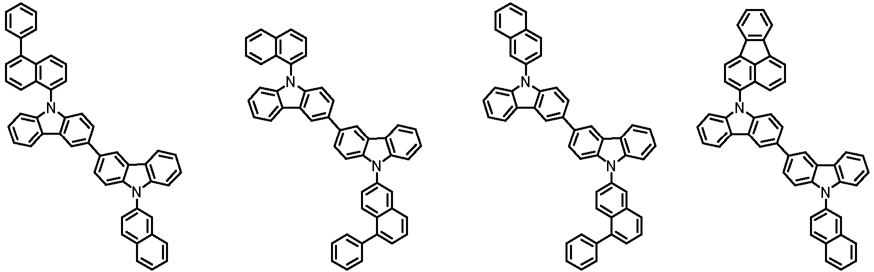

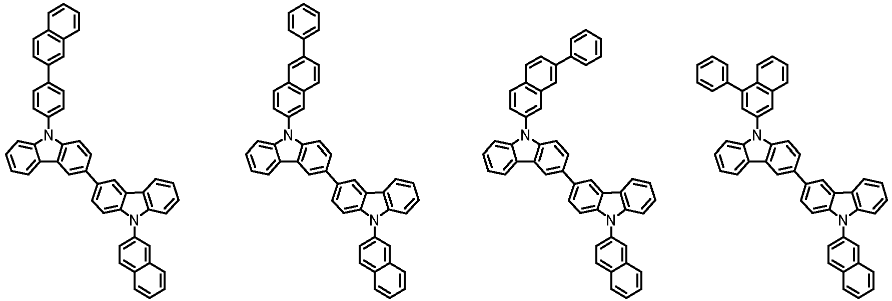

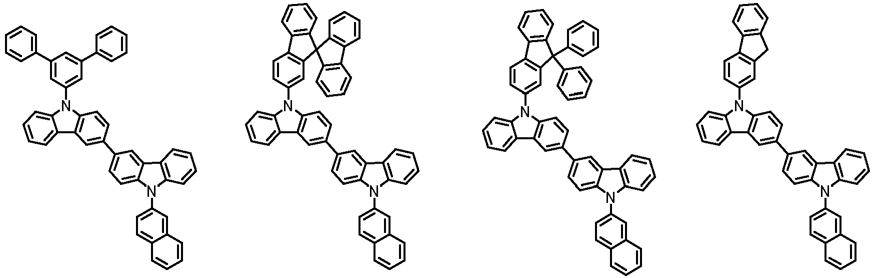

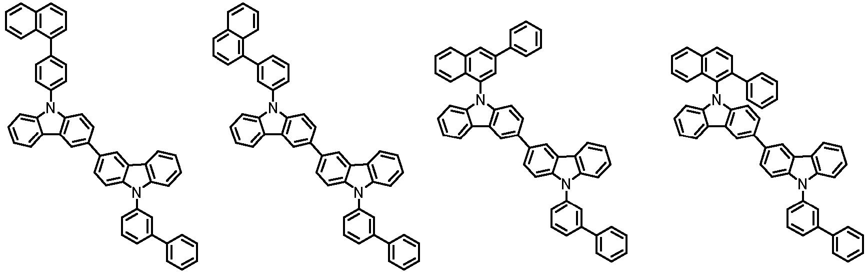

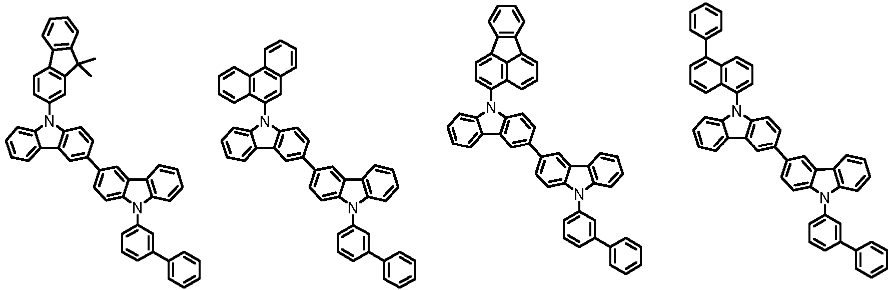

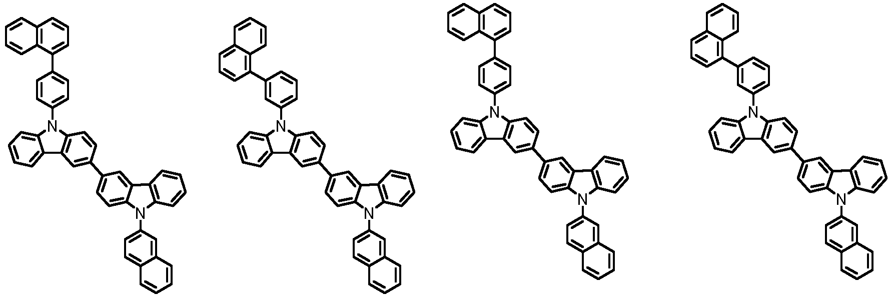

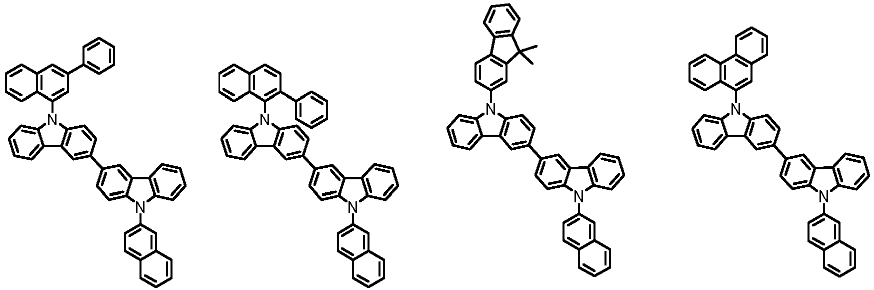

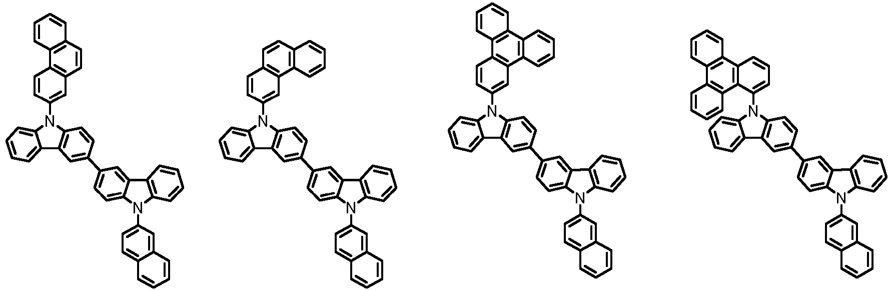

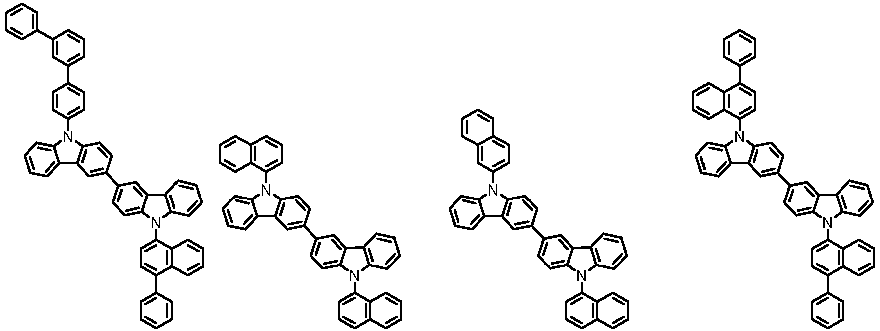

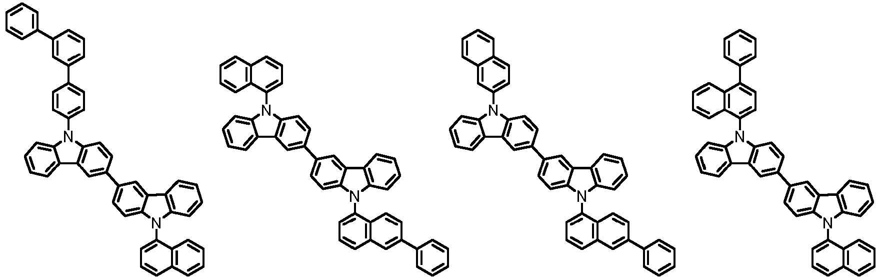

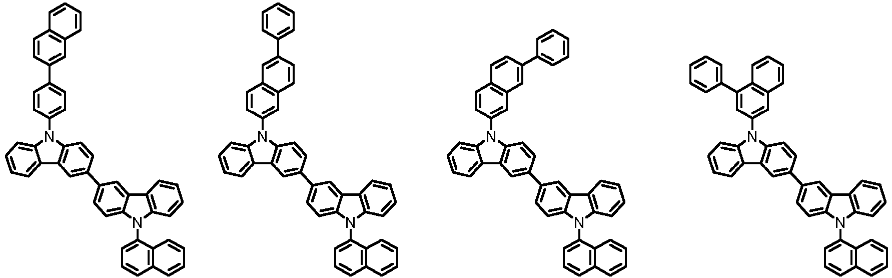

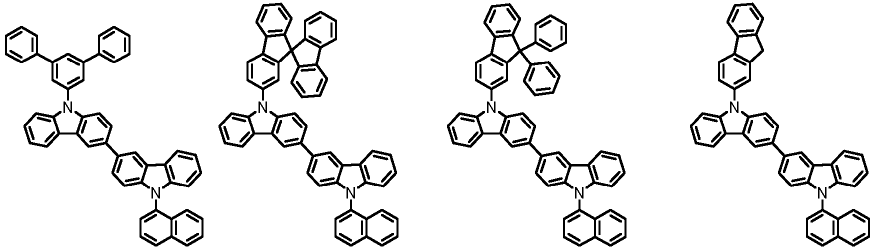

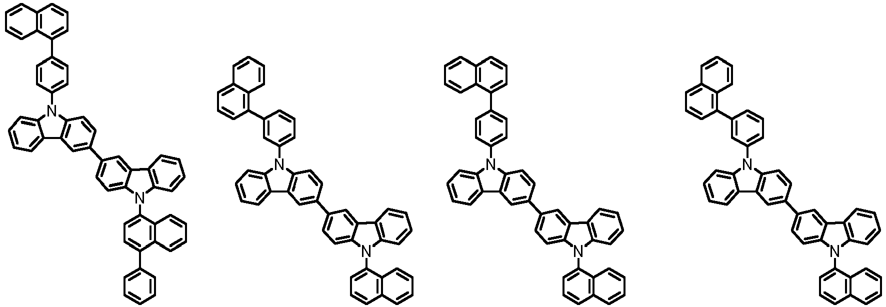

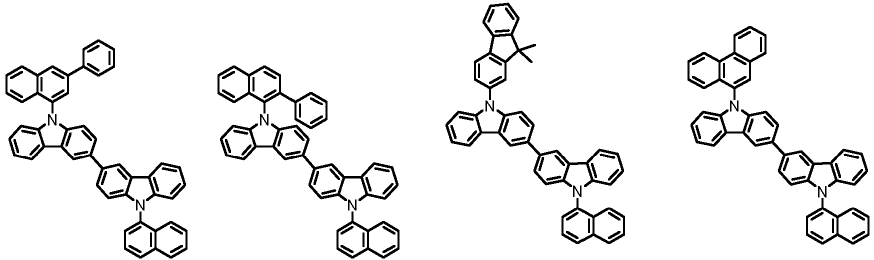

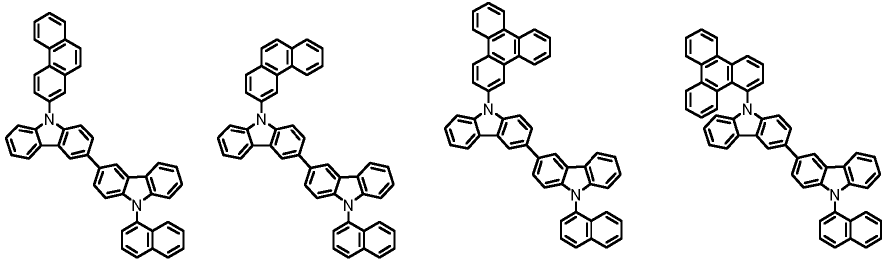

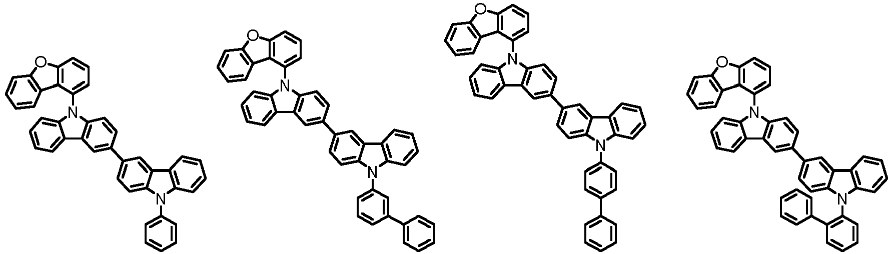

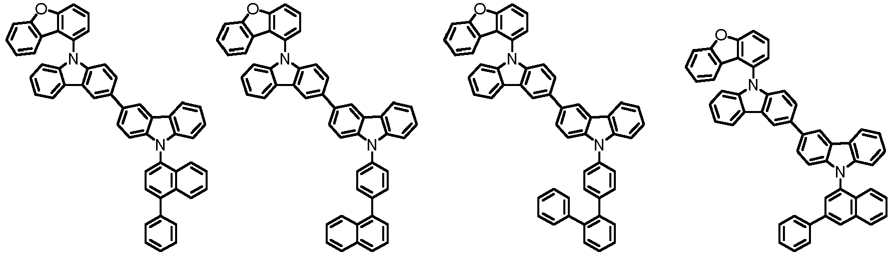

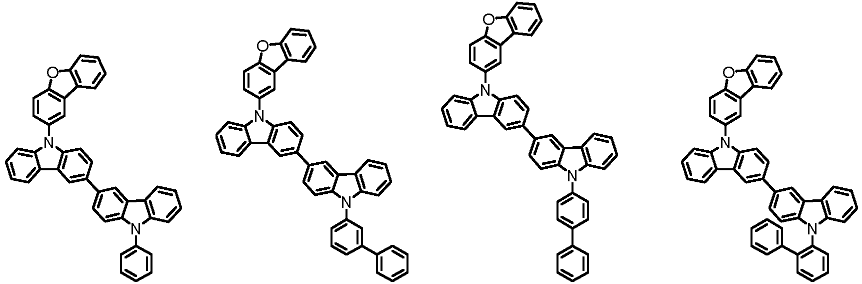

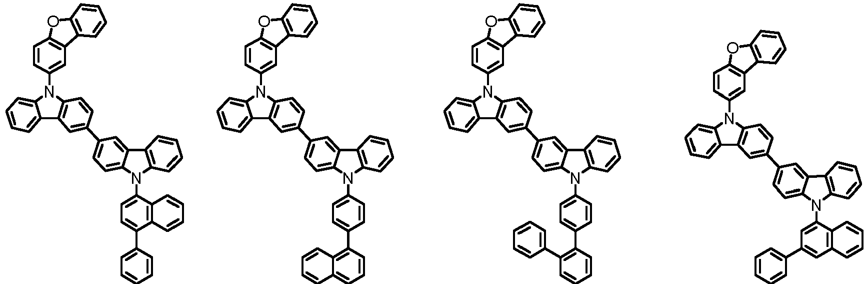

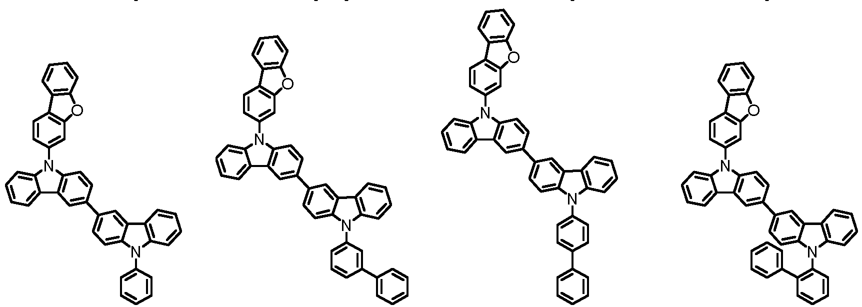

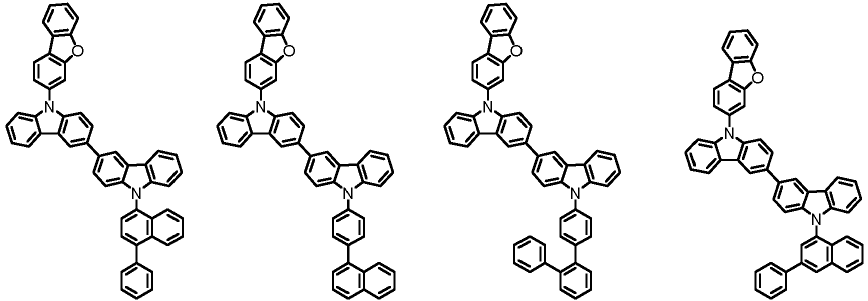

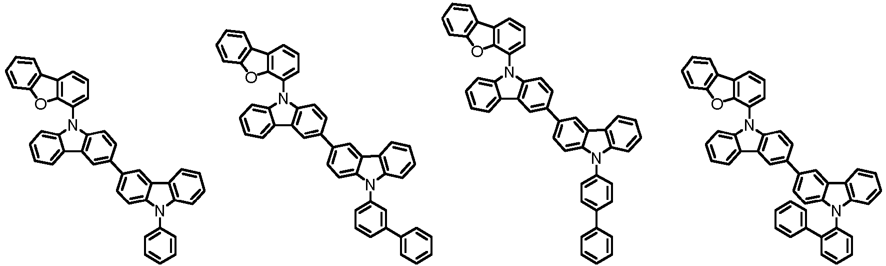

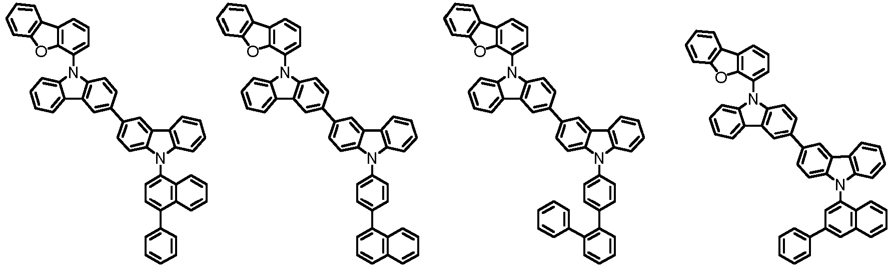

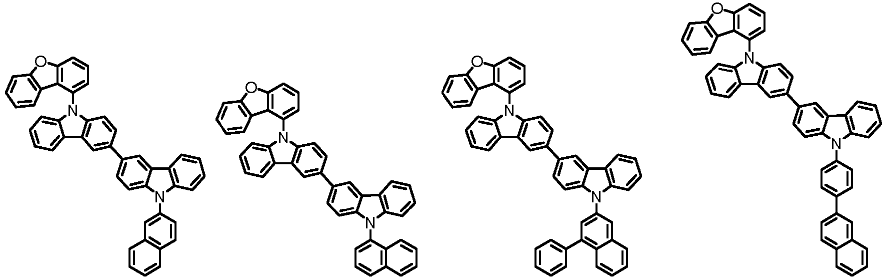

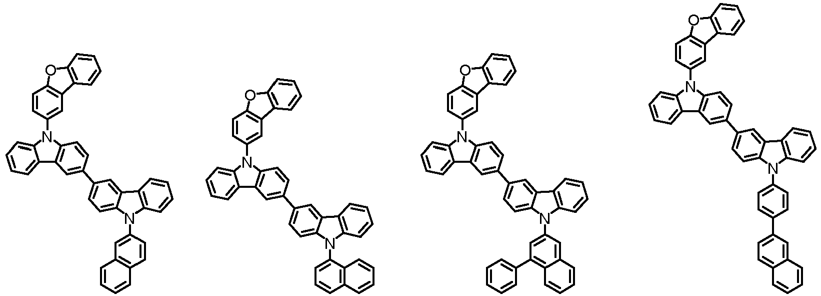

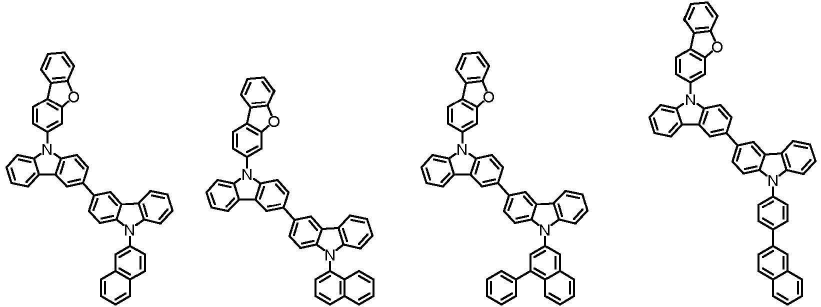

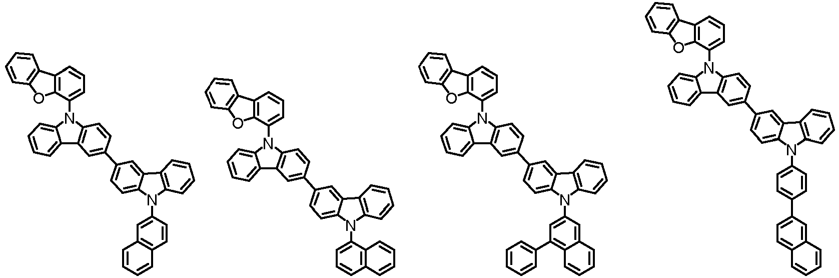

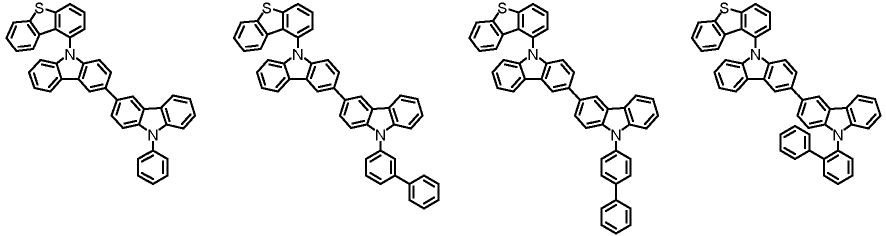

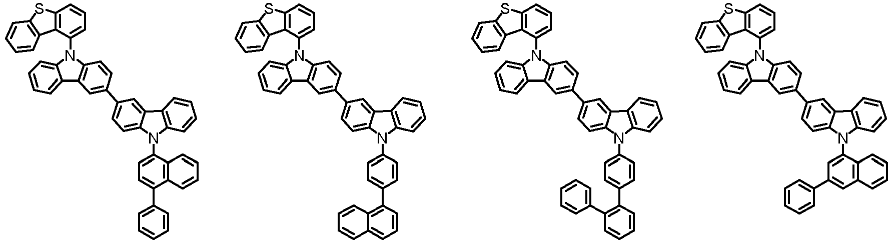

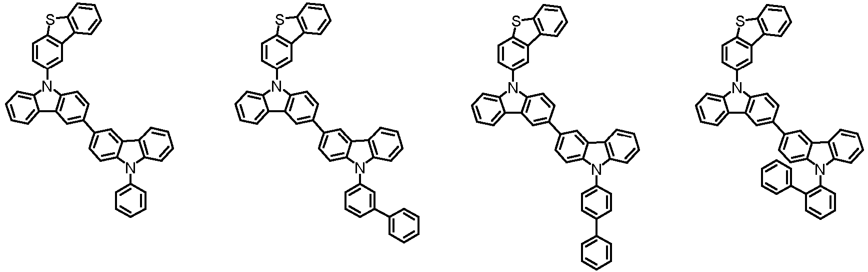

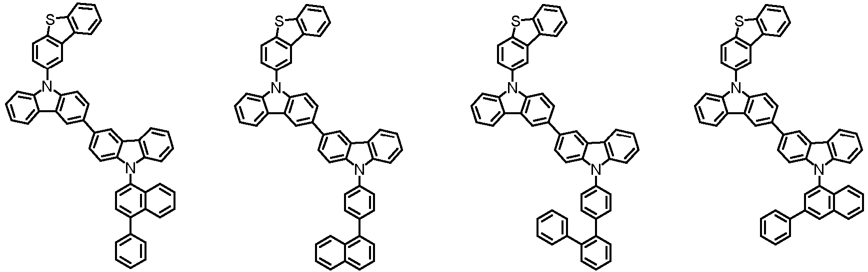

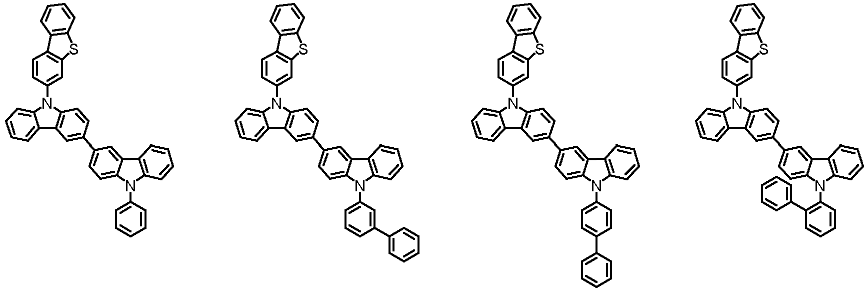

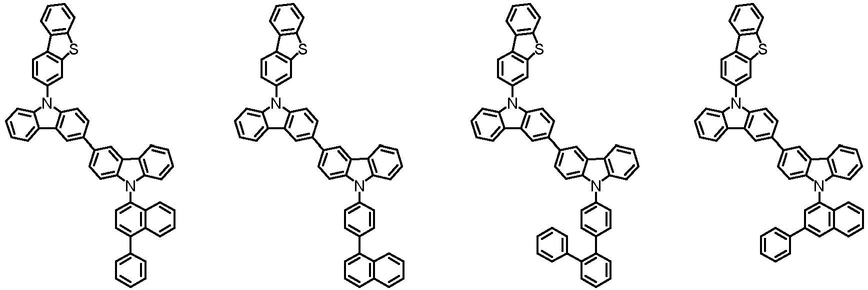

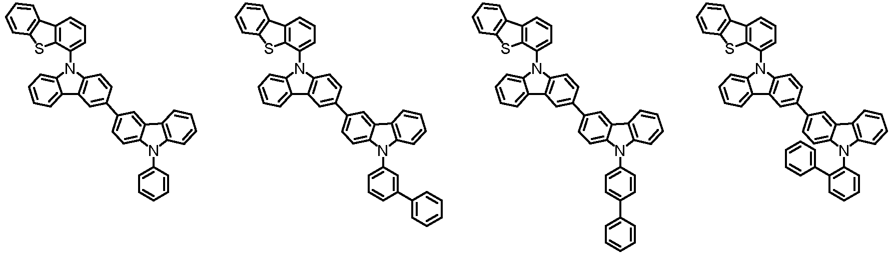

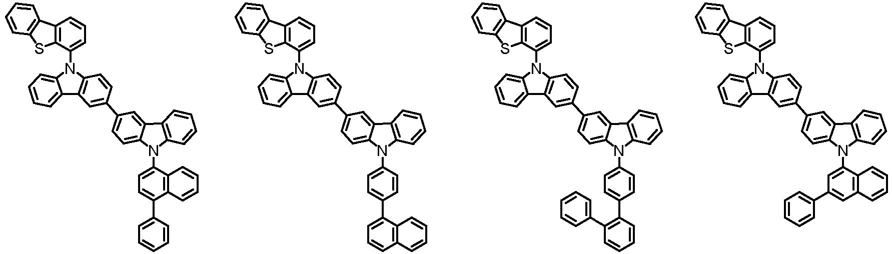

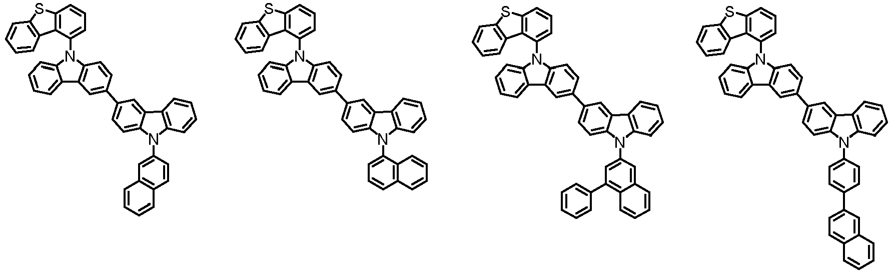

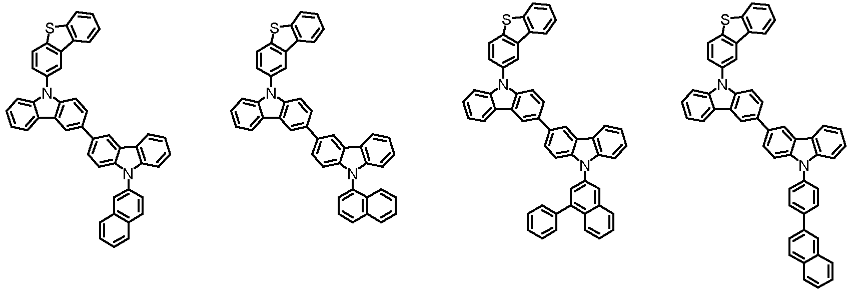

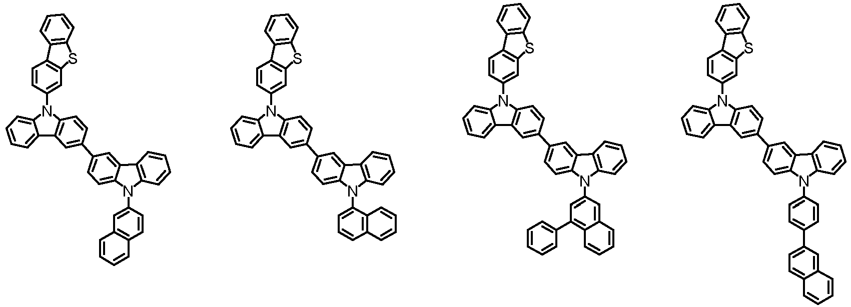

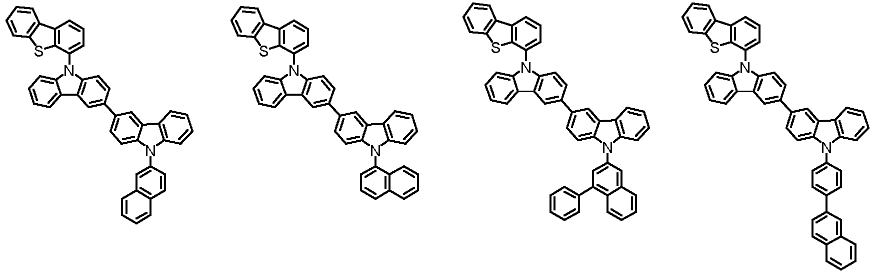

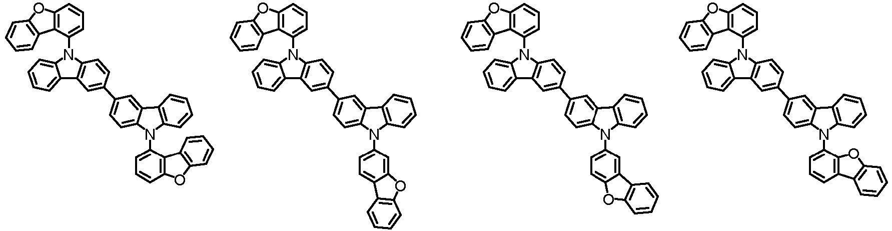

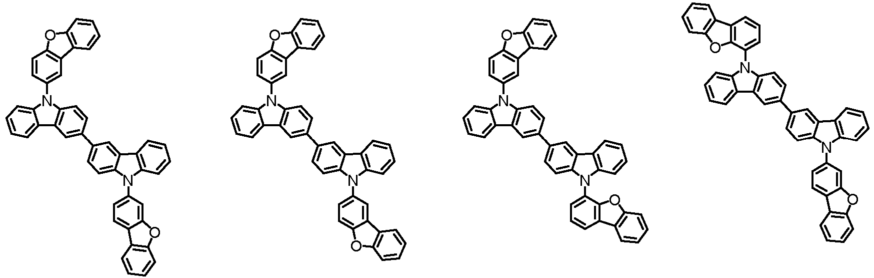

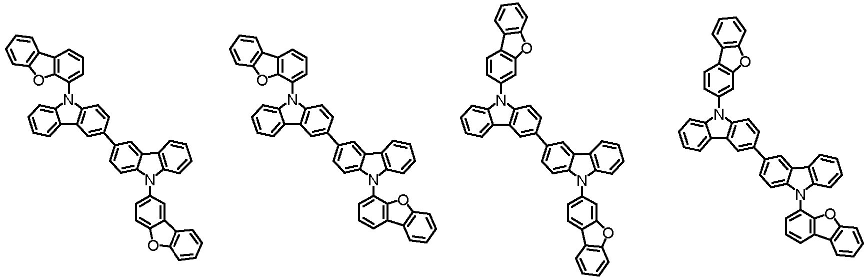

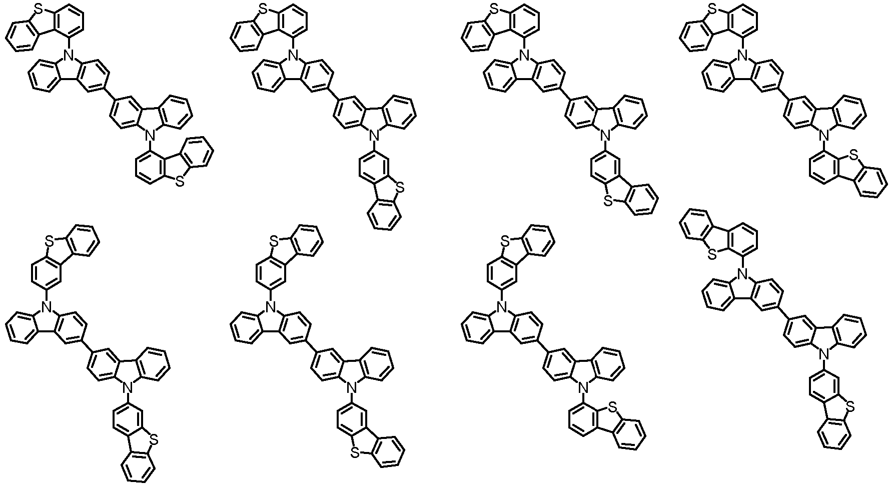

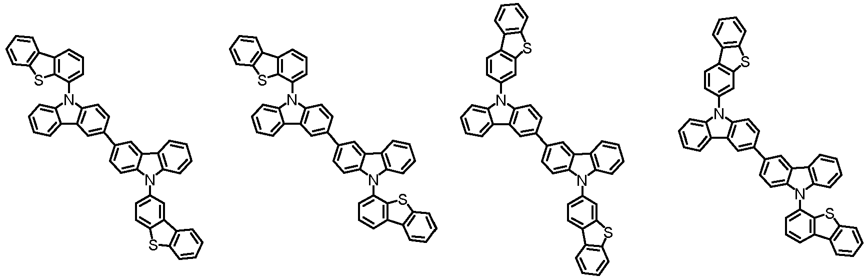

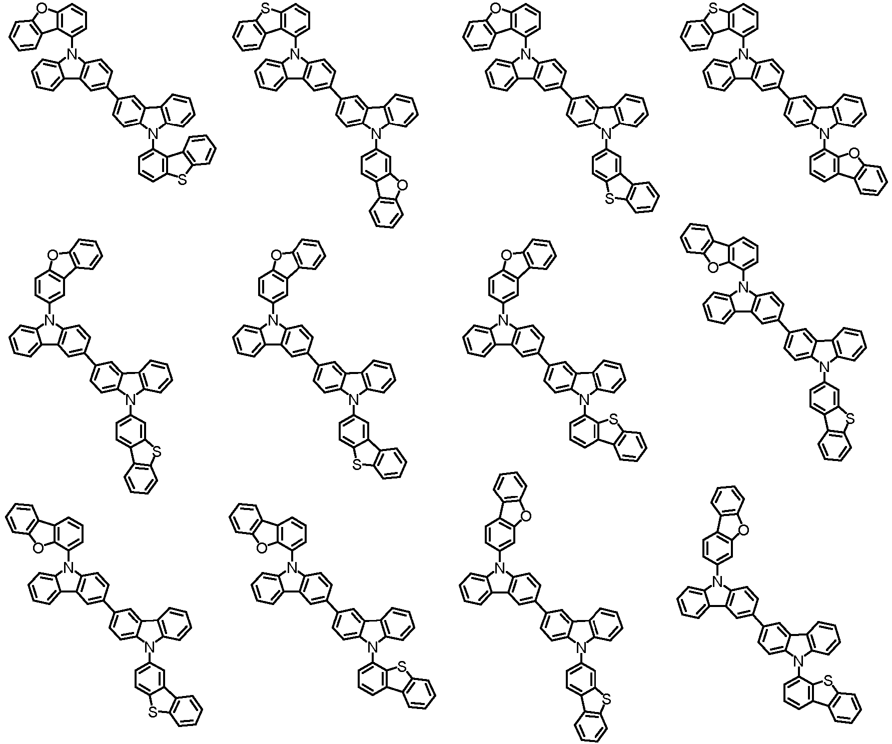

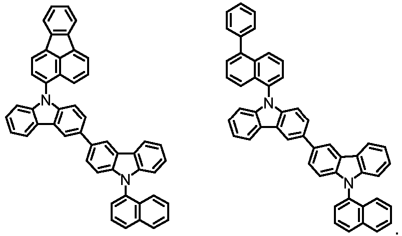

12. Organische lichtemittierende Vorrichtung gemäß Anspruch 1 oder 2,
wobei die Schicht aus organischem Material, die mindestens eine Verbindung, die ausgewählt ist aus den durch die chemischen Formeln 1-A, 1-B und 1-C dargestellten Verbindungen, und die durch die chemische Formel 2 dargestellte Verbindung, enthält, eine lichtemittierende Schicht ist.

13. Organische lichtemittierende Vorrichtung gemäß Anspruch 12,
wobei mindestens eine der Verbindungen, die ausgewählt sind aus den durch die chemischen Formeln 1-A, 1-B und 1-C dargestellten Verbindungen, und die durch die chemische Formel 2 dargestellte Verbindung, Wirtsmaterialien in der lichtemittierenden Schicht sind.

14. Organische lichtemittierende Vorrichtung gemäß Anspruch 12,
wobei die lichtemittierende Schicht ferner ein Dotiermaterial umfasst.

## Revendications

1. Dispositif électroluminescent organique comprenant :
une anode (2) ; une cathode (4) qui est fournie à l'opposé de l'anode ; et une ou plusieurs couches de matériau organique qui sont fournies entre l'anode et la cathode,
dans lequel la couche de matériau organique inclut l'un quelconque sélectionné parmi les composés représentés par les Formules chimiques 1-B et 1-C suivantes et un composé représenté par la Formule chimique 2 suivante : 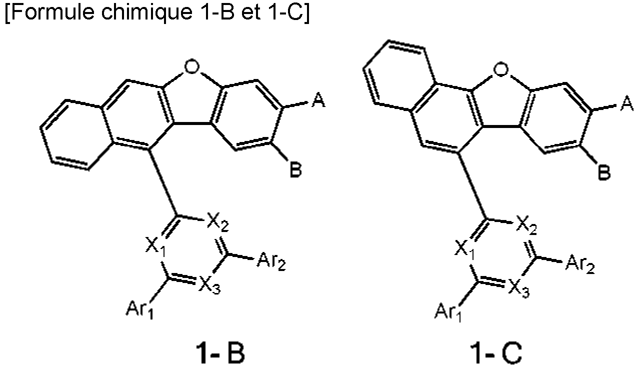
dans les Formules chimiques 1-B et 1-C,
X₁ à X₃ sont chacun indépendamment N ou CR₅', à condition qu'au moins un parmi X₁ à X₃ soit N,
Ar₁ et Ar₂ sont chacun indépendamment un aryle en C₆₋₆₀ substitué ou non substitué ; ou un hétéroaryle en C₂₋₆₀ substitué ou non substitué contenant au moins un parmi O, N, Si et S,
R₅' est un hydrogène ; un deutérium ; un halogène ; un hydroxy ; un nitrile ; un nitro ; un amino ; un alkyle en C₂₋₆₀ substitué ou non substitué ; un alcoxy en C₂₋₆₀ substitué ou non substitué ; un alcényle en C₂₋₆₀ substitué ou non substitué ; un aryle en C₆₋₆₀ substitué ou non substitué ; ou un hétéroaryle en C₂₋₆₀ substitué ou non substitué contenant au moins un parmi O, N, Si et S,
l'un parmi A et B est un substituant représenté par la Formule chimique 1-1 suivante, et l'autre est un hydrogène ou un deutérium, 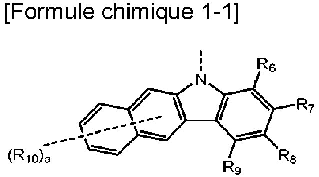
dans la Formule chimique 1-1,
R₆ à R₁₀ sont chacun indépendamment un hydrogène ; un deutérium ; un halogène ; un hydroxy ; un nitrile ; un nitro ; un amino ; un alkyle en C₂₋₆₀ substitué ou non substitué ; un alcoxy en C₂₋₆₀ substitué ou non substitué ; un alcényle en C₂₋₆₀ substitué ou non substitué ; un aryle en C₆₋₆₀ substitué ou non substitué ; un hétéroaryle en C₂₋₆₀ substitué ou non substitué contenant au moins un parmi O, N, Si et S, ou R₆ à R₉ se combinent avec un groupe adjacent pour former un cycle condensé,
a est un nombre entier de 1 à 6, 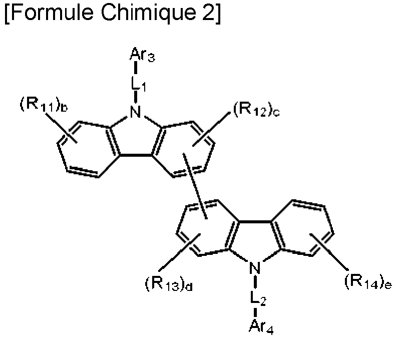
dans la Formule chimique 2,
Ar₃ et Ar₄ sont chacun indépendamment un aryle en C₆₋₆₀ substitué ou non substitué ; ou un hétéroaryle en C₂₋₆₀ substitué ou non substitué contenant au moins un parmi O, N, Si et S,
L₁ et L₂ sont chacun indépendamment une liaison simple ; ou un arylène en C₆₋₆₀ substitué ou non substitué,
R₁₁ à R₁₄ sont chacun indépendamment un hydrogène ; un deutérium ; un halogène ; un hydroxy ; un nitrile ; un nitro ; un amino ; un alkyle en C₂₋₆₀ substitué ou non substitué ; un alcoxy en C₂₋₆₀ substitué ou non substitué ; un alcényle en C₂₋₆₀ substitué ou non substitué ; un aryle en C₆₋₆₀ substitué ou non substitué ; un hétéroaryle en C₂₋₆₀ substitué ou non substitué contenant au moins un parmi O, N, Si et S,
b et e sont chacun indépendamment un nombre entier de 1 à 4, et
c et d sont chacun indépendamment un nombre entier de 1 à 3.

2. Dispositif électroluminescent organique comprenant :
une anode ; une cathode qui est fournie à l'opposé de l'anode ; et une ou plusieurs couches de matériau organique qui sont fournies entre l'anode et la cathode,
dans lequel la couche de matériau organique inclut un composé représenté par la Formule chimique 1-A suivante et un composé représenté par la Formule chimique 2 suivante : 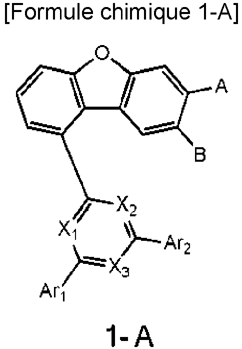
dans la Formule chimique 1-A,
X₁ à X₃ sont chacun indépendamment N ou CR₅', à condition qu'au moins un parmi X₁ à X₃ soit N,
Ar₁ et Ar₂ sont chacun indépendamment un aryle en C₆₋₆₀ substitué ou non substitué ; ou un hétéroaryle en C₂₋₆₀ substitué ou non substitué contenant au moins un parmi O, N, Si et S,
R₅' est un hydrogène ; un deutérium ; un halogène ; un hydroxy ; un nitrile ; un nitro ; un amino ; un alkyle en C₂₋₆₀ substitué ou non substitué ; un alcoxy en C₂₋₆₀ substitué ou non substitué ; un alcényle en C₂₋₆₀ substitué ou non substitué ; un aryle en C₆₋₆₀ substitué ou non substitué ; ou un hétéroaryle en C₂₋₆₀ substitué ou non substitué contenant au moins un parmi O, N, Si et S,
l'un parmi A et B est un substituant sélectionné dans le groupe consistant en les substituants suivants, et l'autre est un hydrogène ou un deutérium, 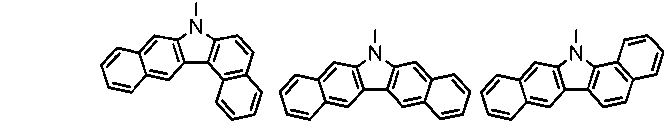 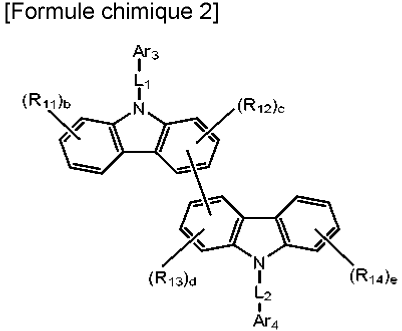
dans la Formule chimique 2,
Ar₃ et Ar₄ sont chacun indépendamment un aryle en C₆₋₆₀ substitué ou non substitué ; ou un hétéroaryle en C₂₋₆₀ substitué ou non substitué contenant au moins un parmi O, N, Si et S,
L₁ et L₂ sont chacun indépendamment une liaison simple ; ou un arylène en C₆₋₆₀ substitué ou non substitué,
R₁₁ à R₁₂ sont chacun indépendamment un hydrogène ; un deutérium ; un halogène ; un hydroxy ; un nitrile ; un nitro ; un amino ; un alkyle en C₂₋₆₀ substitué ou non substitué ; un alcoxy en C₂₋₆₀ substitué ou non substitué ; un alcényle en C₂₋₆₀ substitué ou non substitué ; un aryle en C₆₋₆₀ substitué ou non substitué ; un hétéroaryle en C₂₋₆₀ substitué ou non substitué contenant au moins un parmi O, N, Si et S,
b et e sont chacun indépendamment un nombre entier de 1 à 4, et
c et d sont chacun indépendamment un nombre entier de 1 à 3.

3. Dispositif électroluminescent organique selon la revendication 1 ou la revendication 2,
dans lequel X₁ à X₃ sont tous N.

4. Dispositif électroluminescent organique selon la revendication 1 ou la revendication 2,
dans lequel Ar₁ et Ar₂ sont chacun indépendamment l'un quelconque sélectionné dans le groupe consistant en :

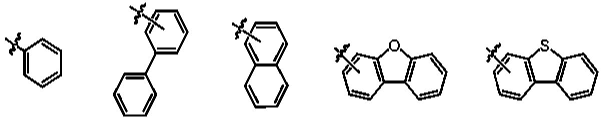

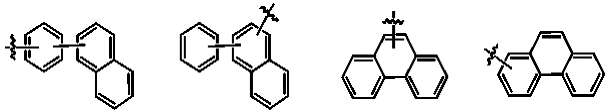

5. Dispositif électroluminescent organique selon la revendication 1,
dans lequel la Formule chimique 1-1 est sélectionnée dans le groupe consistant en les substituants suivants :

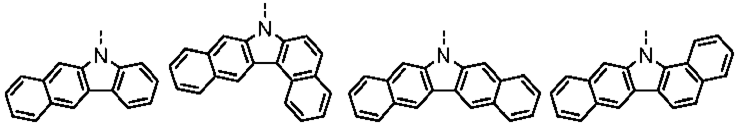

6. Dispositif électroluminescent organique comprenant :
une anode ; une cathode qui est fournie à l'opposé de l'anode ; et une ou plusieurs couches de matériau organique qui sont fournies entre l'anode et la cathode,
dans lequel la couche de matériau organique inclut un composé représenté par la Formule chimique 2 suivante et n'importe quel composé sélectionné dans le groupe (G) consistant en les composés suivants : 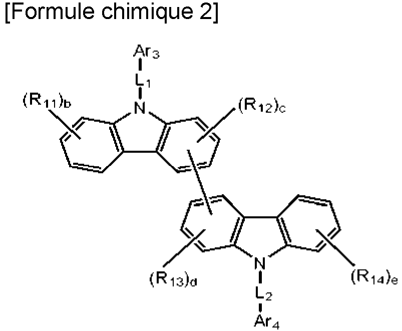
dans la Formule chimique 2,
Ar₃ et Ar₄ sont chacun indépendamment un aryle en C₆₋₆₀ substitué ou non substitué ; ou un hétéroaryle en C₂₋₆₀ substitué ou non substitué contenant au moins un parmi O, N, Si et S,
L₁ et L₂ sont chacun indépendamment une liaison simple ; ou un arylène en C₆₋₆₀ substitué ou non substitué,
R₁₁ à R₁₄ sont chacun indépendamment un hydrogène ; un deutérium ; un halogène ; un hydroxy ; un nitrile ; un nitro ; un amino ; un alkyle en C₂₋₆₀ substitué ou non substitué ; un alcoxy en C₂₋₆₀ substitué ou non substitué ; un alcényle en C₂₋₆₀ substitué ou non substitué ; un aryle en C₆₋₆₀ substitué ou non substitué ; un hétéroaryle en C₂₋₆₀ substitué ou non substitué contenant au moins un parmi O, N, Si et S,
b et e sont chacun indépendamment un nombre entier de 1 à 4, et
c et d sont chacun indépendamment un nombre entier de 1 à 3,

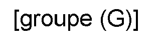

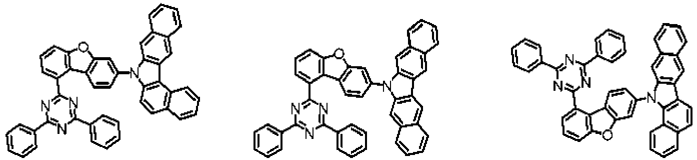

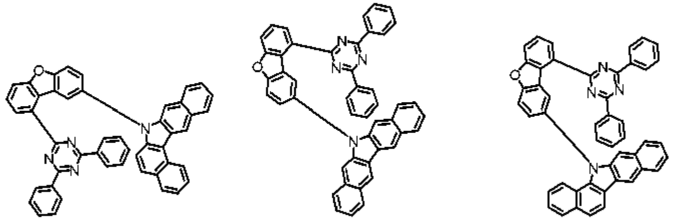

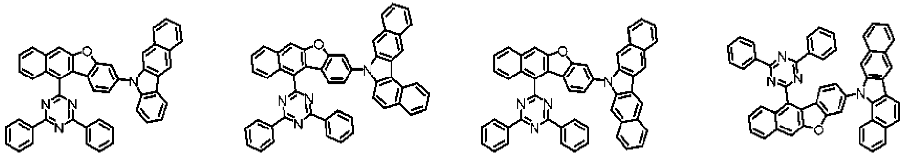

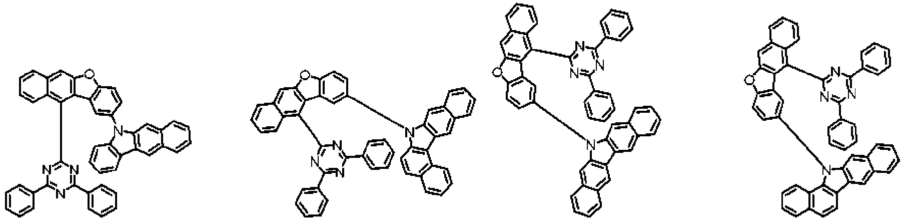

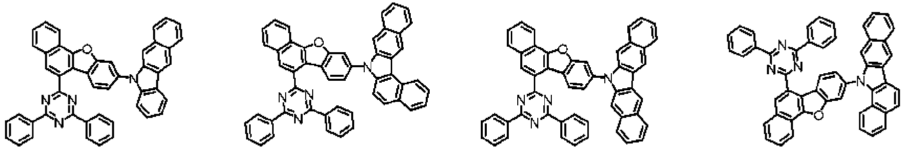

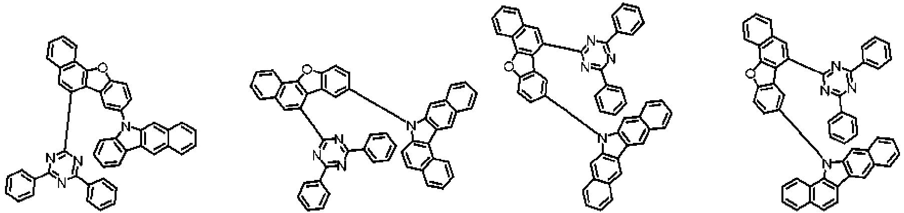

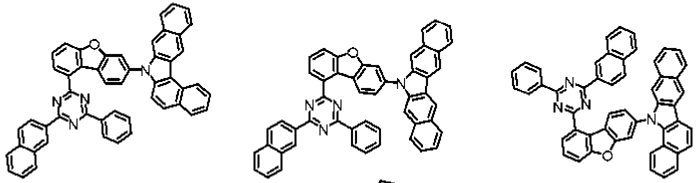

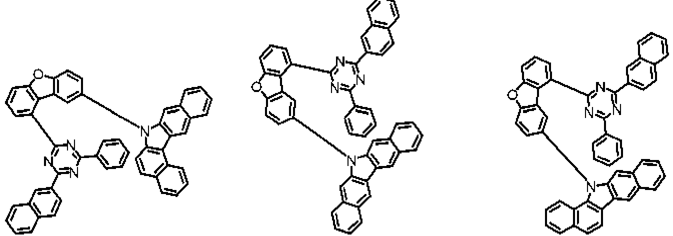

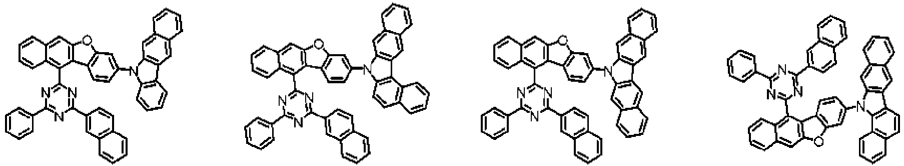

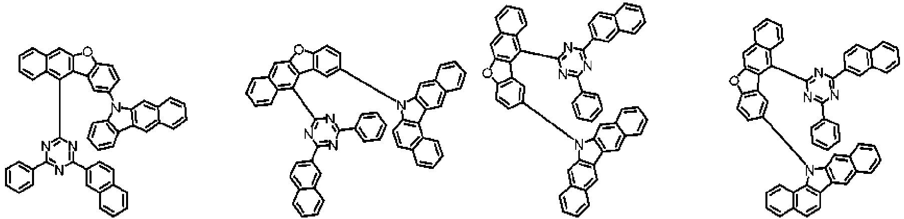

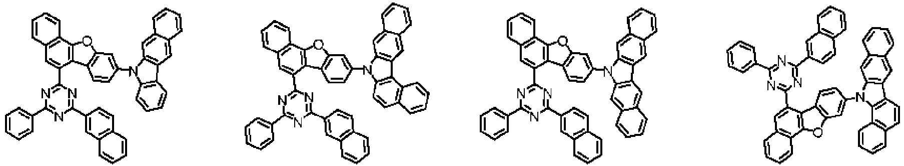

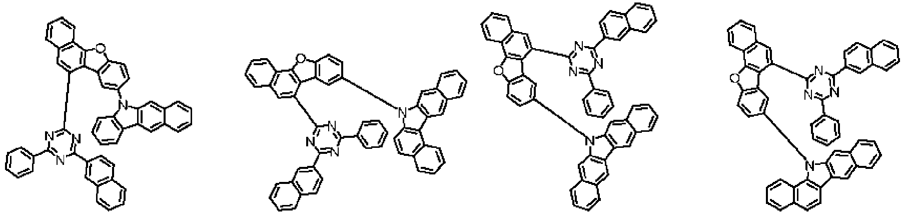

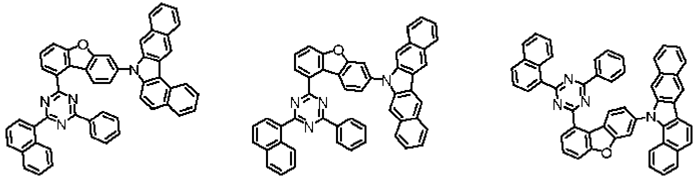

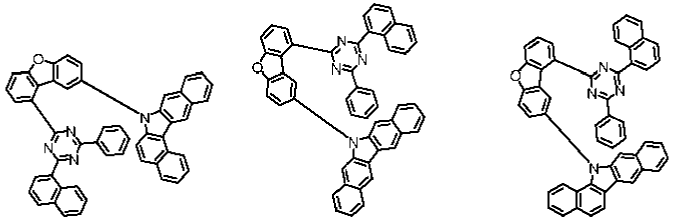

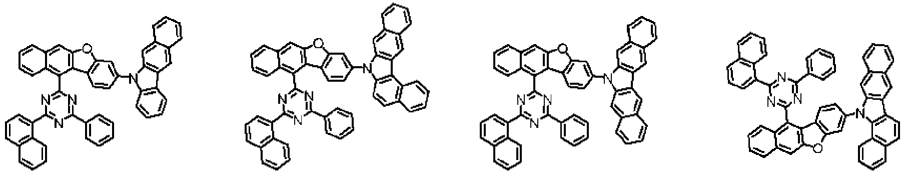

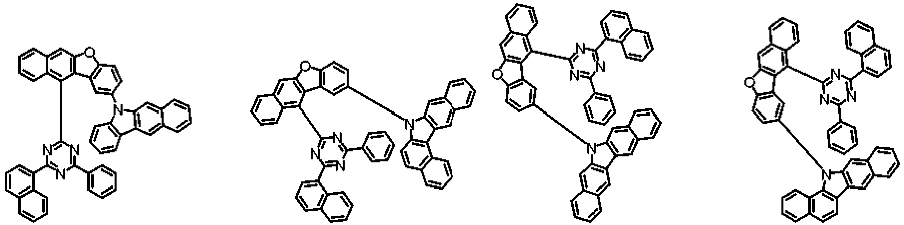

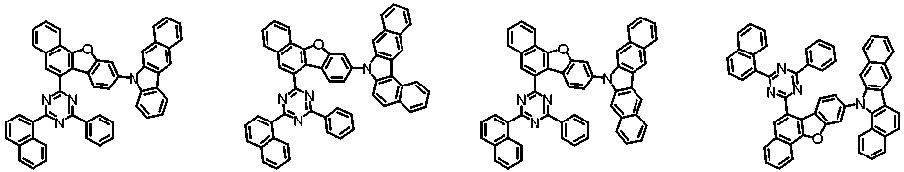

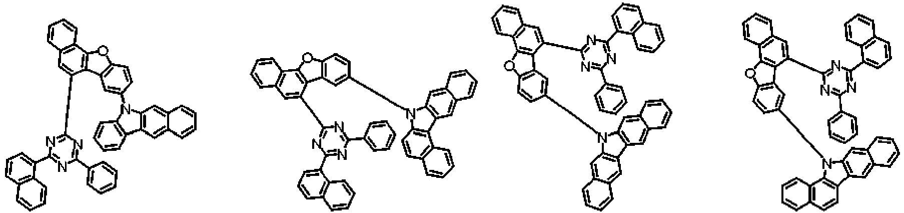

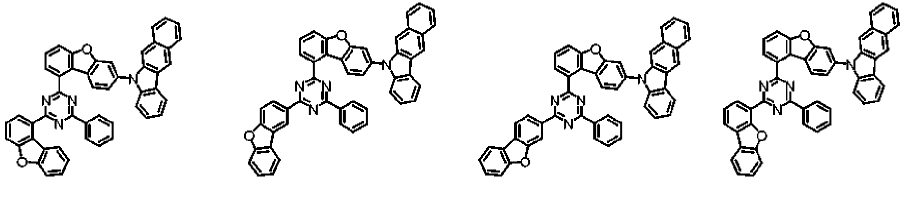

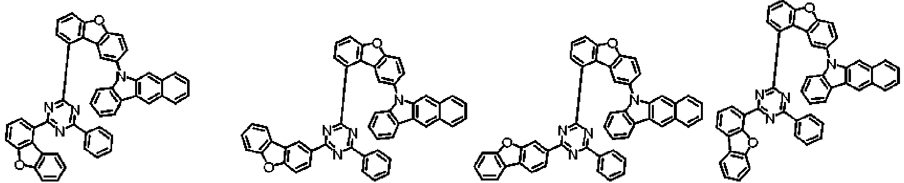

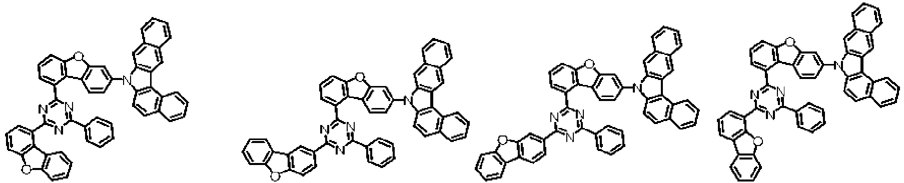

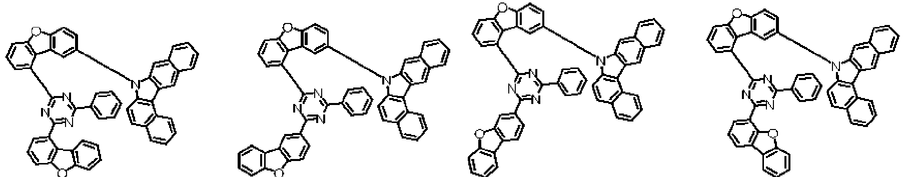

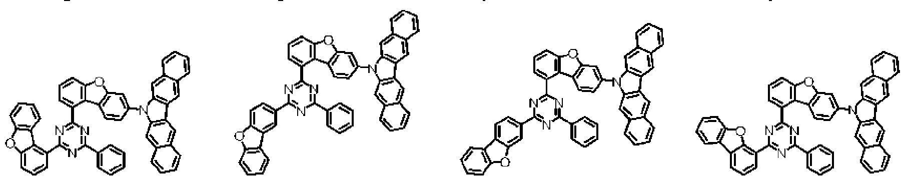

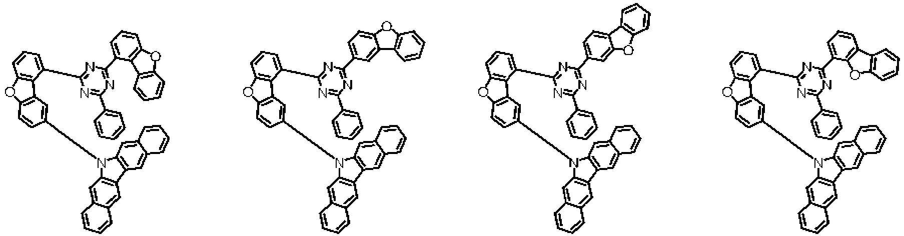

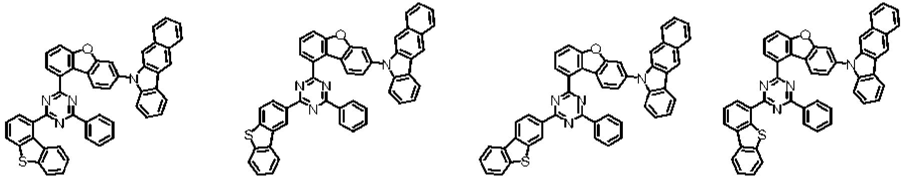

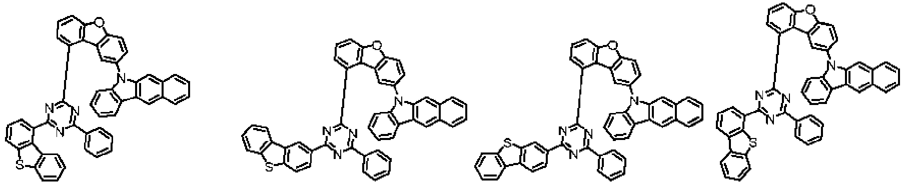

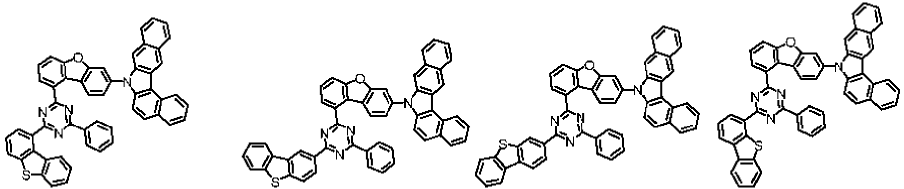

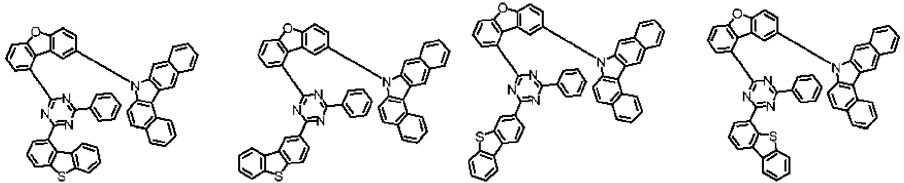

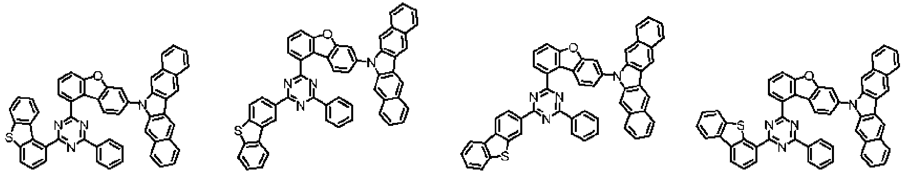

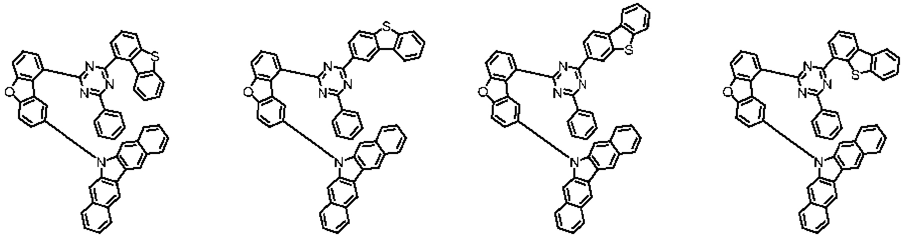

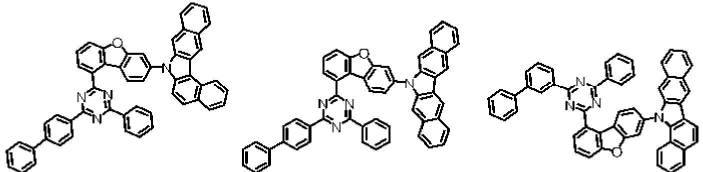

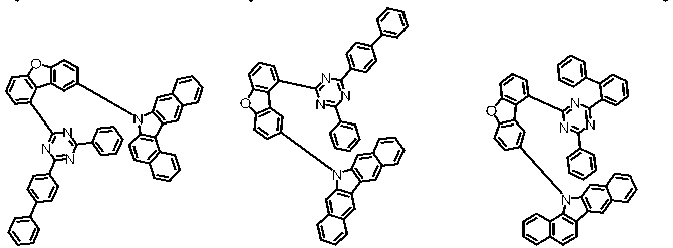

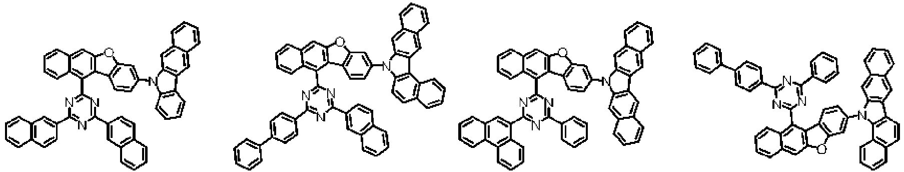

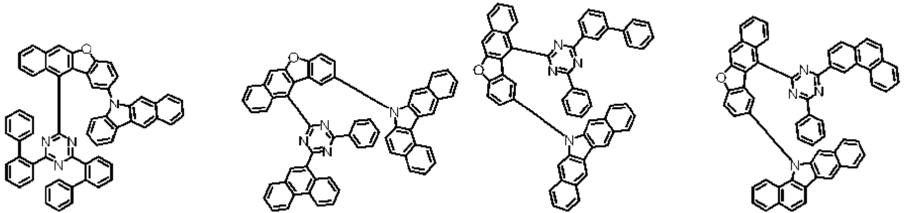

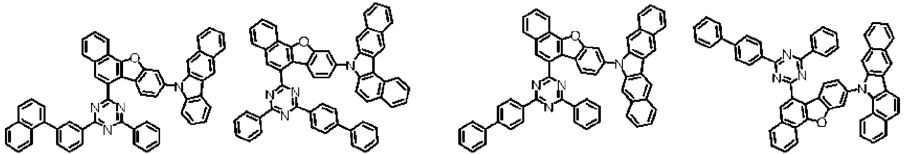

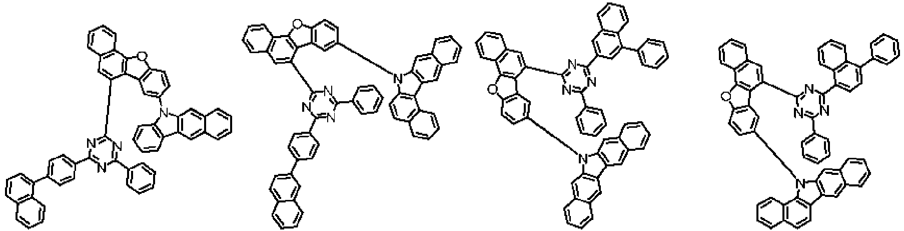

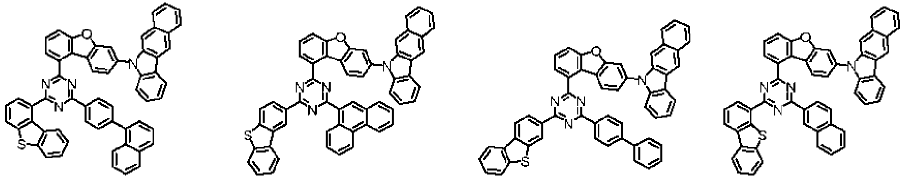

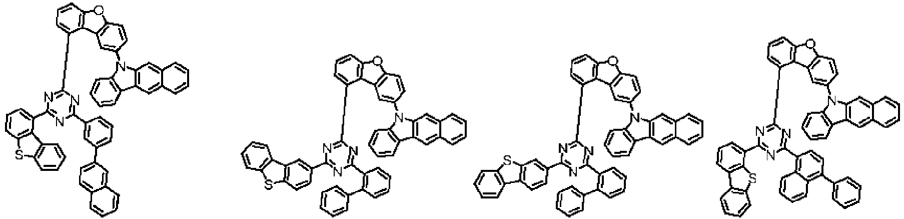

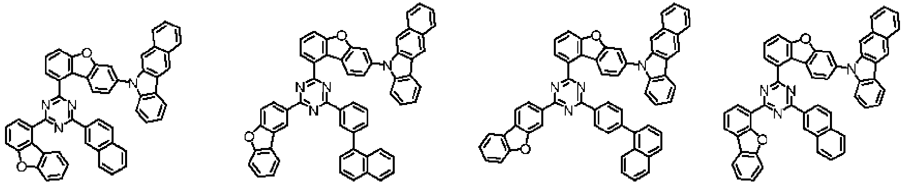

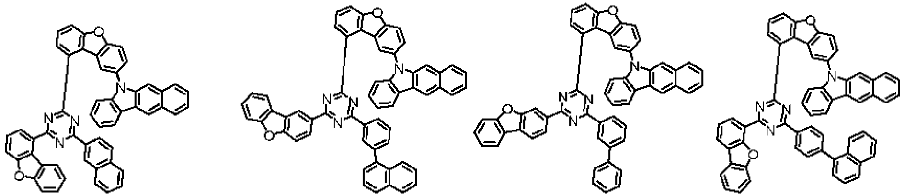

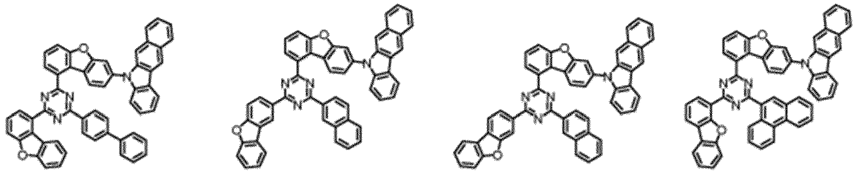

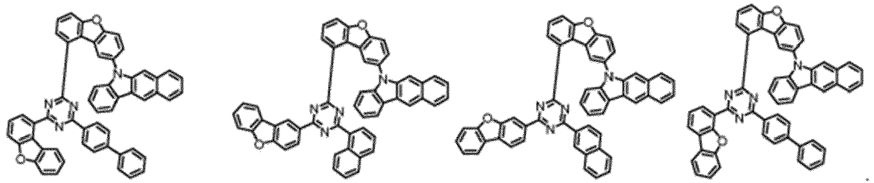

7. Dispositif électroluminescent organique selon la revendication 1 ou la revendication 2,
dans lequel le composé représenté par la Formule chimique 2 est un composé représenté par la Formule chimique 2-1 : 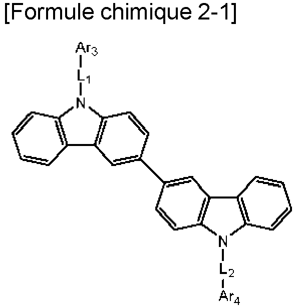
dans la Formule chimique 2-1,
Ar₃, Ar₄, L₁ et L₂ sont tels que définis dans la revendication 1 ou la revendication 2.

8. Dispositif électroluminescent organique selon la revendication 1 ou la revendication 2,
dans lequel Ar₃ et Ar₄ sont chacun indépendamment l'un quelconque sélectionné dans le groupe consistant en :

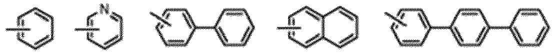

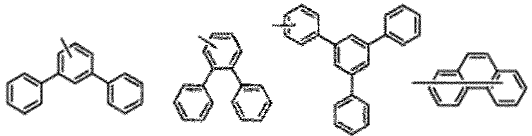

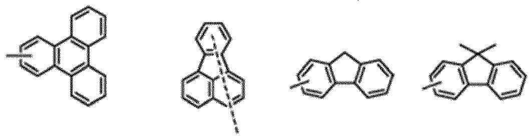

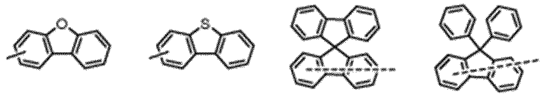

9. Dispositif électroluminescent organique selon la revendication 1 ou la revendication 2,
dans lequel L₁ et L₂ sont chacun indépendamment une liaison simple ou l'un quelconque sélectionné dans le groupe consistant en :

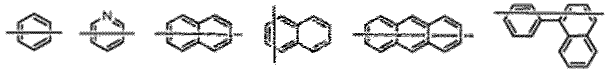

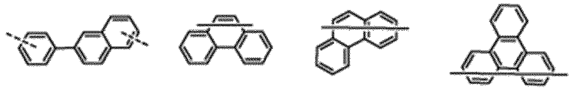

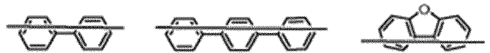

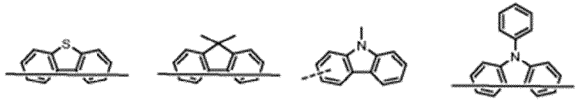

10. Dispositif électroluminescent organique selon la revendication 1 ou la revendication 2,
dans lequel R₁₁ à R₁₄ sont des hydrogènes.

11. Dispositif électroluminescent organique selon la revendication 1 ou la revendication 2,
dans lequel le composé représenté par la Formule chimique 2 est sélectionné dans le groupe consistant en les composés suivants :

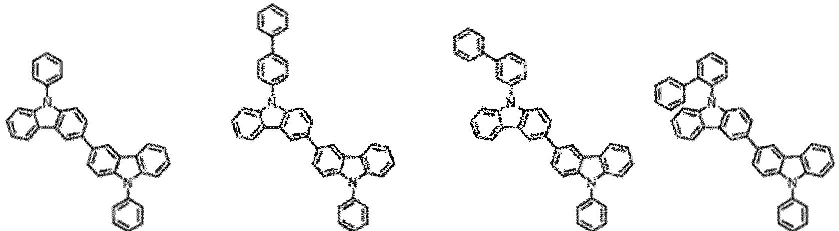

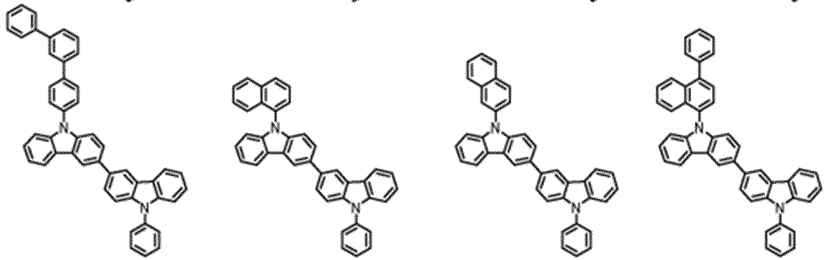

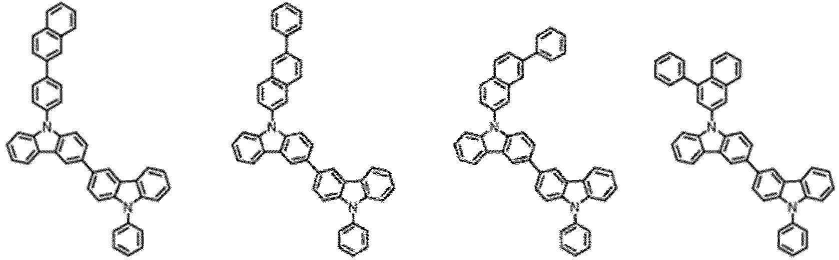

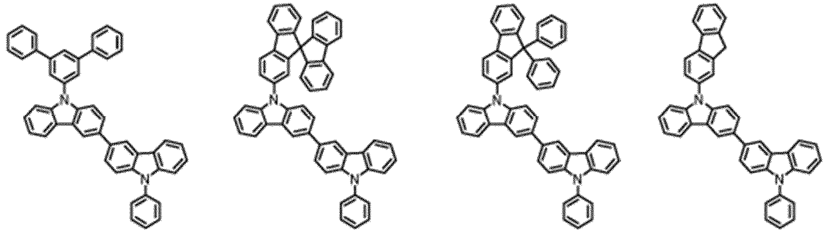

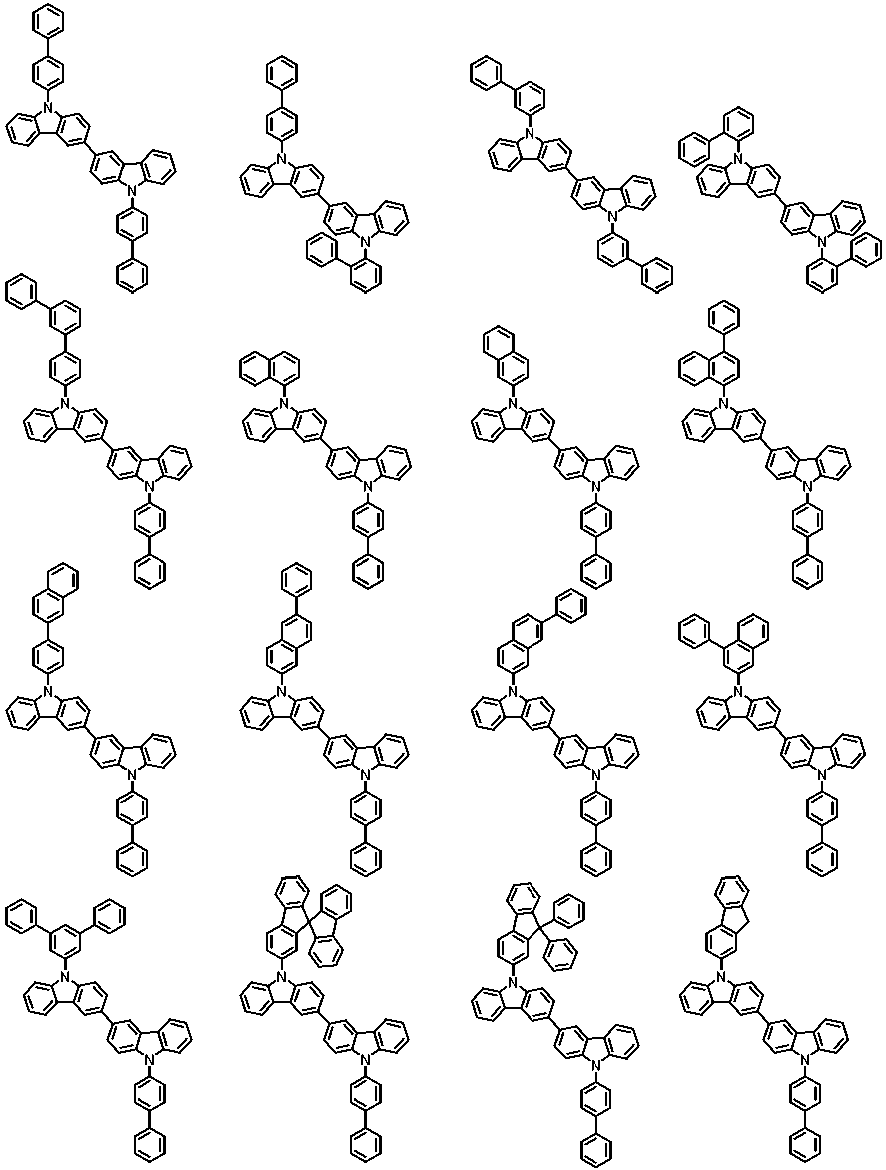

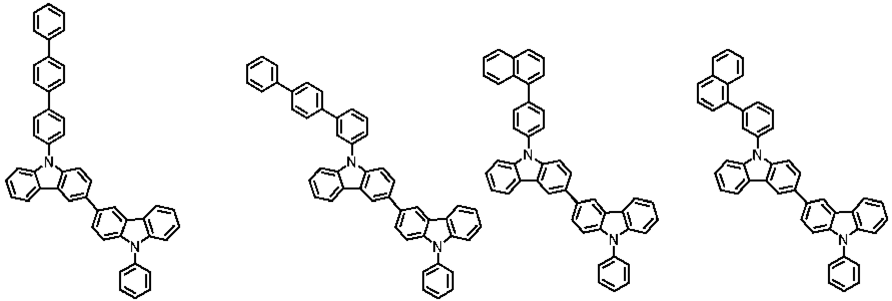

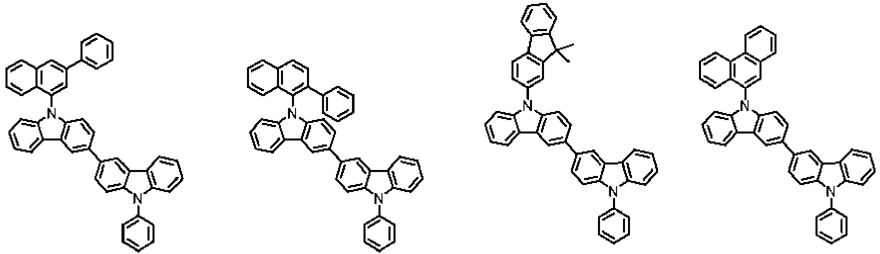

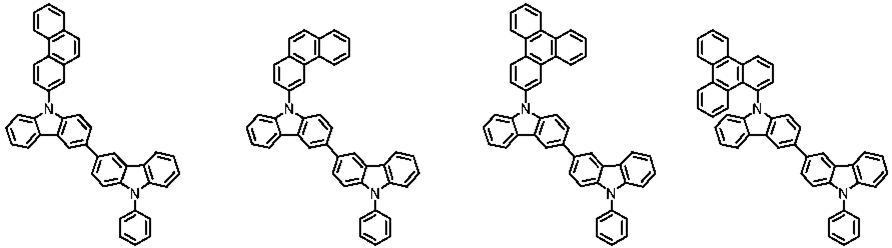

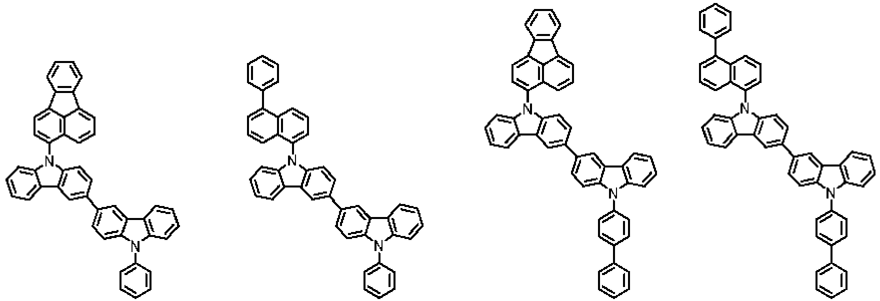

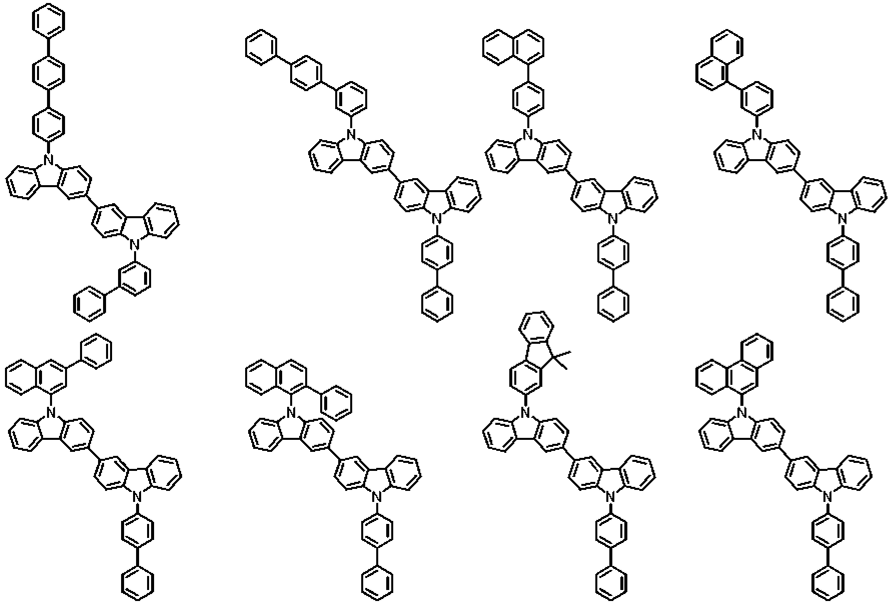

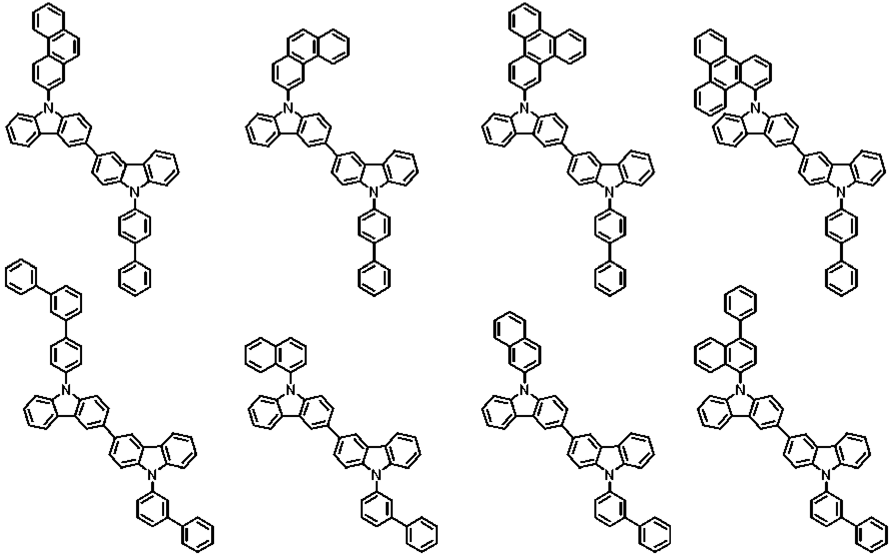

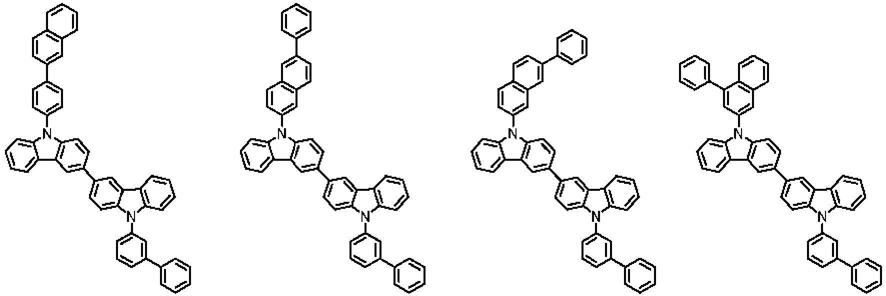

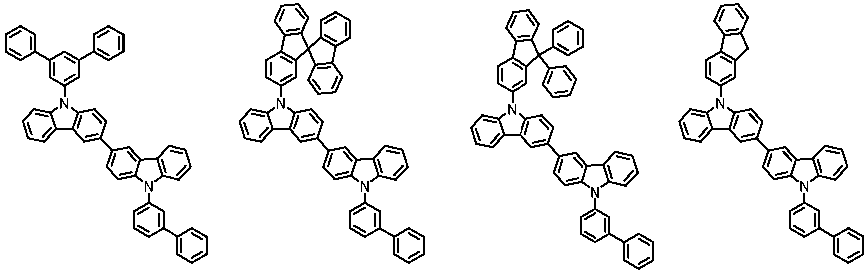

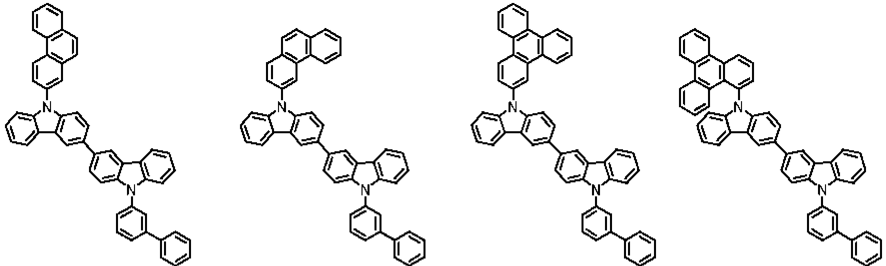

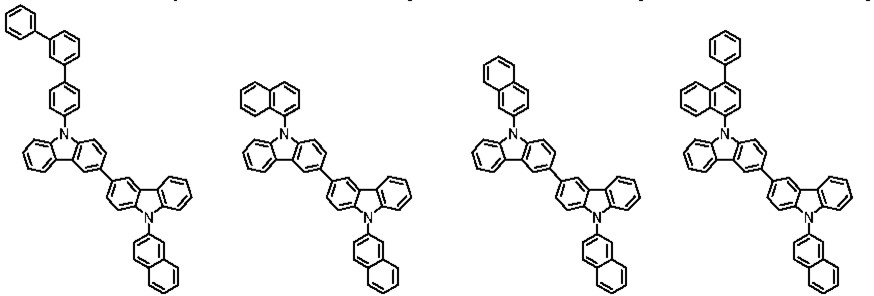

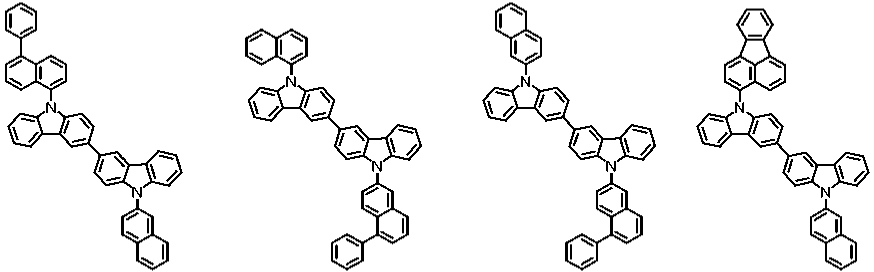

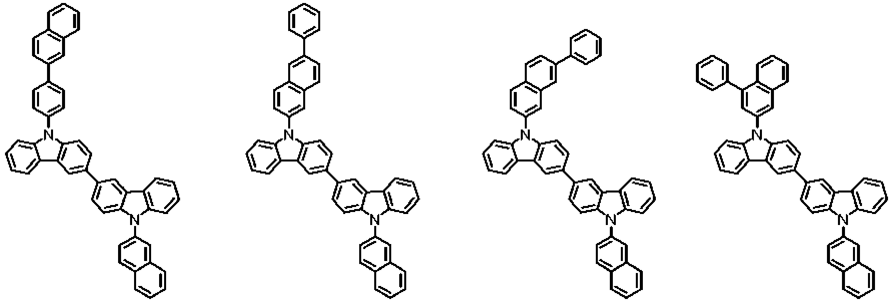

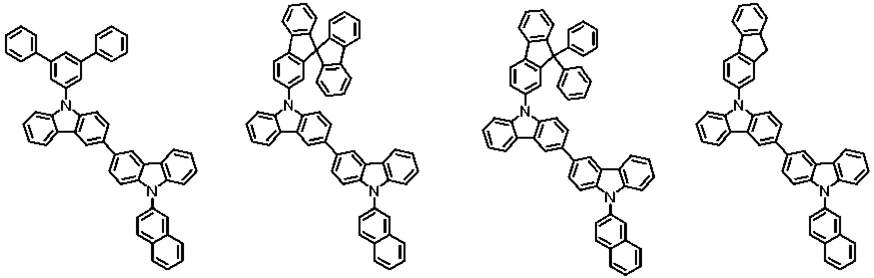

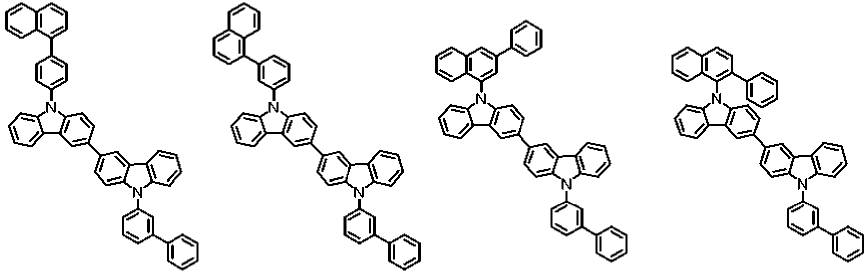

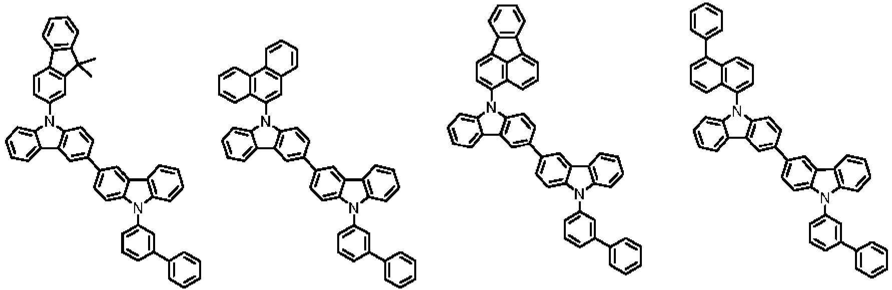

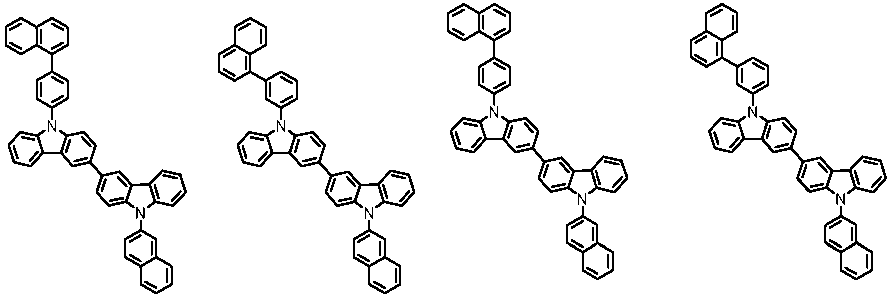

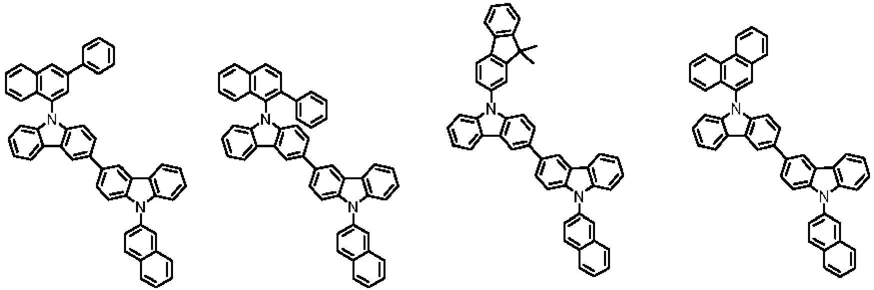

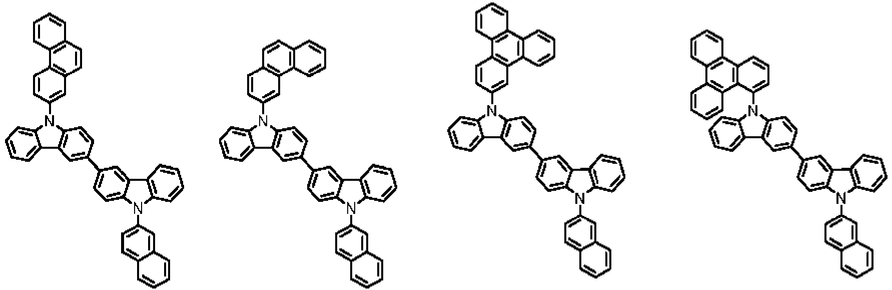

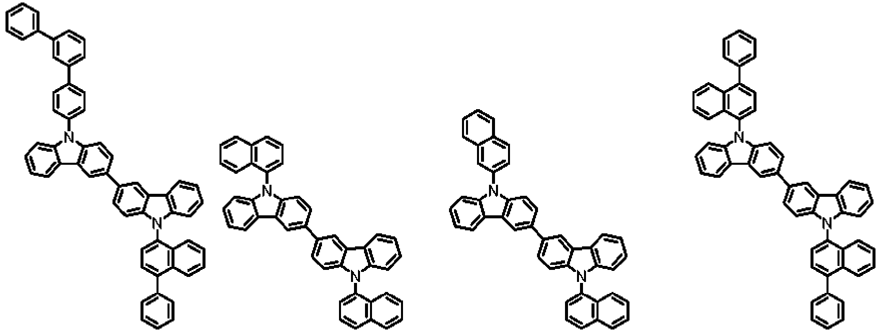

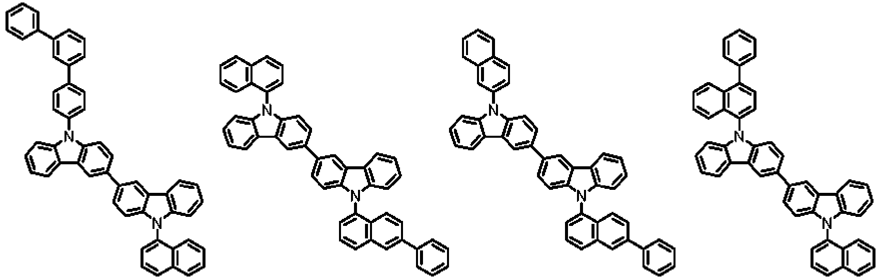

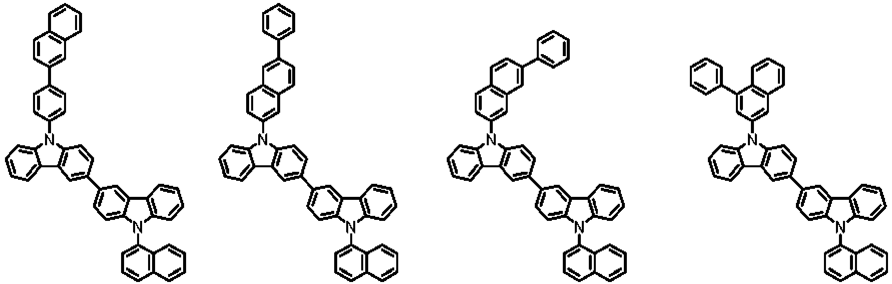

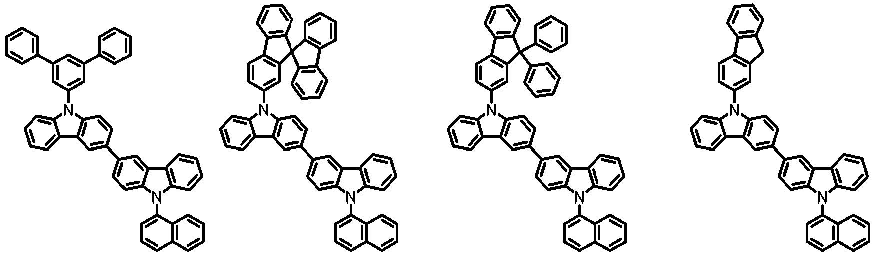

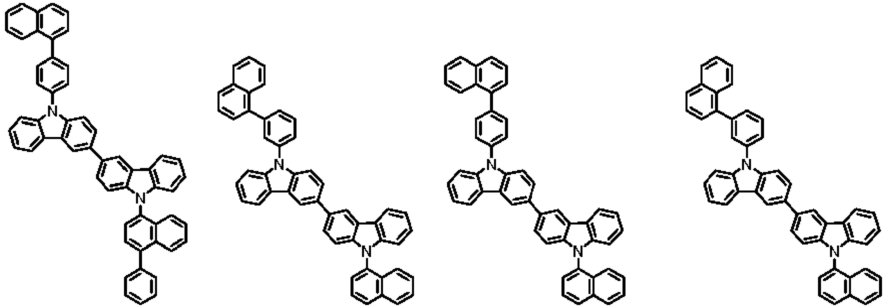

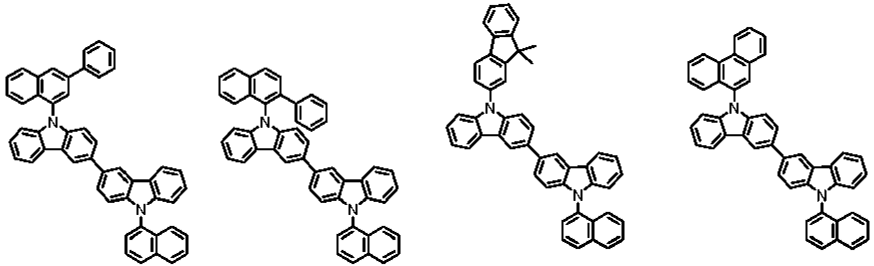

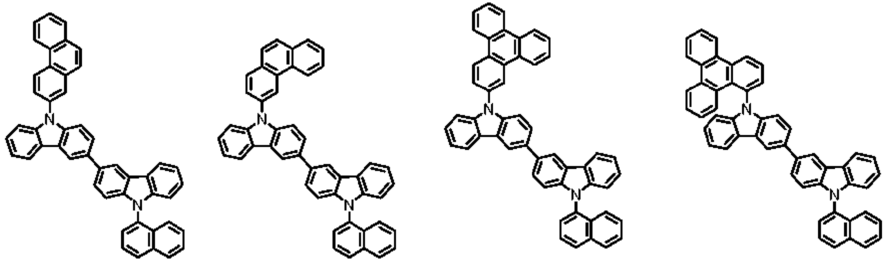

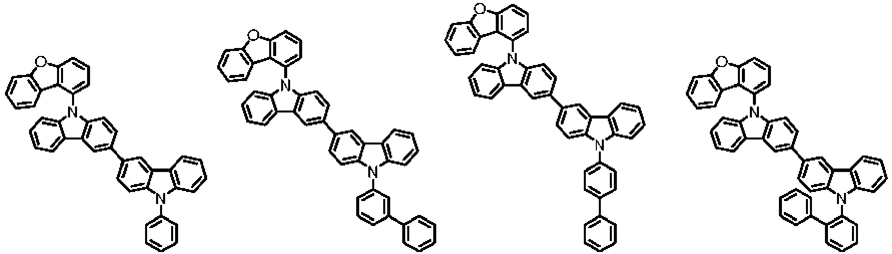

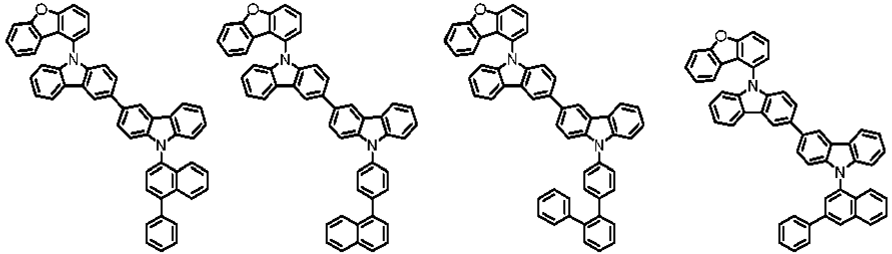

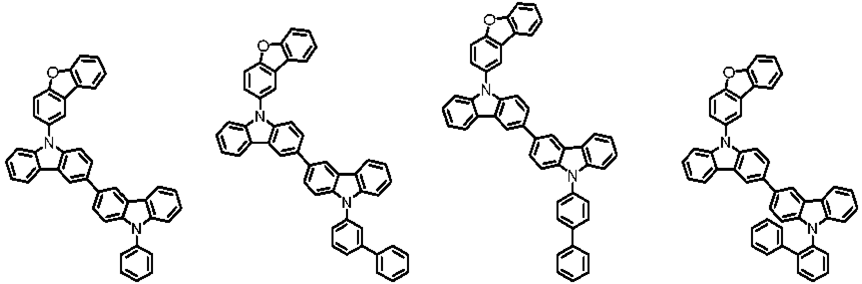

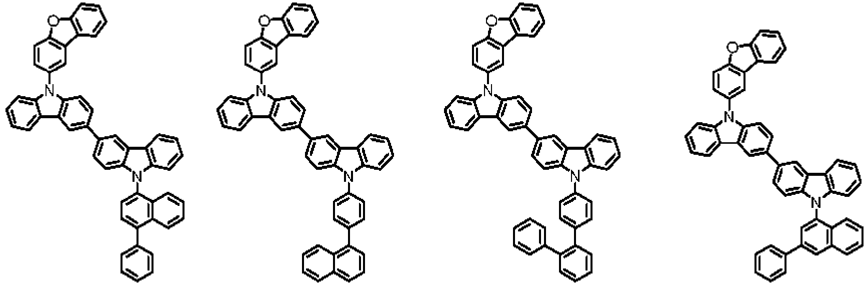

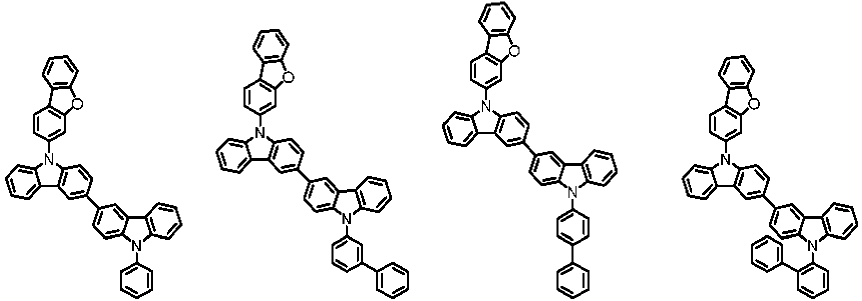

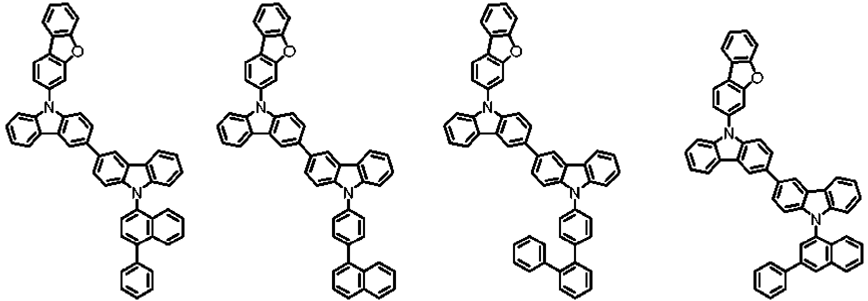

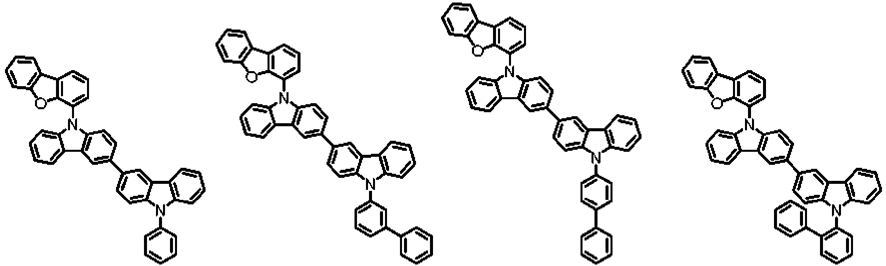

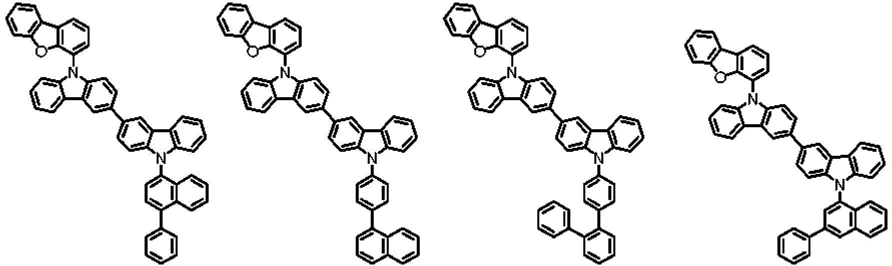

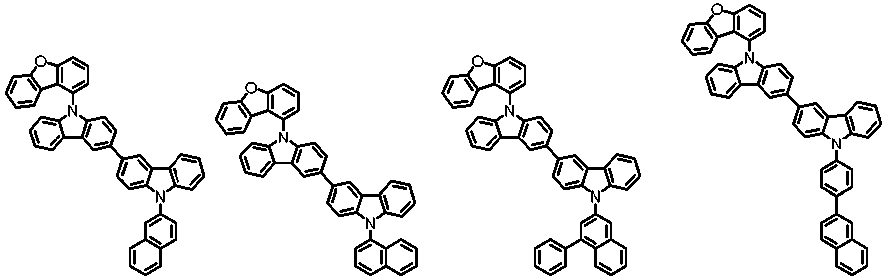

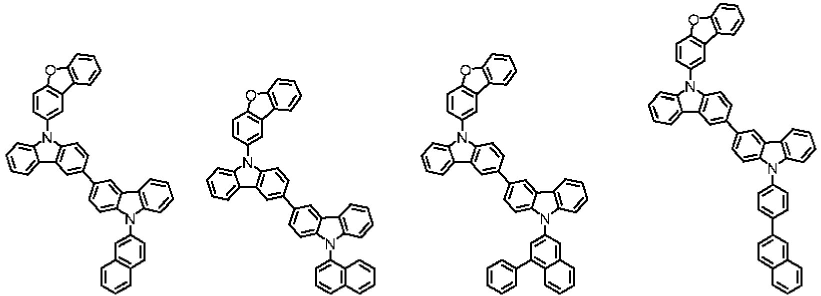

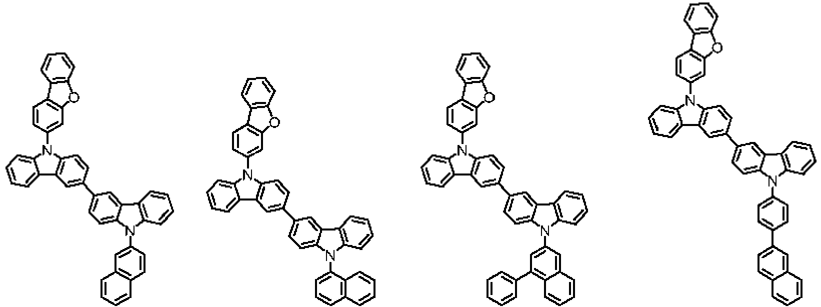

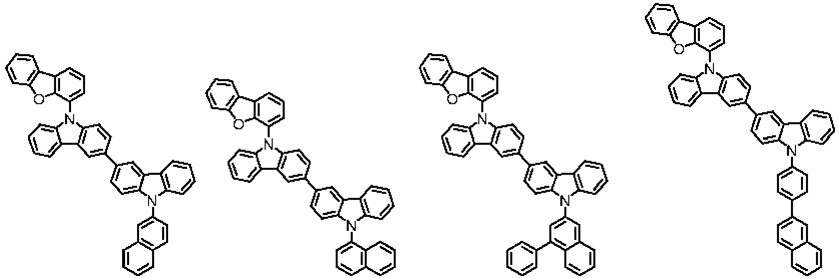

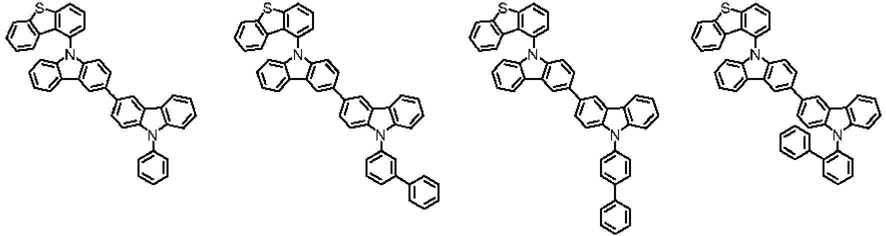

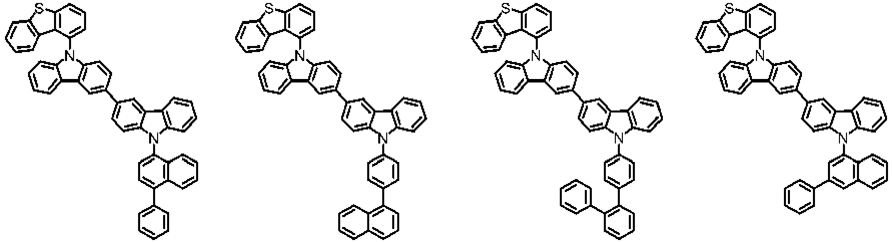

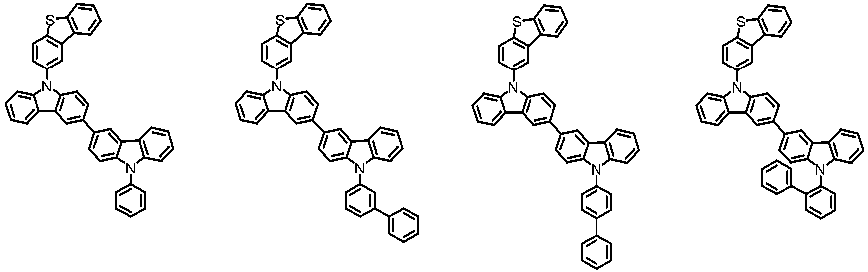

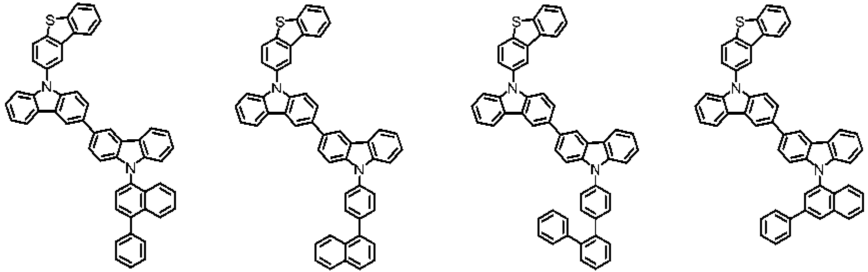

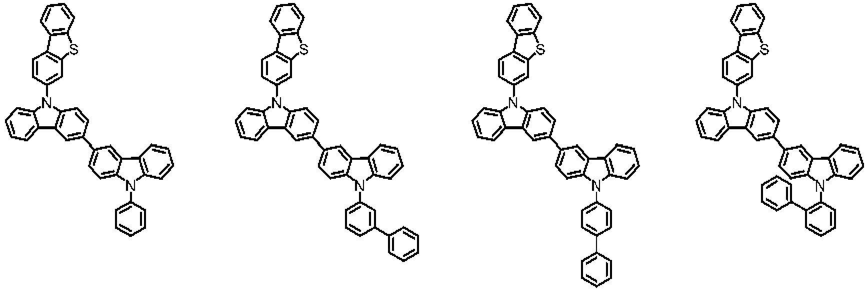

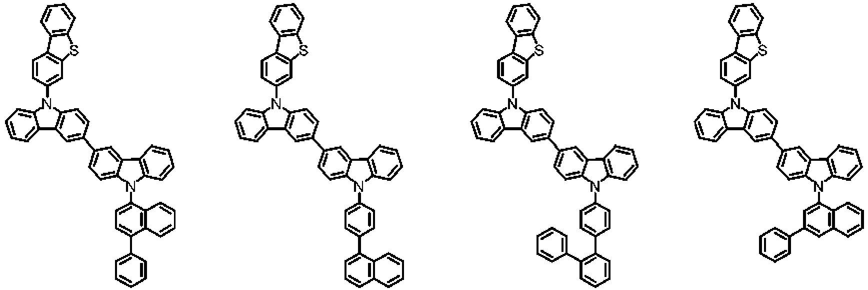

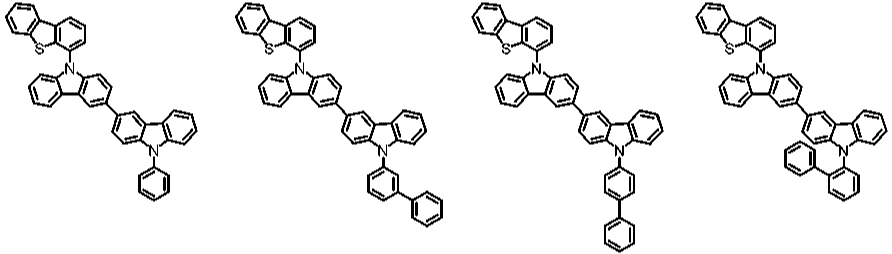

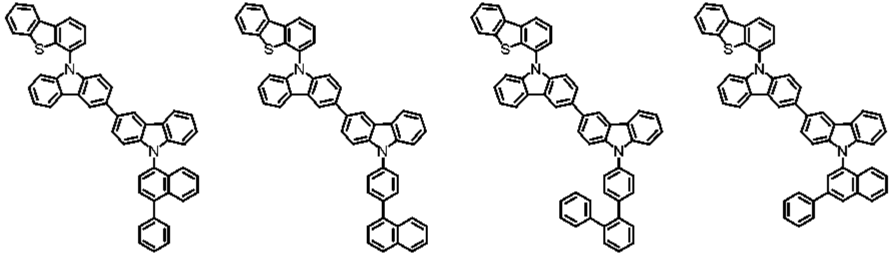

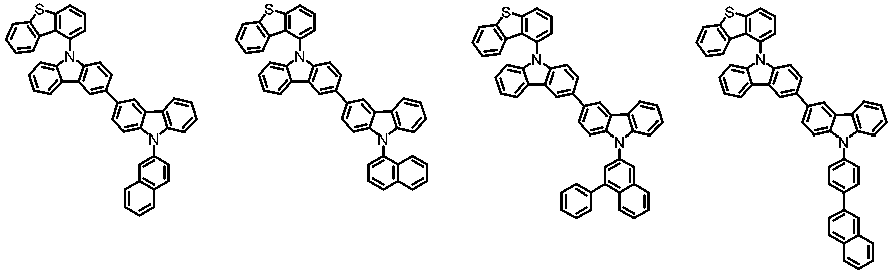

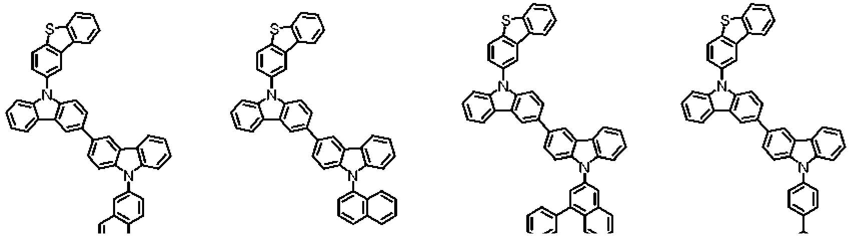

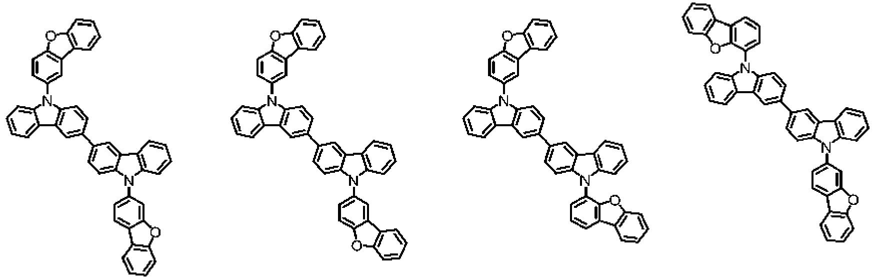

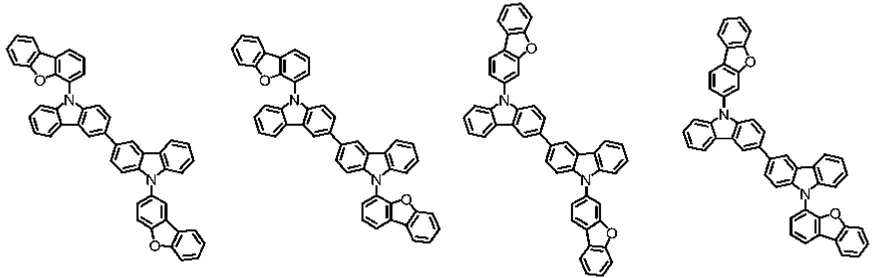

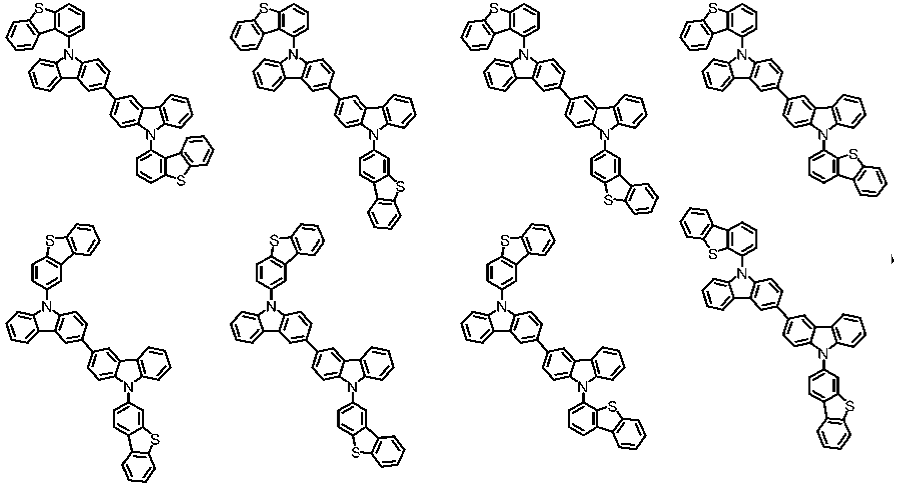

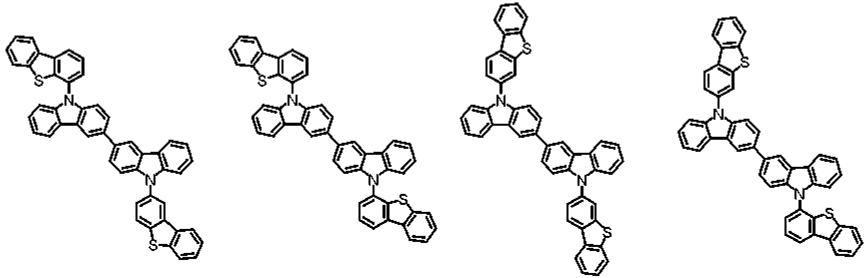

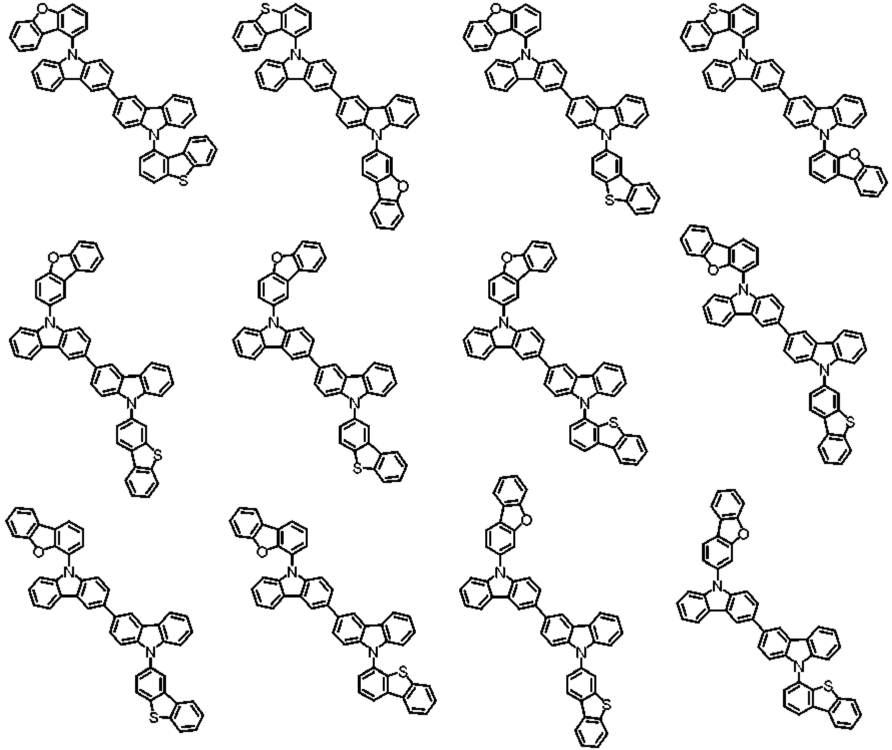

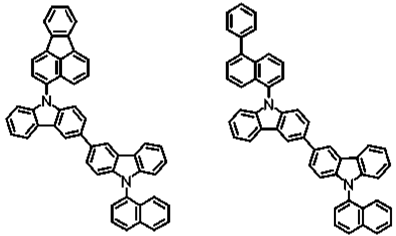

12. Dispositif électroluminescent organique selon la revendication 1 ou la revendication 2,
dans lequel la couche de matériau organique incluant l'un quelconque sélectionné parmi les composés représentés par les Formules chimiques 1-A, 1-B et 1-C et le composé représenté par la Formule chimique 2 est une couche électroluminescente.

13. Dispositif électroluminescent organique selon la revendication 12,
dans lequel l'un quelconque sélectionné parmi les composés représentés par les Formules chimiques 1-A, 1-B et 1-C et le composé représenté par la Formule chimique 2 sont des matériaux hôtes dans la couche électroluminescente.

14. Dispositif électroluminescent organique selon la revendication 12,
dans lequel la couche électroluminescente comprend en outre un matériau dopant.
